(19) Europäisches Patentamt
European Patent Office
Office européen des brevets

(11) EP 4 349 366 A1

(12) # EUROPEAN PATENT APPLICATION
published in accordance with Art. 153(4) EPC

(43) Date of publication:
**10.04.2024 Bulletin 2024/15**

(21) Application number: **22816130.3**

(22) Date of filing: **31.05.2022**

(51) International Patent Classification (IPC):
***A61K 39/00*** *(2006.01)* ***A61K 35/17*** *(2015.01)*
***A61K 39/39*** *(2006.01)* ***A61K 47/61*** *(2017.01)*
***A61P 35/00*** *(2006.01)* ***A61P 43/00*** *(2006.01)*
***C07K 7/06*** *(2006.01)*

(52) Cooperative Patent Classification (CPC):
**A61K 35/17; A61K 39/00; A61K 39/39; A61K 47/61; A61P 35/00; A61P 43/00;** C07K 7/06

(86) International application number:
**PCT/JP2022/022211**

(87) International publication number:
**WO 2022/255384 (08.12.2022 Gazette 2022/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.05.2021 JP 2021091520**

(71) Applicants:
- **Mie University**
  **Tsu-shi, Mie 514-8507 (JP)**
- **Asahi Kasei Kabushiki Kaisha**
  **Tokyo 1000006 (JP)**

(72) Inventors:
- **MOMOSE, Fumiyasu**
  **Tsu-shi, Mie 514-8507 (JP)**
- **SHIKU, Hiroshi**
  **Tsu-shi, Mie 514-8507 (JP)**
- **NAKAI, Takashi**
  **Tokyo 100-0006 (JP)**
- **YABUUCHI, Kohei**
  **Tokyo 100-0006 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **PHARMACEUTICAL COMPOSITION**

(57) Provided is a pharmaceutical composition that can induce an antitumor effect more potently, more efficiently, more sustainably, and/or over a wider range of areas. A composition containing a hyaluronic acid derivative having a hydrophobic group introduced and an antigen is used in combination with a lymphocyte expressing an immune receptor for the antigen

EP 4 349 366 A1

Fig. 1-1

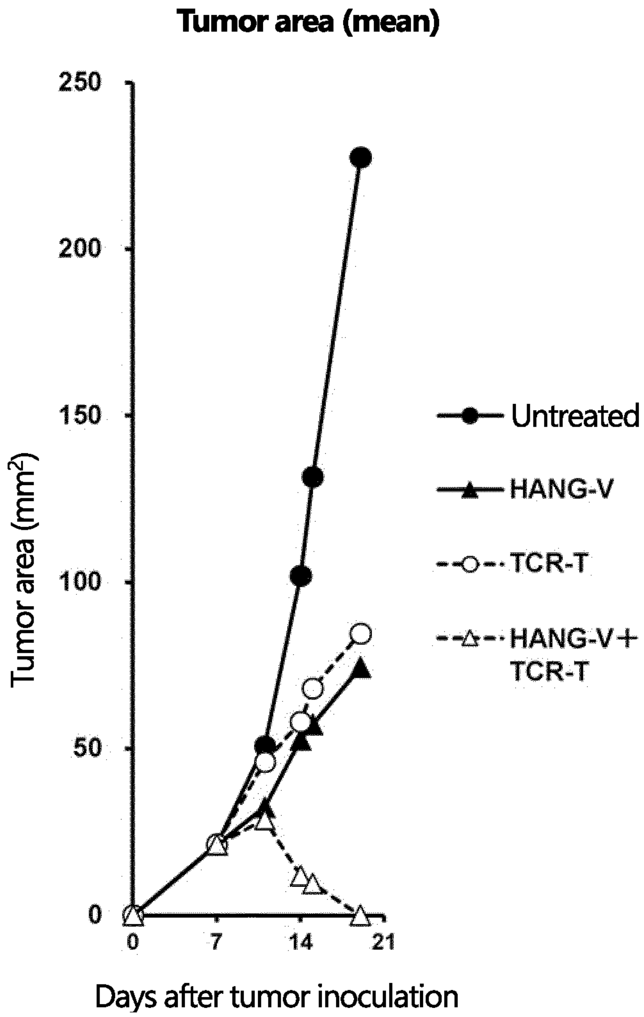
Tumor area (mean)
Tumor area (mm2)
250
200
150
100
50
0
0
7
14
21
Days after tumor inoculation
Untreated
HANG-V
TCR-T
HANG-V+
TCR-T

**Description**

Technical Field

The present invention relates to a pharmaceutical composition.

Background Art

Cancer vaccines are generally administered subcutaneously, intradermally, or intramuscularly. To induce a tumor-specific immune response in the immunosuppressive environment of cancer patients, it is important to efficiently deliver a cancer vaccine to nearby lymph nodes and establish an environment in which antigen-presenting cells can present an antigen to T cells in the lymph nodes. This is also thought to be resulted in the improvement of therapeutic effects. Many of the proteins, peptides, and nucleic acids used in cancer vaccines are generally unstable and easily degraded or inactivated when administered to the body. Thus, it has been required for the development of drug delivery systems (DDS) that can stably deliver a vaccine to the target site and release it slowly.

Hyaluronic acid derivatives having hydrophobic groups introduced self-aggregate to form a complex, typically a nano-particulate nanogel. Due to its cross-linked three-dimensional network structure and hydrophobic interaction, the complex can easily encapsulate a protein, peptide, or poorly soluble low-molecular-weight compound, and is thus stably delivers and slowly releases the encapsulated substance at the target site. Therefore, such complexes are expected to be applied to new DDSs. PTL 1 reports that a cancer vaccine composed of the complex was prepared and subcutaneously administered to mice, and that the cancer vaccine rapidly and highly efficiently migrated to neighboring lymph nodes and induced antitumor effects.

However, cancer vaccines, which are currently undergoing worldwide research and development, are not yet fully satisfactory in terms of their effectiveness.

Citation List

Patent Literature

PTL 1: WO2020/158771A

Summary of Invention

Technical Problem

An object of the present invention is to provide a pharmaceutical composition that can induce an antitumor effect more potently, more efficiently, more sustainably, and/or over a wider range of areas.

Solution to Problem

In light of the object, the present inventors conducted extensive research and found that the object can be achieved by using a composition containing a hyaluronic acid derivative having hydrophobic groups introduced and an antigen in combination with a lymphocyte expressing an immune receptor for the antigen. The present inventors further conducted extensive research based on the finding, and completed the present invention. Specifically, the present invention includes the following subject manner.

Item 1. A pharmaceutical composition for use in combined administration with a lymphocyte,

the pharmaceutical composition comprising

a hyaluronic acid derivative having a hydrophobic group introduced, and
an antigen,

the lymphocyte expressing an immune receptor for the antigen.

Item 2. The pharmaceutical composition according to Item 1, wherein the hydrophobic group is a steryl group.
Item 3. The pharmaceutical composition according to Item 1 or 2, wherein the hyaluronic acid derivative and the

antigen form a complex.

Item 4. The pharmaceutical composition according to any one of Items 1 to 3, wherein the antigen is a cancer antigen.

Item 5. The pharmaceutical composition according to any one of Items 1 to 4, wherein the antigen is an antigen peptide or an antigenic protein.

Item 6. The pharmaceutical composition according to Item 5, wherein the antigen contains a CD8-positive cytotoxic T-cell recognition epitope and/or a CD4-positive helper T-cell recognition epitope.

Item 7. The pharmaceutical composition according to any one of Items 1 to 6, wherein the immune receptor is a T-cell receptor or a chimeric antigen receptor.

Item 8. The pharmaceutical composition according to any one of Items 1 to 7, further comprising an adjuvant, or the pharmaceutical composition being for use in combined administration with an adjuvant.

Item 9. The pharmaceutical composition according to any one of Items 1 to 8, for use in the prevention or treatment of cancer.

Item 10. A pharmaceutical composition comprising a lymphocyte expressing an immune receptor for an antigen, the pharmaceutical composition being for use in combined administration with a composition comprising a hyaluronic acid derivative having a hydrophobic group introduced and the antigen.

Item 11. A cancer treatment kit comprising

a pharmaceutical composition containing

a hyaluronic acid derivative having a hydrophobic group introduced, and
a cancer antigen, and

a lymphocyte expressing an immune receptor for the cancer antigen.

Item 12. A T-cell activation vaccine comprising

a hyaluronic acid derivative having a hydrophobic group introduced, and
an antigen.

Item 13. A cancer treatment kit comprising

a T cell expressing an immune receptor for a cancer antigen, and
a pharmaceutical composition comprising

a hyaluronic acid derivative having a hydrophobic group introduced,
an antigen peptide or an antigenic protein derived from the cancer antigen, and
an adjuvant,
the hyaluronic acid derivative comprising one or more repeating units represented by formula (I) :

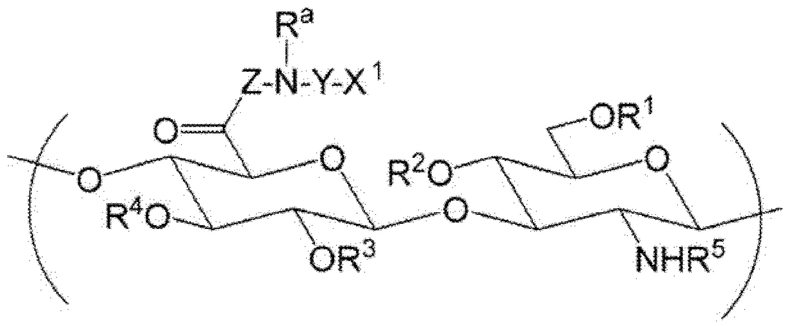

(I)

wherein $R^1$, $R^2$, $R^3$, and $R^4$ are each independently selected from the group consisting of a hydrogen atom, $C_{1-6}$ alkyl, formyl, and $C_{1-6}$ alkyl carbonyl;
$R^5$ represents a hydrogen atom, formyl, or $C_{1-6}$ alkyl carbonyl;
Z represents a direct bond, or a peptide linker composed of 2 to 30 any amino acid residues;
$X^1$ represents a hydrophobic group selected from the groups represented by the following formulas:

$-NR^b-R$,

$-NR^b-COO-R$,

$-NR^b-CO-R$,

$-NR^b-CO-NR^c-R$,

-COO-R,

-O-COO-R,

-S-R,

$-CO-Y^a-S-R$,

$-O-CO-Y^b-S-R$,

$-NR^b-CO-Y^b-S-R$, and

-S-S-R;

$R^a$, $R^b$, and $R^c$ are each independently selected from the group consisting of a hydrogen atom, $C_{1-20}$ alkyl, amino $C_{2-20}$ alkyl, and hydroxy $C_{2-20}$ alkyl, wherein an alkyl moiety of $R^a$, $R^b$, and $R^c$ may have 1 to 3 groups inserted, the 1 to 3 groups being selected from the group consisting of-O- and $-NR^f-$;
$R^f$ is selected from the group consisting of a hydrogen atom, $C_{1-12}$ alkyl, amino $C_{2-12}$ alkyl, and hydroxy $C_{2-12}$ alkyl, wherein an alkyl moiety of $R^f$ may have 1 or 2 groups inserted, the 1 or 2 groups being selected from the group consisting of -O- and -NH-;
R represents a steryl group;
Y represents $C_{2-30}$ alkylene or - $(CH_2CH_2O)_m-CH_2CH_2-$, wherein the alkylene may have 1 to 5 groups inserted, the 1 to 5 groups being selected from the group consisting of -O-, $-NR^g-$, and-S-S-;
$R^g$ is selected from the group consisting of a hydrogen atom, $C_{1-20}$ alkyl, amino $C_{2-20}$ alkyl, and hydroxy $C_{2-20}$ alkyl, wherein an alkyl moiety of $R^g$ may have 1 to 3 groups inserted, the 1 to 3 groups being selected from the group consisting of -O- and -NH-;
$Y^a$ represents $C_{1-5}$ alkylene;
$Y^b$ represents $C_{2-8}$ alkylene or $C_{2-8}$ alkenylene; and m represents an integer selected from 1 to 100.

Item 14. The cancer treatment kit according to Item 13, wherein a modification rate of a carboxy group of a glucuronic acid moiety of the hyaluronic acid derivative with the hydrophobic group is 5 to 50%.
Item 15. The cancer treatment kit according to Item 14, wherein Z represents a direct bond, Y represents $C_{2-12}$ alkylene, $X^1$ represents-NH-COO-R, and R represents a cholesteryl group.
Item 16. The cancer treatment kit according to Item 13, wherein the hyaluronic acid derivative has a weight average molecular weight of 5,000 to 2,000,000.
Item 17. The cancer treatment kit according to Item 13, wherein the hyaluronic acid derivative comprises one or more repeating units represented by formula (IIIc):

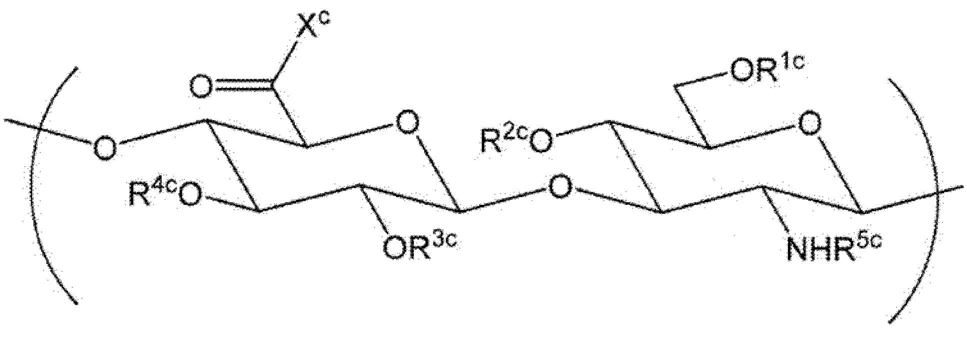

(IIIc)

wherein

$R^{1c}$, $R^{2c}$, $R^{3c}$, and $R^{4c}$ are each independently selected from the group consisting of a hydrogen atom, $C_{1-6}$ alkyl, formyl, and $C_{1-6}$ alkyl carbonyl;
$R^{5c}$ is selected from the group consisting of a hydrogen atom, formyl, and $C_{1-6}$ alkyl carbonyl; and
$X^c$ is selected from the group consisting of hydroxy and -O-$Q^+$, wherein $Q^+$ represents a countercation.

Item 18. The cancer treatment kit according to Item 13, wherein the pharmaceutical composition comprises a complex of the cancer antigen and the hyaluronic acid derivative.
Item 19. The cancer treatment kit according to Item 13, wherein the antigen peptide contains two or more CD8-positive cytotoxic T-cell recognition epitopes and/or CD4-positive helper T-cell recognition epitopes.
Item 20. The cancer treatment kit according to Item 19, wherein the antigen peptide is a long-chain peptide antigen containing

two or more CD8-positive cytotoxic T-cell recognition epitopes, or
one or more CD8-positive cytotoxic T-cell recognition epitopes and one or more CD4-positive helper T-cell recognition epitopes.

Item 21. The cancer treatment kit according to Item 13, which is for use in T-cell infusion therapy for low immunogenic cancer, metastatic cancer, or cancer with a low intratumoral T-cell count.
Item 22. The cancer treatment kit according to Item 13, wherein the pharmaceutical composition is administered before the T cell is administered.
Item 23. The cancer treatment kit according to Item 22, wherein the pharmaceutical composition is administered at least once after one or two units of administration wherein a single unit of administration consists of one-time administration of the pharmaceutical composition and subsequent one-time infusion of the T-cell.
Item 24. The cancer treatment kit according to Item 22, wherein the pharmaceutical composition is subcutaneously or intravenously administered at least twice, and the T-cell is transfused after the first administration of the pharmaceutical composition and before the second administration of the pharmaceutical composition.
Item 25. A method for treating cancer, comprising administering a pharmaceutical composition and a lymphocyte in combination to a subject with cancer,

the pharmaceutical composition containing a hyaluronic acid derivative having a hydrophobic group introduced and a cancer antigen,
the lymphocyte expressing an immune receptor for the cancer antigen.

Item 26. The cancer treatment kit for use in cancer treatment, the cancer treatment kit comprising

a pharmaceutical composition containing a hyaluronic acid derivative having a hydrophobic group introduced and a cancer antigen, and
a lymphocyte expressing an immune receptor for the cancer antigen.

Item 27. A pharmaceutical composition comprising a hyaluronic acid derivative having a hydrophobic group introduced and a cancer antigen,
the pharmaceutical composition being for use in cancer treatment comprising administering a lymphocyte expressing an immune receptor for the cancer antigen.
Item 28. A pharmaceutical composition comprising a lymphocyte expressing an immune receptor for a cancer antigen, the pharmaceutical composition being for use in cancer treatment comprising administering a pharmaceutical composition containing a hyaluronic acid derivative having a hydrophobic group introduced and the cancer antigen.
Item 29. Use of a pharmaceutical composition and a lymphocyte in combination for the production of a cancer treatment kit,

the pharmaceutical composition comprising a hyaluronic acid derivative having a hydrophobic group introduced and a cancer antigen,
the lymphocyte expressing an immune receptor for the cancer antigen.

Item 30. Use of a pharmaceutical composition comprising a hyaluronic acid derivative having a hydrophobic group introduced and a cancer antigen,
the use being for the production of a cancer treatment agent for combined administration with a lymphocyte expressing an immune receptor for the cancer antigen.
Item 31. Use of a lymphocyte expressing an immune receptor for a cancer antigen,

the use being for the production of a cancer treatment agent for combined administration with a pharmaceutical composition containing a hyaluronic acid derivative having a hydrophobic group introduced and the cancer antigen.

Advantageous Effects of Invention

The present invention provides a pharmaceutical composition that can induce an antitumor effect more potently, more efficiently, more sustainably, and/or over a wider range of areas.

Brief Description of Drawings

Fig. 1-1 is a graph showing therapeutic effects (tumor growth inhibition) on CMS5a tumors when CMS5a tumors subcutaneously inoculated into BALB/c mice were untreated, or treated by administration of an HA nanogel cancer vaccine loaded with a long-chain peptide antigen and CpG oligo DNA (a HANG-V group), by antigen-specific adoptive T-cell therapy (a TCR-T group), or by a combination of both of the above treatments (a HANG-V + TCR-T group) (Example 1). Each plot represents the mean tumor area of the mice in each group.

Fig. 1-2 is graphs showing therapeutic effects (tumor growth inhibition) on CMS5a tumors when CMS5a tumors subcutaneously inoculated into BALB/c mice were untreated, or treated by administration of an HA nanogel cancer vaccine loaded with a long-chain peptide antigen and CpG oligo DNA (a HANG-V group), by antigen-specific adoptive T-cell therapy (a TCR-T group), or by both of the above treatments (a HANG-V + TCR-T group) (Example 1). Each plot represents the measured tumor area of the mice in each group.

Fig. 1-3 is photographs showing tumor recurrence inhibition effects (tumor growth inhibition) when CMS5a tumors subcutaneously inoculated into BALB/c mice were treated by administration of an HA nanogel cancer vaccine loaded with a long-chain peptide antigen, CpG oligo DNA, and antigen-specific T cells, and CMS5a tumors were then re-inoculated into the mice with no recurrence after complete tumor regression (CR-Re). WT is photographs showing the tumor size when the same tumor was inoculated into wild-type BALB/c mice (WT) at the same time (Example 1).

Fig. 2-1 is graphs showing the number of cells in the draining lymph nodes at the vaccine administration site (inguinal lymph nodes) when CMS5a tumors subcutaneously inoculated into BALB/c mice were untreated, or treated by administration of an HA nanogel cancer vaccine loaded with a long-chain peptide antigen and CpG oligo DNA (a HANG-V group), by antigen-specific adoptive T-cell therapy (a TCR-T group), or by a combination of both of the above treatments (a HANG-V + TCR-T group) (Example 2).

Fig. 2-2 is photographs showing the size of the draining lymph node at the vaccine administration site (inguinal lymph node) and tumors when CMS5a tumors subcutaneously inoculated into BALB/c mice were untreated, or treated by administration of an HA nanogel cancer vaccine loaded with a long-chain peptide antigen and CpG oligo DNA (a HANG-V group), by antigen-specific adoptive T-cell therapy (a TCR-T group) or by a combination of both of the above treatments (a HANG-V + TCR-T group) (Example 2).

Fig. 2-3 is graphs showing the percentage of dendritic cells or macrophages in the draining lymph node at the vaccine administration site (inguinal lymph node) when CMS5a tumors subcutaneously inoculated into BALB/c mice were untreated, or treated by administration of an HA nanogel cancer vaccine loaded with a long-chain peptide antigen and CpG oligo DNA (a HANG-V group), by antigen-specific adoptive T-cell therapy (a TCR-T group), or by a combination of both of the above treatments (a HANG-V + TCR-T group) (Example 2). The graph also shows the percentage of CD207-positive Langerhans cells in total CD11c-positive cells.

Fig. 2-4-1 is a graph showing the number of T cells, B cells, NK cells, dendritic cells, and macrophages in the draining lymph nodes at the vaccine administration site (inguinal lymph nodes) when CMS5a tumors subcutaneously inoculated into BALB/c mice were untreated, or treated by administration of an HA nanogel cancer vaccine loaded with a long-chain peptide antigen and CpG oligo DNA (a HANG-V group) (Example 2).

Fig. 2-4-2 is graphs showing the number of T cells, B cells, or NK cells in the draining lymph nodes at the vaccine administration site (inguinal lymph nodes) when CMS5a tumors subcutaneously inoculated into BALB/c mice were untreated, or treated by administration of an HA nanogel cancer vaccine loaded with a long-chain peptide antigen and CpG oligo DNA (a HANG-V group) (Example 2).

Fig. 2-4-3 is graphs showing the number of dendritic cells and macrophages in the draining lymph nodes at the vaccine administration site (inguinal lymph nodes) when CMS5a tumors subcutaneously inoculated into BALB/c mice were untreated, or treated by administration of an HA nanogel cancer vaccine loaded with a long-chain peptide antigen and CpG oligo DNA (a HANG-V group) (Example 2).

Fig. 2-5 is a graph showing the percentage of CD90.1-positive infused cells in total CD8-positive T cells in the draining lymph nodes at the vaccine administration site (inguinal lymph nodes) when CMS5a tumors subcutaneously inoculated in BALB/c mice were untreated, or treated by administration of an HA nanogel cancer vaccine loaded

with a long-chain peptide antigen and CpG oligo DNA (a HANG-V group), by antigen-specific adoptive T-cell therapy (a TCR-T group), or by a combination of both of the above treatments (a HANG-V + TCR-T group) (Example 2).

Fig. 2-6 is graphs showing the percentage of interferon gamma (IFN-$\gamma$) producing cells in total CD90.1-positive, CD8-positive T cells or CD90.1-negative, CD8-positive T cells in the draining lymph nodes at the vaccine administration site (inguinal lymph nodes) when CMS5a tumors subcutaneously inoculated into BALB/c mice were untreated, or treated by administration of an HA nanogel cancer vaccine loaded with a long-chain peptide antigen and CpG oligo DNA (a HANG-V group), by antigen-specific adoptive T-cell therapy (a TCR-T group) or by a combination of both of the above treatments (a HANG-V + TCR-T group) (Example 2).

Fig. 3-1 is a graph showing therapeutic effects (tumor growth inhibition) on CMS5a tumors subcutaneously inoculated into BALB/c mice when the CMS5a tumors were untreated, or treated by administration of an HA nanogel cancer vaccine loaded with a long-chain peptide antigen and CpG oligo DNA (a HANG-V group) alone or in combination with antigen-specific adoptive T-cell therapy at a different number of cells ($2 \times 10^6$ cells, $1 \times 10^6$ cells, $5 \times 10^5$ cells, or $2.5 \times 10^6$ cells) (a HANG-V + TCR-T group) (Example 3). Each plot represents the mean tumor area of the mice in each group.

Fig. 3-2 is graphs showing therapeutic effects (tumor growth inhibition) on CMS5a tumors subcutaneously inoculated into BALB/c mice when CMS5a tumors were untreated, or treated by administration of an HA nanogel cancer vaccine loaded with a long-chain peptide antigen and CpG oligo DNA (a HANG-V group) alone or in combination with antigen-specific adoptive T-cell therapy at a different number of cells ($2 \times 10^6$ cells, $1 \times 10^6$ cells, $5 \times 10^5$ cells, or $2.5 \times 10^6$ cells) (a HANG-V + TCR-T group) (Example 3). The plots represent measured tumor areas of the mice in each group.

Fig. 3-3 is a graph showing the number of cell in the draining lymph node at the vaccine administration site (inguinal lymph node) when CMS5a tumors subcutaneously inoculated into BALB/c mice were untreated, or treated by administration of an HA nanogel cancer vaccine loaded with a long-chain peptide antigen and CpG oligo DNA (a HANG-V group) alone or in combination with antigen-specific adoptive T-cell therapy (TCR-T) at a different number of cells ($2 \times 10^6$ cells, $1 \times 10^6$ cells, $5 \times 10^5$ cells, or $2.5 \times 10^5$ cells) (a HANG-V + TCR-T group) (Example 3).

Fig. 3-4 is graphs showing the percentage of dendritic cells or macrophages in the draining lymph nodes at the vaccine administration site (inguinal lymph nodes) when CMS5a tumors subcutaneously inoculated into BALB/c mice were untreated, or treated by administration of an HA nanogel cancer vaccine loaded with a long-chain peptide antigen and CpG oligo DNA (a HANG-V group) alone or in combination with antigen-specific adoptive T-cell therapy (TCR-T) at a different number of cells ($2 \times 10^6$ cells, $1 \times 10^6$ cells, $5 \times 10^5$ cells, or $2.5 \times 10^5$ cells) (a HANG-V + TCR-T group) (Example 3). The graph also shows the percentage of CD207-positive Langerhans cells in the total CD11c-positive cells.

Fig. 3-5 is graphs showing the percentage of CD90.1-positive cells or CD90.2-negative cells in the total CD8-positive T cells in the draining lymph nodes at the vaccine administration site (inguinal lymph nodes) when CMS5a tumors subcutaneously inoculated into BALB/c mice were untreated, or treated by administration of a HA nanogel cancer vaccine loaded with a long-chain peptide antigen and CpG oligo DNA (a HANG-V group) alone or in combination with antigen-specific adoptive T-cell therapy (TCR-T) at a different number of cells ($2 \times 10^6$ cells, $1 \times 10^6$ cells, $5 \times 10^5$ cells, or $2.5 \times 10^5$ cells) (a HANG-V + TCR-T group) (Example 3).

Fig. 3-6 is graphs showing the percentage of interferon gamma (IFN-$\gamma$)-producing cells in the total CD90.1-positive, CD8-positive T cells or CD90.2-negative, CD-8-positive cells in the draining lymph nodes at the vaccine administration site (inguinal lymph nodes) when CMS5a tumors subcutaneously inoculated into BALB/c mice were untreated, or treated by administration of an HA nanogel cancer vaccine loaded with a long-chain peptide antigen and CpG oligo DNA (a HANG-V group) alone or in combination with antigen-specific adoptive T-cell therapy (TCR-T) at a different number of cells ($2 \times 10^6$ cells, $1 \times 10^6$ cells, $5 \times 10^5$ cells, or $2.5 \times 10^5$ cells)(a HANG-V + TCR-T group) (Example 3).

Fig. 3-7 is graphs showing the percentage of interferon gamma (IFN-$\gamma$)-producing cells in the total CD90.2-positive, CD8-positive T cells or CD90.1-negative, CD-8-positive cells in the draining lymph nodes at the vaccine administration site (inguinal lymph nodes) when CMS5a tumors subcutaneously inoculated into BALB/c mice were untreated, or treated by administration of an HA nanogel cancer vaccine loaded with a long-chain peptide antigen and CpG oligo DNA (a HANG-V group) alone or in combination with antigen-specific adoptive T-cell therapy (TCR-T) at a different number of cells ($2 \times 10^6$ cells, $1 \times 10^6$ cells, $5 \times 10^5$ cells, or $2.5 \times 10^5$ cells) (a HANG-V + TCR-T group) (Example 3).

Fig. 4 is a micrographs of CD8-positive T cells in CMS5a tumors subcutaneously inoculated into BALB/c mice when the CMS5a tumors were untreated, or treated by administration of an HA nanogel cancer vaccine loaded with a long-chain peptide antigen and CpG oligo DNA (a HANG-V group), by antigen-specific adoptive T-cell therapy (a TCR-T group), or by a combination of both of the above treatments (a HANG-V + TCR-T group) (Example 4) .

Fig. 5 is graphs showing the percentage of CD90.1-positive cells or CD90.2-negative cells in the total CD8-positive T cells in peripheral blood cells when CMS5a tumors subcutaneously inoculated into BALB/c mice were untreated, or treated by administration of a HA nanogel cancer vaccine loaded with a long-chain peptide antigen and CpG oligo DNA (a HANG-V group) alone or in combination with antigen-specific adoptive T-cell therapy (TCR-T) at a

different number of cells ($2 \times 10^6$ cells, $1 \times 10^6$ cells, $5 \times 10^5$ cells, or $2.5 \times 10^5$ cells) (a HANG-V + TCR-T group) (Example 5).

Fig. 6-1 is a graph showing therapeutic effects (tumor growth inhibition) on B16F10 when B16F10 tumors subcutaneously inoculated into C57BL/6 mice were untreated or treated by administration of an HA nanogel cancer vaccine loaded with a long-chain peptide antigen and CpG oligo DNA (a HANG-V group), by antigen-specific adoptive T-cell therapy (a TCR-T group), or by a combination of both of the above treatments (a HANG-V + TCR-T group) (Example 6). Each plot represents the mean tumor area of the mice in each group.

Fig. 6-2 is graphs showing therapeutic effects (tumor growth inhibition) on B16F10 when B16F10 tumors subcutaneously inoculated into C57BL/6 mice were untreated or treated by administration of an HA nanogel cancer vaccine loaded with a long-chain peptide antigen and CpG oligo DNA (a HANG-V group), by antigen-specific adoptive T-cell therapy (a TCR-T group), or by a combination of both of the above treatments (a HANG-V + TCR-T group) (Example 6). Each plot represents the measured tumor area of the mice in each group.

Fig. 6-3 is photographs showing the size and shape of tumors when B16F10 tumors subcutaneously inoculated into C57BL/6 mice were treated by administration of an HA nanogel cancer vaccine loaded with a long-chain peptide antigen and CpG oligo DNA (a HANG-V group) alone or in combination with antigen-specific adoptive T-cell therapy (a HANG-V + TCR-T group) (Example 6).

Fig. 6-4 is a graph showing therapeutic effects (extended survival) on B16F10 when B16F10 tumors subcutaneously inoculated into C57BL/6 mice were untreated or treated by administration of an HA nanogel cancer vaccine loaded with a long-chain peptide antigen and CpG oligo DNA (a HANG-V group), by antigen-specific adoptive T-cell therapy (a TCR-T group), or by a combination of both of the above treatments (a HANG-V + TCR-T group) (Example 6). Each plot represents the survival rate of mice in each group.

Fig. 6-5 is photographs showing mice with complete tumor regression when B16F10 tumors subcutaneously inoculated into C57BL/6 mice were treated by administration of a cancer vaccine loaded with a long-chain peptide antigen and CpG oligo DNA in combination with antigen-specific adoptive T-cell therapy (Example 6).

Fig. 7-1 is a graph showing therapeutic effects (tumor growth inhibition) on B16F10 when B16F10 tumors subcutaneously inoculated into C57BL/6 mice were untreated or treated by administration of a cancer vaccine loaded with a long-chain peptide antigen (HANG-V or CHP-V) and CpG oligo DNA in combination with antigen-specific adoptive T-cell therapy (a HANG-V + TCR-T group or a CHP-V + TCR-T group) (Example 7). Each plot represents the mean tumor area of the mice in each group.

Fig. 7-2 is graphs showing therapeutic effects (tumor growth inhibition) on B16F10 when B16F10 tumors subcutaneously inoculated into C57BL/6 mice were untreated or treated by administration of a cancer vaccine loaded with a long-chain peptide antigen (HANG-V or CHP-V) and CpG oligo DNA in combination with antigen-specific adoptive T-cell therapy (a HANG-V + TCR-T group or a CHP-V + TCR-T group) (Example 7). Each plot represents the measured tumor area of the mice in each group.

Fig. 7-3 is examples of photographs showing the size and shape of tumors when B16F10 tumors subcutaneously inoculated into C57BL/6 mice were treated by administration of a cancer vaccine loaded with a long-chain peptide antigen (HANG-V or CHP-V) and CpG oligo DNA in combination with antigen-specific adoptive T-cell therapy (a HANG-V + TCR-T group or a CHP-V + TCR-T group) (Example 7).

Fig. 7-4 is a graph showing therapeutic effects (extended survival) on B16F10 when B16F10 tumors subcutaneously inoculated into C57BL/6 mice were untreated or treated by administration of a cancer vaccine loaded with a long-chain peptide antigen (HANG-V or CHP-V) and CpG oligo DNA in combination with antigen-specific adoptive T-cell therapy (a HANG-V + TCR-T group or a CHP-V + TCR-T group) (Example 7). Each plot represents the survival rate of mice in each group.

Fig. 8-1 is a graph showing the number of cells in the draining lymph nodes at the vaccine administration site (inguinal lymph nodes) when B16F10 tumors subcutaneously inoculated into C57BL/6 mice were untreated or treated by administration of an HA nanogel cancer vaccine loaded with a long-chain peptide antigen and CpG oligo DNA (a HANG-V group), by antigen-specific adoptive T-cell therapy (a TCR-T group), or by a combination of both of the above treatments (a HANG-V + TCR-T group) (Example 8).

Fig. 8-2 is a graph showing the percentage of gp100 tetramer-positive (tet+) CD8-positive T cells in total CD8-positive T cells in the draining lymph nodes at the vaccine administration site (inguinal lymph nodes) when B16F10 tumors subcutaneously inoculated into C57BL/6 mice were untreated or treated by administration of an HA nanogel cancer vaccine loaded with a long-chain peptide antigen and CpG oligo DNA (a HANG-V group), by antigen-specific adoptive T-cell therapy (a TCR-T group), or by a combination of both of the above treatments (a HANG-V + TCR-T group) (Example 8).

Fig. 8-3 is a graph showing the expression intensity of CD44 or KLRG1 of gp100 tetramer-positive CD8-positive T cells in the draining lymph nodes at the vaccine administration site (inguinal lymph nodes) when B16F10 tumors subcutaneously inoculated into C57BL/6 mice were untreated or treated by administration of an HA nanogel cancer vaccine loaded with a long-chain peptide antigen and CpG oligo DNA (a HANG-V group), by antigen-specific adoptive

T-cell therapy (a TCR-T group), or by a combination of both of the above treatments (a HANG-V + TCR-T group) (Example 8).

Fig. 8-4 is graphs showing the percentage of interferon gamma (IFN-γ)-producing cells in CD90.1-positive CD8-positive T cells in the draining lymph nodes at the vaccine administration site (inguinal lymph nodes) when B16F10 tumors subcutaneously inoculated into C57BL/6 mice were untreated or treated by administration of an HA nanogel cancer vaccine loaded with a long-chain peptide antigen and CpG oligo DNA (a HANG-V group), by antigen-specific adoptive T-cell therapy (a TCR-T group), or by a combination of both of the above treatments (a HANG-V + TCR-T group) (Example 8).

Fig. 9-1 is graphs showing the percentage of CD90.1-positive CD8-positive infused T cells in total CD8-positive T cells in each tissue (draining lymph node (dLN) at the vaccine administration site, non-draining lymph node (NdLN), spleen (Spleen), tumor (TIL), and peripheral blood (PBMC)) when B16F10 tumors subcutaneously inoculated into C57BL/6 mice were treated by administration of a cancer vaccine loaded with a long-chain peptide antigen and CpG oligo DNA in combination with antigen-specific adoptive T-cell therapy (Example 9).

Fig. 9-2 is graphs showing the percentage of gp100 tetramer-positive (tet+) CD8-positive T cells in total CD8-positive T cells in each tissue (draining lymph node (dLN) at the vaccine administration site, non-draining lymph node (NdLN), spleen (Spleen), tumor (TIL), and peripheral blood (PBMC)) when B16F10 tumors subcutaneously inoculated into C57BL/6 mice were treated by administration of a cancer vaccine loaded with a long-chain peptide antigen and CpG oligo DNA in combination with antigen-specific adoptive T-cell therapy (Example 9).

Fig. 10-1 is a graph showing the percentage of CD90.1-positive or/and gp100 tetramer (tet)-positive CD8-positive infused T cells in total CD8-positive T cells in the peripheral blood of mice with no recurrence after complete tumor regression (CR) after B16F10 tumors subcutaneously inoculated into C57BL/6 mice were treated by administration of an HA nanogel cancer vaccine loaded with a long-chain peptide antigen, CpG oligo DNA, and antigen-specific T cells (Example 10).

Fig. 10-2 is graphs showing the percentage of CD90.1-positive or/and gp100 tetramer (tet)-positive CD8-positive infused T cells in total CD8-positive T cells in the peripheral blood of mice with no recurrence after complete tumor regression after B16F10 tumors subcutaneously inoculated into C57BL/6 mice were treated by administration of an HA nanogel cancer vaccine loaded with a long-chain peptide antigen, CpG oligo DNA, and antigen-specific T cells, followed by re-inoculated with B16F10 tumors (CR-Re) or re-vaccinated (CR-Vac) (Example 10).

Fig. 10-3 is graphs showing the percentage of CD44-positive CD62L-negative (effector memory T cells) and CD44-positive CD62L-positive (central memory T cells) in gp100 tetramer-positive CD8-positive CD90.1-positive infused T cells in the peripheral blood of mice with no recurrence after complete tumor regression after B16F10 tumors subcutaneously inoculated into C57BL/6 mice were treated by administration of an HA nanogel cancer vaccine loaded with a long-chain peptide antigen, CpG oligo DNA, and antigen-specific T cells, followed by re-inoculated with B16F10 tumors (CR-Re) or re-vaccinated (CR-Vac) (Example 10).

Fig. 10-4 is graphs showing the expression intensity of CD44-positive cells (High/Mid/Low) in CD8-positive T cells in the peripheral blood of mice with no recurrence after complete tumor regression after B16F10 tumors subcutaneously inoculated into C57BL/6 mice were treated by administration of an HA nanogel cancer vaccine loaded with a long-chain peptide antigen, CpG oligo DNA, and antigen-specific T cells, followed by re-inoculated with B16F10 tumors (CR-Re) or re-vaccinated (CR-Vac) (Example 10).

Fig. 10-5 is photographs showing recurrence-inhibitory effects (tumor growth inhibition) when B16F10 tumors subcutaneously inoculated into C57BL/6 mice were treated by administration of an HA nanogel cancer vaccine loaded with a long-chain peptide antigen, CpG oligo DNA, and antigen-specific T cells, and then mice with no recurrence after complete tumor regression were re-inoculated with C57BL/c tumors (CR-Re) or re-inoculated with C57BL/c tumors after re-vaccination (CR-Vac). WT is a photograph showing the tumor size when the same tumor was inoculated into wild-type B57BL/6 mice (WT) at the same time (Example 10).

Fig. 11-1 is a graph showing therapeutic effects (tumor growth inhibition) on CMS5a when CMS5a tumors subcutaneously inoculated into BALB/c mice were untreated or treated by administration of an HA nanogel cancer vaccine loaded with a long-chain peptide antigen, CpG oligo DNA, and antigen-specific T cells (Example 11). Each plot represents the mean tumor area of the mice in each group.

Fig. 11-2 is a graph comparing therapeutic effects (tumor growth inhibition) on CMS5a tumors when CMS5a tumors subcutaneously inoculated into BALB/c mice were untreated or treated by administration of an HA nanogel cancer vaccine loaded with a long-chain peptide antigen, CpG oligo DNA, and antigen-specific T cells, resulted in successful effects changing to tumor regrowth, and then mice with tumor regrowth after successful effects were treated by a combination of vaccine re-administration, antigen-specific T cells, and an immune checkpoint inhibitors (ICIs) (figure on left) (Example 11). Fig. 11-2 also is a graph showing tumor growth when a test similar to Example 1 or Example 11 was separately conducted, and additional treatment, such as vaccine re-administration, was not performed for mice with tumor regrowth (figure on right). Each plot represents the measured tumor area of the mice in each group.

Fig. 12-1 is a graph showing recurrence-inhibitory effects (tumor growth inhibition) when CMS5a tumors subcutaneously inoculated into BALB/c mice were treated by administration of an HA nanogel cancer vaccine loaded with a long-chain peptide antigen, CpG oligo DNA, and antigen-specific T cells, and then mice with complete tumor regression (CR) were re-inoculated with CMS5a tumors. WT is a graph showing tumor growth when the same tumor was inoculated into wild-type B57BL/6 mice (WT) at the same time (Example 12). Each plot represents the mean tumor area of the mice in each group.

Fig. 12-2 is graphs showing recurrence-inhibitory effects (tumor growth inhibition) when CMS5a tumors subcutaneously inoculated into BALB/c mice were treated by administration of an HA nanogel cancer vaccine loaded with a long-chain peptide antigen, CpG oligo DNA, and antigen-specific T cells, and then mice with complete tumor regression (CR) were re-inoculated with CMS5a tumors. WT is a graph showing tumor growth when the same tumor was inoculated into wild-type B57BL/6 mice (WT) at the same time (Example 12). Each plot represents the measured tumor area of the mice in each group.

Fig. 13-1 is a graph showing therapeutic effects (tumor growth inhibition) on CMS5a when CMS5a tumors subcutaneously inoculated into BALB/c mice were untreated or treated by administration of an HA nanogel cancer vaccine loaded with a long-chain peptide antigen, CpG oligo DNA, and antigen-specific T cells (single infusion) (Example 13). Each plot represents the mean tumor area of the mice in each group.

Fig. 13-2 is graphs showing therapeutic effects (tumor growth inhibition) on CMS5a when CMS5a tumors subcutaneously inoculated into BALB/c mice were untreated or treated by administration of an HA nanogel cancer vaccine loaded with a long-chain peptide antigen, CpG oligo DNA, and antigen-specific T cells (single infusion) (Example 13). Each plot represents the mean tumor area of the mice in each group.

Fig. 14 is a graph comparing therapeutic effects (tumor growth inhibition) on CMS5a tumors when CMS5a tumors subcutaneously inoculated into BALB/c mice were untreated or treated by administration of an HA nanogel cancer vaccine loaded with a long-chain peptide antigen, CpG oligo DNA, and antigen-specific T cells, resulted in successful effects changing to tumor regrowth, and then treated by vaccine re-administration and additional treatment with an immune checkpoint inhibitors (ICIs) (Example 14). Each plot represents the measured tumor area of the mice in each group.

Fig. 15 is graphs showing the percentage of CD44-positive CD62L-negative (effector memory T cells) and CD44-positive CD62L-positive (central memory T cells) in total CD8-positive T cells in peripheral blood cells when CMS5a tumors subcutaneously inoculated into BALB/c mice were treated by a combination of an HA nanogel cancer vaccine loaded with a long-chain peptide antigen, CpG oligo DNA, and antigen-specific adoptive T-cell therapy, and then mice with complete tumor regression (CR) were re-administered with the HA vaccine (Vac) or not re-administered (UT) (Example 15).

Fig. 16 is a graph showing the percentage of CD44-positive CD62L-negative cells (effector memory T cells) and CD44-positive CD62L-positive cells (central memory T cells) in total CD8-positive T cells in peripheral blood cells when CMS5a tumors subcutaneously inoculated into BALB/c mice were treated by a combination of an HA nanogel cancer vaccine loaded with a long-chain peptide antigen, CpG oligo DNA, and antigen-specific adoptive T-cell therapy, and then mice who received additional treatment due to tumor regrowth after successful effects (PD) were re-administered with the HA vaccine (Vac) or not re-administered (UT) (Example 16).

Fig. 17-1a shows the sequence of mMAGE#17 zG CAR.

Fig. 17-1b shows a schematic diagram of the structure of mMAGE#17 zG CAR.

Fig. 17-2a shows the sequence of mMAGE 28z CAR.

Fig. 17-2b shows a schematic diagram of the structure of mMAGE 28z CAR.

Fig. 17-3a shows the sequence of a MAGE-A4 expression vector.

Fig. 17-3b shows a schematic diagram of the structure of the MAGE-A4 expression vector.

Fig. 17-4a shows the sequence of a HHD-A2 expression vector.

Fig. 17-4b shows a schematic diagram of the structure of the HHD-A2 expression vector.

Fig. 17-5 is graphs showing the percentage of CD4-positive or CD8-positive T cells in cultured cells and the percentage of CAR expression relative to each T cell when MAGE#17 zG CAR gene was introduced into HLA-A2 Tg mouse-derived spleen cells cultured using anti-mouse CD3 antibody and CD28 antibody (Example 17).

Fig. 17-6 is graphs showing the percentage of expression of MAGE-A4 gene and HHD-A2 gene when both genes were introduced into MC38 and CT26 cells (Example 17).

Fig. 17-7 is a graph showing the amount of IFN-γ production in the culture supernatant when mMAGE#17 zG CAR-introduced T cells or CAR gene-free cells were added to MC38 and CT26 cells, and co-cultured (Example 17).

Description of Embodiments

In an embodiment, the present invention relates to a combination of a composition containing a hyaluronic acid derivative having a hydrophobic group introduced and an antigen ("HA-antigen composition" in the present specification) with a lymphocyte expressing an immune receptor for the antigen ("antigen-specific lymphocyte" in the present specification). The following describes the combination.

1. Definition

In the present specification, the terms "comprising" and "containing" include the concepts of comprising, containing, consisting essential of, and consisting of.

The term "$C_{1-20}$ alkyl" as used in the present specification refers to a linear or branched alkyl group having 1 to 20 carbon atoms. Examples include $C_{1-4}$ alkyl, such as methyl, ethyl, n-propyl, i-propyl, n-butyl, s-butyl, i-butyl, and t-butyl; and further include n-pentyl, 3-methylbutyl, 2-methylbutyl, 1-methylbutyl, 1-ethylpropyl, n-hexyl, 4-methylpentyl, 3-methylpentyl, 2-methylpentyl, 1-methylpentyl, 3-ethylbutyl, and 2-ethylbutyl. $C_{1-20}$ alkyl includes $C_{1-12}$ alkyl, which has 1 to 12 carbon atoms, and $C_{1-6}$ alkyl, which has 1 to 6 carbon atoms.

The term "$C_{1-6}$ alkyl" as used in the present specification refers to a linear or branched alkyl group having 1 to 6 carbon atoms. Examples include $C_{1-4}$ alkyl, such as methyl, ethyl, n-propyl, i-propyl, n-butyl, s-butyl, i-butyl, and t-butyl.

The term "$C_{1-6}$ alkyl carbonyl" as used in the present specification refers to an alkyl carbonyl group, the alkyl moiety of which is $C_{1-6}$ alkyl described above. Examples include $C_{1-4}$ alkyl carbonyl, such as acetyl, propionyl, n-propylcarbonyl, i-propylcarbonyl, n-butylcarbonyl, s-butylcarbonyl, i-butylcarbonyl, and t-butylcarbonyl.

The term "$C_{1-6}$ alkoxy" as used in the present specification refers to an alkyloxy group, the alkyl moiety of which is $C_{1-6}$ alkyl described above. Examples include $C_{1-4}$ alkoxy, such as methoxy ($H_3C$-O-), ethoxy, n-propoxy, i-propoxy, n-butoxy, s-butoxy, i-butoxy, and t-butoxy.

The term "$C_{1-6}$ alkylthio" as used in the present specification refers to an alkylthio group, the alkyl moiety of which is $C_{1-6}$ alkyl described above. Examples include methylthio ($H_3C$-S-), ethylthio, n-propylthio, i-propylthio, n-butylthio, s-butylthio, i-butylthio, and t-butylthio, with methylthio being preferred.

The term "amino $C_{2-20}$ alkyl" as used in the present specification refers to a linear or branched alkyl group having 2 to 20 carbon atoms with an amino group as a substituent. For example, the amino group may be at the terminal carbon atom of the alkyl group. Amino $C_{2-20}$ alkyl includes amino $C_{2-12}$ alkyl, which has 2 to 12 carbon atoms.

The term "hydroxy $C_{2-20}$ alkyl" as used in the present specification refers to a linear or branched alkyl group having 2 to 20 carbon atoms with a hydroxy group as a substituent. For example, the hydroxy group may be at the terminal carbon atom of the alkyl group. Hydroxy $C_{2-20}$ alkyl includes hydroxy $C_{2-12}$ alkyl, which has 2 to 12 carbon atoms.

The term "$C_{2-30}$ alkylene" as used in the present specification refers to a linear or branched divalent saturated hydrocarbon group having 2 to 30 carbon atoms. Examples include ethylene and propylene, and also $C_{2-20}$ alkylene, $C_{2-8}$ alkylene, and a $-(CH_2)n-$ group (wherein n is 2 to 30, preferably 2 to 20, and more preferably 2 to 15).

The term "$C_{1-5}$ alkylene" as used in the present specification refers to a linear or branched divalent saturated hydrocarbon group having 1 to 5 carbon atoms. Examples include methylene, ethylene (ethane-1,2-diyl and ethane-1,1-diyl), and propylene (propane-1,1-diyl and propane-1,2-diyl, butane-1,4-diyl, and pentane-1,5-diyl.

The term "$C_{2-10}$ alkylene" as used in the present specification refers to a linear or branched divalent saturated hydrocarbon group having 2 to 10 carbon atoms. Examples include ethylene (ethane-1,2-diyl and ethane-1,1-diyl), propylene (propane-1,1-diyl, propane-1,2-diyl, and propane-1,3-diyl), butane-1,4-diyl, pentane-1,5-diyl, hexane-1,6-diyl, heptane-1,7-diyl, and octane-1, 8-diyl. $C_{2-10}$ alkylene includes $C_{2-8}$ alkylene, which has 2 to 8 carbon atoms, and $C_{2-6}$ alkylene, which has 2 to 6 carbon atoms.

The term "$C_{2-8}$ alkylene" as used in the present specification refers to a linear or branched divalent saturated hydrocarbon group having 2 to 8 carbon atoms. Examples include ethylene (ethane-1,2-diyl and ethane-1,1-diyl), propylene (propane-1,1-diyl, propane-1,2-diyl, and propane-1,3-diyl), butane-1,4-diyl, pentane-1,5-diyl, hexane-1,6-diyl, heptane-1,7-diyl, and octane-1,8-diyl.

The term "$C_{2-8}$ alkenylene" as used in the present specification refers to a linear or branched divalent saturated hydrocarbon group having 2 to 8 carbon atoms with one or more double bonds. Examples include -CH=CH-, $-C(CH_3)=CH-$, 2-butene-1,4-diyl, hepta-2,4-diene-1,6-diyl, and octa-2,4,6-triene-1,8-diyl. $C_{2-8}$ alkenylene also includes their geometric isomers and mixtures thereof if such geometric isomers are present.

The term "aryl" as used in the present invention refers to an aromatic carbocyclic group, such as an aromatic carbocyclic group having 6 to 14 carbon atoms. Examples of aryl include phenyl and naphthyl (1-naphthyl and 2-naphthyl). Examples of aryl substituted with one or more hydroxy groups include 4-hydroxyphenyl.

The term "heteroaryl" in the present invention refers to an aromatic cyclic group containing one or more heteroatoms selected from the group consisting of a nitrogen atom, an oxygen atom, and a sulfur atom in the atoms constituting a ring, and the heteroaryl may be partially saturated. The ring may be a monocyclic ring or a bicyclic heteroaryl having a fused benzene ring or monocyclic heteroaryl ring. The number of atoms constituting the ring is, for example, 4 to 15, preferably 5 to 14, and more preferably 6 to 10. Examples of heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzoimidazolyl, indolyl, isoindolyl, indazolyl, quinolyl, isoquinolyl, cinnolinyl, quinazolinyl, quinoxalinyl, benzodioxolyl, indolizinyl, and imidazopyridyl, with indol-2-yl being preferred.

The term "divalent $C_{2-50}$ hydrocarbon group" as used in the present specification is not particularly limited. Examples include linear, branched, cyclic, or partially cyclic alkylene, alkenylene, or alkynylene groups having 2 to 50 carbon atoms. The divalent $C_{2-50}$ hydrocarbon group may be a divalent aromatic ring or may contain an aromatic ring as part of its structure.

The term "divalent $C_{2-50}$ polyalkyleneoxy" as used in the present specification is not particularly limited, and the alkylene group of the repeating unit may be a linear or branched chain. Examples of divalent $C_{2-50}$ polyalkyleneoxy include divalent $C_{2-50}$ polyethyleneoxy groups, $C_{3-48}$ polypropyleneoxy groups, and $C_{3-48}$ polybutyleneoxy groups. The divalent $C_{2-50}$ polyalkyleneoxy group may be linked to another group via an oxygen atom or a carbon atom. Examples of $C_{2-50}$ polyethyleneoxy groups include $-O(CH_2CH_2O)_{1-25}-$, $-(CH_2CH_2O)_{1-25}-$, $-(OCH_2CH_2)_{1-25}-$, and $-(CH_2CH_2O)_{1-24}-(CH_2CH_2)-$.

The term "salt substance" as used in the present specification is not particularly limited as long as the salt substance is a water-soluble inorganic substance. Examples include calcium salts, such as calcium chloride and calcium phosphate; magnesium salts, such as magnesium sulfate and magnesium chloride; aluminum salts, such as aluminum sulfate and aluminum chloride; potassium salts, such as potassium sulfate, potassium carbonate, potassium nitrate, potassium chloride, potassium bromide, and potassium iodide; sodium salts, such as sodium hydrogen carbonate, sodium carbonate, sodium sulfate, sodium nitrate, sodium chloride, sodium bromide, sodium iodide, sodium silicate, trisodium phosphate, disodium phosphate, sodium borate, sodium acetate, and sodium citrate; and lithium salts, such as lithium chloride, lithium bromide, lithium iodide, and lithium carbonate; with sodium chloride, trisodium phosphate, disodium phosphate, potassium chloride, calcium chloride, and magnesium chloride being preferred.

The term "antigen" as used in the present specification refers to a substance that evokes immunity. For example, the antigen is a substance that can induce activation of lymphocytes, such as T cells and B cells, when presented to antigen-presenting cells in the lymph nodes or spleen. The term "antigenic protein" refers to an antigen that is a protein. The term "antigen peptide" refers to an antigen that is a peptide. For example, the antigen peptide is part of an amino acid sequence contained in an antigenic protein and includes a T-cell recognition epitope. The antigen peptide may be a combination of two or more T cell recognition epitopes. The substance recognized as an antigen can be, for example, a protein or peptide itself, a complex of a protein or peptide with another molecule (e.g., major histocompatibility antigen (MHC)), or a major histocompatibility antigen (MHC) itself.

The term "adjuvant" as used in the present specification refers to a substance that enhances an immune response in a subject when administered to the subject in combination with a vaccine containing an antigen.

## 2. HA-Antigen Composition

### 2-1. Hyaluronic Acid Derivative Having a Hydrophobic Group Introduced

The hyaluronic acid derivative having a hydrophobic group introduced is hyaluronic acid or a derivative thereof, and is not particularly limited as long as the hyaluronic acid derivative has a hydrophobic group and is capable of forming a complex with an antigen (in particular, an antigen peptide or an antigenic protein).

The hydrophobic group has hydrophobicity and is not particularly limited as long as the hyaluronic acid derivative can form a complex with an antigen (in particular, an antigen peptide or an antigenic protein). The hydrophobic group preferably includes a hydrophobic group with a sterol skeleton (steryl group) and a hydrocarbon group, with a steryl group being particularly preferred.

The sterol skeleton is an alcohol whose hydroxy group is attached to a cyclopentahydrophenanthrene ring represented by formula I. The letters A to D in formula I each indicate a ring forming the cyclopentahydrophenanthrene ring.

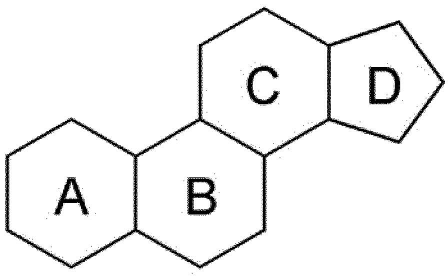

(I)

In the sterol skeleton, the cyclopentahydrophenanthrene ring may have a double bond, and the site at which the hydroxy group is bound is also not particularly limited. A preferable sterol has a hydroxy group attached at the C-3

position with a double bond present in ring B, or has a hydroxy group attached at the C-3 position and is composed of saturated rings. The hydrophobic group includes a group formed of a modified sterol skeleton, such as a group derived from a compound (steroid) substituted with a hydrocarbon group (e.g., a linear or branched, $C_{1-20}$ alkyl group) at a ring-forming carbon. The phrase "group derived from" means a group formed by removing a hydrogen atom or a functional group, such as a hydroxy group, from a compound.

Specifically, the steroid includes cholesterol, dehydrocholesterol, coprostenol, coprosterol, cholestanol, campestanol, ergostanol, stigmastanol, coprostanol, stigmasterol, sitosterol, lanosterol, ergosterol, simialenol, bile acids (cholanic acid, lithocholic acid, hyodeoxycholic acid, chenodeoxycholic acid, ursodeoxycholic acid, deoxycholic acid, apocholic acid, cholic acid, dehydrocholic acid, glycocholic acid, and taurocholic acid), testosterone, estradiol, progesterone, cortisol, cortisone, aldosterone, corticosterone, and deoxycortisterone. The steryl group includes a cholesteryl group, a stigmasteryl group, a lanosteryl group, an ergosteryl group, a cholanoyl group, and a choloyl group, with a cholesteryl group (in particular, the cholest-5-en-3β-yl group shown in the formula below) and a cholanoyl group (in particular, the 5β-cholan-24-oil group shown in the formula below) being preferred. In the formulas, two asterisks represent a binding site.

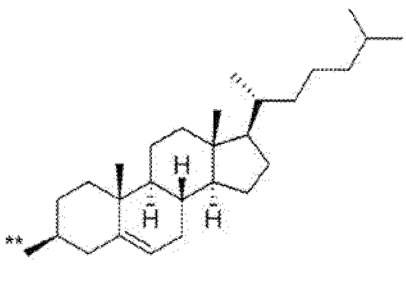

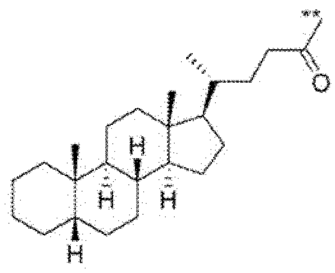

The hydrocarbon group is not particularly limited, and examples include a chain (preferably linear) hydrocarbon group (preferably an alkyl group) having 8 to 50 (preferably 10 to 30, and more preferably 12 to 20) carbon atoms.

The weight average molecular weight of the hyaluronic acid derivative having a hydrophobic group introduced is not particularly limited as long as the hyaluronic acid derivative can form a complex with an antigen. The hyaluronic acid derivative having a hydrophobic group introduced has a weight average molecular weight of, for example, 5,000 to 2,000,000, preferably 10,000 to 1,000,000, more preferably 20,000 to 500,000, still more preferably 40,000 to 250,000, and yet more preferably 80,000 to 125,000.

The introduction rate of the hydrophobic group is, for example, 1 to 50%, preferably 7 to 50%, more preferably 17 to 50%, and still more preferably 20 to 45%. An introduction rate falling within these ranges allows the hyaluronic acid derivative to efficiently form a complex with an antigen in a solution. The introduction rate of the hydrophobic group can be measured and calculated according to or in accordance with the method described below.

Specific examples of the hyaluronic acid derivative having a hydrophobic group introduced include a hyaluronic acid derivative containing one or more disaccharide units (which are also repeating units) represented by formula (I) or a hyaluronic acid derivative containing one or more disaccharide units (which are also repeating units) represented by formula (II). The hyaluronic acid derivative is, for example, a hyaluronic acid derivative comprising one or more repeating units represented by formula (I):

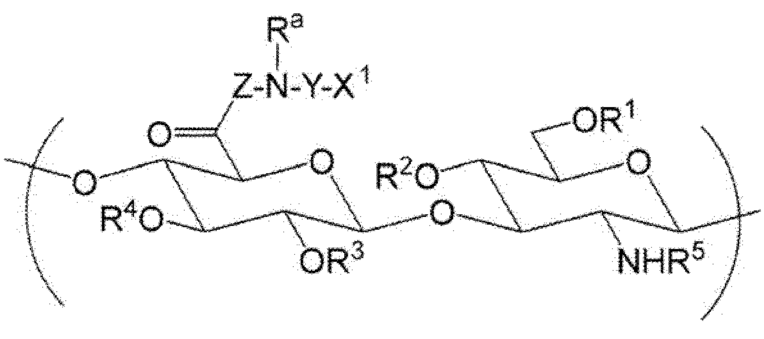 (I)

wherein $R^1$, $R^2$, $R^3$, and $R^4$ are each independently selected from the group consisting of a hydrogen atom, $C_{1-6}$ alkyl, formyl, and $C_{1-6}$ alkyl carbonyl;

$R^5$ represents a hydrogen atom, formyl, or $C_{1-6}$ alkyl carbonyl;

Z represents a direct bond or a peptide linker composed of 2 to 30 any amino acid residues;
$X^1$ represents a hydrophobic group selected from the groups represented by the following formulas:

$-NR^b-R$,

$-NR^b-COO-R$,

$-NR^b-CO-R$,

$-NR^b-CO-NR^c-R$,

-COO-R,

-O-COO-R,

-S-R,

$-CO-Y^a-S-R$,

$-O-CO-Y^b-S-R$,

$-NR^b-CO-Y^b-S-R$, and

-S-S-R;

$R^a$, $R^b$, and $R^c$ are each independently selected from the group consisting of a hydrogen atom, $C_{1-20}$ alkyl, amino $C_{2-20}$ alkyl, and hydroxy $C_{2-20}$ alkyl, wherein an alkyl moiety of $R^a$, $R^b$, and $R^c$ may have 1 to 3 groups inserted, the 1 to 3 groups being selected from the group consisting of -O- and $-NR^f-$;
$R^f$ is selected from the group consisting of a hydrogen atom, $C_{1-12}$ alkyl, amino $C_{2-12}$ alkyl, and hydroxy $C_{2-12}$ alkyl, wherein an alkyl moiety of $R^f$ may have 1 or 2 groups inserted, the 1 or 2 groups being selected from the group consisting of -O- and -NH-;
R represents a steryl group;
Y represents $C_{2-30}$ alkylene or - $(CH_2CH_2O)_m-CH_2CH_2-$, wherein the alkylene may have 1 to 5 groups inserted, the 1 to 5 groups being selected from the group consisting of -O-, $-NR^g-$, and -S-S-;
$R^g$ is selected from the group consisting of a hydrogen atom, $C_{1-20}$ alkyl, amino $C_{2-20}$ alkyl, and hydroxy $C_{2-20}$ alkyl, wherein an alkyl moiety of $R^g$ may have 1 to 3 groups inserted, the 1 to 3 groups being selected from the group consisting of -O- and -NH-;
$Y^a$ represents $C_{1-5}$ alkylene;
$Y^b$ represents $C_{2-8}$ alkylene or $C_{2-8}$ alkenylene; and m represents an integer selected from 1 to 100; or a hyaluronic acid derivative comprising a repeating unit represented by formula (II) :

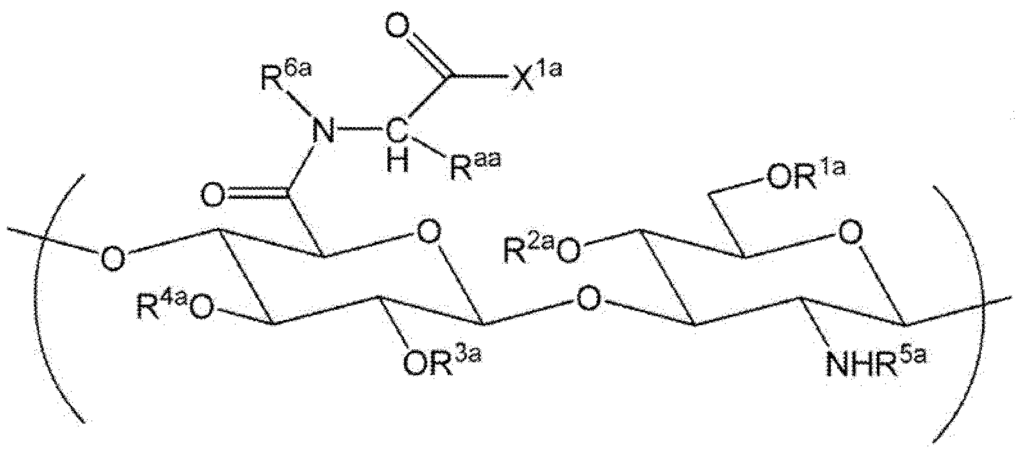

(II)

wherein $R^{1a}$, $R^{2a}$, $R^{3a}$, and $R^{4a}$ are each independently selected from the group consisting of a hydrogen atom,

$C_{1-6}$ alkyl, formyl, and
$C_{1-6}$ alkyl carbonyl;
$R^{5a}$ represents a hydrogen atom, formyl, or $C_{1-6}$ alkyl carbonyl;
$X^{1a}$ represents hydroxy, $-O-Q^+$, $C_{1-6}$ alkoxy, $-NR^7R^8$, or $-NR^9-Z^1-Z^2$;
$Q^+$ represents a countercation;
$R^{6a}$, $R^7$, $R^8$, and $R^9$ are each independently selected from the group consisting of a hydrogen atom and $C_{1-6}$ alkyl;
$R^{aa}$ represents a hydrogen atom or $C_{1-6}$ alkyl, wherein the alkyl may be substituted with one or more groups independently selected from the group consisting of hydroxy, carboxy, carbamoyl, $C_{1-6}$ alkylthio, aryl, and heteroaryl, and the aryl may be substituted with one or more hydroxy groups;
$Z^1$ represents $C_{2-30}$ alkylene or - $(CH_2CH_2O)_{ma}-CH_2CH_2-$, wherein the alkylene may have 1 to 5 groups inserted, the 1 to 5 groups being independently selected from the group consisting of -O-, $-NR^{ga}-$, and -S-S-, and ma is an integer selected from 1 to 100;
$Z^2$ represents a group selected from the groups represented by the following formulas:

$-NR^{ba}-Z^3$,

$-NR^{ba}-COO-Z^3$,

$-NR^{ba}-CO-Z^3$,

$-NR^{ba}-CO-NR^{ca}-Z^3$,

$-COO-Z^3$,

$-CO-NR^{ca}-Z^3$,

$-O-CO-NR^{ca}-Z^3$,

$-O-COO-Z^3$,

$-S-Z^3$,

$-CO-Z^a-S-Z^3$,

$-O-CO-Z^b-S-Z^3$,

$-NR^{ba}-CO-Z^b-S-Z^3$, and

$-S-S-Z^3$;

$R^{ba}$ and $R^{ca}$ are each independently selected from the group consisting of a hydrogen atom, $C_{1-20}$ alkyl, amino $C_{2-20}$ alkyl, and hydroxy $C_{2-20}$ alkyl, wherein an alkyl moiety of $R^{ba}$ and $R^{ca}$ may each independently have 1 to 3 groups inserted, the 1 to 3 groups being selected from the group consisting of -O- and $-NR^{fa}-$;
$R^{fa}$ is independently selected from the group consisting of a hydrogen atom, $C_{1-12}$ alkyl, amino $C_{2-12}$ alkyl, and hydroxy $C_{2-12}$ alkyl, wherein an alkyl moiety of $R^{fa}$ may independently have 1 or 2 groups inserted, the 1 or 2 groups being selected from the group consisting of -O- and -NH-;
$R^{ga}$ is independently selected from the group consisting of a hydrogen atom, $C_{1-20}$ alkyl, amino $C_{2-20}$ alkyl, and hydroxy $C_{2-20}$ alkyl, wherein an alkyl moiety of $R^{ga}$ may independently have 1 to 3 groups inserted, the 1 to 3 groups being selected from the group consisting of -O- and -NH-;
$Z^3$ represents a steryl group;
$Z^a$ represents $C_{1-5}$ alkylene; and
$Z^b$ represents $C_{2-8}$ alkylene or $C_{2-8}$ alkenylene. If the hyaluronic acid derivative does not contain a repeating unit represented by formula (II) in which $X^{1a}$ is $-NR^9-Z^1-Z^2$, the hyaluronic acid derivative further comprises a repeating unit represented by formula (III):

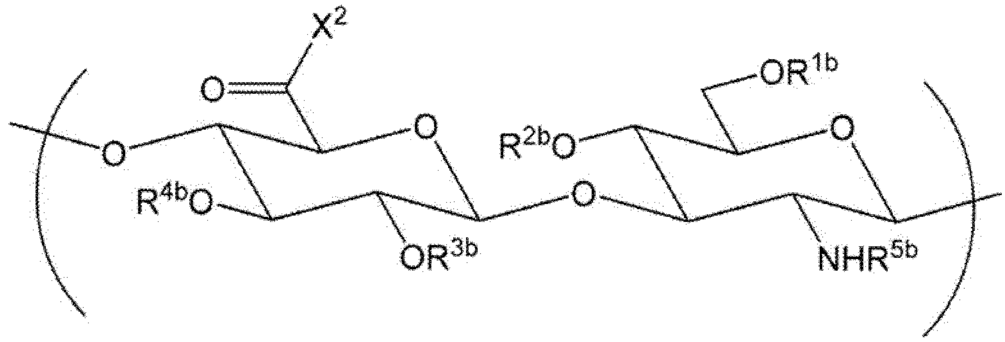

(III)

wherein $R^{1b}$, $R^{2b}$, $R^{3b}$, and $R^{4b}$ are each independently selected from the group consisting of a hydrogen atom, $C_{1-6}$ alkyl, formyl, and $C_{1-6}$ alkyl carbonyl;
$R^{5b}$ represents a hydrogen atom, formyl, or $C_{1-6}$ alkyl carbonyl; and $X^2$ represents $-NR^9-Z^1-Z^2$, wherein $R^9$, $Z^1$, and $Z^2$ are as defined above.

In the hyaluronic acid derivatives above, the hyaluronic acid derivative having a hydrophobic group introduced preferably further contains a repeating unit represented by formula (IIIc):

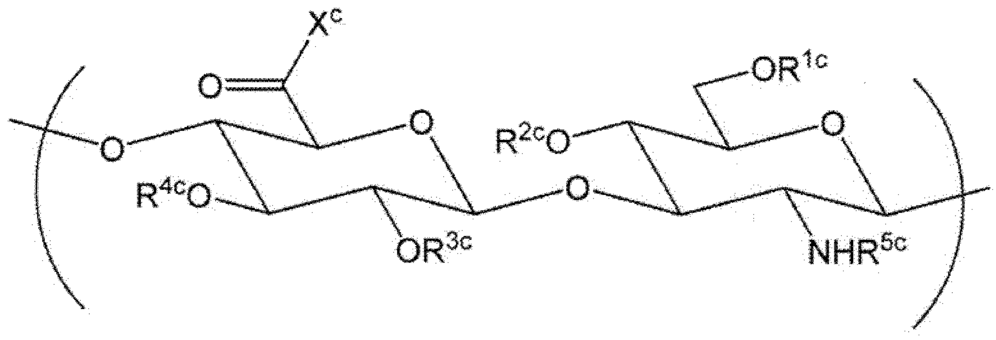

(IIIc)

wherein $R^{1c}$, $R^{2c}$, $R^{3c}$, and $R^{4c}$ are each independently selected from the group consisting of a hydrogen atom, $C_{1-6}$ alkyl, formyl, and $C_{1-6}$ alkyl carbonyl;
$R^{5c}$ is selected from the group consisting of a hydrogen atom, formyl, and $C_{1-6}$ alkyl carbonyl; and
$X^c$ is selected from the group consisting of hydroxy and $-O-Q^+$, wherein $Q^+$ represents a countercation.

In an embodiment, the hyaluronic acid derivative containing the repeating unit represented by formula (I) is substantially composed of (1) the repeating unit represented by formula (I), or composed of (2) the repeating unit represented by formula (I) and the repeating unit represented by formula (IIIc).

If the hyaluronic acid derivative containing the repeating unit represented by formula (I) contains two or more repeating units represented by formula (I), these repeating units may be the same or different. The hyaluronic acid derivative may be modified at a site other than the repeating unit represented by formula (I). For example, a hydroxy group may be replaced with -O ($C_{1-6}$ alkyl), -O (formyl), -O($C_{1-6}$ alkyl carbonyl), or a like group, and a carboxy group may be replaced with an amide group or an ester group, or may form a salt.

In an aspect, the $-Z-N(R^a)Y-X^1$ group in formula (I) is selected from the groups represented by the following formulas:

$-NH-(CH_2)_{mz}-NH-R$;

$-NH-(CH_2)_{mz}-NH-COO-R$;

$-NH-(CH_2CH_2O)_m-CH_2CH_2-NH-COO-R$;

$-NH-(CH_2)_{mz}-COO-R$;

$-NH-(CH_2CH_2O)_m-CH_2CH_2-COO-R$;

$-NH-(CH_2)_{mz}-O-COO-R;$

$-NH-(CH_2CH_2O)_m-CH_2CH_2-O-COO-R;$

$-NH-(CH_2)_{mz}-S-R;$

$-NH-(CH_2CH_2O)_m-CH_2CH_2-S-R;$

$-NH-(CH_2)_{mz}-O-CO-CH(R^{10})-CH_2-S-R;$

$-NH-(CH_2)_{mz}-NHCO-CH(R^{10})-CH_2-S-R;$

$-NH-(CH_2CH_2O)_m-CH_2CH_2-NHCO-CH(R^{10})-CH_2-S-R;$

$-NH- (CH_2CH_2O)_m-CH_2CH_2-O-CO-CH(R^{10})-CH_2-S-R;$

$-NH-(CH_2)_{mz}-S-S-R;$

and

$-Z-NR^a-Y-NR^b-COO-R$

wherein mz represents an integer of 2 to 30, $R^{10}$ represents a hydrogen atom or methyl group, and R and m are as defined above in this specification.

The $-Z-N(R^a)$ $Y-X^1$ group is preferably selected from the group consisting of

$-NH-(CH_2)_{mz}-NH-COO-R;$

$-NH-(CH_2CH_2O)_m-CH_2CH_2-NH-COO-R;$

and

$-NH-(CH_2)_{mz}-S-S-R$

wherein mz, R, and m are as defined above in this specification.

In a preferable embodiment, Z in formula (I) represents a direct bond. In an embodiment, when Z in formula (I) is a peptide linker, $X^1$ is $-NR^b-COO-R$.

Specific examples of Y in formula (I) include $-CH_2CH_2O-CH_2CH_2-S-S-CH_2CH_2O-CH_2CH_2-$, $-(CH_2CH_2O)_2-CH_2CH_2-S-S-CH_2CH_2O-CH_2CH_2-$, $-CH_2CH_2O-CH_2CH_2-S-S-(CH_2CH_2O)_2-CH_2CH_2-$, and $-(CH_2CH_2O)_2-CH_2CH_2-S-S-(CH_2CH_2O)_2-CH_2CH_2-$.

$Y^a$ in formula (I) is preferably $-CH_2-$ and $-CH_2-CH_2-$.

$Y^b$ in formula (I) is more preferably $-CH_2-CH_2-$, $-CH(CH_3)CH_2-$, 2-butene-1,4-diyl, hepta-2,4-diene-1,6-diyl, and octa-2,4,6-triene-1,8-diyl, and more preferably $-CH_2-CH_2-$ and $-CH(CH_3)CH_2-$.

In an embodiment, Z in formula (I) is a peptide linker represented by $-NH-[CH(-Z^a)-CONH]_n-1-CH(-Z^a)-CO-$ wherein n is an integer of 2 to 30, and each $Z^a$ is independently a substituent in α-amino acid represented by $H_2N-CH(-Z^a)-COOH$. The peptide linker binds to the carboxy group of the glucuronic acid moiety at the N-terminus and binds to the $-N(-R^a)-Y-X^1$ group at the C-terminus. Examples of the amino acid usable as an amino acid residue for the peptide linker include α-amino acids, such as wild (L-) amino acids, such alanine, arginine, asparagine (Asn), aspartic acid, cysteine, glutamine, glutamic acid, glycine (Gly), histidine, isoleucine, leucine (Leu), lysine, methionine, phenylalanine (Phe), proline, serine, threonine, tryptophan, tyrosine, and valine, and D-forms of these amino acids. All of the α-amino acids, including synthesized amino acids, are usable. Specifically, examples of $Z^a$ include $-CH_3$, $H_2NC(NH)NH(CH_2)_3-$, and $H_2NCOCH_2-$. When Z is present in the number n, Z may be the same or different. n is an integer of 2 to 30, preferably 2 to 10, and more preferably 2 to 4. Preferable examples of the peptide linker include -Gly-Phe-Leu-Gly-, -Asn-Phe-Phe-, -Phe-Phe-, and -Phe-Gly-.

Specific examples of the $-Z-N(R^a)Y-X^1$ group in formula (I) include $-NH-(CH_2)_2-NH-CO-$cholesteryl, $-NH-(CH_2)_4-NH-(CH_2)_3-NH-(CH_2)_3-NH-COO-$cholesteryl, $-NH-(CH_2)_3-NH-(CH_2)_4-NH-(CH_2)_3-NH-COO-$cholesteryl, $-NH-(CH_2)_4-NH-(CH_2)_3-NH-COO-$cholesteryl, $-NH-(CH_2)_4-N(-(CH_2)_3-NH_2)-COO-$cholesteryl, $-NH-(CH_2)_3-NH-(CH_2)_4-N(-(CH_2)_3-NH_2)-COO-$cholesteryl, $-NH-(CH_2)_3-NH-(CH_2)_4-N(-(CH_2)_3-NH-(CH_2)_3-NH_2)-COO-$

cholesteryl, $-NH-(CH_2)_3-NH-(CH_2)_4-N(-(CH_2)_3-NH_2)-CO-NH-$cholesteryl, $-NH-(CH_2)_3-NH-(CH_2)_4-N(-(CH_2)_3-NH_2)-CO-$cholesteryl, and $-NH-(CH_2)_3-NH-(CH_2)_4-N(-(CH_2)_3-NH_2)-$cholesteryl. In a preferable embodiment of the $-Z-N(R^a)Y-X^1$ group, $R^a$, $R^b$, and $R^c$ are a hydrogen atom, Y is linear $C_{2-30}$ alkylene or - $(CH_2CH_2O)_m-CH_2CH_2-$, and $Y^a$ is linear $C_{1-5}$ alkylene or $Y^b$ is linear $C_{2-8}$ alkylene or linear $C_{2-8}$ alkenylene.

In an embodiment of the $Z-N(R^a)Y-X^1$ group, Z is a direct bond, $R^a$ is a hydrogen atom, Y is, for example, $C_{2-12}$ alkylene, preferably $C_{2-6}$ alkylene, and more preferably $C_6$ alkylene, $X^1$ is $-NR^b-COO-R$, $R^b$ is a hydrogen atom, and R is a cholesteryl group.

In an embodiment, the hyaluronic acid derivative containing the repeating unit represented by formula (I) further contains the repeating unit represented by formula (IIIc). If the hyaluronic acid derivative contains two or more repeating units represented by formula (IIIc), these repeating units may be the same or different.

$Q^+$ in formula (IIIc) is not particularly limited as long as $Q^+$ is a countercation that forms a salt with a carboxy group in water. If $Q^+$ is divalent or higher valent, $Q^+$ forms a salt with multiple carboxy groups according to its valence. Examples of countercations include metal ions, such as a lithium ion, a sodium ion, a rubidium ion, a cesium ion, a magnesium ion, and a calcium ion; and ammonium ions represented by formula: $N+R^jR^kR^lR^m$ (wherein $R^j$, $R^k$, $R^l$, and $R^m$ are each independently selected from the group consisting of a hydrogen atom and $C_{1-6}$ alkyl) . The countercation is preferably a sodium ion, a potassium ion, and a tetraalkyl ammonium ion (e.g., tetra-n-butyl ammonium ion). $R^j$, $R^k$, $R^l$, and $R^m$ are preferably the same group selected from $C_{1-6}$ alkyl, and preferably an n-butyl group.

It is preferred that $R^1$, $R^2$, $R^3$, and $R^4$ in formula (I) and $R^{1a}$, $R^{2a}$, $R^{3a}$, and $R^{4a}$ in formula (IIIc) be all a hydrogen atom. It is also preferred that $R^a$ and $R^b$ both be a hydrogen atom.

$R^5$ in formula (I) is preferably acetyl.

In an embodiment, the hyaluronic acid derivative containing the repeating unit represented by formula (I) is substantially composed of the repeating units represented by formulas (I) and (IIIc). In the hyaluronic acid derivative, the repeating unit represented by formula (I) or (IIIc) accounts for, for example, 80% or more, preferably 90% or more, and more preferably 95% or more, of the repeating units, which are disaccharides formed of D-glucuronic acid and N-acetylglucosamine contained in the derivative. In an embodiment, the hyaluronic acid derivative is composed of only the repeating units represented by formula (I) and formula (IIIc).

Y defined in formula (I) may be, for example, $-(CH_2)_{na}-$(wherein na is an integer selected from 2 to 20, preferably 2 to 15, more preferably 2 to 12, and still more preferably 2 to 10), preferably $-(CH_2)_2-$, $-(CH_2)_6-$, $-(CH_2)_8-$, and $-(CH_2)_{12}-$, and more preferably $-(CH_2)_6-$. These Ys are preferred from the viewpoint of precipitation formation and stable dispersion, as described later.

In an aspect, the hyaluronic acid derivative containing the repeating unit represented by formula (I) has an introduction rate of the hydrophobic group relative to the disaccharide repeating units present in the derivative of, for example, 1 to 50%, preferably 7 to 50%, more preferably 17 to 50%, and still more preferably 20 to 45%. An introduction rate within these ranges allows the hyaluronic acid derivative containing the repeating unit represented by formula (I) to efficiently form a complex with an antigen in a solution.

The introduction rate of the hydrophobic group is calculated according to the following formula:

$$\text{(The introduction rate of the hydrophobic group)} = \frac{\text{(The number of disaccharide repeating units having the hydrophobic group introduced)}}{\text{(The number of disaccharide repeating units present)}} \times 100$$

"The disaccharide repeating units present in the derivative" include the repeating unit represented by formula (I) having the carboxy group replaced with an amide group and having a hydrophobic group introduced and the repeating unit represented by formula (IIIc) having a hydrophobic group not introduced. The introduction rate can be controlled by reaction conditions, such as the proportion of reagents, and can be determined, for example, by NMR measurement.

The hyaluronic acid derivative containing the repeating unit represented by formula (I) is preferably synthesized from hyaluronic acid composed of only the repeating unit represented by formula (IIIc) or a derivative thereof as a starting material that has a weight average molecular weight of, for example, 1 kDa to 500 kDa, preferably 3 kDa to 500 kDa, and more preferably 5 kDa to 200 kDa, when $R^{1c}$, $R^{2c}$, $R^{3c}$, and $R^{4c}$ are all a hydrogen atom, $R^{5c}$ is acetyl, and $X^c$ is $-O-N^{a+}$. In an embodiment, from the viewpoint of forming a complex with an antigen, the weight average molecular weight of the starting material is preferably 1 kDa to 1000 kDa, and more preferably 3 kDa to 200 kDa. From the viewpoint of transfer of the complex to lymph nodes, the weight average molecular weight of the starting material is preferably 1 kDa to 1000 kDa, more preferably 3 kDa to 500 kDa, still more preferably 5 kDa to 200 kDa, and yet more preferably 5 kDa to 150 kDa.

Because it is generally difficult to obtain hyaluronic acid or a derivative thereof as a single product, their molecular

weight is calculated as the number average molecular weight or weight average molecular weight. In the present invention, the molecular weight is calculated as weight average molecular weight. For example, various known methods, such as the light scattering method, osmotic pressure method, or viscosity method, as described in Essential Kobunshi Kagaku [Essential Polymer Science] (published by Kodansha Ltd, ISBN4-06-153310-X) by Seiichi Nakahama et al., can be used to measure the weight average molecular weight. The viscometric average molecular weight indicated in the present specification can also be measured according to a method commonly used in the technical field to which the present invention pertains, such as an Ubbelohde viscometer. If hyaluronic acid and a derivative thereof for use are commercially marketed with their molecular weights clearly indicated, the indicated value may be taken as a molecular weight.

The hyaluronic acid derivative containing the repeating unit represented by formula (I) incorporates a hydrophobic group by replacing the carboxy group in the glucuronic acid (one saccharide of the disaccharide constituting the repeating unit) with an amide group. The degree of modification of the hyaluronic acid derivative can be adjusted to control the disposition of the formulation produced by using the hyaluronic acid derivative.

If the hyaluronic acid derivative containing the repeating unit represented by formula (I) has a high modification rate of the carboxy group in the glucuronic acid moiety, its binding to hyaluronic acid receptors, including CD44, is inhibited, and the hyaluronic acid derivative becomes a drug carrier (including vaccines) that stays longer in vivo. Furthermore, a target element introduced into the hyaluronic acid derivative can target various organs, including lymph nodes, and cells. Examples of target elements include target tissue-specific peptides, antibodies, fragmented antibodies, aptamers, R9D peptides for cancer cells, folic acid, anisamide, transferrin, galactose for the liver, and tocopherol.

The hyaluronic acid derivative containing the repeating unit represented by formula (I) has a modification rate of the carboxy group of the glucuronic acid moiety with a hydrophobic group (i.e., the introduction rate of a hydrophobic group) of preferably 4 to 60%, more preferably 5 to 50%, still more preferably 7 to 50%, and even more preferably 7 to 45%, from the viewpoint of forming a complex with an antigen. From the viewpoint of transfer of the complex into lymph nodes, the hyaluronic acid derivative has a modification rate of preferably 6 to 60%, more preferably 17 to 50%, still more preferably 20 to 50%, and even more preferably 20 to 45%.

From the viewpoint of forming a complex with an antigen, the combination of the molecular weight of the starting material for the hyaluronic acid derivative containing the repeating unit represented by formula (I) and the introduction rate of the hydrophobic group is preferably a combination of 3 kDa to 500 kDa and 4 to 60%, more preferably 3 kDa to 200 kDa and 6 to 50%, still more preferably 5 kDa to 200 kDa and 6 to 50%, and even more preferably 5 kDa to 150 kDa and 6 to 45%. From the viewpoint of transfer of the complex into lymph nodes, the combination is preferably a combination of 3 kDa to 500 kDa and 4 to 60%, more preferably 3 kDa to 200 kDa and 6 to 50%, still more preferably 5 kDa to 200 kDa and 6 to 50%, and even more preferably 5 kDa to 150 kDa and 20 to 45%. From the viewpoint of stable dispersion, the combination is preferably a combination of 3 kDa to 200 kDa and 4 to 60%, more preferably 3 kDa to 200 kDa and 6 to 50%, still more preferably 5 kDa to 200 kDa and 6 to 50%, and even more preferably 5 kDa to 150 kDa and 17 to 45%. From the viewpoint of improving blood retention, the combination is preferably a combination of 5 kDa to 27 kDa and 2 to 50%, more preferably 5 kDa to 27 kDa and 8 to 35%, still more preferably 5 kDa to 18 kDa and 8 to 35%, even more preferably 5 kDa to 18 kDa and 8 to 35%, and yet more preferably 5 kDa to 18 kDa and 15 to 22%. From the viewpoint of gelation, the combination is preferably a combination of 5 kDa to 300 kDa and 2 to 30%, more preferably 5 kDa to 50 kDa and 2 to 22%, still more preferably 5 kDa to 27 kDa and 2 to 22%, and even more preferably 5 kDa to 27 kDa and 7 to 22%.

In an embodiment, the hyaluronic acid derivative containing the repeating unit represented by formula (II) is substantially composed of (1) the repeating unit represented by formula (II); (2) the repeating unit represented by formula (II) and the repeating unit represented by formula (III); (3) the repeating unit represented by formula (II) and the repeating unit represented by formula (IIIc); or (4) the repeating unit represented by formula (II), the repeating unit represented by formula (III), and the repeating unit represented by formula (IIIc). In the hyaluronic acid derivative, the repeating unit represented by formula (II), (III), or formula (IIIc) account for, for example, 80% or more, preferably 90% or more, and more preferably 95% or more of the repeating units of the disaccharide formed of D-glucuronic acid and N-acetylglucosamine contained in the hyaluronic acid derivative.

In an embodiment, the hyaluronic acid derivative is composed of only (1) the repeating unit represented by formula (II); (2) the repeating unit represented by formula (II) and the repeating unit represented by formula (III); (3) the repeating unit represented by formula (II) and the repeating unit represented by formula (IIIc); or (4) the repeating unit represented by formula (II), the repeating unit represented by formula (III), and the repeating unit represented by formula (IIIc).

The ratio of a specific disaccharide unit to the disaccharide repeating units present in the hyaluronic acid derivative containing the repeating unit represented by formula (II) means the ratio of a specific disaccharide unit to all of the disaccharide units present in a predetermined amount of the hyaluronic acid derivative containing the repeating unit represented by formula (II), which is a polysaccharide having disaccharide units as a repeating unit.

In formula (II), which represents the disaccharide unit contained in the hyaluronic acid derivative containing the repeating unit represented by formula (II), $R^{1a}$, $R^{2a}$, $R^{3a}$, and $R^{4a}$ are all preferably a hydrogen atom. $R^{5a}$ is preferably a hydrogen atom or $C_{1-6}$ alkyl carbonyl, more preferably a hydrogen atom or acetyl, and still more preferably acetyl. In

formulas (III) and (IIIc), which represent the disaccharide units contained in the hyaluronic acid derivative containing the repeating unit represented by formula (II), $R^{1b}$, $R^{2b}$, $R^{3b}$, and $R^{4b}$, and $R^{1c}$, $R^{2c}$, $R^{3c}$ and $R^{4c}$, are all preferably a hydrogen atom. $R^{5b}$ and $R^{5c}$ are preferably a hydrogen atom or $C_{1-6}$ alkyl carbonyl, more preferably a hydrogen atom or acetyl, and still more preferably both acetyl.

Specific examples of $R^{aa}$ in formula (II) include a hydrogen atom, methyl, hydroxymethy, 1-hydroxyethyl, carbamoylmethyl, carboxymethyl, 1-methylpropyl, 2-methylpropyl, isopropyl, 2-carboxyethyl, 2-methylthioethyl, 2-carbamoylethyl, phenylmethyl, (4-hydroxyphenyl)methyl, and indol-3-ylmethyl.

When the $-CHR^{aa}-$ group is a chiral center, the each optically active form and mixtures thereof are also included in the scope. However, when the $-CHR^{aa}-$ group is described as $H_2N-CHR^{aa}-COOH$ (amino acid), L-forms (natural form) are preferred.

In formula (II), $R^{6a}$, $R^7$, $R^8$, and $R^9$ are, for example, independently a hydrogen atom or methyl, and preferably all a hydrogen atom.

An embodiment of the $-CHR^{aa}-CO-X^{1a}$ group in formula (II) can be, for example, a $-CHR^{aa}-COOH$ group. Specific examples of this group include the following groups.

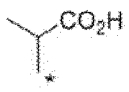
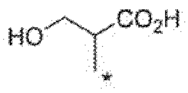
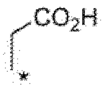
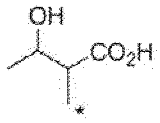
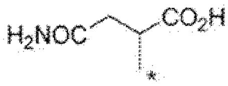

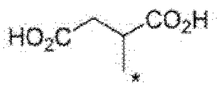
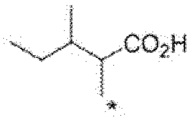
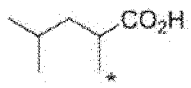
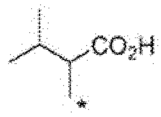

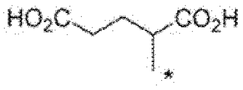
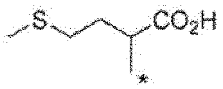
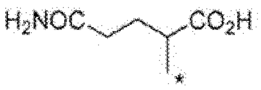

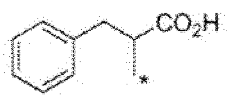
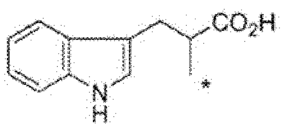

In the formulas above, the asterisk indicates the binding site with $-NR^{6a}-$ (the same applies below) .

Preferable examples of the $-CHR^{aa}-COOH$ group include the following groups.

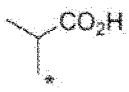
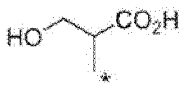
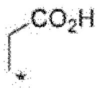
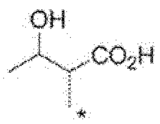
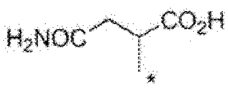

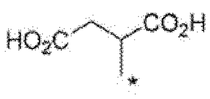
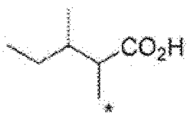
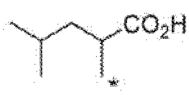
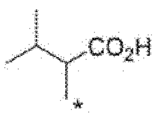

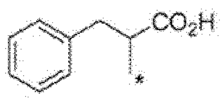
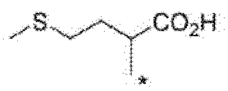
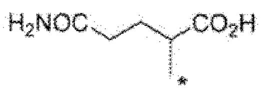

Preferable examples of the $-CHR^{aa}-COOH$ group include the following groups.

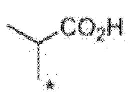
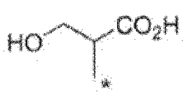
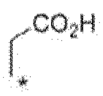
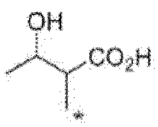
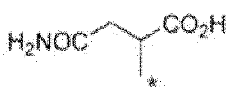

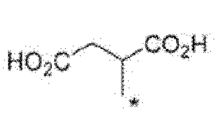 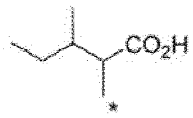 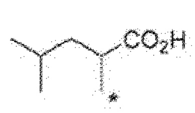 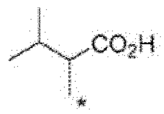

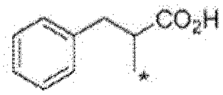

Preferable examples of the -CHR$^{aa}$-COOH group include the following groups.

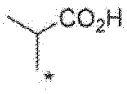 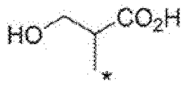 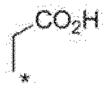 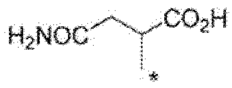 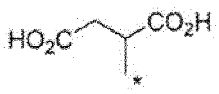

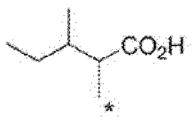 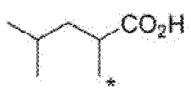 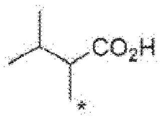 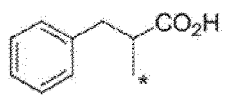

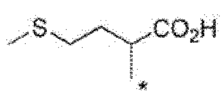 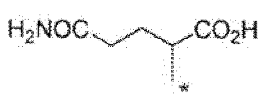

Preferable examples of the -CHR$^{aa}$-COOH group include the following groups.

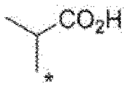 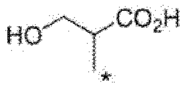 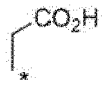 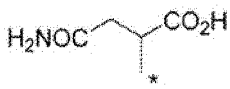 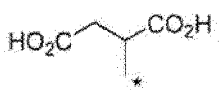

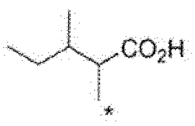 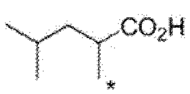 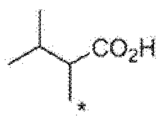 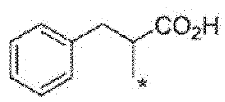

From the viewpoint of delivery of the complex into lymph nodes, preferable examples of the -CHR$^{aa}$-COOH group include the following groups.

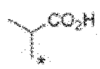 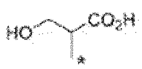 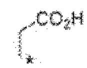 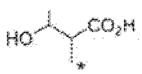 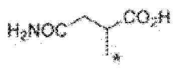 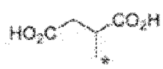

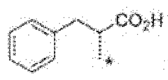 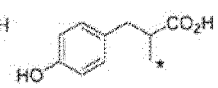 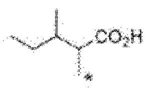 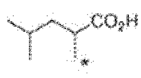 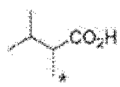

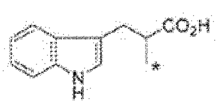 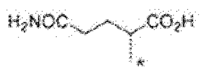 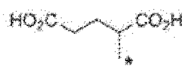

Further preferable examples include the following groups.

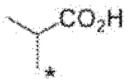 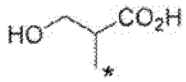 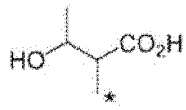 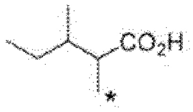 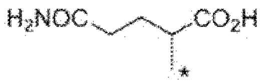

Further preferable examples include the following groups.

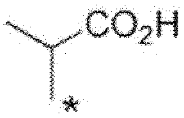 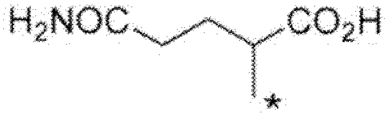

Any of the $-CHR^{aa}-COOH$ groups described above may be entirely or partially converted into a $-CHR^{aa}-CONH-Z^1-Z^2$ group. Examples of the $-Z^1-Z^2$ group are as described later. In another embodiment, examples of the $-CHR^{aa}-CO-X^{1a}$ group in formula (II) include a $-CHR^{aa}-CONH_2$ group. Specific examples of this group include the following groups.

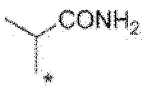 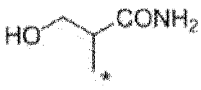 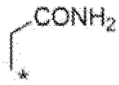 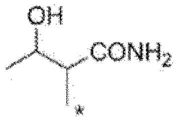 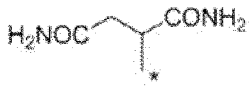

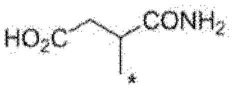 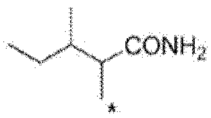 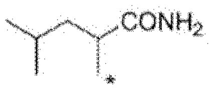 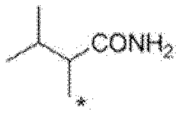

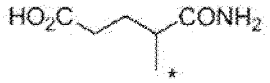 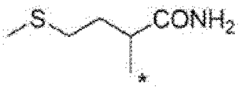 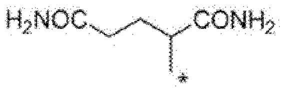

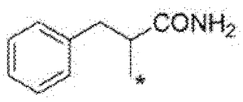 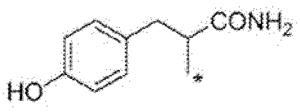 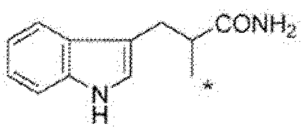

Preferable examples of the $-CHR^{aa}-CONH_2$ group include the following groups.

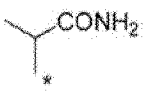 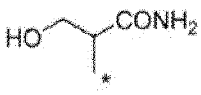 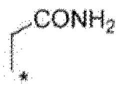 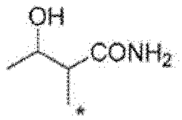 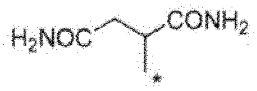

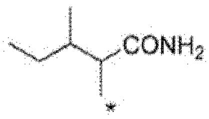 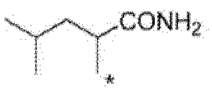 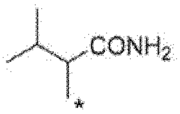

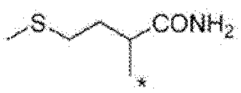 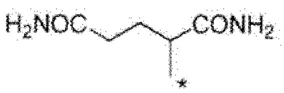

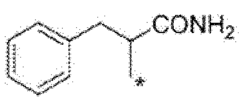 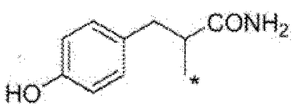 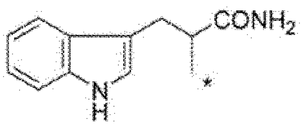

Preferable examples of the $-CHR^{aa}-CONH_2$ group include the following groups.

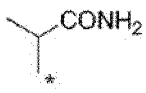 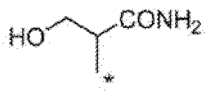 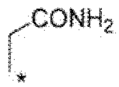 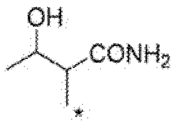 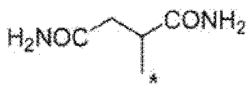

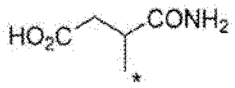 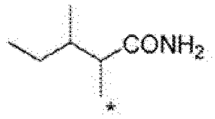 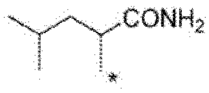 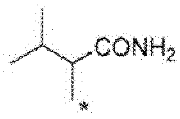

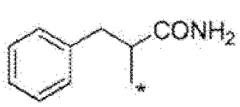 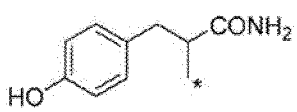

Preferable examples of the $-CHR^{aa}-CONH_2$ group include the following groups.

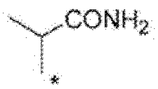 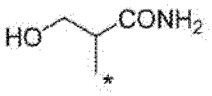 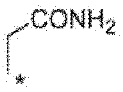 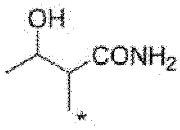 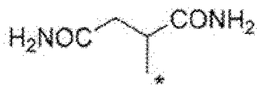

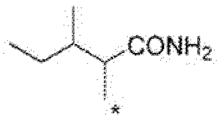 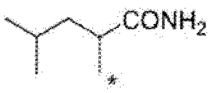 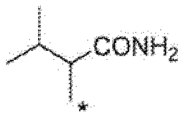

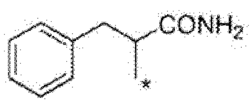 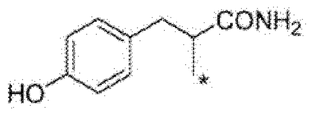

Preferable examples of the $-CHR^{aa}-CONH_2$ group include the following groups.

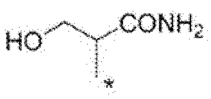 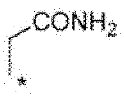 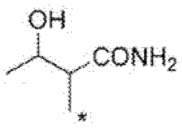 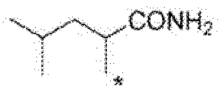 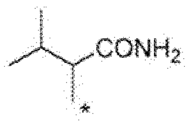

These groups are also preferred from the viewpoint of properties of both biodegradability and blood retention.

From the viewpoint of properties of both biodegradability and blood retention, preferable examples of the $-CHR^{aa}-CONH_2$ group also include the following groups.

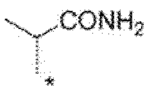 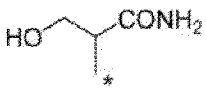 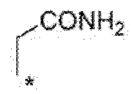 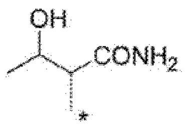 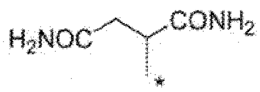

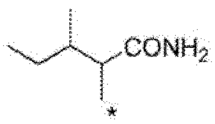 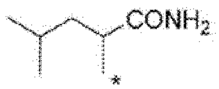 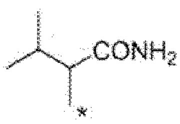

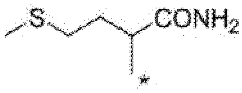
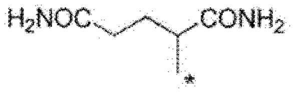

From the viewpoint of properties of both biodegradability and blood retention, further preferable examples of the -$CHR^{aa}$-$CONH_2$ group include the following groups.

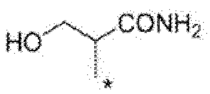
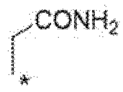
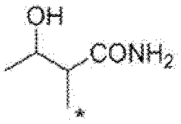
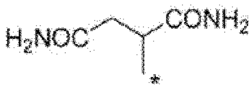

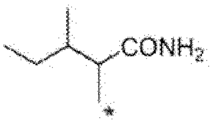
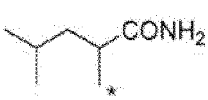
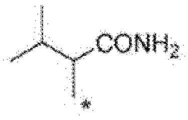

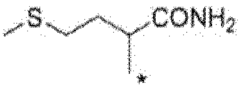

From the viewpoint of more suitable dispersibility in pure water, preferable examples the -$CHR^{aa}$-$CONH_2$ group include the following groups.

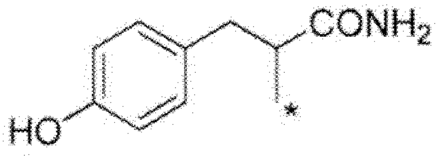
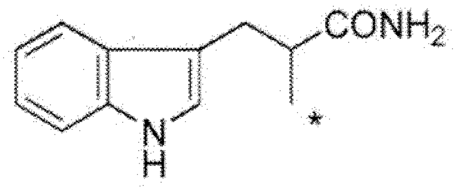

These two groups are also preferable examples as a base for long-acting subcutaneous injection formulations.

From the viewpoint of a base for long-acting subcutaneous injection formulations, preferable examples of the - $CHR^{aa}$-$CONH_2$ group include the following group.

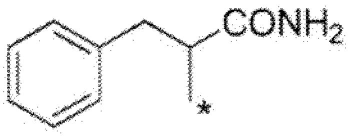

In the formula, $R^7$ is more preferably a hydrogen atom and methyl, and more preferably a hydrogen atom.

The carboxy defined by formulas (II), (III), and (IIIc) may form a salt represented by formula -COO-$Q^+$. $Q^+$ is not particularly limited as long as $Q^+$ is a countercation that forms a salt with carboxy in water. If $Q^+$ is divalent or higher valent, $Q^+$ forms a salt with multiple carboxy groups according to its valence. Examples of countercations include metal ions, such as a lithium ion, a sodium ion, a rubidium ion, a cesium ion, a magnesium ion, and a calcium ion; and ammonium ions represented by formula $N+R^jR^kR^lR^m$ (wherein $R^j$, $R^k$, $R^l$, and $R^m$ are each independently selected from the group consisting of a hydrogen atom and $C_{1-6}$ alkyl), with a sodium ion, a potassium ion, and a tetraalkylammonium ion (e.g., a tetra-n-butyl ammonium ion) being preferred. $R^j$, $R^k$, $R^l$, and $R^m$ are preferably the same group selected from $C_{1-6}$ alkyl, and preferably n-butyl.

In another embodiment, examples of the -$CHR^{aa}$-CO-$X^{1a}$ group in formula (II) include a -$CHR^{aa}$-CONH-$Z^1$-$Z^2$ group. Specific examples of this group include the following groups.

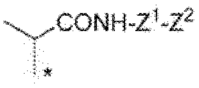 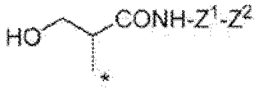 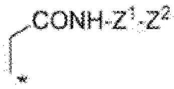 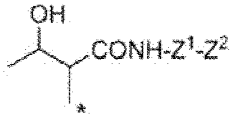

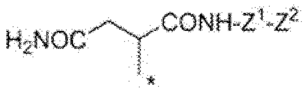 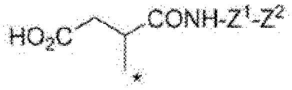 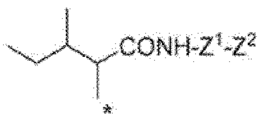

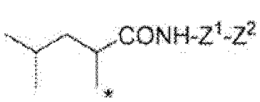 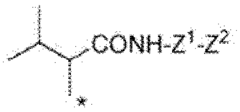 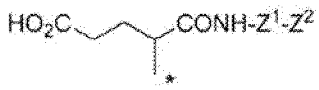

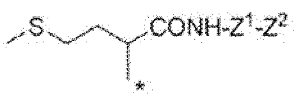 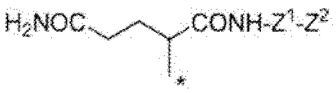

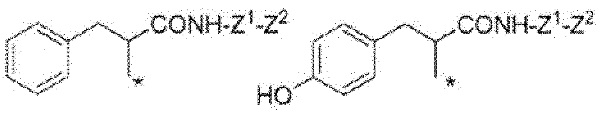 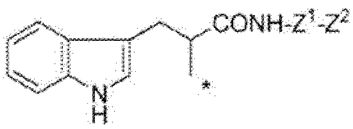

Other specific examples of the group include the following groups.

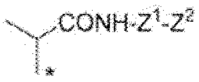 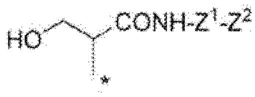 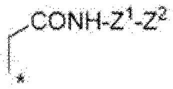 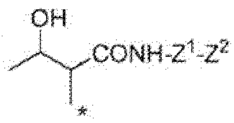

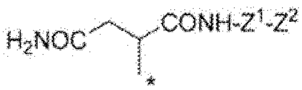 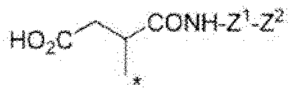 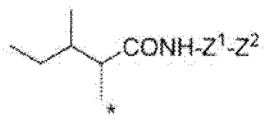

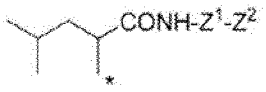 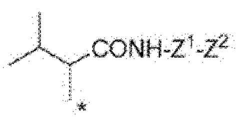 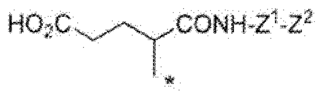

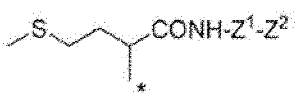 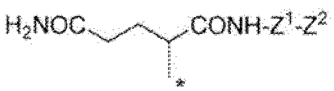

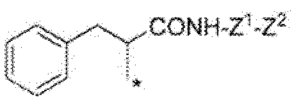 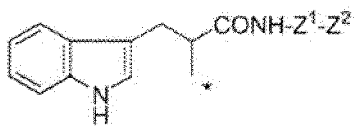

Preferable examples of the -$CHR^{aa}$-CONH-$Z^1$-$Z^2$ group include the following groups.

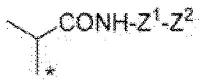 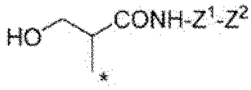 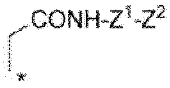 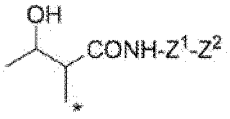

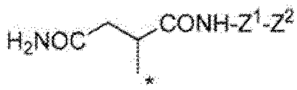 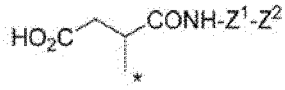 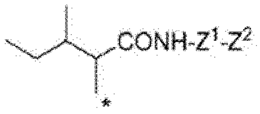

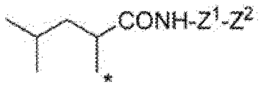 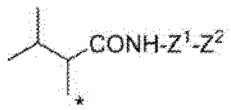

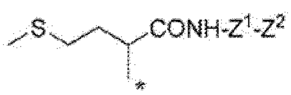 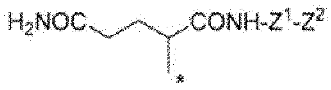

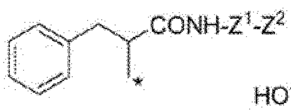 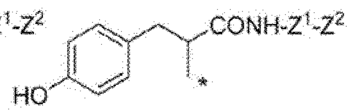 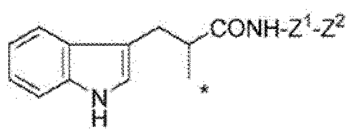

Preferable examples of the -$CHR^{aa}$-CONH-$Z^1$-$Z^2$ group include the following groups.

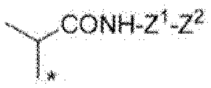 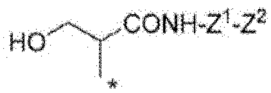 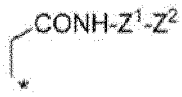 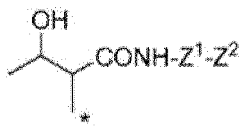

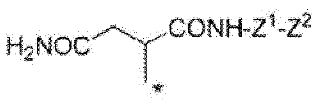 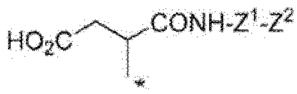 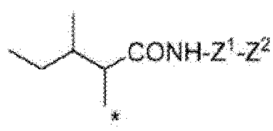

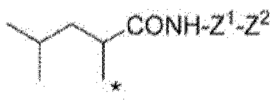 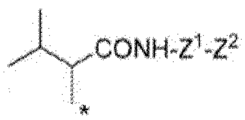 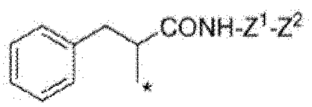

Preferable examples of the -$CHR^{aa}$-CONH-$Z^1$-$Z^2$ group include the following groups.

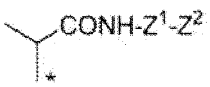 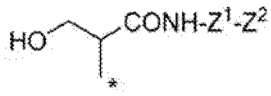 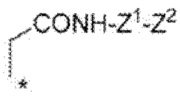

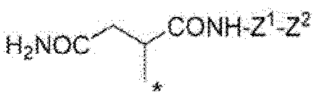 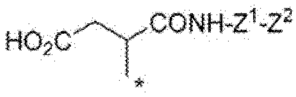 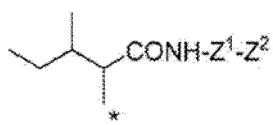

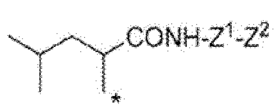 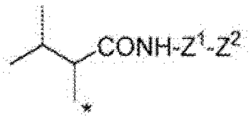 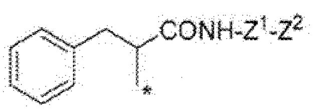

From the viewpoint of delivery of the complex into lymph nodes, preferable examples of the $-CHR^{aa}-CONH-Z^1-Z^2$ group include the following groups.

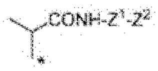 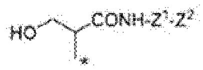 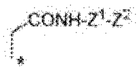 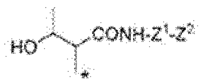

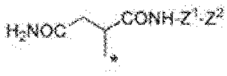 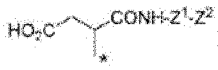 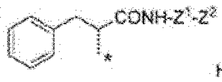 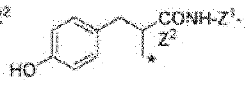

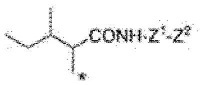 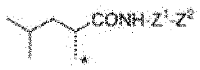 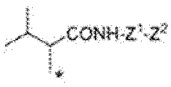 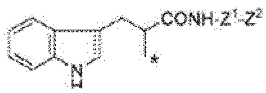

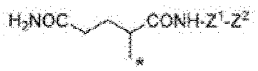 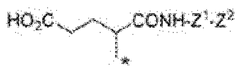

Further preferable groups include the following groups.

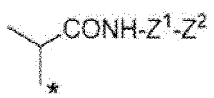 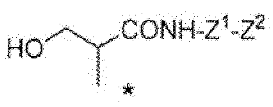 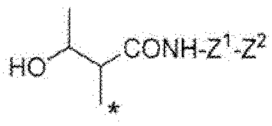

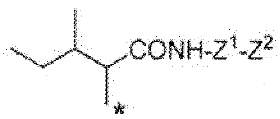 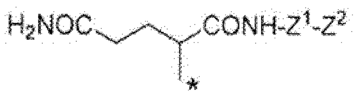

Further preferable examples include the following groups.

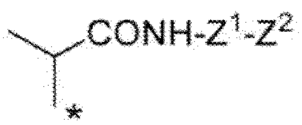 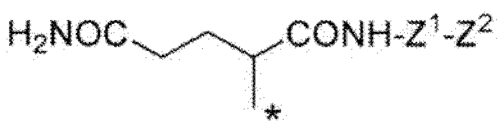

From the viewpoint of properties of both biodegradability and blood retention, preferable examples of the $-CHR^{aa}-CONH-Z^1-Z^2$ group include the following groups.

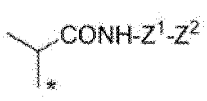 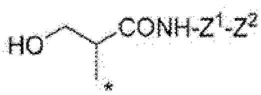 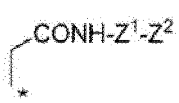 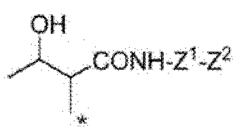

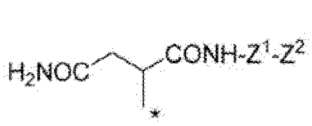 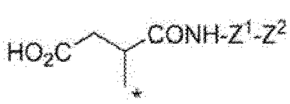 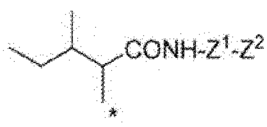

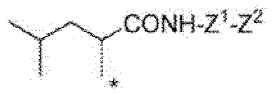
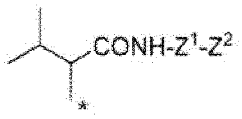

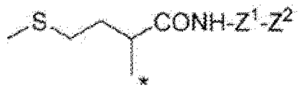
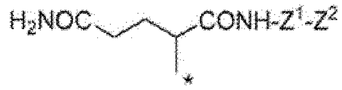

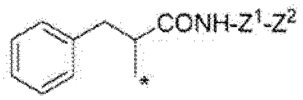

Examples of the $-Z^1-Z^2$ group include a $-(C_{2-10}$ alkylene) - $NH-COO-Z^3$ group and also a $-(C_{2-12}$ alkylene)-$NH-COO-Z^3$ group. For example, $C_{2-12}$ alkylene is preferably $-(CH_2)_2-$, $-(CH_2)6-$, $-(CH_2)8-$, $-(CH_2)10-$, and $-(CH_2)12-$, and more preferably $-(CH_2)_2-$and $-(CH_2)6-$. Examples of the $-Z^1-Z^2$ group further include a $-(CH_2CH_2O)_{ma}-CH_2CH_2-NH-Z^3$ group, wherein ma is preferably 1 to 20, more preferably 1 to 10, and still more preferably 1 to 3. A specific example of ma is preferably 2. Examples of the $-Z^1-Z^2$ group are preferably a -(hexane-1,6-diyl)-$NH-COO-Z^3$ group, a -(ethane-1,2-diyl)-$NH-COO-Z^3$ group, and a $-(CH_2CH_2O)_2-CH_2CH_2-NH-Z^3$ group, more preferably a -(hexane-1,6-diyl)-NH-COO-cholesteryl group, a -(ethane-1,2-diyl)-NH-COO-cholesteryl group, and a $-(CH_2CH_2O)\ _2-CH_2CH_2$-NH-cholanoyl group, and still more preferably a -(hexane-1,6-diyl)-NH-COO-cholesteryl group. Examples of $Z^1$, $Z^2$, and the $-Z^1-Z^2$ group can be respectively those corresponding to Y, $X^1$, and the $-Y-X^1$ group of the hyaluronic acid derivative containing the repeating unit represented by formula (I). Examples of the $-CO-NR^{ca}-Z^3$ group and the $-O-CO-NR^{ca}-Z^3$ group are those individually in which $R^{ca}$ is a hydrogen atom.

In an embodiment of the hyaluronic acid derivative containing the repeating unit represented by formula (II), in the $X^{1a}$ group, $X^{1a}$ is $-NR^9-Z^1-Z^2$, $R^9$ is a hydrogen atom, $Z^1$ is, for example, $C_{2-12}$ alkylene, preferably $C_{2-6}$ alkylene, and more preferably $C_6$ alkylene, $Z^2$ is $-NR^{ba}-COO-Z^3$, $R^{ba}$ is a hydrogen atom, and $Z^3$ is a steryl group; and the $R^{aa}$ group is a hydrogen atom or $C_{1-6}$ alkyl wherein the alkyl may be independently substituted with one or more groups selected from the group consisting of hydroxy, carboxy, carbamoyl, $C_{1-6}$ alkylthio, aryl, and heteroaryl wherein the aryl may be substituted with one or more hydroxy groups.

From the viewpoint of delivery of the complex into lymph nodes, specific preferable examples of the $R^{aa}$ group include methyl, hydroxymethy, a hydrogen atom, 1-hydroxyethyl, carbamoylmethyl, carboxymethyl, benzyl, (4-hydroxyphenyl)methyl, 1-methylpropyl, 2-methylpropyl, isopropyl, indol-3-ylmethyl, 2-carbamoylethyl, and 2-carboxyethyl, with methyl, hydroxymethy, 1-hydroxyethyl, 1-methylpropyl, and 2-carbamoylethyl being preferred, and methyl and 2-carbamoylethyl being more preferred.

In an embodiment, the hyaluronic acid derivative containing the repeating unit represented by formula (III) and the repeating unit represented by formula (II) is also preferable. In a more preferable embodiment, $X^{1a}$ in formula (II) and $X^2$ in formula (III) are identical. In an aspect, the hyaluronic acid derivative containing the repeating unit represented by formula (II) may contain the repeating unit represented by formula (II) wherein $X^{1a}$ is $-NR^9-Z^1-Z^2$, the repeating unit represented by formula (III), and the repeating unit represented by formula (IIIc).

In still another aspect, the hyaluronic acid derivative containing the repeating unit represented by formula (II) may have a ratio of the repeating units represented by formula (II) and/or formula (III) having the $-NR^9-Z^1-Z^2$ group ("hydrophobic group" below) to the disaccharide repeating units present (the introduction rate of the hydrophobic group) of 3 to 50%.

The introduction rate of the hydrophobic group is calculated according to the following formula:

$$\text{(The introduction rate of the hydrophobic group)}$$
$$= \frac{\text{(The number of disaccharide repeating units having the hydrophobic group introduced)}}{\text{(The number of disaccharide repeating units present)}} \times 100$$

"The disaccharide repeating units present in the derivative" include the repeating units represented by formula (II) and formula (III), and the repeating unit represented by formula (IIIc). The introduction rate can be controlled by reaction conditions, such as the proportion of reagents, and can be determined, for example, by NMR measurement.

The hyaluronic acid derivative containing the repeating unit represented by formula (II) has an introduction rate of the hydrophobic group of, for example, 3 to 50%, preferably 5 to 40%, more preferably 5 to 35%, still more preferably 5 to 25%, even more preferably 5 to 20%, and yet more preferably 5 to 10%. From the viewpoint of forming a complex with an antigen, the introduction rate of the hydrophobic group is preferably 3 to 60%, more preferably 7 to 50%, still more preferably 18 to 45%, and even more preferably 20 to 35%. From the viewpoint of transfer of the complex into lymph nodes, the introduction rate of the hydrophobic group is preferably 3 to 60%, more preferably 7 to 50%, still more preferably 18 to 45%, and even more preferably 20 to 35%.

In an embodiment of the hyaluronic acid derivative containing the repeating unit represented by formula (II), $X^{1a}$ is $-NR^9-Z^1-Z^2$ in formula (II) . In this case, the ratio of the disaccharide unit represented by formula (II) to the disaccharide repeating units present in the hyaluronic acid derivative is, for example, 70% or more, preferably 75% or more, and more preferably 90% or more from the viewpoint of properties of both biodegradability and blood retention. The upper limit may be 100% or less. The range of the ratio is, for example, 70 to 100%, preferably 75 to 100%, and more preferably 90 to 100%. From the viewpoint of properties of forming a complex with an antigen, the range of the ratio is, for example, 10% or more, preferably 20% or more, more preferably 50% or more, still more preferably 70% or more, and even more preferably 90% or more. The upper limit may be 100% or less. The range of the ratio is, for example, 10 to 100%, preferably 20 to 100%, more preferably 50 to 100%, still more preferably 70 to 100%, and even more preferably 90 to 100%. Further, from the viewpoint of properties of the complex transferring to lymph nodes, the range of the ratio is, for example, 10% or more, preferably 20% or more, more preferably 50% or more, still more preferably 70% or more, and even more preferably 90% or more. The upper limit may be 100% or less. The range of the ratio is, for example, 10 to 100%, preferably 20 to 100%, more preferably 50 to 100%, still more preferably 70 to 100%, and even more preferably 90 to 100%. The hyaluronic acid derivative containing the repeating unit represented by formula (II) may further contain the repeating unit represented by formula (III).

In an embodiment, the hyaluronic acid derivative containing the repeating unit represented by formula (II) does not contain the repeating unit represented by formula (II) wherein $X^1$ is $-NR^9-Z^1-Z^2$. In this case, the sum of the ratio of the repeating unit represented by formula (II) and the ratio of the repeating unit represented by formula (III) to the disaccharide repeating units present is, for example, 70 to 100%, preferably 80 to 100%, and more preferably 90 to 100%. From the viewpoint of forming a complex with an antigen, the sum is, for example, 7 to 100%, preferably 20 to 100%, and more preferably 30 to 100%. From the viewpoint of transfer of the complex into lymph nodes, the sum is, for example, 20 to 100%, preferably 30 to 100%, and more preferably 70 to 100%.

In the hyaluronic acid derivative containing the repeating unit represented by formula (II), the ratio of the repeating unit represented by formula (III) to the disaccharide repeating units present is preferably 3 to 50%, more preferably 5 to 40%, still more preferably 5 to 35%, even more preferably 5 to 25%, yet more preferably 5 to 20%, and further more preferably 5 to 10%. From the viewpoint of forming a complex with an antigen, the ratio is preferably 3 to 60%, more preferably 7 to 50%, still more preferably 18 to 45%, and even more preferably 20 to 35%. From the viewpoint of transfer of the complex into lymph nodes, the ratio is preferably 3 to 60%, more preferably 7 to 50%, still more preferably 18 to 45%, and even more preferably 20 to 35%.

In the hyaluronic acid derivative containing the repeating unit represented by formula (II), the ratio of the repeating unit represented by formula (II) to the disaccharide repeating units present is preferably 20 to 97%, more preferably 30 to 95%, still more preferably 35 to 95%, even more preferably 45 to 95%, yet more preferably 50 to 95%, and further more preferably 60 to 95%. From the viewpoint of forming a complex with an antigen, the ratio is, for example, 7 to 100%, preferably 20 to 100%, and more preferably 30 to 100%. From the viewpoint of transfer of the complex into lymph nodes, the ratio is, for example, 20 to 100%, preferably 30 to 100%, and more preferably 70 to 100%.

The hyaluronic acid derivative having a hydrophobic group introduced can be produced according to, for example, the method disclosed in WO2010/053140A or WO2014/038641A.

In an aspect, the hyaluronic acid derivative having a hydrophobic group introduced forms fine particles because of aggregation in water. Without wishing to be bound by theory, it is believed that the hydrophobic interaction of introduced hydrophobic groups causes self-aggregation in water to thereby form fine particles. Because of this property, the hyaluronic acid derivative having a hydrophobic group can be used as a vaccine carrier, a lymph node delivery carrier, an in-blood sustained-release carrier, or a targeting carrier. The particle size of the fine particles is not particularly limited, and is, for example, 1 $\mu$m or less, preferably 500 nm, more preferably 200 nm or less, still more preferably 100 nm or less, and even more preferably 50 nm or less. The hyaluronic acid derivative having a hydrophobic group introduced can be formed into fine particles according to, for example, the method disclosed in WO2010/053140A or the method disclosed in WO2014/038641A.

The starting material for producing the hyaluronic acid derivative having a hydrophobic group introduced can be hyaluronic acid, a salt thereof, or a derivative thereof. Examples of hyaluronates include alkali metal salts, such as sodium salts, potassium salts, and lithium salts. Particularly preferable salts are sodium salts, which are widely used in pharmaceuticals. Hyaluronic acid or a pharmaceutically acceptable salt thereof can be produced according to various known methods, such as extraction from biological sources (e.g., chicken combs or porcine skin) or biofermentation, or

produced from commercially available products (e.g., products from Denka Company Limited, Shiseido Japan Co., Ltd., Seikagaku Corporation, or R&D Systems).

An antigen forming a complex with a fine particle of the hyaluronic acid derivative having a hydrophobic group may be released from the complex after administration because of the degradation or disintegration of fine particles of the hyaluronic acid derivative, or replacement of a biological component, such as albumin, with the antigen. The release rate can be controlled by the introduction rate or molecular weight of the hydrophobic group of the hyaluronic acid derivative, or the introduction rate of amino acids. Additionally, the release rate of the antigen can be controlled by chemically crosslinking the hyaluronic acid derivative to form a gel according to a method disclosed in WO2010/053140A. Additionally, the release rate of the antigen can also be controlled by conjugating the hyaluronic acid derivative with the antigen according to a method disclosed in WO2010/053140A.

2-2. Antigen

Examples of antigens to be combined with the hyaluronic acid derivative having a hydrophobic group introduced include antigen peptides, antigenic proteins, and DNA or mRNA encoding these antigen sequences, with antigen peptides and antigenic proteins being preferred, and antigen peptides being more preferred.

The antigen may be a cancer antigen. Cancer antigens are those often expressed in cancer cells. Some cancer antigens are expressed only in cancer cells. A cancer antigen can be expressed within a cancer cell or on the surface of a cancer cell.

Although the antigenic protein to be used in the present invention is not limited, the antigenic protein can be selected from the following: ERK1, ERK2, MART-1/Melan-A, gp100, binding protein for adenosine deaminase (ADAbp), FAP, cyclophilin b, colorectum-associated antigen (CRC)-C017-1A/GA733, carcinoembryonic antigen (CEA), CAP-1, CAP-2, etv6, AML1, prostate-specific antigen (PSA), PSA-1, PSA-2, PSA-3, prostate-specific membrane antigen (PSMA), T-cell receptor/CD3-zeta-chain, CD20, MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5, GAGE-1, GAGE-2, GAGE-3, GAGE-4, GAGE-5, GAGE-6, GAGE-7, GAGE-8, GAGE-9, RAGE, RAGE, LAGE-1, NAG, GnT-V, MUM-1, CDK4, tyrosinase, p53, MUC family, HER2/neu, p21ras, RCAS1, α-fetoprotein, E-cadherin, α-catenin, β-catenin, γ-catenin, p120ctn, gp100Pmel117, PRAME, NY-ESO-1, cdc27, adenomatous polyposis protein (APC), fodrin, connexin 37, Ig idiotype, p15, gp75, GM2 ganglioside, GD2 ganglioside, human papillomavirus protein, Smad family of tumor antigens, lmp-1, P1A, EBV-encoding nuclear antigen (EBNA)-1, brain glycogen phosphorylase, SSX-1, SSX-2 (HOM-MEL-40), SSX-1, SSX-4, SSX-5, SCP-1, CT-7, CD20, and c-erbB-2.

The antigenic proteins above may be used in their entire sequences or in sequences with some deletions.

The antigen peptide contains one or more CD8-positive cytotoxic T-cell recognition epitopes and/or CD4-positive helper T-cell recognition epitopes within antigenic protein sequences. In an embodiment, from the viewpoint of being loaded onto the MHC class I or MHC class II molecule after degradation in an antigen-presenting cell, the antigen peptide preferably contains two or more (e.g., 2, 3, 4, 5, 6, 7, or 8) epitopes. Specifically, the antigen peptide is, for example, an antigen peptide containing an epitope of an antigenic protein of a tumor cell.

In an embodiment, the antigen peptide has, for example, 8 to 120 amino acids, preferably 8 to 80 amino acids, more preferably 15 to 80 amino acids, more preferably 16 to 80 amino acids, more preferably 23 to 80 amino acids, more preferably 23 to 60 amino acids, and more preferably 23 to 50 amino acids.

In an embodiment, from the viewpoint of inducing activation of cytotoxic T cells (CTL) by helper T cells, the antigen peptide contains one or more CD8-positive cytotoxic T-cell recognition epitopes and one or more CD4-positive helper T-cell recognition epitopes.

In an aspect, if the antigen peptide contains two or more epitopes, an amino acid linker may be placed between epitopes. The linker has, for example, 2 to 10 amino acids, preferably 4 to 10 amino acids, and more preferably 4 to 8 amino acids. Examples of amino acids for use in the linker include glycine (G), tyrosine (Y), leucine (L), and tryptophan (W), with tyrosine (Y), leucine (L), and tryptophan (W) being preferred. Specific examples of amino acid linkers include -YYYY-(4Y), -LLLL-(4L), -WWWW-(4W), -GGGGGG-(6G), -YYYYYY-(6Y), -LLLLLL-(6L), -WWWWWW-(6W), -YYYYYYYY-(8Y), -LLLLLLLL-(8L), and -WWWWWWWW-(8W), with 6Y, 6L, and 6W being preferred.

2-3. Complex

A complex of a hyaluronic acid derivative having a hydrophobic group introduced and an antigen can be produced by mixing the hyaluronic acid derivative having a hydrophobic group introduced with the antigen in an appropriate solution. Examples of solutions for use include water, physiological saline, various buffers, sugar solutions, DMSO, ethanol, DMF, and combinations of these. Solvent or buffer replacement may be performed according to a method such as dialysis.

For example, the complex of the present invention can be produced by mixing fine particles of the hyaluronic acid derivative having a hydrophobic group introduced with an antigen. Although there is no particular limitation, a complex formed of a fine particle of the hyaluronic acid derivative and the antigen is considered to be a complex of a hyaluronic acid derivative and an antigen. A complex of a hyaluronic acid derivative and an antigen can also be formed by encapsulating an antigen in a fine particle of the hyaluronic acid derivative. The complex encompasses an inclusion of an antigen in a fine particle of the hyaluronic acid derivative. In the inclusion, the antigen is coated with the hyaluronic acid derivative.

The complex can be produced, for example, by a method of adding an antigen solution to pre-formed fine particles of the hyaluronic acid derivative having a hydrophobic group introduced. In this method, the formed fine particles of the hyaluronic acid derivative and the antigen form a complex through hydrophobic interaction, electrostatic interaction, or interaction, such as a hydrogen bond. Interaction can occur on the surface of fine particles and/or inside the fine particles. Although there is no particular limitation, conditions such as solvents, salt concentration, pH, temperature, time, and addition of a denaturant may be determined as appropriate to ensure that the antigen stably forms a complex at high yields. For example, because the salt concentration and pH at the time of complex formation will change the degree of swelling and density of the particles of the hyaluronic acid derivative, as well as the ionization state of the antigen, suitable conditions may be applied according to the combination of these factors. Forming complexes at low salt concentrations causes the electrostatic repulsion between carboxy groups of the hyaluronic acid derivative to decrease the density of particles, increase the amount of complexes, and enable formation of complexes with a higher molecular-weight antigen. After complexes are formed, the salt concentration can be increased to weaken electrostatic repulsion, increase the particulate density, and make the gel mesh size smaller than the size of the antigen, thereby allowing complexes to firmly retain the antigen and delaying its release. In this case, the salt concentration may be the concentration of physiological saline. For micronization and size uniformity, ultrasonic irradiation may be performed with an ultrasound homogenizer or a single-point-focused ultrasound irradiation device. Ultrasonic irradiation may be performed after the hyaluronic acid derivative and a drug are mixed, or may be performed only on the hyaluronic acid derivative. Free antigens that are not in the form of a complex can be separated and removed by dialysis or size-exclusion chromatography (SEC).

As a method for forming a complex, the hyaluronic acid derivative having a hydrophobic group introduced can be dissolved in an aprotic polar organic solvent, such as DMSO or DMF, mixed with an antigen, and then allowed to undergo substitution with water, an aqueous salt solution, or an aqueous buffer solution to simultaneously form fine particles and a complex. Substitution can be performed, for example, by dialysis. For micronization and size uniformity, ultrasonic irradiation may be performed with an ultrasound homogenizer or a single-point-focused ultrasound irradiation device. Ultrasonic irradiation may be performed after the hyaluronic acid derivative and an antigen are mixed, or may be performed only on the hyaluronic acid derivative. Ultrasonic irradiation may also be performed before dialysis or after dialysis. This method makes it less likely for the antigen complexed with the hyaluronic acid derivative to be released from the complex. Although there is no particular limitation, conditions such as solvent type, salt concentration, pH, temperature, time, addition of a denaturant, the concentration of the hyaluronic acid derivative, the concentration of the antigen, and the ratio of the hyaluronic acid derivative to the antigen during mixing of the hyaluronic acid derivative and the antigen may be determined as appropriate to ensure that the antigen stably forms a complex at high yields. Although there is no particular limitation, conditions such as the solvent type for substitution to water, an aqueous salt solution, or various aqueous buffer solutions, salt concentration, pH, temperature, time, frequency, whether to add a denaturant, concentration of the hyaluronic acid derivative, concentration of the antigen, and the ratio of the hyaluronic acid derivative to the antigen are determined as appropriate to ensure that the antigen stably forms a complex at high yields. The desired size of fine particles can also be achieved by selecting these conditions as appropriate. Free antigens that are not in the form of a complex can be separated and removed by dialysis or size-exclusion chromatography (SEC).

If the complex is a particle, the particle size is not particularly limited. From the viewpoint of transfer into lymph nodes, the particle size is, for example, 20 to 200 nm, preferably 20 to 100 nm, and more preferably 20 to 50 nm.

## 3. Antigen-Specific Lymphocyte

### 3-1. Immune Receptor

The immune receptor is not particularly limited as long as it is a receptor capable of transmitting signals necessary for exhibiting the effector function of immune cells, i.e., a receptor capable of activating immune cells when an antigen-binding domain binds to an antigen. Preferable examples of the immune receptor include T-cell receptors and chimeric antigen receptors (CAR). The antigen recognized by a chimeric antigen receptor can be a cell surface antigen or a complex of an intracellular antigen and a major histocompatibility gene complex.

The immune receptor typically comprises an antigen-binding domain, a transmembrane domain, and an intracellular domain.

The antigen-binding domain is a domain that is located extracellularly when an immune receptor is located on a cell membrane. The antigen-binding domain is not particularly limited as long as it is a domain capable of recognizing an antigen and binding to the antigen. The antigen-binding domain usually has one or more, preferably two, three, four, five, or more, more preferably all six, of the CDRs of the antibody or T-cell receptor against an antigen (for example, in the case of antibody CDRs, heavy-chain CDR1, heavy-chain CDR2, heavy-chain CDR3, light-chain CDR1, light-chain CDR2, and light-chain CDR3). The antigen-binding region more preferably includes variable regions of the antibody or T-cell receptor against an antigen (for example, in the case of antibodies, heavy-chain variable regions and/or light-chain variable regions).

The antigen-binding domain can use a single-chain antibody structure, such as an scFv structure. When a heavy-chain variable region and a light-chain variable region are included as in the scFv structure, the heavy-chain variable region and the light-chain variable region are usually linked via a linker. The linker is not particularly limited, and any linker can be used as long as antigen binding ability is not significantly impaired. The linker is preferably a linker composed of glycine or composed of glycine and serine (e.g., GGS linkers, GS linkers, and GGG linkers). The length of the linker is not particularly limited. The number of amino acid residues in the linker is, for example, 5 to 30, and preferably 10 to 25. The arrangement relationship between the heavy-chain variable region and the light-chain variable region is not particularly limited. Any of the following embodiments can be used: an embodiment in which the light-chain variable region is on the N-terminal side and an embodiment in which the heavy-chain variable region is on the N-terminal side.

The transmembrane domain is a domain that is located in the plasma membrane when an immune receptor is placed on the plasma membrane. The transmembrane domain is not particularly limited as long as it can constitute an immune receptor. The transmembrane domain can be a transmembrane region, such as a transmembrane region, such as TCR $\alpha$ chain/$\beta$ chain, CD28, CD3$\varepsilon$, CD8$\alpha$, CD3, CD4, and 4-1BB. The transmembrane region of each factor is known or can be easily determined from known sequence information (for example, by using a transmembrane region prediction program). It is also possible to use the transmembrane domain comprising (or consisting of) an artificially constructed polypeptide. Such transmembrane domains can be mutated as appropriate as long as the mutation does not significantly interfere with the function of the chimeric antigen receptor.

The antigen-binding domain and the transmembrane domain are linked directly or via a spacer. When a spacer is used, the sequence is not particularly limited. For example, a sequence such as the hinge or part of the hinge of IgG, preferably human IgG (e.g., subtype IgG1 or IgG4), the hinge or part of CH2, or part of a factor (e.g., CD28) used in a transmembrane domain can be used as a spacer. When a sequence of the hinge or part of the hinge is used, a flexible spacer can be expected to be constructed.

The intracellular signal domain is a domain that is located intracellularly when an immune receptor is located on the plasma membrane and that is capable of transmitting signals necessary to exhibit the effector function of immune cells, i.e., capable of transmitting signals necessary for activating immune cells when an antigen-binding domain binds to an antigen.

The intracellular signal domain preferably comprises an intracellular activation domain. Examples of usable intracellular activation domains include intracellular domains, such as CD3$\zeta$ and Fc$\varepsilon$RI$\gamma$, and preferably CD3$\zeta$. CD3 may be mutated as appropriate as long as the mutation does not interfere with the function of the immune receptor. When CD3 is mutated, the mutation is preferably performed in such a manner that an immunoreceptor tyrosine-based activation motif (ITAM) is contained.

The immune receptor may comprise a region other than those described above. Examples of such other regions include co-stimulatory factor intracellular domains, leader sequences (signal peptides) for promoting the transport of the immune receptor onto the cell membrane (for example, a leader sequence of a GM-CSF receptor), and a spacer/linker (for example, between the transmembrane region and the intracellular signal domain or between each domain within the intracellular signal domain). The co-stimulatory factor of the intracellular domain is not particularly limited as long as it is an intracellular domain derived from co-stimulatory factors possessed by T cells or other cells. For example, at least one member selected from the group consisting of OX40, 4-1BB, GITR, CD27, CD278, and CD28 can be used.

The immune receptor may be a molecule formed of one type of polypeptide or a molecule formed of a complex of two or more types of polypeptides. The immune receptor may be a molecule formed of a polypeptide or of a complex of polypeptide, or a molecule formed of a polypeptide or complex of polypeptide to which another substance (e.g., a fluorescent substance, a radioactive substance, or an inorganic particle) is linked.

3-2. Lymphocytes

The antigen-specific lymphocyte is not particularly limited as long as it expresses an immune receptor against an antigen (i.e., capable of binding (preferably specifically binding) to an antigen) in the HA-antigen composition. In a more specific embodiment, the antigen receptor is expressed on the cell membrane, and preferably expressed in such a state that the antigen-binding domain is exposed outside the cell membrane.

Examples of lymphocytes include T cells (e.g., CD8-positive cells, CD4-positive cells, CD4-positive, CD8-negative T cells, CD4-negative CD8-positive T cells, T cells prepared from iPS cells, αβ-T cells, and γδ-T cells), NK cells, and NKT cells.

The antigen-specific lymphocyte can be obtained by introducing a polynucleotide comprising the coding sequence of an immune receptor into the cells.

It is preferable to activate cells for immunoreceptor expression before the introduction of the polynucleotide described above. For example, the cells for immunoreceptor expression can be activated by stimulation with anti-CD3 antibody and anti-CD28 antibody. For example, stimulation with anti-CD3 antibody and anti-CD28 antibody can be applied by culturing the cells in a culture vessel (e.g., culture dish) having a culture surface coated with anti-CD3 antibody and anti-CD28 antibody. The stimulation can also be applied by using magnetic beads coated with anti-CD3 antibody and anti-CD28 antibody (e.g., Dynabeads T-Activator CD3/CD28 provided by Veritas Corporation).

In order to increase the survival rate and/or proliferation rate of the cells, it is preferred to use a culture medium containing a T-cell growth factor in activation treatment. As the T-cell growth factor, IL-2, IL-15, IL-7, and the like can be used. T-cell growth factors, such as IL-2, IL-15, and IL-7, can be prepared according to a usual method. Commercially available products can also be used. Although the use of T-cell growth factors from animal species other than humans is not excluded, T-cell growth factors derived from humans (which may be recombinants) are usually used.

Typically, for their application (administration to a patient), cells after introduction of the polynucleotide described above (the polynucleotide-transduced lymphocytes) are grown. For example, the polynucleotide-transduced lymphocytes are cultured (subcultured as necessary) using a culture medium to which a T-cell growth factor has been added. In addition to this culture, the same treatment (reactivation) as in the case of the activation of cells for immunoreceptor expression may be performed.

Although a medium to which serum (human serum, fetal bovine serum, or the like) is added may be used for culturing the cells for immunoreceptor expression and polynucleotide-transduced lymphocytes described above, using a serum-free medium or medium with a low serum concentration enables the preparation of cells with the advantages of having a high level of safety in clinical application and being less likely to have a difference in culture efficiency due to a difference between serum lots. When serum is used, it is preferable to use autologous serum, i.e., serum collected from a patient to whom immune receptor gene-introduced lymphocytes are administered. Feeder cells, such as peripheral blood mononuclear cells (for example, peripheral blood mononuclear cell cells from several pooled healthy individuals) inactivated by irradiation (e.g., 25-35 Gy), can be used for culture.

More specifically, the method for obtaining antigen-specific lymphocytes is as follows.

The nucleic acid encoding the immune receptor can be linked to another nucleic acid in such a manner that the nucleic acid is expressed under the control of an appropriate promoter. Examples of usable promoters include promoters that promote expression constitutively and promoters induced by a drug or the like (e.g., tetracycline or doxorubicin). For example, it is possible to use mammalian promoters, such as phosphoglycerate kinase (PGK) promoter, Xist promoter, β-actin promoter, and RNA polymerase II promoter; virus-derived promoters, such as SV40 early promoter, cytomegalovirus promoter, herpes simplex virus thymidine kinase promoter, and LTR promoters of various retroviruses. To achieve efficient transcription of the nucleic acid, a nucleic acid comprising another regulatory element that cooperates with a promoter or a transcription initiation site (e.g., an enhancer sequence or a terminator sequence) may be linked. Further, a gene that can be used as a marker for confirmation of the expression of the nucleic acid (e.g., a drug resistance gene, a gene encoding a reporter enzyme, or a gene encoding a fluorescent protein) may be incorporated.

A nucleic acid encoding the immune receptor can be introduced into cells by using a vector.

The vector is operatively linked to an appropriate control sequence to express the nucleic acid in a suitable host. Such a control sequence comprieses a promoter for transcription of nucleic acids, an operator sequence for controlling transcription, a sequence encoding a ribosome binding site, an enhancer, a polyadenylation sequence, and a sequence for controlling termination of transcription and translation. Various known sequences (e.g., restriction enzyme cleavage site, marker genes, such as a drug resistance gene (selection gene), signal sequence, and leader sequence) can be used in the vector. Various sequences can be appropriately selected and used according to the type of polypeptide to be expressed, host cell, medium, and other conditions.

Examples of usable vectors include vectors incorporated into the host genome, vectors not incorporated into the host genome, and episomal vectors present in the cytoplasm to replicate autonomously. Examples of such vectors include retrovirus vectors (including oncoretrovirus vectors, lentivirus vectors, and pseudotyped vectors), adenoviral vectors, adeno-associated virus (AAV) vectors, simian virus vectors, vaccinia virus vectors, Sendai virus vectors, Epstein-Barr virus (EBV) vectors, HSV vectors, and like virus vectors. As viral vectors, those deficient in replication ability so that the virus cannot replicate itself in infected cells are preferably used. Further, non-viral vectors can also be used in combination with a liposome and a condensing agent, such as cationic lipid. Further, the nucleic acid can be introduced into cells by calcium phosphate transfection, DEAE-dextran, electroporation, particle bombardment, and the like.

The antigen-specific lymphocyte can be prepared by the process of introducing a nucleic acid encoding the immune receptor into a cell. When a cell expressing the immune receptor binds to an antigen via the immune receptor,

a signal is transmitted to the cell and activated. Activation of cells can be confirmed by using the release of cytokines, increased proliferation rate, the change of cell surface molecules, or the like as an indicator, depending on the host cell type and the intracellular domain. For example, the release of cytotoxic cytokines (e.g., tumor necrosis factor, lymphotoxin, etc.) from activated cells results in destruction of tumor cells expressing a complex. Further, cytokine release or the change of cell surface molecules stimulates other immune cells, such as B cells, dendritic cells, NK cells, and macrophages. Accordingly, immune receptor-expressing cells are useful in adoptive immunotherapy.

The process of introducing the nucleic acid encoding the CAR to cells is carried out in vitro (ex vivo) or in vivo. Examples of cells into which the nucleic acid is introduced include mammalian cells, such as human-derived cells, and cells derived from non-human mammals, such as monkeys, mice, rats, pigs, cattle, and dogs. For the types of cells, for example, cells collected, isolated, purified, or derived from blood (e.g., peripheral blood and umbilical cord blood), bone marrow, or other body fluids, tissues, or organs can be used. Usable cells include PBMCs, immune cells (dendritic cells, B cells, hematopoietic stem cells, macrophages, monocytes or NK cells, hematopoietic cells (neutrophils, basophils, and monocytes)), ematopoietic stem cells, cord blood mononuclear cells, fibroblasts, proadipocytes, hepatocytes, blood cells, skin keratinocytes, mesenchymal stem cells, hematopoietic stem cells, adipose stem cells, pluripotent stem cells, various cancer cell lines, and neural stem cells. In the present invention, in particular, T cells, T-cell precursors (e.g., hematopoietic stem cells and lymphoid progenitor cells), and pluripotent stem cells or cell populations containing such cells are preferably used. T cells include CD8-positive T cells, CD4-positive T cells, regulatory T cells, cytotoxic T cells, and tumor-infiltrating lymphocytes. The cell population containing T cells and T-cell precursors includes PBMCs. The cells used in the present invention can be any of the following: cells sampled from a living body, those expanded by culture, and those established as cell lines. When cells transfected with a nucleic acid or cells differentiated from the transfected cells are to be transplanted into a living body, cells collected from the living subject or the living organism of the same species as the subject are preferably transplanted with the nucleic acid.

Antigen-specific lymphocytes may be cultured and/or stimulated by using appropriate culture media and/or stimulatory molecules before administration to a subject. The stimulatory molecules include cytokines, appropriate proteins, and other components. Examples of cytokines include IL-2, IL-7, IL-12, IL-15, and IFN-$\gamma$, and preferably IL-2. The concentration of IL-2 in the culture medium is not particularly limited. For example, the concentration is 0.01 to $1 \times 10^5$ U/mL, and preferably 1 to $1 \times 10^4$ U/mL. Examples of appropriate proteins include CD3 ligand, CD28 ligand, and anti-IL-4 antibody. In addition, lymphocyte-stimulating factors, such as lectins, can also be added. Further, serum or plasma can be added to the culture medium. The amount of such components added to the medium is not particularly limited, and can be, for example, from 0 to 20 vol%. The amount of serum or plasma used can be appropriately changed according to the culture stage. The concentration of serum or plasma can be decreased stepwise. The origin of serum or plasma can be the same as the origin of cultured cells (autologous) or different from the origin of cultured cells (non-autologous). The serum or plasma from the same origin as cultured cells is preferably used in terms of safety.

The cell culture equipment to be used for cell culture is not particularly limited. For example, a Petri dish, a flask, a bag, a large culture bath, a bioreactor, and the like can be used. As the bag, a $CO_2$ gas-permeable bag for cell culture can be used. When a cell population is produced on a large industrial scale, a large culture bath can be used. The culture can be conducted in an open system or a closed system. The closed system is preferably used in terms of safety of the obtained cell population.

## 4. Use

A combination of the HA-antigen composition and antigen-specific lymphocytes can exert antitumor effects more potently, more efficiently, more persistently, and/or more extensively. Therefore, some embodiments of the present invention relate to a pharmaceutical composition for use in combined administration with an antigen-specific lymphocyte, the composition comprising an HA-antigen composition; a pharmaceutical composition for use in combined administration with an HA-antigen composition, the composition comprising an antigen-specific lymphocyte; a pharmaceutical composition characterized by comprising a combination of an HA-antigen composition and an antigen-specific lymphocyte; etc. Further, the HA-antigen composition can also be used as a T cell activation vaccine etc.

The target of administration (e.g., humans, mice, monkeys, dogs, cats, and other animals (in particular, mammalian animals)) can be, for example, a subject with cancer.

The pharmaceutical composition described above can be a composition for prevention or treatment (improvement) of cancer (target disease). The target disease includes solid tumors and blood cancers. Examples of the target disease include various B-cell malignant lymphomas (B-cell acute lymphocytic leukemia, follicular lymphoma, diffuse large-cell lymphoma, mantle cell lymphoma, MALT lymphoma, intravascular B-cell lymphoma, CD20-positive Hodgkin's lymphoma), myeloproliferative diseases, myelodysplastic and myeloproliferative tumors (CMML, JMML, CML, MDS/MPN-UC), myelodysplastic syndrome, acute myeloid leukemia, multiple myeloma, lung cancer, colorectal cancer, ovarian cancer, breast cancer, brain tumor, stomach cancer, liver cancer, tongue cancer, thyroid cancer, kidney cancer, prostate cancer, uterine cancer, osteosarcoma, chondrosarcoma, and rhabdomyosarcoma. "Treatment" includes alleviation (moderation) of symptoms or associated symptoms characteristic to the target diseases, and inhibition or retardation of deterioration of symptoms. "Prevention" means prevention or retardation of development or expression of diseases (disorders) or their symptoms, or decrease in the risk of development or expression. On the other hand, "improvement" means alleviation (moderation), change for the better, amelioration, or healing (including partial healing).

Although immune checkpoint inhibitors show excellent effects on melanoma and lung cancer, their therapeutic effect is only about 20%. Currently, treatments for cold tumors with an intratumoral T low T-cell count, called "immune-desert" or "immune-excluded," are desired.

Mouse melanoma cell B16F10 is a typical cold tumor with very low MHC expression (Non-patent Literature: Oncoimmunology, 2017; 6(6): e1259049), low immunogenicity (Non-patent Literature: Cancer Res., 2000; 60: 5514-5521; Non-patent Literature: Curr Opin Genet Dev. 2014; 24: 46-51), and low intratumoral T cell count (Non-patent Literature: Genomics 21, 2 (2020)), and is also highly metastatic (Non-patent Literature: J Immunogenet 1989; 16(4-5): 291-303). Nonclinical models using B16F10 have been widely used as nonclinical models for low immunogenic cancers, highly metastatic cancers, and cancers with a low intratumoral T cell count. In such models, it has been difficult to demonstrate antitumor effects.

The pharmaceutical composition showed a high degree of effects in the model using B16F10 and thus can exhibit excellent effects on low immunogenic cancers as well.

The low immunogenic cancer as referred to therein means a cancer in which the expression of MHC class I in cancer cells is reduced or absent, tumor antigen presentation is reduced or absent, and immunogenicity is low. Specifically, the percentage of cancer cells expressing MHC class I in the total cancer cells is, for example, 50% or less. The lower the expression level of MHC class I, the more difficult the treatment of cancer is. When the percentage of cancer cells expressing MHC class I is 40% or less, 30% or less, 20% or less, 10% or less, 5% or less, and particularly 1% or less, treatment is difficult. The expression level of MHC class I can be measured, for example, by immunohistochemical staining and FCS analysis.

The pharmaceutical composition of the present invention showed a high degree of effects in the model using B16F10. Thus, the pharmaceutical composition of the present invention can exhibit excellent effects on metastatic cancer as well.

Examples of primary tumor sites of metastatic cancers include, but are not limited to, bladder, bone, bone marrow, brain, breast, cervix, colon, endometrium, esophagus, intestine, kidney, liver, lung, mouth, muscle, ovary, skin, pancreas, prostate, skin, stomach, testis, thyroid gland, and uterus, as well as any hyperproliferative tissues, including vascular structures (e.g., endothelial cells, smooth muscle cells, pericytes, scars, fibrotic tissue, surgically adherent tissue, or hyperproliferative bone lesions). Metastatic sites of metastatic cancers include, but are not limited to, adrenal glands, bone, brain, kidney, liver, lung, lymph node, ovary, peritoneum, skin, and spleen.

The pharmaceutical composition of the present invention showed a high degree of effects in the model using B16F10. Thus, the pharmaceutical composition of the present invention can exhibit a high degree of effects on cancers with a low intratumoral T cell count as well.

The cancer with a low intratumor T-cell count refers to a cancer with a very small number of or no T cells in tumors. Specifically, the ratio of T cells to cancer cells is, for example, 10% or less. The lower the ratio of T cells, the less effective anticancer drugs, such as immuno-checkpoint inhibitors, are. When the ratio is 5% or less, 3% or less, 1% or less, 0.5% or less, 0.3% or less, or 0.1% or less, the treatment is difficult. The ratio of T cells can be measured by immunohistochemical staining and FCS analysis.

The combined administration of the HA-antigen composition and the antigen-specific lymphocyte can be performed by administering the HA-antigen composition and the antigen-specific lymphocyte at the same time, on the same day, or at intervals. In one preferred embodiment, the antigen-specific lymphocyte is preferably administered after administration of the HA-antigen composition (more preferably after administration of the HA-antigen composition and an adjuvant). In this case, the dosing interval between the HA-antigen composition and the antigen-specific lymphocyte (for multiple doses, interval between doses) can be, for example, 1 day to 1 week, 1 to 3 days, or 1 to 2 days.

The pharmaceutical composition can also be in the form of a kit, characterized by comprising a combination of the HA-antigen composition and the antigen-specific lymphocyte. The pharmaceutical composition can be, for example, a cancer treatment kit that contains the HA-antigen composition and the antigen-specific lymphocyte.

4-1. Use of HA-antigen Composition

The HA-antigen composition may be administered, for example, via an oral, extraintestinal, intranasal, vaginal, intraocular, subcutaneous, intravenous, intramuscular, intradermal, intraperitoneal, intraarticular, intracerebral, intraoral, intratissue, or intramammary route, or by inhalation. Preferably, the HA-antigen composition may be administered via a subcutaneous, intramuscular, intravenous, or intradermal route.

The HA-antigen composition may be administered as a pharmaceutical composition containing one or more pharmaceutically acceptable diluents, wetting agents, emulsifiers, dispersants, auxiliary agents, preservatives, buffers,

binders, stabilizers, or the like in any suitable form according to the intended route of administration. The route of administration may be a parenteral or oral route.

The dosage of the HA-antigen composition can be determined as needed. For example, the usual dosage is about 0.1 to 100 mg/dose in terms of the antigen. The number of doses is appropriately 2 to 20 doses. Dosing intervals can be selected between 1 day and 2 weeks.

The HA-antigen composition may be administered in combination with one or more adjuvants. The adjuvant used can be a substance having an effect of potentiating the activity of antigen-presenting cells and is more specifically selected from substances activating an innate immune receptor (pattern recognition receptor) and other antigen-presenting cell-stimulating substances, and substances having an effect of inhibiting antigen-presenting cells from acquiring immunosuppressive activity. The innate immune receptor is classified into Toll-like receptors (TLR), C-type lectin receptors (CLR), NOD-like receptors (NLR), RIG-I-like receptors (RLR), and cytoplasmic DNA sensors. The adjuvant that is an innate immune receptor agonist can be selected from inactivated microbial cells, microbial cell extracts, nucleic acids, lipopolysaccharides, lipopeptides, synthetic low-molecular compounds, and the like. Preferably, CpG oligo DNA, polyIC RNA, imidazoquinoline (e.g., R848 and imiquimod), saponin (e.g., QuilA and QS21), a STING agonist (e.g., cyclic di-GMP), monophosphoryl lipid, a lipopeptide, or the like is used. A taxane drug or an anthracycline drug can be used as the adjuvant that is an antigen-presenting cell stimulant. The adjuvant that is an agent having an effect of inhibiting antigen-presenting cells from acquiring immunosuppressive activity is selected from a substance inhibiting JAK/STAT, a substance inhibiting indole dioxygenase (IDO), a substance inhibiting tryptophan dioxygenase (TDO), and the like. These inhibiting substances include compounds having antagonism on the factors as well as neutralizing antibodies, small interfering RNA (siRNA), and antisense DNA against the factors.

The adjuvant that is used for the HA-antigen composition is preferably an adjuvant that is an innate immune receptor agonist, more preferably an agonist of a Toll-like receptor (TLR) or cytoplasmic DNA sensor. The TLR agonist is preferably CpG oligo DNA, polyIC RNA, imidazoquinoline, such as R848 or imiquimod, saponin, such as QuilA or QS21, a STING agonist, or monophosphoryl lipid.

The adjuvant and the HA-antigen composition may be administered as separate formulations or may be administered as one formulation. The adjuvant may be conjugated with a hyaluronic acid derivative, which is prepared by introducing a hydrophobic group thereinto, by the method described in WO2010/053140 and used. For administration in combination, the amount of the HA-antigen composition is, for example, 0.01 to 100 mg/dose, preferably 0.1 to 50 mg/dose, and more preferably, for example, 0.1 to 20 mg/dose, and the amount of the adjuvant administered is, for example, 0.01 to 100 mg/kg body weight, preferably 0.1 to 50 mg/kg body weight, and more preferably 0.1 to 10 mg/kg of the body weight. The adjuvant may be administered at the same time as the HA-antigen composition (including the case in which the vaccine formulation contains the adjuvant), or the adjuvant and the HA-antigen composition may be administered at different times. When the adjuvant or the HA-antigen composition are administered at different times, it is preferable that one is first administered and the other is then administered, for example, within 1 minute to 5 hours, preferably within 1 minute to 1 hour, after the first administration.

The HA-antigen composition of the present invention may be administered in combination with at least one antibody for use in cancer treatment. The antibody is, for example, an antibody inhibiting an immunosuppressive signal from a tumor, or at least one antibody activating a costimulatory signal of immune cells, preferably an antibody inhibiting an immunosuppressive signal from a tumor or an antibody activating a costimulatory signal of immune cells. Specifically, the antibody is at least one antibody selected from the group consisting of anti-CTLA4 antibody, anti-PD1 antibody, anti-PDL1 antibody, anti-OX40 antibody, and anti-4-1BB antibody. For the combined administration, the amount of the HA-antigen composition administered is, for example, 0.01 to 100 mg/dose, preferably 0.1 to 50 mg/dose, and more preferably 0.1 to 20 mg/dose, and the amount of the antibody administered is, for example, 0.01 to 200 mg/kg body weight, preferably 0.1 to 100 mg/kg body weight, and more preferably 1 to 40 mg/kg body weight. The antibody may be administered at the same time as the HA-antigen composition (including the case in which the antibody is contained in the formulation), or the antibody and the HA-antigen composition may be administered at different times. When the antibody and the HA-antigen composition are administered at different times, it is preferable that one is first administered and the other is then administered, for example, within 1 minute to 24 hours, preferably within 1 minute to 5 hours, after the first administration.

The HA-antigen composition may be administered in combination with both the adjuvant and the antibody. In this case, the amount of the HA-antigen composition administered is, for example, 0.01 to 100 mg/dose, preferably 0.1 to 50 mg/dose, and more preferably, for example, 0.1 to 20 mg/dose; the amount of the adjuvant administered is, for example, 0.01 to 100 mg/kg body weight, preferably 0.1 to 50 mg/kg body weight, and more preferably 0.1 to 10 mg/kg body weight; and the amount of the antibody administered is, for example, 0.01 to 200 mg/kg body weight, preferably 0.1 to 100 mg/kg body weight, and more preferably 1 to 40 mg/kg body weight. The adjuvant and the antibody may be administered at the same time as in the vaccine formulation of the present invention (including the case in which the formulation contains the adjuvant and the antibody), or the adjuvant, the antibody, and the HA-antigen composition may be administered at different times. When the vaccine formulation, the adjuvant, and the antibody are administered at

different times, it is preferable that they are all administered, for example, within 1 minute to 24 hours, preferably within 1 minute to 5 hours.

In one preferred embodiment of the present invention, the HA-antigen composition is preferably administered before administration of the antigen-specific lymphocyte. In a preferred embodiment of the present invention, the HA-antigen composition is preferably administered at least once after one or two units of administration wherein a single unit of administration consists of one-time administration of the HA-antigen composition and subsequent one-time infusion of the antigen-specific lymphocyte.

In one preferred embodiment of the invention, the interval between the first administration and the second administration of the HA-antigen composition is, for example, not less than 1 day, 2 days, 3 days, 4 days, 5 days, 6 days, 7 days, 8 days, 9 days, 10 days, 11 days, 12 days, 13 days, or 14 days. On the other hand, the interval can be, for example, not more than 28 days, 24 days, 21 days, 17 days, 14 days, 13 days, 12 days, 11 days, 10 days, 9 days, 8 days, 7 days, 6 days, 5 days, 4 days, 3 days, or 2 days.

4-2. Use of Antigen-Specific Lymphocyte

The antigen-specific lymphocyte can be widely used as a cell preparation for the treatment, prevention, or improvement of a tumor or cancer (target disease) that expresses a target antigen of an antigen-binding domain.

The content of the antigen-specific lymphocyte in the cell preparation is not particularly limited, and is preferably $1 \times 10^3$ to $1 \times 10^{11}$ cells/mL, more preferably $1 \times 10^4$ to $1 \times 10^{10}$ cells/mL, and even more preferably $1 \times 10^5$ to $2 \times 10^9$ cells/mL. The dosage of the therapeutic agent containing the cell population as an active ingredient is not particularly limited, and is preferably $1 \times 10^6$ to $1 \times 10^{12}$ cells/day per adult, more preferably $1 \times 10^7$ to $5 \times 10^{11}$ cells/day per adult, and even more preferably $1 \times 10^8$ to $2 \times 10^{11}$ cells/day per adult. The cell preparation can contain, for example, dimethyl sulfoxide (DMSO), serum albumin, etc. for the purpose of protecting cells, antibiotics etc. for the purpose of preventing bacterial contamination, and various components for the purpose of activating cells or inducing proliferation or differentiation (e.g., vitamins, cytokines, growth factors, and steroids).

The route of administration of the antigen-specific lymphocyte is not particularly limited. For example, the antigen-specific lymphocyte is administered by intravenous injection, intraarterial injection, portal vein injection, intradermal injection, subcutaneous injection, intramuscular injection, or intraperitoneal injection. Local administration may be used in place of systemic administration. Examples of the local administration include direct injection into target tissues, body parts, or organs. The dosing schedule may be made according to the sex, age, body weight, and pathological condition of the subject (patient). A single dose or continuous or periodic multiple doses may be used. For the number of doses, 2 to 20 doses are appropriate. The dosing interval can be selected from 1 day to 2 weeks.

Examples

The present invention is described in detail below based on Examples. However, the scope of the invention is not limited to these Examples.

Materials and Methods (Examples 1 to 5)

(1) Cells

RPMI 1640 medium (supplemented with 2-mercaptoethanol) was purchased from Cell Science & Technology Institute. Fetal bovine serum (FBS) was purchased from Gibco. Mouse fibrosarcoma CMS5a cell line obtained from Memorial Sloan-Kettering Cancer Institute subcultured at Mie University was used. The mouse fibrosarcoma CMS5a cell line expressed mutated ERK2 protein. The peptide containing the mutation site of mutated ERK2 protein (QYIHSANVL (SEQ ID NO: 1)) is recognized by CD8-positive cytotoxic T cells of BALB/c mice. A T-cell receptor (TCR) capable of recognizing this peptide was isolated, and a TCR gene-transfected mouse (DUC18 mouse) was established. The long-chain peptide antigens used in the Examples contain the CD8-positive cytotoxic T-cell recognizing epitope sequence of mutated ERK2

(QYIHSANVL).

(2) Test Animal

Female BALB/c mice (CD90.2-positive) at the age of 6 to 12 weeks were purchased from Japan SLC. DUC18 transgenic mice obtained from the University of Washington and bred at Mie University were used. Both mice were bred at Experimental Animal Facilities in the Advanced Science Research Promotion Center of Mie University. Protocols of

animal experiments were approved by the ethics committee of Mie University School of Medicine.

(3) Hyaluronic Acid Nanogel (HANG)

A hyaluronic acid nanogel into which a cholesteryl group was introduced as described in Examples 1 and 2 of WO2010/053140 (weight average molecular weight: 99 kDa, cholesteryl group introduction rate: 42%) was synthesized.

(4) Long-chain Peptide Antigen

A synthetic long-chain peptide was purchased from Sigma Genosys. The peptide had the following sequence: NDHIAYFLYQILRGLQYIHSANVLHRDLKPSNLLLNT (SEQ ID NO: 2).

The sequence contained a 9-amino acid sequence (SEQ ID NO: 1: QYIHSANVL) from Q at position 16 to L at position 24 as a CD8-positive cytotoxic T-cell recognition epitope sequence, and a 17-amino acid sequence (SEQ ID NO: 3: RGLQYIHSANVLHRDLK) from R at position 13 to K at position 29 as a CD4-positive helper T-cell recognition epitope sequence.

(5) Preparation of Complex of Hyaluronic Acid Nanogel and Antigen

The hyaluronic acid nanogel was dissolved at 12 mg/mL in ultrapure water and ultrasonicated (E220X, produced by Covaris Inc.,). The antigenic peptide was dissolved at 50 mg/mL in DMSO. The solution of the antigenic peptide in DMSO was added to the aqueous HA derivative solution, and the mixture was ultrasonicated in a bath sonicator for 30 minutes. The mixture was transferred to a dialysis kit (Slide-A-Lyzer, molecular weight cutoff: 3.5 K) and dialyzed against a 5 mM carbonate buffer (pH: 9), a 10 mM phosphate buffer (pH: 7), and a 10 mM phosphate buffer (pH: 7) containing 10% sucrose in this order. The antigenic peptide concentration was quantified by reversed-phase chromatography analysis. The concentration was adjusted to 250 to 500 ug/mL to prepare a dosing solution. If the concentration did not reach the intended concentration, the solution was concentrated with an ultrafiltration concentrator (Vivaspin 6, 5000 MwCO, produced by Sartorius AG). The obtained complex may be referred to as the "HA nanogel cancer vaccine loaded with a long-chain peptide antigen" or the "HA nanogel cancer vaccine."

(6) Other Reagents

-Adjuvant

CpG oligo DNA1668 was purchased from Ajinomoto Biopharma Service Gene Design.

- Fluorescence-Labeled Antibodies

eFluor 450-labeled anti-mouse CD8a antibody (clone 53-6.7), eFluor 450-labeled anti-mouse KLRG1 antibody (clone 2F1), and APC-labeled anti-mouse CD45R (B220) antibody (clone RA3-6B2) were purchased from eBioscience. APC-labeled anti-mouse CD8a antibody (clone 53-6.7), APC-labeled anti-mouse CD4 antibody (clone RM4-5), PerCP-Cy5.5-labeled anti-mouse CD90.1 antibody (clone OX-7), PerCP-Cy5.5-labeled anti-mouse F4/80 antibody (clone BM8), PE-labeled anti-mouse CD207 antibody (clone 4C7), Pacific Blue-labeled anti-mouse CD90.2 antibody (clone 30-H12), and Purified CD16/32 antibody (clone 93) were purchased from BioLegend. FITC-labeled anti-mouse CD11c antibody (clone HL3), and PerCP-Cy5.5-labeled anti-mouse CD3 antibody (clone 145-2C11) were purchased from BD Bioscience. APC-labeled anti-mouse IFN-$\gamma$ antibody (clone XMG1.2) was purchased from eBioscience or TONBO. Anti-mouse CD8a antibody (clone 4SM15) for tissue staining was purchased from Invitrogen, and Alexa488-labeled anti-rat IgG (H+L) antibody was purchased from Cell Signaling.

- Short-chain Peptide Antigen

A synthetic short-chain peptide for CD8-positive T-cell stimulation was purchased from Sigma Genosys. The peptide has the following sequence: QYIHSANVL (SEQ ID NO: 1).

Example 1. Tumor Growth Test

Mouse fibrosarcoma CMS5a cell line was cultured in 10% FBS-containing RPMI 1640 medium using T75 culture flasks (Corning). The cell line was detached from the flasks by using 0.5% trypsin-containing PBS and suspended in 10% FBS-containing RPMI 1640 medium. The suspension was centrifuged (400$\times$g, 5 min, 4°C) to remove the supernatant. The cells were then washed twice with RPMI 1640 medium and suspended in RPMI 1640 medium at a concentration of 1 × $10^6$/100 μL. The suspension was subcutaneously inoculated into the right anterodorsal part of BALB/c mice at a dose of 100 μL/mouse (5 mice per group).

When an HA nanogel cancer vaccine loaded with a long-chain peptide antigen was administered, 50 μg of the HA nanogel cancer vaccine loaded with a long-chain peptide antigen was subcutaneously administered together with 50 μg of CpG oligo DNA dissolved in PBS into the right posterodorsal part of the mice 7 days and 11 days after the tumor inoculation (HANG-V group).

When the antigen-specific T cell was administered, CD8-positive T cells were isolated from the spleens of mutated ERK 2-specific TCR transgenic DUC18 mice (CD90.1-positive or CD90.2-negative) 8 and 12 days after the tumor inoculation using CD8a MicroBeads (produced by Miltenyi Biotec). After the cells were suspended in RPMI 1640 medium at a concentration of 2 × $10^6$ cells/200 μL, 200 μL of the suspension was infused through the tail vein of the mice (TCR-T group). The tumor area was then measured over time.

Example 1: Results

To clarify the antitumor effect achieved by combined use of the HA nanogel cancer vaccine and antigen-specific adoptive T-cell therapy, an HA nanogel cancer vaccine loaded with a long-chain peptide antigen, which is a composite of an HA nanogel and a long-chain peptide antigen containing the CD8 epitope of mutated ERK2 antigen (mERK2) expressed in CMS5a, was prepared, and using an immune checkpoint inhibitors (ICIs)-resistant fibrosarcoma cell line CMS5a tumor-bearing mice, therapeutic effects of combined use of the HA nanogel cancer vaccine and mERK2-recognition TCR gene-transfected adoptive T-cell therapy were examined. As shown in Fig. 1-1 and Fig. 1-2, the results showed that the combination treatment group treated with HANG-V and TCR-T significantly inhibited CMS5a tumor growth and made the tumor regress as compared to the single-agent treatment group treated with HANG-V alone (the HANG-V treatment group) or the single-agent treatment group treated with TCR-T alone (the TCR-T treatment group), and tumors regressed in all of the mice 7 days after the treatment (Fig. 1-1 and Fig. 1-2). This effect was long lasting with no tumor growth being observed after re-inoculation of CMS5a into the same mice, and the mice survived for a long period of time without recurrence (Fig. 1-3). These results indicate that combined use of the HA nanogel cancer vaccine and antigen-specific adoptive T-cell therapy has potent antitumor effects and can cure ICI-resistant tumors. Furthermore, the results reveal that this combination can inhibit tumor recurrence after tumor regression, suggesting that the HA nanogel cancer vaccine enhances the antitumor effect of antigen-specific T cells.

Example 2. Analysis of Antitumor Immune Response in the draining Lymph Nodes at the Vaccine Administration Site

Fourteen days after tumor inoculation in the test in Example 1, the draining lymph nodes at the vaccine administration site (right inguinal lymph node) were collected. After grinding the lymph nodes by using a glass slide, the cells were suspended in RPMI 1640 medium. At this time, cells from 2 mice in each group were pooled. The suspension was centrifuged (400×g, 5 min, 4°C) to remove the supernatant. The cells were washed twice using RPMI 1640 medium and then suspended in RPMI 1640 medium containing 10% FBS. The number of cells was counted using a cell hemocytometer (Fig. 2-1), and the cell suspension was adjusted to a cell concentration of 2 × $10^6$ cells/mL.

Staining of Myeloid Cells (Fig. 2-3 and Fig. 2-4)

The cell suspension was added to a 96-well V-bottom microplate (Nunc) at 4 × $10^5$ cells/200 μL per well. After the cell suspension was centrifuged (2000 rpm, 2 min, 4°C) to remove the supernatant, the cells were washed twice using 200 μL of staining buffer (PBS containing 0.5% FBS) and then suspended. After the suspension was centrifuged to remove the supernatant, 50 μL of a solution containing 50-fold diluted anti-mouse CD16/CD32 antibody was added. The mixture was incubated in the dark at 4°C for 10 minutes. After 150 μL of staining buffer was added and the mixture was centrifuged to remove the supernatant, the cells were further washed twice with 200 μL of staining buffer. After 50 μL of a solution containing FITC-labeled anti-mouse CD11c antibody, PerCP-Cy5.5-labeled anti-mouse F4/80 antibody, and PE-labeled anti-mouse CD207 antibody at use concentrations recommended by the manufacturer of each antibody was added to the cells and mixed. The mixture was incubated in the dark at 4°C for 15 minutes. After 150 μL of staining buffer was added and the mixture was centrifuged to remove the supernatant, the cells were further washed twice with 200 μL of staining buffer. The cells were centrifuged to remove the supernatant, then resuspended in 200 μL of staining buffer and transferred to round-bottom polystyrene tubes (produced by BD Biosciences). The cells were analyzed on a FACS Canto II flow cytometer (produced by BD Biosciences) using analysis software (FACSDiva). Dendritic cells were detected as a CD11c-positive population, macrophages as a F4/80-positive population, and Langerhans cells as a CD11c-positive and CD207-positive population.

Staining of Lymphocytes (Fig. 2-4)

The cell suspension was added to 96-well V-bottom microplates (Nunc) at 4 × $10^5$ cells/200 μL per well. After the cell suspension was centrifuged (2000 rpm, 2 min, 4°C) to remove the supernatant, the cells were washed twice with staining buffer (PBS containing 0.5% FBS). After 50 μL of a solution containing PerCP-Cy5.5-labeled anti-mouse CD3 antibody, APC-labeled anti-mouse CD45R (B220) antibody, and eFluor450-labeled anti-mouse KLRG1 antibody at use concentrations recommended by the manufacturer of each antibody was added to the cell suspension and mixed, the mixture was incubated in the dark at 4°C for 15 minutes. The cells were washed twice with 200 μL of staining buffer, then resuspended in 200 μL of staining buffer and transferred to round-bottom polystyrene tubes (produced by BD Biosciences). The cells were analyzed on a FACS Canto II flow cytometer (produced by BD Biosciences) using analysis software (FACSDiva). T cells were detected as a CD3-positive population, B cells as a CD45R (B220)-positive population, and NK cells as a KLRG1-positive population.

Staining for CD8-Positive Infused T Cells (Fig. 2-5)

A cell suspension was added to a 96-well V-bottom microplate (Nunc) at 4 × $10^5$ cells/200 μL per well. After the cell suspension was centrifuged (400 × g, 5 min, 4°C) to remove the supernatant, the cells were washed twice with staining buffer (PBS containing 0.5% FBS) and then suspended. After 50 μL of a solution containing eFluor450-labeled anti-mouse CD8 antibody, PerCP-Cy5.5-labeled anti-mouse CD90.1 antibody, and APC-labeled anti-mouse CD4 antibody at use concentrations recommended by the manufacturer of each antibody was added to the cell suspension and mixed, the mixture was incubated in the dark at 4°C for 15 minutes. The cells were washed twice with 200 μL of staining buffer, then resuspended in 200 μL of staining buffer and transferred to round-bottom polystyrene tubes (produced by BD Biosciences). The cells were analyzed on a FACS Canto II flow cytometer (produced by BD Biosciences) using analysis software (FACSDiva). Infused CD8-positive T cells were detected as a CD90.1-positive, CD8-positive population.

Antigen-Specific T Cell Induction Test (Intracellular Cytokine Staining) (Fig. 2-6):

The cell suspension was added to 48-well culture plates (Nunc) at 4 × $10^5$ cells/200 μL per well. As a short-chain peptide for CD8-positive T-cell stimulation, 50 μL of mERK2 9m peptide was added at a concentration of 10 μg/mL, and the cells were cultured at 37°C, 5% $CO^2$ for 1 hour. GolgiPlug (produced by BD Biosciences) diluted 167-fold with RPMI 1640 medium containing 10% FBS was then added at 50 μL per well, and the cells were cultured at 37°C, 5% $CO_2$, for 5 hour. The cells were collected and transferred to a 96-well V-bottom microplate (Nunc). The cells were centrifuged (2000 × rpm, 2 min, 4°C) to remove the supernatant, and then suspended in 200 μL of staining buffer per well. After the cells were washed twice with 200 μL of staining buffer, eFluor450-labeled anti-CD8 antibody and PerCP-Cy5.5-labeled anti-CD90.1 antibody were added to the cell suspension at use concentrations recommended by the manufacturer of each antibody and mixed, and the mixture was incubated in the dark at 4°C for 15 minutes. After 150 μL of staining buffer was added and the mixture was centrifuged to remove the supernatant, the cells were further washed twice with 200 μL of staining buffer. Thereafter, 100 μL of Cytofix/Cytoperm buffer (produced by BD Biosciences) was added, and the mixture was gently mixed. After the mixture was incubated in the dark at room temperature for 20 minutes, the cells were washed twice with staining buffer, suspended in 200 μL of staining buffer, and then kept refrigerated overnight away from light. After the suspension was centrifuged to remove the supernatant, the cells were washed with 200 μL of Perm/Wash buffer (BD Biosciences). After APC-labeled anti-mouse IFN-γ antibody diluted 50-fold with Perm/Wash buffer was added to the cells and mixed, the mixture was incubated in the dark at room temperature for 15 minutes. The cells were washed twice with 200 μL of Perm/Wash buffer, then resuspended in 200 μL of staining buffer, and transferred to round-bottom polystyrene tubes (produced by BD Biosciences). The cells were analyzed on a FACS Canto II flow cytometer (produced by BD Biosciences) using analysis software (FACSDiva). IFN-γ-producing CD8-positive infused T cells were detected as an IFN-γ-positive CD90.1-positive, CD8-positive population, or an IFN-γ-positive, CD90.2-negative, CD8-positive population. IFN-γ-producing CD8-positive host T cells were detected as an IFN-γ-positive, CD90.1-negative, CD8-positive population or an IFN-γ-positive, CD90.2-positive, CD8-positive population.

Example 2: Results

To clarify the effect of the HA nanogel cancer vaccine on antitumor immune response in combined use of the HA nanogel cancer vaccine and antigen-specific adoptive T-cell therapy, the draining lymph nodes at the HA vaccine administration site were collected 14 days after the tumor inoculation, and the lymph node cells were analyzed. As a result, the draining lymph nodes in the HANG-V treatment group and the combined HANG-V and TCR-T treatment group were significantly enlarged, and lymph node cells in both groups increased approximately 4 to 8 times compared to the

untreated group (Fig. 2-1). The enlarged lymph nodes were continuously observed even 22 days after tumor inoculation (Fig. 2-2). Next, the percentage of myeloid antigen-presenting cells that influxed and accumulated in the draining lymph nodes were examined. The results showed that an increase in myeloid antigen-presenting cells was observed in both the HANG-V treatment group and the combined HANG-V + TCR-T treatment group. In particular, the combined HANG-V and TCR-T treatment group showed a significant increase in Langerhans cells (Fig. 2-3). Furthermore, to clarify the effect of the HA nanogel cancer vaccine on the number of the cells in draining lymph nodes, lymphocytes and myeloid cells of the draining lymph nodes were analyzed. As a result, as shown in Fig. 2-4, a several-fold to several dozen-fold increase in lymphocytes (T cells, B cells, and NK cells) and myeloid cells was observed in the HANG-V group (Fig. 2-4). Furthermore, the influx of CD8-positive infused T cells into the draining lymph nodes was examined. The results revealed that in the combined HANG-V + TCR treatment group, the percentage of CD8-positive infused T cells in the draining lymph nodes were significantly increased as compared to the TCR-T treatment group (Fig. 2-5), and about 80% of the CD8-positive infused T cells specifically response to the tumor antigen and produced IFN-$\gamma$ (Fig. 2-6). Furthermore, an increase in the percentage of IFN-$\gamma$-producing, CD8-positive T cells in the host mice was also observed in the combined HANG-V + TCR-T treatment group (Fig. 2-6). These results suggest that in the immunosuppressive environment of a tumor-bearing host mice, the HA nanogel cancer vaccine induces a large influx and accumulation of antigen-presenting cells and lymphocytes, such as host dendritic cells, macrophages, and Langerhans cells, in the draining lymph nodes and thus established an efficient tumor antigen-presenting environment. The results further suggest that when this environment of improved tumor antigen presentation is provided with T cells by antigen-specific adoptive T-cell therapy, CD8-positive infused T cells recruiting the draining lymph nodes can efficiently receive antigen presentation, thus leading to the induction and proliferation of CD8-positive infused T cells into qualitatively and quantitatively potent tumor antigen-specific cytotoxic T cells (CTLs) to thereby sustainably establish a synergistic antitumor immunity environment in the draining lymph nodes.

Example 3: Therapeutic Effect of Gradual Reduction in the Number of Infused Cells and Analysis of Antitumor Immune Response

The mouse tumor growth test and the antitumor immune response analysis were performed in the same manner as Example 1 and Example 2, respectively, except that the dose was tapered in such a manner that the number of antigen-specific T cells was gradually reduced from $2 \times 10^6$ cells/200 $\mu$L to $1 \times 10^6$ cells/200 $\mu$L, $5 \times 10^5$ cells/200 $\mu$L, and $2.5 \times 10^5$ cells/200 $\mu$L.

Example 3: Results

To clarify the effect of the number of CD8-positive infused T cells on the therapeutic effect achieved by combined use of the HA nanogel cancer vaccine with antigen-specific adoptive T-cell therapy, the dose was tapered in such a manner that the number of CD8-positive infused T cells was gradually reduced, and the therapeutic effects and antitumor immune response were examined. As a result, even when the number of CD8-positive infused T cells was gradually reduced to $1 \times 10^6$, $5 \times 10^5$, and $2.5 \times 10^5$ cells and administered in a small amount, therapeutic effects equivalent to those provided by conventional the dose of $2 \times 10^6$ cells were achieved (Figs. 3-1 and 3-2) . Furthermore, the effect on the total number of draining lymph node cells and myeloid antigen-presenting cells 14 days after the tumor inoculation was examined. Even at gradually reduced low doses, these cells were significantly observed (Fig. 3-3 and Fig. 3-4). Furthermore, the percentages of CD8-positive infused cells and tumor antigen-specific IFN-$\gamma$-producing cells in the draining lymph nodes were tested. The results showed that the percentage of CD8-positive infused cells was the highest when the cells were administered at a conventional concentration of $2 \times 10^6$ cells on the other hand, tumor antigen-specific IFN-$\gamma$-producing cells exhibited equivalent activity even when CD8-positive infused T cells were administered in gradually reduced small amounts in the infused cells and the host T cells (Figs. 3-5 and 3-6). These results indicate that the HA nanogel vaccine can induce a tumor antigen-specific immune response and strongly cure ICI-resistant tumors even when used in combination with a much smaller number of CD-positive infused T cells, which is nearly 1/10 the number of CD8-positive infused T cells conventionally used.

Example 4: Observation of Tumor-Infiltrating CD8-Positive T Cells in Tumors

Fourteen days after the tumor inoculation in the test of Example 1, tumors were collected and subjected to immunohistofluorescent staining by the following method. The tumor was embedded in O.C.T. compound (Sakura Finetech), frozen, and sliced into a thickness of 5 um. The sliced tumor sections were air-dried. The dried tumor sections were fixed with ice-cold acetone for 10 minutes and used for immunostaining. The tumor sections were washed 3 times with PBS, then immersed in Blocking One Histo (produced by Nacalai Tesque), and blocked in an incubation chamber for 30 minutes. Subsequently, anti-mouse CD8 antibody diluted 100-fold in Antibody Diluent with Background Reducing

Component (Dako) was used to stain the tumor sections in an incubation chamber at room temperature for 1 hour. Furthermore, after the tumor sections were washed with PBS containing 0.1% Tween 20, Alexa488-labeled anti-rat antibody diluted 1000-fold in PBS was used to stain the tumor sections in an incubation chamber at room temperature for 1 hour. Finally, the tumor sections were washed 3 times with PBS containing 0.1% Tween 20, and immersed in Prolong Gold antifade reagent with DAPI (produced by Life Technologies). CD8-positive T cells infiltrating the tumor were observed using a BZ-710 fluorescence microscope (produced by Keyence Corporation).

Example 4: Results

To clarify the effect of combined use of the HA nanogel cancer vaccine and antigen-specific adoptive T-cell therapy on the intratumoral environment of tumor-bearing host mice, the tumor of each mouse was removed 14 days after the tumor inoculation. CD8-positive T cells infiltrating the tumor were observed under a fluorescence microscope. The results showed that CD8-positive T cells in the tumor were more prominently observed in the combined HANG-V and TCR-T treatment group than those in the untreated group or in the single-agent treatment group (Fig. 4). The results suggest that combined use of the HA nanogel cancer vaccine and antigen-specific adoptive T-cell therapy induces the tumor antigen-specific cytotoxic T cell (CTL) into the tumor and eliminates the tumor.

Example 5: Analysis of CDS-Positive Infused T Cells in Peripheral Blood

Twenty-one days after tumor inoculation in the test of Example 3, peripheral blood was collected from the mouse orbit and suspended in RPMI containing 10% heparin and mixed. The suspension was then slowly superposed on 500 uL of Ficoll (produced by GE Healthcare) placed in an Eppendorf tube. After centrifugation (4000 rpm × 10 min, 25°C, AC: 1, DE: 1), the mononuclear cell layer was collected in staining buffer (PBS containing 0.5% BSA). After the cell suspension was centrifuged (5000 rpm × 1 min, 25°C), the supernatant was removed with an aspirator and the cells were washed twice with PBS containing 0.5% BSA. After APC-labeled anti-mouse CD8 antibody, PerCP-Cy5.5-labeled anti-mouse CD90.1 antibody, and Pacific Blue-labeled anti-mouse CD90.2 antibody were added to the cell suspension at use concentrations recommended by the manufacturer of each antibody and mixed, the mixture was incubated in the dark at 4°C for 15 minutes. The cells were washed twice with 200 μL of staining buffer, then resuspended in 200 μL of staining buffer and transferred to round-bottom polystyrene tubes (BD Biosciences). The cells were analyzed on a FACS Canto II flow cytometer (produced by BD Biosciences) using analysis software (FACSDiva) .

Example 5: Results

To clarify the survivability (persistence) of CD8-positive infused T cells in the total body after HA nanogel cancer vaccine administration and antigen-specific T-cell infusion, peripheral blood cells were collected 21 days after tumor inoculation, and the percentage of CD8 positive infused T cells present in the peripheral blood was examined. As a result, it was found that CD8-positive infused T cells were still circulating throughout the body and were present in the blood even 21 days after the tumor inoculation. When the number of CD8-positive infused T cells was gradually reduced to $2.5 \times 10^5$ and administered at a low dose, CD8-positive infused T cells were observed in the blood at a concentration equivalent to that achieved by infusion at a concentration of $2 \times 10^6$ cells (Fig. 5) . These results showed that CD8-positive infused T cells continue to circulate in the peripheral blood and be present throughout the body after infusion, regardless of the number of infused cells.

Examples 1 to 5: Conclusion

The above results clearly show the following. Combined use of the HA nanogel cancer vaccine and antigen-specific adoptive T-cell therapy promotes influx and accumulation of antigen-presenting cells and infused T cells into the draining lymph nodes, and induces infused T cells into tumor antigen-specific cytotoxic T cells (CTLs) via antigen presentation resulted in regressed ICI-resistant tumors and cure the disease. In addition to the advantage of qualitatively and quantitatively enhancing infused T cells, this therapy also acts on T cells of the host mice, thus altering the immunosuppressive environment in the host mice to an antitumor environment.

Furthermore, the results clearly show that this combined therapy induces an equivalent level of effect by using a much less number of infused cells than the number of infused cells conventionally used, and the effects persisted systemically after healing, thus suppressing tumor recurrence and allowing the host mice to survive for a long period of time.

In general, the cancer vaccine is a therapy that enhances the antigen-presenting capacity of antigen-presenting cells and is expected to induce antitumor effects of T cells. Since the immune environment of many cancer patients is suppressive, even if a vaccine can present tumor antigens to T cells, the therapeutic effect is limited if the number of T

cells present in the total body. On the other hand, antigen-specific adoptive T-cell therapy is a therapy in which T cells of a cancer patient are modified to antigen-specific T cells in vitro and returned to the body. However, the infused T cells become gradually exhausted and weakened. To prolong the therapeutic effect, it is important to maintain continuous infusion of T cells and the presence of high-quality T cells in the body.

In the present invention, each treatment method complements and compensates for weakness of the other, that is, the HA nanogel cancer vaccine establishes an efficient tumor antigen presentation environment in the draining lymph nodes and the adoptive T-cell therapy provides T cells into the efficient tumor antigen presentation environment. As a result, an antitumor immunity environment in which tumor antigen-specific CTLs are qualitatively, quantitatively, and sustainably induced is established, whereby a synergistic therapeutic effect is considered to be successfully provided by the present invention.

Materials and Methods (Examples 6 to 16)

(1) Cells

RPMI 1640 medium (containing 2-mercaptoethanol) was purchased from Cell Science & Technology Institute, Inc. D-MEM medium was purchased from FUJIFILM Wako Pure Chemical Corporation, and fetal bovine serum (FBS) was purchased from Gibco. Mouse fibrosarcoma cell line CMS5a was provided by the Memorial Sloan Kettering Cancer Center, and mouse metastatic melanoma cell line B16F10 was purchased from ATCC and passaged at Mie University for use. The mouse fibrosarcoma cell line CMS5a has expressed mutated ERK2 protein. A peptide (QYIHSANVL (SEQ ID NO: 1)) containing the mutated part of the mutated ERK2 protein is recognized by CD8-positive cytotoxic T cells of BALB/c mice. A T-cell receptor (TCR) that recognizes this peptide has been isolated, and transgenic mice (DUC18 mice) with the TCR have been established. The long-chain peptide antigen used in the Examples comprises the CD8-positive cytotoxic T-cell recognition epitope sequence (QYIHSANVL) of the mutated ERK2. The mouse metastatic melanoma cell line B16F10 has expressed a tumor antigen gp100 protein, p25-33 peptide (EGSRNQDWL (SEQ ID NO: 4)) contained in this protein is recognized by CD8-positive cytotoxic T cells of C57BL/6 mice, and transgenic mice (Pmel-1 mice) with a TCR that recognizes the CD8-positive cytotoxic T-cell recognition epitope sequence have been established. The long-chain peptide antigen used in the Examples comprises this sequence.

(2) Test Animals

Female BALB/c mice and female C57BL/6 mice (6 weeks old to 8 weeks old) were purchased from Japan SLC. DUC18 transgenic mice were provided by the University of Washington and bred at the Animal Experiment Facility of Mie University for use. Pmel-1 transgenic mice were purchased from the Jackson Laboratory and bred at the Animal Experiment Facility of Mie University for use. All the mice were raised at the Animal Experiment Facility of Mie University. Protocols of animal experiments were approved by the ethics committee of Mie University School of Medicine.

(3) Production of Complex Using HA Nanogel with a Molecular Weight of 10k and Peptide mERK2 (Used in Examples 11 to 16)

(3-1) Hyaluronic Acid Nanogel (HANG)

As described in Examples 1 and 2 of WO2010/053140, a cholesteryl group-introduced hyaluronic acid nanogel (weight average molecular weight: 10 kDa, introduction ratio of cholesteryl groups: 44 and 41%) was synthesized.

(3-2) Long-Chain Peptide Antigen

A synthetic long-chain peptide was purchased from Biologica. The sequence was as follows: NDHIAYFLYQILRGLQYIHSANVLHRDLKPSNLLLNT (SEQ ID NO: 2). This sequence includes a 9-amino acid sequence from the 16th Q to the 24th L (QYIHSANVL (SEQ ID NO: 1)) as a CD8-positive cytotoxic T-cell recognition epitope sequence, and a 17-amino acid sequence from the 13th R to the 29th K (RGLQYIHSANVLHRDLK (SEQ ID NO: 3)) as a CD4-positive helper T-cell recognition epitope sequence.

(3-3) Production of Complex of Hyaluronic Acid Nanogel and Antigen

A lyophilized product of the hyaluronic acid nanogel was weighed, water for injection was added to a concentration of 5 mg/mL, and the mixture was stirred overnight to fully dissolve the lyophilized product. On the other hand, in another container, the peptide was dissolved in dimethyl sulfoxide so that the peptide concentration was 50 mg/mL.

Subsequently, the hyaluronic acid nanogel aqueous solution and the dimethyl sulfoxide solution of the peptide were mixed at a volume ratio of 100:1, followed by stirring at room temperature for 24 hours, thereby obtaining a composition containing a peptide-hyaluronic acid nanogel complex. Then, solid purified sucrose (produced by FUJIFILM Wako Pure Chemical Corporation, for production only, product number: 198-18385) was added so that the composition containing a peptide-hyaluronic acid nanogel complex was 10 mass% sucrose, followed by stirring for 1 hour or more. Subsequently, the composition was diluted with 10 mass% sucrose-containing 25 mM phosphate buffer (pH: 7.4) so that the theoretical peptide concentration was 0.3 mg/mL, and sterile filtration was performed using 0.2 um PES (polyether sulfone) (produced by Pall, Acrodisc syringe filter, 25-mm diameter), thereby obtaining a composition containing a peptide-hyaluronic acid nanogel complex. After the peptide concentration in the composition containing a peptide-hyaluronic acid nanogel complex at this time was quantified by reversed-phase chromatography analysis, the concentration was adjusted to a peptide concentration of 250 ug/mL using 10% sucrose 10 mM phosphate buffer (pH: 7.4) to prepare a dosing solution.

(4) Production of Complex Using HA Nanogel with a Molecular Weight of 10k and Peptide gp100 (Used in Examples 6 to 10)

(4-1) Hyaluronic Acid Nanogel (HANG)

As described in Examples 1 and 2 of WO2010/053140, a cholesteryl group-introduced hyaluronic acid nanogel (weight average molecular weight: 10 kDa, introduction ratio of cholesteryl groups: 44%) was synthesized.

(4-2) Long-Chain Peptide Antigen

Using the hyaluronic acid nanogel obtained above, a peptide-hyaluronic acid nanogel complex was prepared as follows. gp100 TRP2 TRP1_6Y peptide was purchased from Biologica. The sequence is as follows: SVYDFFVWLYYYYYYTWHRYHLLYYYYYYEGSRNQDWL (SEQ ID NO: 5). This sequence includes the following TRP2 (amino acid sequence: SVYDFFVWL (SEQ ID NO: 6)), TRP1 (amino acid sequence: TWHRYHLL (SEQ ID NO: 7)), and gp100 (amino acid sequence: EGSRNQDWL (SEQ ID NO: 4)) as CD8-positive cytotoxic T-cell recognition epitope sequences, and the epitopes are connected by six Ys.

(4-3) Production of Complex of Hyaluronic Acid Nanogel and Antigen

A lyophilized product of the hyaluronic acid nanogel was weighed, water for injection was added to a concentration of 5 mg/mL, and the mixture was stirred overnight to fully dissolve the lyophilized product. On the other hand, in another container, the peptide was dissolved in dimethyl sulfoxide so that the peptide concentration was 50 mg/mL. After confirming dissolution, the hyaluronic acid nanogel aqueous solution and a 1 mol/L sodium hydroxide aqueous solution were mixed at a volume ratio of 100:2 at room temperature. Subsequently, the hyaluronic acid nanogel aqueous solution and the dimethyl sulfoxide solution of the peptide were mixed at a volume ratio of 102:1, followed by stirring at room temperature for 24 hours, thereby obtaining a composition containing a peptide-hyaluronic acid nanogel complex. Then, solid purified sucrose (produced by FUJIFILM Wako Pure Chemical Corporation, for production only, product number: 198-18385) was added so that the composition containing a peptide-hyaluronic acid nanogel complex was 10 mass% sucrose, followed by stirring for 1 hour or more. Subsequently, the composition was diluted with 10 mass% sucrose-containing 25 mM phosphate buffer (pH: 7.4) so that the theoretical peptide concentration was 0.3 mg/mL, and sterile filtration was performed using 0.2 um PES (polyether sulfone) (produced by Pall, Acrodisc syringe filter, 25-mm diameter), thereby obtaining a composition containing a peptide-hyaluronic acid nanogel complex. After the peptide concentration in the composition containing a peptide-hyaluronic acid nanogel complex at this time was quantified by reversed-phase chromatography analysis, the concentration was adjusted to a peptide concentration of 250 ug/mL using 10% sucrose 10 mM phosphate buffer (pH: 7.4) to prepare a dosing solution.

(5) Production of Complex Using CHP and Peptide gp100 (Used in

Example 7)

(5-1) Cholesteryl-Modified Pullulan (CHP)

CHP (cholesteryl-modified pullulan, product number: CHP-80T) produced by NOF Corporation was used.

(5-2) Long-Chain Peptide Antigen

The above gp100 peptide (SEQ ID NO: 5) was used.

(5-3) Production of Complex of Hyaluronic Acid Nanogel and Antigen

CHP powder was dissolved in 6M urea-containing phosphate buffered saline (pH: 7.4) to a concentration of 10 mg/mL. On the other hand, in another container, the peptide was dissolved in dimethyl sulfoxide so that the peptide concentration was 50 mg/mL. The 6M urea-containing CHP aqueous solution and the dimethyl sulfoxide solution of the peptide were mixed at a volume ratio of 100:1, followed by incubation at room temperature for 12 hours or more, thereby obtaining a composition containing a peptide-CHP complex. Then, the composition was transferred to a dialysis cassette (produced by Thermo, Slide-A-Lyzer G2 Dialysis Cassettes, 3.5K MWCO, 15 mL) and dialyzed with 0.6M urea-containing phosphate buffered saline (pH: 7.4). Subsequently, dialysis was performed in the order of 0.06M urea-containing phosphate buffered saline (pH: 7.4) and phosphate buffered saline (pH: 7.4). Furthermore, the composition was concentrated to a desired concentration using an ultraconcentrator (Vivaspin 20, MWCO: 10,000, Sartorius). Finally, sterile filtration was performed using 0.2 μm PES (polyether sulfone) (produced by Pall, Acrodisc syringe filter, 25-mm diameter), thereby obtaining a dosing composition containing a CHP-antigen complex. The peptide concentration was quantified by reversed-phase chromatography analysis.

(6) Other Reagents

- Adjuvant

CpG oligo DNA1668 was purchased from Ajinomoto Bio Pharma Services, Gene Design.

- Fluorescent-Labeled Antibody

eFluor450-labeled anti-mouse CD8a antibody (clone 53-6.7), eFluor450-labeled anti-mouse KLRG1 antibody (clone 2F1), APC-labeled anti-mouse CD44 (clone IM7), and APC-labeled anti-mouse IFN-γ antibody (clone XMG1.2) were purchased from eBioscience. APC-labeled anti-mouse CD8a antibody (clone 53-6.7), APC-labeled anti-mouse CD4 antibody (clone RM4-5), PerCP-Cy5.5-labeled anti-mouse CD90.1 antibody (clone OX-7), Pacific Blue-labeled anti-mouse CD90.2 antibody (clone 30-H12), FITC-labeled anti-mouse CD8 antibody (clone 53-6.7), Brilliant Violet 510-labeled anti-mouse CD90.1 antibody (clone OX-7), Pacific Blue-labeled anti-mouse CD44 (clone IM7), and purified CD16/32 antibody (clone 93) were purchased from BioLegend. PE-labeled anti-mouse CD62L antibody (clone MEL-14) was purchased from BD Biosciences. APC-labeled H-2Db gp100 Tetramer for tetramer staining and Clear Back were purchased from MBL.

- Immune Checkpoint Inhibitors (ICIs)

An anti-mouse PD-1 antibody (clone RMPI-14) was purchased from BioCell. An anti-mouse CTLA-4 antibody (clone 9D9) was produced at Mie University using hybridoma provided by the MD Anderson Cancer Center.

- Short-Chain Peptide Antigen

A synthetic short-chain peptide for CD8-positive T-cell stimulation (QYIHSANVL (SEQ ID NO: 1) was purchased from Sigma Genosys. EGSRNQDWL (SEQ ID NO: 4) and VYDGREHTV (SEQ ID NO: 9) were purchased from Eurofins.

Example 6. Tumor Growth Test (Metastatic Melanoma Cell Line B16F10)

Using a T75 culture flask (Corning), the metastatic melanoma cell line B16F10 was cultured in 10% FBS-containing D-MEM medium. The cultured cells were removed with 0.5% trypsin-containing PBS and suspended in 10% FBS-containing D-MEM medium. After the suspension was centrifuged (400×g, 5 minutes, 4°C), the supernatant was removed. Then, the cells were washed twice with D-MEM medium and suspended in D-MEM medium at a concentration of $2\times10^5$ cells/100 μL. The cells were inoculated subcutaneously into the right anterodorsal part of the C57BL/6 mice (5 mice per group) at a dose of 100 μL per mouse.

When an HA nanogel cancer vaccine loaded with a long-chain peptide antigen was administered, 50 μg (75 μg on day 15 only) of the HA nanogel cancer vaccine loaded with a long-chain peptide antigen was administered subcutaneously into the right posterodorsal part of the mice together with 50 μg of CpG oligo DNA dissolved in PBS 7,

11, and 15 days after the tumor inoculation (HANG-V group).

When antigen-specific T cells were administered, CD8-positive T cells were isolated from the spleen of gp100 recognition TCR gene introduced transgenic mice Pmel-1 (CD90.1 positive) using CD8a microbeads (Miltenyi) 8 and 12 days after the tumor inoculation, and suspended in RPMI 1640 medium at a concentration of $2\times10^6$ cells/200 μL. Then, 200 μL of the suspension was infused from the mouse tail vein (TCR-T group). Then, the tumor area was measured over time.

Example 6: Results

As in Example 1 (ICI-resistant fibrosarcoma cell line CMS5a), to clarify the antitumor effects of the combined use of the HA nanogel cancer vaccine and antigen-specific adoptive T-cell therapy on metastatic malignant melanoma, using metastatic melanoma cell line B16F10 tumor-bearing C57BL/6 mice and producing an HA nanogel cancer vaccine loaded with a long-chain peptide antigen by compounding an HA nanogel and a long-chain peptide antigen containing CD8 epitope of gp100 tumor antigen overexpressed in B16F10, the therapeutic effect of the combined use of the vaccine with gp100 recognition TCR gene introduction adoptive T-cell therapy was examined. As a result, the growth inhibition of B16F10 tumor was observed in the HANG-V treatment group, compared to the untreated group and the TCR-T treatment group (Figs. 6-1 and 6-2). In particular, in the combined HANG-V and TCR-T treatment group, antitumor effects were demonstrated even for B16F10 tumors that had once reached a size of about 100 $mm^2$, with significant tumor regression in all individual mice (Fig. 6-2). Complete tumor regression was observed in two of them. In both the HANG-V treatment group and the combined HANG-V and TCR-T treatment group, a crater-like cavity (tumor necrosis) was observed in the center of the tumor in all mice, which is considered to be caused by the tumor-cytotoxic effect of cytotoxic T cells (CTLs) (Fig. 6-3). Furthermore, in the untreated group and the TCR-T treatment group, all mice died within 25 days of tumor inoculation, whereas 100% of mice survived in the combined HANG-V and TCR-T treatment group, and 80% of mice survived in the HANG-V group (Fig. 6-4). This effect lasted for a long period of time, and the two mice with complete tumor regression remained alive without recurrence even after 5 months or more after the tumor inoculation.

The above results suggested that the combined use of the HA nanogel cancer vaccine and antigen-specific adoptive T-cell therapy exhibits strong antitumor effects against not only immune checkpoint inhibitors (ICIs) treatment-resistant tumors, but also malignant melanoma with metastasis and poor prognosis, such as B16F10, can cure tumors, and can be a revolutionary treatment for "cold tumors" and "immune-desert tumors."

Example 7. Tumor Growth Test (Comparison between HA Nanogel Vaccine and CHP Nanogel Vaccine)

For the mouse tumor growth test (Example 6), a nanogel cancer vaccine loaded with a long-chain peptide antigen (HANG-V or CHP-V) was used in combination with antigen-specific adoptive T-cell therapy to perform the same test. 50 μg (75 μg after the third dose) of the nanogel cancer vaccine loaded with a long-chain peptide antigen, for both HANG-V and CHP-V, was administered subcutaneously into the right posterodorsal part of the mice 7, 11, 15, 29, and 36 days after the tumor inoculation together with 50 μg of CpG oligo DNA dissolved in PBS, and antigen-specific T cells ($4\times10^6$ cells) were infused.

Example 7: Results

In order to clarify whether the combined use of the HA nanogel cancer vaccine and antigen-specific adoptive T-cell therapy is superior in antitumor effects to the CHP nanogel cancer vaccine and antigen-specific adoptive T-cell therapy (prior art), a tumor growth test was performed to compare their therapeutic effects. As a result, as in Example 6, in the combined HANG-V and TCR-T treatment group, the growth of B16F10 tumor was significantly inhibited in all mice, and the tumor strongly regressed, whereas in the combined CHP-V and TCR-T treatment group, similar therapeutic effects were not observed (Figs. 7-1 and 7-2). Furthermore, in the combined HANG-V and TCR-T treatment group, compared to the combined CHP-V and TCR-T treatment group, not only tumor size (area) but also upward progression and growth (height) were significantly inhibited, and a crater-like cavity (tumor necrosis) was observed in the center of the tumor in all mice, which is considered to be caused by the tumor-cytotoxic effect of CTLs (Fig. 7-3). Then, two out of all mice with regression showed a complete regression of tumors with the tumors becoming scabrous, and the tumors of the other three mice also regressed to such an extent that the tumors were not visible without re-aggravation. On the other hand, in the combined CHP-V and TCR-T treatment group, tumor growth continued after that without being inhibited, and all mice died within 50 days, whereas in the combined HANG-V and TCR-T treatment group, 100% survival rate was maintained (Fig. 7-4).

The above results revealed that the combined use of the HA nanogel cancer vaccine and antigen-specific adoptive T-cell therapy had superior therapeutic effects in terms of all of tumor growth inhibition, tumor-cytotoxic effect, and survival prolongation, compared to the prior art, i.e., the CHP nanogel cancer vaccine and adoptive T-cell combination

therapy.

Example 8. Analysis of gp100 Antigen-specific CD8 T Cells in the Draining Lymph Nodes at the Vaccine Administration Site

A test similar to Example 6 was conducted, draining lymph nodes (right inguinal lymph nodes) at the vaccine administration site were collected 14 days after the tumor inoculation (2 or 3 mice per group), the lymph nodes were ground with a glass slide, and the cells were then suspended in RPMI 1640 medium. After centrifugation (400×g, 5 minutes, 4°C), the supernatant was removed, and the cells were washed twice with RPMI 1640 medium and then suspended in 10% FBS-containing RPMI 1640 medium. The number of cells was counted (Fig. 8-1), and the suspension was adjusted to a cell concentration of $2\times10^6$ cells/mL.

Staining of gp100 tetramer cells (Figs. 8-2 and 8-3): The cell suspension was added to a 96-well V-bottom microplate (Nunc) at $4\times10^5$ cells/200 μL per well. The cell suspension was centrifuged (2000 rpm, 2 minutes, 4°C), and the supernatant was removed. After washing twice using 200 μL of staining buffer (0.5% FBS-containing PBS), the cells were suspended, and the cell suspension was added at 50 μL per well.

Clear Back (MBL) was added to the cell suspension each at 10 μL/well, and after incubation for 10 minutes, H-2Db gp100 Tetramer (MBL) was added each at 10 μL/well and reacted for 30 minutes in the dark. Next, FITC-labeled anti-mouse CD8 antibody, Brilliant Violet 510-labeled anti-mouse CD90.1 antibody, and Pacific Blue-labeled anti-mouse CD44 or eFlow450-labeled anti-mouse KLRG1 antibody were added and mixed so that the final concentration was as recommended by the manufacturer of each antibody, and the mixture was then incubated in the dark at 4°C for 15 minutes. 150 μL of staining buffer was added, and after centrifugation to remove the supernatant, the cells were further washed twice with 200 μL of staining buffer. After centrifugation to remove the supernatant, the cells were resuspended in 200 μL of staining buffer and transferred to a round-bottom polystyrene tube (BD Biosciences). The cells were analyzed on a FACS Canto II flow cytometer (produced by BD Biosciences) using analysis software (FACSDiva). gp100 antigen-specific CD8-positive infused T cells were detected as a gp100 tetramer-positive CD90.1-positive CD8-positive population.

Antigen-specific T-cell induction test (intracellular cytokine staining) (Fig. 8-4): The cell suspension was added to a 48-well culture plate (Nunc) at $4\times10^5$ cells/200 μL per well. As a short-chain peptide for stimulating CD8-positive T cells, 50 μL of gp100 peptide was added to a final concentration of 10 μM, and the cells were cultured in a 37°C, 5% $CO_2$ incubator for 1 hour. Then, Goldi Plug (BD Biosciences) diluted 167-fold with 10% FBS-containing RPMI 1640 medium was added at 50 μL per well, and the cells were cultured in a 37°C, 5% $CO_2$ incubator for 5 hours. The cells were collected, transferred to a 96-well V-bottom microplate (Nunc), centrifuged (2000×rpm, 2 minutes, 4°C) to remove the supernatant, and then suspended in a staining buffer (200 μL per well). After the cells were washed twice with 200 pL of staining buffer, FITC-labeled anti-CD8 antibody and Brilliant Violet 510-labeled anti-mouse CD90.1 antibody were added and mixed with the cell suspension according to the use concentration recommended by the manufacturer of each antibody, and the mixture was then incubated in the dark at 4°C for 15 minutes. 150 pL of staining buffer was added, and after centrifugation to remove the supernatant, the cells were further washed twice with 200 μL of staining buffer, and 100 μL of Cytofix/Cytoperm buffer (BD Biosciences) was then added and gently mixed. After incubation in the dark at room temperature for 20 minutes, the cells were washed twice with a staining buffer, suspended in 200 μL of staining buffer, and then stored in a refrigerator protected from light overnight. After centrifugation to remove the supernatant, the cells were washed with 200 μL of Perm/Wash buffer (BD Biosciences), APC-labeled anti-mouse IFN-γ antibody diluted 50-fold with Perm/Wash buffer was added and mixed with the cells, and the mixture was incubated in the dark at room temperature for 15 minutes. The cells were washed twice with 200 μL of Perm/Wash buffer, then resuspended in 200 μL of staining buffer, and transferred to a round-bottom polystyrene tube (BD Biosciences). The cells were analyzed on a FACS Canto II flow cytometer (produced by BD Biosciences) using analysis software (FACSDiva). IFN-γ-producing CD8-positive infused T cells were detected as an IFN-γ-positive CD90.1-positive CD8-positive population.

Example 8: Results

In order to clarify the effect of an HA nanogel cancer vaccine on gp100 antigen-specific CD8-positive T cells in the combined use of the HA nanogel cancer vaccine and antigen-specific adoptive T-cell therapy, the draining lymph nodes were collected 14 days after the tumor inoculation, and the CD8 T cells were analyzed. As a result, the lymph nodes in the HANG-V treatment group and the combined HANG-V and TCR-T treatment group were significantly enlarged, and lymph node cells in both groups increased approximately 9 to 16 times compared to the untreated group (Fig. 8-1). In addition, as a result of the analysis of the percentage of gp100 antigen-specific tetramer-positive CD8 T cells that flowed into and accumulated in the draining lymph nodes, it was revealed that a significant increase in the CD8 T cells was observed in both groups compared to the HANG-V untreated group (Fig. 8-2), and that these cells exhibited highly

activated effector T-cell phenotypes (KLRG1, CD44) (Fig. 8-3). Furthermore, it was revealed that in the combined HANG-V and TCR-T treatment group, IFN-γ was significantly produced by CD8-positive infused T cells specifically for gp100 antigen (Fig. 8-4) .

The above results suggested that the HA nanogel cancer vaccine strongly induces CD8-positive infused T cells to gp100 antigen-specific CTLs in the draining lymph nodes in the immunosuppressive environment of tumor-bearing host mice, resulted in regress B16F10 tumor.

Example 9. Examination of Tumor Local site and Systemic Localization of Antigen-Specific CD8-Positive Infused T Cells

After a test similar to Example 6 was conducted, the draining lymph nodes at the vaccine administration site (right inguinal lymph node), non- draining lymph nodes (left inguinal lymph nodes), spleen, tumor, and peripheral blood were collected 19 days after the tumor inoculation, each cell was separated by the method shown below, and the percentage of CD8-positive infused T cells and gp100 tetramer-positive CD8-positive T cells present in each tissue was examined.

Separation of lymph node cells and spleen cells: After the lymph nodes (draining and non-draining) and spleen were ground with a glass slide, cells were collected in RPMI 1640 medium. After centrifugation (400×g, 5 minutes, 4°C), the supernatant was removed. For the spleen cells, 2 mL of red blood cell hemolysis solution was added to treat the cells for 1 minute. Then, 18 mL of RPMI 1640 medium was added, and after centrifugation (400×g, 5 minutes, 4°C) to remove the supernatant, the cells were suspended in RPMI 1640 medium (primary cell suspension).

Separation of tumor-infiltrating immune cells (TILs): Tumor-infiltrating immune cells were suspended in RPMI 1640 medium after the tumor tissue was ground with a homogenator. Collagenase D (final concentration: 2 mg/mL, Roche) was added to the suspended cells and reacted at 37°C for 30 minutes. Then, while adding RPMI 1640 medium, the cell suspension was passed through a filtration filter (pore size: 0.22 um). After centrifugation (400×g, 5 minutes, 4°C), the supernatant was removed, and the cells were suspended in RPMI 1640 medium (primary cell suspension).

Separation of peripheral blood mononuclear cells (PBMCs): Peripheral blood mononuclear cells were collected in the same manner as in Example 5. A primary cell suspension of each of the collected tissues was subjected to staining and measurement in the same manner as in Example 7. CD8-positive infused T cells were detected as a CD90.1-positive CD8-positive population, and gp100 antigen-specific CD8-positive T cells were detected as a gp100 tetramer-positive CD8-positive population.

Example 9: Results

In order to clarify the persistence of survival of antigen-specific CD8-positive infused T cells in tumor local site and systemic areas after the HA nanogel cancer vaccine and antigen-specific T-cell infusion, secondary lymph tissues, tumor tissues, and peripheral blood were collected 19 days after the tumor inoculation, and the percentage of the T cells was examined. As a result, it was revealed that the infused T cells were significantly present in other secondary lymph tissues (spleen and non-regional lymph nodes), tumor tissues, and peripheral blood throughout the body, in addition to the draining lymph nodes (Fig. 9-1). Furthermore, the gp100 antigen-specific CD8 T cells were also significantly present widely in each tissue (Fig. 9-2), suggesting that due to the antigen-specific CD8 T cells localized throughout the body, this treatment inhibits not only local tumor growth, but also systemic metastasis.

Example 10. Recurrence-Inhibitory Effect after Treatment

After Example 6 was conducted, peripheral blood was collected from two mice with complete tumor regression (5 months after the tumor inoculation), and the percentage of gp100 antigen-specific CD8-positive infused T cells was examined. Then, B16F10 tumor ($2\times10^5$ cells) was re-inoculated to one out of the two mice. For the other mice, after subcutaneous administration of 75 μg of the HA nanogel cancer vaccine loaded with a long-chain peptide antigen into the right posterodorsal part of the mouse 3 times in total (intervals of 3 to 4 days) together with 50 μg of CpG oligo DNA dissolved in PBS, peripheral blood was collected again, and changes in the percentage of gp100 antigen-specific CD8-positive infused T cells, activation and phenotype of these cells were examined. Furthermore, this mouse was re-inoculated with B16F10 tumor ($2\times10^5$ cells), and the presence or absence of recurrence was examined. Peripheral blood mononuclear cells (PBMCs) were collected in the same manner as in Example 5, and tetramer staining was performed in the same manner as in Example 8. Then, the cells were stained and measured by the following method.

According to the use concentration recommended by the manufacturer of each antibody, 50 μL of a solution containing FITC-labeled anti-mouse CD8 antibody, Brilliant Violet 510-labeled anti-mouse CD90.1 antibody, and Pacific Blue-labeled anti-mouse CD44 and PE-labeled anti-mouse CD62L antibody was added and mixed, and incubated in the dark at 4°C for 15 minutes. 150 μL of staining buffer was added, and after centrifugation to remove the supernatant, the cells were further washed twice with 200 μL of staining buffer. After centrifugation to remove the supernatant, the

cells were resuspended in 200 μL of staining buffer and transferred to a round-bottom polystyrene tube (BD Biosciences). The cells were analyzed on a FACS Canto II flow cytometer (produced by BD Biosciences) using analysis software (FACSDiva). gp100 antigen-specific CD8-positive infused T cells were detected as a gp100 tetramer-positive CD90.1-positive CD8-positive population, effector memory cells were detected as a CD44-positive CD62L-negative population, and central memory cells were detected as a CD44-positive CD62L-positive population.

Example 10: Results

As a result of treating the HA nanogel cancer vaccine and antigen-specific adoptive T-cell therapy, and measuring gp100 antigen-specific CD8-positive infused T cells in the peripheral blood of mice without recurrence after complete tumor regression, it was revealed that the CD8-positive infused T cells were present in the peripheral blood even after 5 months of remission (Fig. 10-1). Furthermore, after these mice were re-inoculated with B16F10 tumor or re-vaccinated, the percentage of gp100 antigen-specific CD8-positive infused T cells in the peripheral blood was measured again. As a result, a significant increase in these cells was observed in the peripheral blood (Fig. 10-2). These cells exhibited effector memory T-cell (CD44-positive CD62L-negative) and central memory T-cell (CD44-positive CD62L-positive) phenotypes (Fig. 10-3). In particular, it became clear that these cells including a CD44 strongly positive effector cells proliferated explosively in re-vaccinated mice (Fig. 10-4). In addition, even when these mice were re-inoculated with B16F10, no tumor engraftment or growth was observed, indicating no tumor recurrence in these mice (Fig. 10-5).

The above results revealed that by treating the HA nanogel cancer vaccine and antigen-specific adoptive T-cell therapy, a certain amount of antigen-specific CD8-positive infused T cells remained in the peripheral blood of mice who responded successfully to treatment, for a long time even after tumor regression, and that when the tumor recurred, the memory cells quickly grew, recognized the tumor antigen, exhibited antitumor effects, and inhibited the recurrence. It was also revealed that periodic vaccination after successful treatment can induce highly activated antigen-specific effector/memory T cells and prevent tumor recurrence.

By being used in combination with antigen-specific adoptive T-cell therapy, this vaccine is expected to exhibit strong antitumor effects against malignant melanoma with metastasis and poor prognosis, such as B16F10, also have the effect of preventing tumor recurrence, and be able to be an innovative cancer vaccine with both therapeutic and preventive aspects.

Example 11. Therapeutic Effects of Vaccine etc. on Individuals with Regrowth after Successful Effects

After a test similar to the mouse tumor growth test (Example 1) was conducted (9 mice per treatment group), for the mouse with tumor regrowth (1 out of 9 mice), 75 μg of the HA nanogel cancer vaccine loaded with a long-chain peptide antigen was administered subcutaneously into the right posterodorsal part of the mice together with 50 μg of CpG oligo DNA dissolved in PBS 27, 32, 36, 40, and 43 days after the tumor inoculation. Furthermore, antigen-specific CD8-positive T cells ($2\times10^6$ cells) isolated from the spleen of mutated ERK2 recognition TCR transgenic mice (DUC18) using CD8a microbeads (Miltenyi) were injected into the mouse tail vein 41 days after the tumor inoculation. In addition, 49 days after the tumor inoculation, ICIs (200 μg of anti-mouse PD-1 antibody and 100 μg of anti-mouse CTLA-4 antibody) was administered intraperitoneally, and the tumor area was measured over time.

Example 11: Results

Vaccine re-administration, Re-infusion of antigen-specific CD8-positive T cells, and ICIs were treated for the mouse who had been successful effects after treating with the HA nanogel cancer vaccine and antigen-specific adoptive T-cell therapy, but whose tumor then grew again. As a result, in general, relapsed tumors have a rapid growth rate and a poor prognosis (figure on right in Fig. 11-2) however, it was revealed that repeated administration of the vaccine can significantly inhibit tumor growth, and that the combination with other immunotherapy (ICI) can inhibit tumor progression (Fig. 11-1 and figure on left in Fig. 11-2).

From the above results, even if the tumor re-grows even after successful effects treated with the HA nanogel cancer vaccine and antigen-specific adoptive T-cell therapy, re-administration of the vaccine, reinfusion, and additional ICI therapy can significantly inhibit tumor growth and may be an effective treatment for tumor regression. In this test, hyaluronic acid nanogels with an introduction ratio of cholesteryl groups of 44 or 41% were used, and both showed high antitumor effects.

Example 12. Recurrence-Inhibitory Effect on Individuals with Complete Response (Complete Regression) after Treatment

After Example 11 was conducted, mice with complete tumor regression (n=8) were re-inoculated with CMS5a tumor ($1\times10^6$ cells) two months after the tumor inoculation, and the tumor area was measured.

Example 12: Results

Mice with complete tumor regression after treating the HA nanogel cancer vaccine and antigen-specific adoptive T-cell therapy were re-inoculated with CMS5a tumor. As a result, tumor growth was inhibited in all mice, and 6 out of 8 mice achieved complete tumor regression (75%) (Figs. 12-1 and 12-2).

From the above results, this treatment can cure ICI treatment-resistant tumors, and as in the results of Example 10, memory immunity against the tumor was acquired (memory formation) after the treatment was successful. It is considered that when the tumor recurs, antigen-specific memory T cells quickly recognize and attack them, thereby inhibiting tumor recurrence. In addition, as in Example 10, re-vaccination is expected to induce antigen-specific CD8 memory T cells having activated strong effector function and further prevent recurrence.

Example 13. Combination Effect of Vaccine and Antigen-specific adoptive T-cell therapy (Single infusion)

For the mouse tumor growth test (Example 1), a similar test was conducted by reducing the frequency of antigen-specific adoptive T-cell therapy from two times to one time (9 mice per treatment group).

Example 13: Results

In order to clarify the effect of the frequency of infusions of CD8-positive T cells on antitumor effects in the combined use of the HA nanogel cancer vaccine and antigen-specific adoptive T-cell therapy, the frequency of infusions of CD8-positive T cells was gradually reduced from two times in the conventional method to one time, and the therapeutic effects were examined. As a result, even in the case of a single infusion of CD8-positive T cells, tumor regression was observed in all mice, as in the case of twice infusion in the conventional method and the prior art, and the tumor completely disappeared in 8 out of 9 mice (response rate: 100%, complete response: 89%, partial response: 11%) (Figs. 13-1 and 13-2).

The above results revealed that the HA nanogel cancer vaccine can cure ICI-resistant tumors strongly enough with a single infusion of antigen-specific T cells without twice infusion. Currently, the physical and financial burden on patients undergoing adoptive T-cell therapy (TCR-T and CAR-T) is a problem and needs to be improved. A regimen of cell infusion (single dose) followed by this vaccine could cure ICI-resistant tumors and be a less physically and financially burdensome treatment for patients.

Example 14. Therapeutic Effects of Vaccine etc. on Individuals with Regrowth after Successful Effects by Treatment

After Example 13 was conducted, for the mouse with tumor regrowth (1 out of 9 mice), 75 μg of the HA nanogel cancer vaccine loaded with a long-chain peptide antigen was administered subcutaneously into the right posterodorsal part of the mice together with 50 μg of CpG oligo DNA dissolved in PBS 26, 30, 33, and 36 days after the tumor inoculation. Furthermore, 33, 36, and 39 days after the tumor inoculation, ICIs (200 μg of anti-mouse PD-1 antibody and 100 μg of anti-mouse CTLA-4 antibody) was administered intraperitoneally, and the tumor area was measured over time.

Example 14: Results

The vaccine and ICIs were administered to the mouse who had been successful effects after treating with the HA nanogel cancer vaccine and antigen-specific adoptive T-cell therapy (single infusion), but whose tumor then grew again. As a result, in general, relapsed tumors have a rapid growth rate and a poor prognosis however it was revealed that tumor growth can be significantly inhibited by repeated administration of the vaccine alone or in combination with ICIs (Fig. 14).

From the above results, even if the tumor re-grows even after successful effects after treating with the HA nanogel cancer vaccine and antigen-specific adoptive T-cell therapy, repeated administration of the vaccine, reinfusion, and additional ICI therapy can significantly inhibit tumor growth and may be an effective treatment for tumor regression.

Example 15. Memory T-Cell-Inducing Effect of Re-vaccination on Individuals with Complete Response (Complete Regression) after Treatment

After Example 13 was conducted, the peripheral blood of mice with complete tumor regression (n=8) was collected in the same manner as in Example 5 (36 days after the tumor inoculation), and the memory phenotype of CD8-positive T cells was analyzed. Furthermore, 4 out of 8 mice, 75 μg of the HA nanogel cancer vaccine loaded with a long-chain peptide antigen was administered subcutaneously into the right posterodorsal part of the mice (36 and 39 days after the tumor inoculation) together with 50 μg of CpG oligo DNA dissolved in PBS, and the memory phenotype of the

cells was reanalyzed (41 days after the tumor inoculation). Cell staining and measurements were performed in the same manner as in Example 10.

Example 15: Results

CD8-positive T cells in the peripheral blood of mice with complete tumor regression after treating the HA nanogel cancer vaccine and antigen-specific adoptive T-cell therapy (single infusion) was analyzed. As a result, mice included CD8-positive T cells expressing effector memory and central memory phenotypes, revealing that re-vaccination significantly increased the percentage of both cells (Fig. 15).

The above results suggested that even after successful effects after treating with the HA nanogel cancer vaccine and antigen-specific adoptive T-cell therapy (single infusion), re-vaccination can significantly increase effector memory and central memory CD8-positive T cells, and can sustainably induce memory T cells.

Example 16. Memory T-Cell-Inducing Effect of Re-vaccination on Individuals (under Additional treatment) from Response to Regrowth after primary treatment

The peripheral blood of the mouse undergoing additional treatment by Example 14 was collected in the same manner as in Example 5 (36 days after the tumor inoculation), and the memory phenotype of CD8-positive T cells was analyzed. Furthermore, to the same mice, 75 μg of the HA nanogel cancer vaccine loaded with a long-chain peptide antigen was administered subcutaneously into the right posterodorsal part of the mice (same day after blood collection) together with 50 μg of CpG oligo DNA dissolved in PBS, and the memory phenotype of the cells was reanalyzed (41 days after the tumor inoculation). Cell staining and measurements were performed in the same manner as in Example 10.

Example 16: Results

CD8-positive T cells in the peripheral blood of the mouse who were once successful effects after treating with the HA nanogel cancer vaccine and antigen-specific adoptive T-cell therapy (single dose), but whose tumor then grew again (under additional treatment) were analyzed. As a result, as in the mouse with complete regression, the mouse undergoing additional treatment also included CD8-positive T cells expressing effector memory and central memory phenotypes, revealing that re-vaccination significantly increased the percentage of both cells (Fig. 16).

The above results suggested that even if the tumor re-grows after successful effects after treating with the HA nanogel cancer vaccine and antigen-specific adoptive T-cell therapy (single dose), re-vaccination can significantly increase and induce effector memory and central memory CD8-positive T cells, and can sustainably induce memory T cells and therapeutic effects.

Discussion of Examples 6 to 16

The results of Examples 6 to 16 particularly revealed or suggested the following eight points.

1. This treatment exhibits strong therapeutic effects on malignant melanoma, which is difficult to treat and has characteristics such as high metastasis, high lethality, rapid lethality, low immunogenicity, and difficulty in infiltrating tumors with T cells (cold tumors and immune-desert tumors) (Example 6).
2. For the difficult-to-treat tumors mentioned above, the prior art CHP vaccine cannot inhibit tumor growth or mortality, whereas this treatment exhibits strong antitumor effects to suppresses tumors and maintain survival (Example 7).
3. This treatment can significantly increase antigen-specific CD8 T cells in the draining lymph nodes at the vaccine administration site, and induce highly activated antigen-specific cytotoxic CD8 T cells (CTLs) (Example 8).
4. The infused antigen-specific CD8 T cells infiltrate well into tumors and are also localized prominently throughout the body, potentially inhibiting tumor metastasis (Example 9).
5. In addition to its strong therapeutic effects, this treatment can prevent recurrence after successful therapeutic effects, and has aspects of both therapeutic cancer vaccines and recurrence prevention vaccines (Examples 10 and 12).
6. Furthermore, this treatment can rapidly induce memory T cells in a strong and sustainable manner by re-administration of the vaccine after successful therapeutic effects or during additional treatment (Examples 10, 15, and 16).
7. In addition, even if tumor re-growth is observed after successful therapeutic effects, tumor growth can be inhibited by repeated administration of the vaccine alone or in combination with other immunotherapies (Examples 11 and 14).
8. Compared to conventional methods and the prior art (infusion therapy twice), this treatment exhibits significant therapeutic effects even in a single infusion, and can reduce the physical and financial burden on patients (Example 13).

Example 17. Antitumor Test Using a Chimeric Antigen Receptor-Expressing Lymphocyte, and Dosing Composition Containing Hyaluronic Acid Derivative and Antigen

From the test results of dosing compositions containing TCR-T, a hyaluronic acid derivative, and antigen in Examples 1 to 16, it is considered that antigen-presenting cells that uptake the hyaluronic acid derivative and antigen efficiently present the antigen on the cell surface, and that one of the mechanisms of the high antitumor effects is the activation and proliferation of T cells infused with the antigen-presenting cells. Report 1 (Blood (2018) 132 (11): 1134-1145, Antitumor activity of CAR-T cells targeting the intracellular oncoprotein WT1 can be enhanced by vaccination) has reported that antitumor effects are enhanced by infusing lymphocytes that express a chimeric antigen receptor, and antigen-presenting cells that have taken up the antigen. Report 2 (Science. 2020 Jan 24; 367(6476):446-453, An RNA vaccine drives expansion and efficacy of claudin-CAR-T cells against solid tumors) has reported that antitumor effects are enhanced by infusing lymphocytes that express a chimeric antigen receptor, and expressing the antigen recognized by the chimeric antigen receptor in antigen-presenting cells. Therefore, when not only TCR-T, but also chimeric antigen receptor-expressing lymphocytes are infused, similarly high antitumor effects can be expected by administering a dosing composition containing a hyaluronic acid derivative and antigen, which produces antigen-presenting cells that have taken up the antigen in the body.

By using the chimeric antigen receptor-expressing lymphocytes shown below, tumors expressing antigens that can be recognized by the chimeric antigen receptor, and HLA transgenic mice, it is possible to prove that a dosing composition containing the lymphocytes expressing the chimeric antigen receptor, a hyaluronic acid derivative, and the antigen has a high antitumor effect.

Materials and Methods

(1) Cells

Mouse colon cancer cell lines MC38 and CT26 were purchased from ATCC and passaged at Mie University for use. Retrovirus packaging cell line Plat-E was purchased from Cell Biolabs. RPMI 1640 medium was purchased from Cell Science & Technology Institute, Inc. High-glucose D-MEM medium was purchased from FUJIFILM Wako Pure Chemical Corporation. Opti-MEM and fetal bovine serum (FBS) were purchased from Gibco. Albuminar was purchased from CSL Behling, and human IL-2 was purchased from Novartis.

(2) Test Animals

HLA-A2 transgenic (Tg) mice were established by injecting HHD-A2 plasmid DNA provided by the Institut Pasteur into fertilized eggs of C57BL/6 mice at the Animal Experiment Facility of Mie University. These mice were bred at the Animal Experiment Facility of Mie University. Furthermore, HLA-A2 Tg mice backcrossed to BALB/c mice (BALB/c background) were established and bred, and these mice were raised at the Animal Experiment Facility of Mie University, and used in the experiments.

(3) Antibodies

Anti-mouse CD3e antibody (clone 145-2C11), anti-mouse CD28 antibody (clone 37.51), and Alexa Fluor 647-labeled anti-rabbit IgG antibody (clone Poly4064) were purchased from Invitrogen. APC-labeled anti-mouse CD4 antibody (clone RM4-5) and APC/Cyanine7-labeled anti-mouse CD8 antibody (clone 53-6.7) were purchased from BioLegend. MAGE-A4 (E7O1U) XP Rabbit mAb was purchased from Cell Signaling Technology. PE-labeled anti-human HLA-A2 antibody (clone BB7.2) was purchased from MBL. Purified Rat Anti-Mouse IFN-γ antibody and Biotin Rat Anti-Mouse IFN-γ antibody were purchased from BD Biosciences.

(4) Reagents

Lipofectamine 3000 Reagent, P300 reagent, and human IFN-γ uncoated ELISA kit were purchased from Invitrogen. pCL-ECO Retrovirus Packaging Vector was purchased from NOVUS. A2 MAGE-A4 tetramer was produced by using Streptavidin-R-Phycoerithrin (Agilent). RetroNectin was purchased from Takara Bio Inc.

1. Production of Retroviral Vector Plasmid

1-1. For Chimeric Antigen Receptor

(1) mMAGE#17 zG CAR

MAGE#17scFv used for CAR production was obtained by reacting an artificially produced HLA-A*02:01 MAGE-A4 p230-239 peptide MHC complex with a human antibody library, followed by isolation (Japanese Patent Application No. 2019-523909 etc.: Antigen-Binding Protein Recognizing MAGE-A4-Derived Peptide). Design was made so that a human VH leader sequence was linked to the 5'-side of the scFv, and mCD8 hinge, mCD28 TM, mCD28 ICD, mCD3z, and mGITR ICD were linked to the 3'-side, thereby creating an artificial gene (ordered from Genscript). This gene was introduced into a retroviral expression plasmid to produce mMAGE zG viral vector plasmid (Figs. 17-1a and 17-1b).

(2) mMAGE 28z CAR

Design was made so that a human VH leader sequence was linked to the 5'-side of the scFv, and mCD8 hinge, mCD28 TM, mCD28 ICD, and mCD3z were linked to the to 3'-side, thereby creating an artificial gene (ordered from Genscript). This gene was introduced into a retroviral expression plasmid to produce mMAGE zG viral vector plasmid (Figs. 17-2a and 17-2b).

1-2. For MAGE-A4, HHD-A2-Expressing MC38 Cells and CT26 Cells

(1) mNGFR-Co-expressing MAGE-A4

Design was made so that a human MAGE-A4 gene, a P2A peptide sequence, and C-terminal truncated mouse NGFR gene were linked, thereby creating an artificial gene (ordered from Genscript). This gene was digested with restriction enzymes NotI and XhoI, and introduced into a retroviral expression plasmid (Figs. 17-3a and 17-3b).

(2) HHD-A2

The intron part was deleted from the MHC sequence of HHD-A2 plasmid, and mRNA type HHD-A2 expression gene was designed to create an artificial gene (ordered from Genscript). This gene was digested with NotI-XhoI, and introduced into a retroviral expression plasmid (Figs. 17-4a and 17-4b).

2. Production of Chimeric Antigen Receptor-Expressing Lymphocyte

2-1. Production of Retrovirus

The retrovirus packaging cell line Plat-E ($1.2\times10^6$ cells) was cultured in a 6-well plate for 24 hours using high-glucose D-MEM medium, and mixed with Opti-MEM (250 μL) and Lipofectamine 3000 Reagent (7 μL) (liquid A). Furthermore, Opti-MEM (250 μL), the mMAGE#17 zG CAR viral vector plasmid (3 pL) prepared in 1-1 (1), pCL-ECO Retrovirus Packaging Vector (1 μL), and P300 reagent (6 μL) were mixed, and after mixing with the liquid A, the mixture was incubated for 15 minutes at room temperature. Half of the medium was removed from the 6-well plate, and the mixed liquid was added, followed by culturing in a 37°C, 5% $CO_2$ incubator for 6 hours. Then, the whole medium in the 6-well plate was exchanged, and the medium was collected after 24 hours, 48 hours, and 72 hours, sterilized by filtration with a 0.45-um filter, and stored frozen.

2-2. Gene Introduction into Mouse Spleen Cells by Retrovirus

The spleen was collected from BALB/c mice and ground with a glass slide, and the cells were then collected in RPMI 1640 medium. After centrifugation (400×g, 5 minutes, 4°C), the supernatant was removed, and 2 mL of red blood cell hemolysis solution was added to treat the cells for 30 seconds. Then, 18 mL of RPMI 1640 medium was added, and after centrifugation (400×g, 5 minutes, 4°C), the supernatant was removed, and the cells were suspended at a concentration of $3\times10^6$ cells/mL in 10% FBS-containing RPMI 1640 medium. Anti-mouse CD3e antibody (1 μg/mL) and anti-mouse CD28 antibody (2.5 ug/mL) were added to a 6-well plate in advance the previous day, and incubated at 4°C overnight to form a solid phase. After the plate was washed three times with PBS, 5 mL ($1.5\times10^7$ cells) of the cell suspension was seeded and cultured in a 37°C, 5% $CO_2$ incubator for 24 hours.

On the day before gene introduction, RetroNectin was adjusted to 20 ug/mL with PBS, and 500 μL was added

to each well of a 24-well plate (Non-Treated Multidishes, Nunc) and incubated at 4°C overnight. On the next day, after washing three times with PBS, the virus liquid produced in 2-1 was diluted 2-fold, and 1 mL of the liquid was added to the plate, followed by centrifugation (2000×g, 2 hours, 32°C). After the plate was washed twice with 1.5% Albuminar-containing PBS, mouse spleen cells were collected from the $CO_2$ incubator and suspended at a concentration of $3.3\times10^5$ cells/mL in 10% FBS-containing RPMI 1640 containing human IL-2 (600 IU/μL), and 1.5 mL of the suspension was seeded on the plate. After centrifugation (1000×g, 10 minutes, 32°C), the cells were cultured in a 37°C, 5% $CO_2$ incubator for 5 days.

2-3. Confirmation of Chimeric Antigen Receptor-Expressing Lymphocyte

The cells were collected, transferred to a 96-well V-bottom microplate (Nunc), centrifuged (2000×rpm, 2 minutes, 4°C) to remove the supernatant, and then suspended in a staining buffer (0.5% BSA-containing PBS) (200 μL per well). After the cells were washed twice with 200 μL of staining buffer, 30 μL of A2 MAGE-A4 tetramer diluted 100-fold was added, and the mixture was incubated in the dark for 30 minutes. After the cells were washed twice with a staining buffer, APC-labeled anti-mouse CD4 antibody and APC/Cyanine7-labeled anti-mouse CD8 antibody were added and mixed with the cell suspension according to the use concentration recommended by the manufacturer of each antibody, and the mixture was then incubated in the dark at 4°C for 15 minutes. 150 μL of staining buffer was added, and after centrifugation to remove the supernatant, the cells were further washed twice with 200 μL of staining buffer, then resuspended in 200 μL of buffer, and transferred to a round-bottom polystyrene tube (BD Biosciences). The cells were analyzed on an LSRFortessa Cell Analyzer flow cytometer (BD Biosciences) using analysis software (FACSDiva) (Fig. 17-5).

3. Production of Target Cells (MAGE-A4, HHD-A2-Expressing MC38 Cells and CT26 Cells) Expressing Antigen That Can Be Recognized by Chimeric Antigen Receptor, and Confirmation of Recognition and Reactivity

3-1. Production of Retrovirus

A retrovirus was produced in the same manner as described in 2-1, except that the mNGFR-co-expressing MAGE-A4 viral vector plasmid prepared in 1-2 (1) or the HHD-A2 viral vector plasmid prepared in 1-2 (2) was used in place of the mMAGE#17 zG CAR viral vector plasmid prepared in 1-1 (1).

3-2. Preparation of MAGEA4, HHD-A2-Expressing MC-38 Cells and CT26 Cells

Using a T75 culture flask (Corning), mouse colon cancer cell lines MC38 and CT26 were cultured in 10% FBS-containing RPMI 1640 medium. The cultured cells were removed with 0.5% trypsin-containing PBS and suspended in 10% FBS-containing RPMI 1640 medium. After the suspension was centrifuged (400×g, 5 minutes, 4°C), the supernatant was removed. Then, the cells were washed twice with 10% FBS-containing RPMI 1640 medium, and suspended at a concentration of $3.3\times10^5$ cells/mL in 10% FBS-containing RPMI 1640 medium.

On the day before gene introduction, RetroNectin was adjusted to 20 ug/mL with PBS, and 500 μL was added to each well of a 24-well plate (Non-Treated Multidishes, Nunc) and incubated at 4°C overnight. On the next day, after washing three times with PBS, each of the virus liquids produced in 3-1 was diluted 2-fold, and 1 mL was added to the plate, followed by centrifugation (2000×g, 2 hours, 32°C). After the plate was washed twice with 1.5% Albuminar-containing PBS, 1.5 mL of MC38 cells suspended in 10% FBS-containing RPMI 1640 medium ($3.3\times10^5$ cells/mL) were seeded on the plate. After centrifugation (1000×g, 10 minutes, 32°C), the cells were cultured in a 37°C, 5% CO2 incubator for 3 days (same for the CT26 cell line).

Both cells were collected, transferred to a 96-well V-bottom microplate (Nunc), centrifuged (2000×rpm, 2 minutes, 4°C) to remove the supernatant, and then suspended in a staining buffer (200 μL per well). After the cells were washed twice with 200 μL of staining buffer, the staining and expression of HHD-A2 and MAGE-A4 were confirmed by the following method.

HHD-A2: PE-labeled anti-HLA-A2 antibody was added to the cell suspension according to the use concentration recommended by the manufacturer, and the mixture was incubated in the dark at 4°C for 15 minutes. 150 μL of staining buffer was added, and after centrifugation to remove the supernatant, the cells were further washed twice with 200 μL of staining buffer, then resuspended in 200 μL of buffer, and transferred to a round-bottom polystyrene tube (BD Biosciences). The cells were analyzed on a FACS Canto II flow cytometer (produced by BD Biosciences) using analysis software (FACSDiva) (Fig. 17-6).

Intracellular staining of MAGE A4: The cells adjusted to $1\times10^5$ cells were mixed well in 100 μL of a cell fixative (Cytofix Cytoperm: BD Biosciences), and the mixture was incubated in the dark at room temperature for 20 minutes. After washing with Perm/Wash buffer (BD Biosciences) diluted 10-fold with PBS, 0.25 μL of MAGE-A4 (E7O1U) XP

Rabbit mAb was added to 50 μL of Perm/Wash buffer, and the mixture was incubated in the dark at room temperature for 20 minutes. After washing twice with Perm/Wash buffer, 0.25 μL of Alexa Fluor 647-labeled anti-rabbit IgG antibody was added and mixed with 50 μL of Perm/Wash buffer, and the mixture was incubated in the dark at room temperature for 20 minutes. After washing twice with Perm/Wash buffer, the cells were suspended in a staining buffer and transferred to a round-bottom polystyrene tube (BD Biosciences). The cells were analyzed on an LSRFortessa Cell Analyzer flow cytometer (BD Biosciences) using analysis software (FACSDiva) (Fig. 17-6).

3-3. Confirmation of Recognition and Reactivity against Target Cells by mMAGE zG CAR T Cells

The target cells prepared in 3-2 (1. MC38 cells, 2. HHD-A2-intorudced MC38 cells, 3. HHD-A2-intorudced MC38 cells (MAGE-A4 CD8 epitope peptide (SEQ ID NO: 9) 10 μM pulse), 4. HHD-A2 and MAGE-A4-introduced MC38 cells) were suspended at a concentration of $5\times10^4$ cells/100 μl in 10% FBS-containing RPMI 1640 medium, and mMAGE zG CAR T cells ($1\times10^5$ cells/100 ul) or CAR gene-free cells similarly suspended in 10% FBS-containing RPMI 1640 medium were added to the plate. After co-culturing for 24 hours, the culture supernatant was collected. After CT26 cells were similarly co-cultured with mMAGE zG CAR T cells, the culture supernatant was collected. The collected culture supernatants were each evaluated for IFN-γ production by the ELISA method shown below.

IFN-γ ELISA: 50 μL of Purified Rat Anti-Mouse IFN-γ antibody adjusted to 10 ug/mL with 0.05% $NaN_3$ PBS was added to a 96-well flat-bottom plate and incubated overnight at 4°C to form a solid phase. On the next day, after the plate was washed three times with PBS, 50 μL of the collected cell culture supernatant and 50 μL of standard solution were added to each well, and incubated at room temperature for 2 hours. The IFN-γ standard solution used was obtained by adjusting a stock solution to 1000 pg/mL with a standard solution, followed by 5-fold dilution in 4 steps. After washing three times with PBS, 100 μL of Biotin Rat Anti-Mouse IFNg antibody adjusted to 0.5 μL/mL with ELISA buffer obtained by diluting 5-fold 5×ELISA/Elispot Diluent (ELISA kit) with sterile water was added, and the mixture was incubated at room temperature for 1 hour. After washing three times with PBS, 100 μL of Streptavidin-HRP adjusted to 0.5 μL/mL with ELISA buffer was added, and the mixture was incubated at room temperature for 30 minutes. After washing three times with 0.05% PBS-T and twice with PBS, 100 μL of TMB substrate solution was added. After reaction in the dark at room temperature for 2 minutes, 100 μL of 0.18M $H_2SO_4$ was added to stop the reaction. Immediately after that, the absorbance was measured at a wavelength of 450 nm using a Model 680 microplate reader (Bio-Rad) (Fig. 17-7).

4. Preparation of HA Nanogel Cancer Vaccine Loaded with Long-Chain Peptide Antigen

4-1. Production of Complex Using HA Nanogel with a Molecular Weight of 10k and Peptide MAGE-A4 29mer

Hyaluronic Acid Nanogel (HANG)

As described in Examples 1 and 2 of WO2010/053140, a cholesteryl group-introduced hyaluronic acid nanogel (weight average molecular weight: 10 kDa, introduction ratio of cholesteryl groups: 44%) was synthesized.

Long-Chain Peptide Antigen

Using the hyaluronic acid nanogel obtained above, a peptide-hyaluronic acid nanogel complex was prepared as shown below. MAGE-A4 peptide was purchased from Eurofins. The sequence is as follows: AMEGDSASEEEIWEELGVMGVYDGREHTV (SEQ ID NO: 8). This sequence includes GVYDGREHTV (SEQ ID NO: 9) as a CD8-positive cytotoxic T-cell recognition epitope sequence.

Production of Complex of Hyaluronic Acid Nanogel and Antigen

A lyophilized product of the hyaluronic acid nanogel was weighed, water for injection was added to a concentration of 5 mg/mL, and the mixture was stirred overnight to fully dissolve the lyophilized product. On the other hand, in another container, the peptide was dissolved in dimethyl sulfoxide so that the peptide concentration was 50 mg/mL. After confirming dissolution, the hyaluronic acid nanogel aqueous solution and a 1 mol/L sodium hydroxide aqueous solution were mixed at a volume ratio of 100:2 at room temperature. Subsequently, the hyaluronic acid nanogel aqueous solution and the dimethyl sulfoxide solution of the peptide were mixed at a volume ratio of 102:1, followed by stirring at room temperature for 24 hours, thereby obtaining a composition containing a peptide-hyaluronic acid nanogel complex. Then, solid purified sucrose (produced by FUJIFILM Wako Pure Chemical Corporation, for production only, product number: 198-18385) was added so that the composition containing a peptide-hyaluronic acid nanogel complex was 10 mass% sucrose, followed by stirring for 1 hour or more. Subsequently, the composition was diluted with 10 mass% sucrose-containing 25 mM phosphate buffer (pH: 7.4) so that the theoretical peptide concentration was 0.3 mg/mL, and

sterile filtration was performed using 0.2 um PES (polyether sulfone) (produced by Pall, Acrodisc syringe filter, 25-mm diameter), thereby obtaining a composition containing a peptide-hyaluronic acid nanogel complex. After the peptide concentration in the composition containing a peptide-hyaluronic acid nanogel complex at this time was quantified by reversed-phase chromatography analysis, the concentration was adjusted to a peptide concentration of 250 ug/mL to prepare a dosing solution.

4-2. Production of Complex Using HA Nanogel with a Molecular Weight of 10k and Peptide MAGE-A4 20mer

This complex was prepared by the method described in 4-1 above, except that the long-chain peptide antigen shown below was used. The concentration was adjusted so that the peptide concentration was 250 ug/mL, thereby preparing a dosing solution.

Long-Chain Peptide Antigen MAGE-A4 20mer

MAGE-A4 20mer was purchased from Eurofins. The sequence is as follows: GVYDGREHTVGVYDGREHTV (SEQ ID NO: 10). This sequence includes the following MAGEA4 (amino acid sequence: GVYDGREHTV (SEQ ID NO: 9) as a CD8-positive cytotoxic T-cell recognition epitope sequence.

5. Antitumor Test

The MAGE-A4/HHD-A2-expressing MC38 cells prepared in 3-2 are inoculated subcutaneously at $5\times10^6$ cells/mouse into the right anterodorsal part side of HLA-A2 Tg mice. 5 days and 10 days after the tumor inoculation, the chimeric antigen receptor-expressing lymphocytes prepared in 2-2 are suspended in PBS at a concentration of $3\times10^6$ cells/200 μL, and 200 μL of the suspension is then injected into the mouse tail vein. 50 μg of the HA nanogel cancer vaccines loaded with long-chain peptide antigens prepared in 4-1 and 4-2 are administered subcutaneously into the right posterodorsal part of the mice 4 and 9 (and 14) days after the tumor inoculation together with 50 μg of CpG oligo DNA dissolved in PBS. Then, the tumor area is measured over time.

Sequence Listing

## Claims

**1.** A pharmaceutical composition for use in combined administration with a lymphocyte,

the pharmaceutical composition comprising

a hyaluronic acid derivative having a hydrophobic group introduced, and
an antigen,

the lymphocyte expressing an immune receptor for the antigen.

**2.** The pharmaceutical composition according to claim 1, wherein the hydrophobic group is a steryl group.

**3.** The pharmaceutical composition according to claim 1 or 2, wherein the hyaluronic acid derivative and the antigen form a complex.

**4.** The pharmaceutical composition according to any one of claims 1 to 3, wherein the antigen is a cancer antigen.

**5.** The pharmaceutical composition according to any one of claims 1 to 4, wherein the antigen is an antigen peptide or an antigenic protein.

**6.** The pharmaceutical composition according to claim 5, wherein the antigen contains a CDS-positive cytotoxic T-cell recognition epitope and/or a CD4-positive helper T-cell recognition epitope.

**7.** The pharmaceutical composition according to any one of claims 1 to 6, wherein the immune receptor is a T-cell receptor or a chimeric antigen receptor.

**8.** The pharmaceutical composition according to any one of claims 1 to 7, further comprising an adjuvant, or the pharmaceutical composition being for use in combined administration with an adjuvant.

**9.** The pharmaceutical composition according to any one of claims 1 to 8, for use in the prevention or treatment of cancer.

**10.** A pharmaceutical composition comprising a lymphocyte expressing an immune receptor for an antigen, the pharmaceutical composition being for use in combined administration with a composition comprising a hyaluronic acid derivative having a hydrophobic group introduced and the antigen.

**11.** A cancer treatment kit comprising

a pharmaceutical composition containing

a hyaluronic acid derivative having a hydrophobic group introduced, and
a cancer antigen, and

a lymphocyte expressing an immune receptor for the cancer antigen.

**12.** A T-cell activation vaccine comprising

a hyaluronic acid derivative having a hydrophobic group introduced, and
an antigen.

**13.** A cancer treatment kit comprising

a T cell expressing an immune receptor for a cancer antigen, and
a pharmaceutical composition comprising

a hyaluronic acid derivative having a hydrophobic group introduced,
an antigen peptide or an antigenic protein derived from the cancer antigen, and
an adjuvant,
the hyaluronic acid derivative comprising one or more repeating units represented by formula (I) :

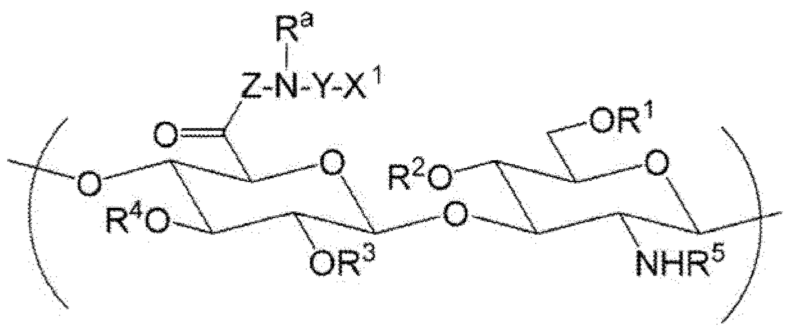

(I)

wherein $R^1$, $R^2$, $R^3$, and $R^4$ are each independently selected from the group consisting of a hydrogen atom, $C_{1-6}$ alkyl, formyl, and $C_{1-6}$ alkyl carbonyl;
$R^5$ represents a hydrogen atom, formyl, or $C_{1-6}$ alkyl carbonyl;
Z represents a direct bond, or a peptide linker composed of 2 to 30 any amino acid residues;
$X^1$ represents a hydrophobic group selected from the groups represented by the following formulas:

$-NR^b-R$,

$-NR^b-COO-R$,

$-NR^b-CO-R$,

$-NR^b-CO-NR^c-R$,

-COO-R,

-O-COO-R,

-S-R,

-CO-$Y^a$-S-R,

-O-CO-$Y^b$-S-R,

-$NR^b$-CO-$Y^b$-S-R, and

-S-S-R;

$R^a$, $R^b$, and $R^c$ are each independently selected from the group consisting of a hydrogen atom, $C_{1-20}$ alkyl, amino $C_{2-20}$ alkyl, and hydroxy $C_{2-20}$ alkyl, wherein an alkyl moiety of $R^a$, $R^b$, and $R^c$ may have 1 to 3 groups inserted, the 1 to 3 groups being selected from the group consisting of -O- and -$NR^f$-;
$R^f$ is selected from the group consisting of a hydrogen atom, $C_{1-12}$ alkyl, amino $C_{2-12}$ alkyl, and hydroxy $C_{2-12}$ alkyl, wherein an alkyl moiety of $R^f$ may have 1 or 2 groups inserted, the 1 or 2 groups being selected from the group consisting of -O- and -NH-;
R represents a steryl group;
Y represents $C_{2-30}$ alkylene or - $(CH_2CH_2O)_m$-$CH_2CH_2$-, wherein the alkylene may have 1 to 5 groups inserted, the 1 to 5 groups being selected from the group consisting of -O-, -$NR^g$-, and-S-S-;
R9 is selected from the group consisting of a hydrogen atom, $C_{1-20}$ alkyl, amino $C_{2-20}$ alkyl, and hydroxy $C_{2-20}$ alkyl, wherein an alkyl moiety of $R^g$ may have 1 to 3 groups inserted, the 1 to 3 groups being selected from the group consisting of -O- and -NH-;
$Y^a$ represents $C_{1-5}$ alkylene;
$Y^b$ represents $C_{2-8}$ alkylene or $C_{2-8}$ alkenylene; and
m represents an integer selected from 1 to 100.

**14.** The cancer treatment kit according to claim 13, wherein a modification rate of a carboxy group of a glucuronic acid moiety of the hyaluronic acid derivative with the hydrophobic group is 5 to 50%.

**15.** The cancer treatment kit according to claim 14, wherein Z represents a direct bond, Y represents $C_{2-12}$ alkylene, $X^1$ represents -NH-COO-R, and R represents a cholesteryl group.

**16.** The cancer treatment kit according to claim 13, wherein the hyaluronic acid derivative has a weight average molecular weight of 5,000 to 2,000,000.

**17.** The cancer treatment kit according to claim 13, wherein the hyaluronic acid derivative comprises one or more repeating units represented by formula (IIIc):

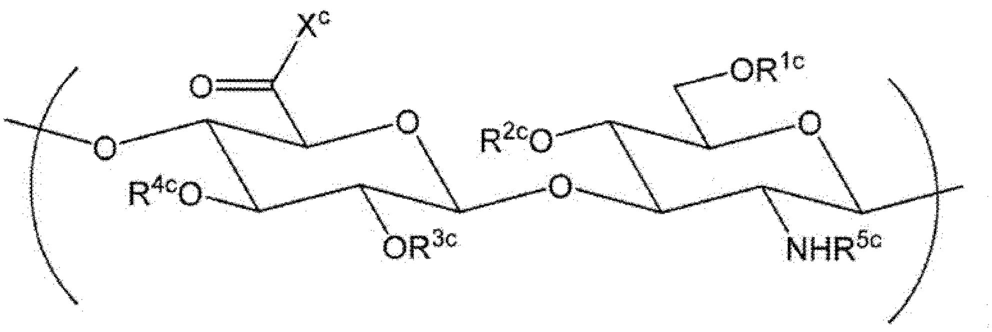

(IIIc)

wherein

$R^{1c}$, $R^{2c}$, $R^{3c}$, and $R^{4c}$ are each independently selected from the group consisting of a hydrogen atom, $C_{1-6}$

alkyl, formyl, and $C_{1-6}$ alkyl carbonyl;
$R^{5c}$ is selected from the group consisting of a hydrogen atom, formyl, and $C_{1-6}$ alkyl carbonyl; and
$X^c$ is selected from the group consisting of hydroxy and $-O-Q^+$, wherein $Q^+$ represents a countercation.

**18.** The cancer treatment kit according to claim 13, wherein the pharmaceutical composition comprises a complex of the cancer antigen and the hyaluronic acid derivative.

**19.** The cancer treatment kit according to claim 13, wherein the antigen peptide contains two or more CD8-positive cytotoxic T-cell recognition epitopes and/or CD4-positive helper T-cell recognition epitopes.

**20.** The cancer treatment kit according to claim 19, wherein the antigen peptide is a long-chain peptide antigen containing

two or more CD8-positive cytotoxic T-cell recognition epitopes, or
one or more CD8-positive cytotoxic T-cell recognition epitopes and one or more CD4-positive helper T-cell recognition epitopes.

**21.** The cancer treatment kit according to claim 13, which is for use in T-cell infusion therapy for low immunogenic cancer, metastatic cancer, or cancer with a low intratumoral T-cell count.

**22.** The cancer treatment kit according to claim 13, wherein the pharmaceutical composition is administered before the T cell is administered.

**23.** The cancer treatment kit according to claim 22, wherein the pharmaceutical composition is administered at least once after one or two units of administration wherein a single unit of administration consists of one-time administration of the pharmaceutical composition and subsequent one-time infusion of the T-cell.

**24.** The cancer treatment kit according to claim 22, wherein the pharmaceutical composition is subcutaneously or intravenously administered at least twice, and the T cell is transfused after the first administration of the pharmaceutical composition and before the second administration of the pharmaceutical composition.

**25.** A method for treating cancer, comprising administering a pharmaceutical composition and a lymphocyte in combination to a subject with cancer,

the pharmaceutical composition containing a hyaluronic acid derivative having a hydrophobic group introduced and a cancer antigen,
the lymphocyte expressing an immune receptor for the cancer antigen.

**26.** The cancer treatment kit for use in cancer treatment, the cancer treatment kit comprising

a pharmaceutical composition containing a hyaluronic acid derivative having a hydrophobic group introduced and a cancer antigen, and
a lymphocyte expressing an immune receptor for the cancer antigen.

**27.** A pharmaceutical composition comprising a hyaluronic acid derivative having a hydrophobic group introduced and a cancer antigen,
the pharmaceutical composition being for use in cancer treatment comprising administering a lymphocyte expressing an immune receptor for the cancer antigen.

**28.** A pharmaceutical composition comprising a lymphocyte expressing an immune receptor for a cancer antigen,
the pharmaceutical composition being for use in cancer treatment comprising administering a pharmaceutical composition containing a hyaluronic acid derivative having a hydrophobic group introduced and the cancer antigen.

**29.** Use of a pharmaceutical composition and a lymphocyte in combination for the production of a cancer treatment kit,

the pharmaceutical composition comprising a hyaluronic acid derivative having a hydrophobic group introduced and a cancer antigen,
the lymphocyte expressing an immune receptor for the cancer antigen.

**30.** Use of a pharmaceutical composition comprising a hyaluronic acid derivative having a hydrophobic group introduced and a cancer antigen,
the use being for the production of a cancer treatment agent for combined administration with a lymphocyte expressing an immune receptor for the cancer antigen.

**31.** Use of a lymphocyte expressing an immune receptor for a cancer antigen,
the use being for the production of a cancer treatment agent for combined administration with a pharmaceutical composition containing a hyaluronic acid derivative having a hydrophobic group introduced and the cancer antigen.

Fig. 1-1

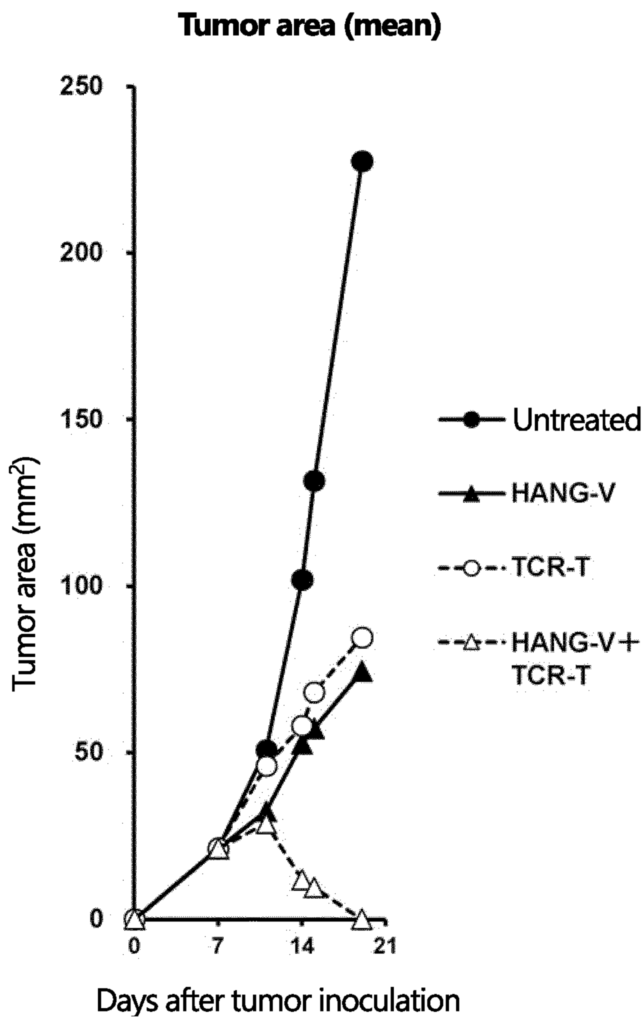
Tumor area (mean)
250
200
150
100
50
0
Tumor area (mm2)
0
7
14
21
Days after tumor inoculation
Untreated
HANG-V
TCR-T
HANG-V+
TCR-T

Fig. 1-2

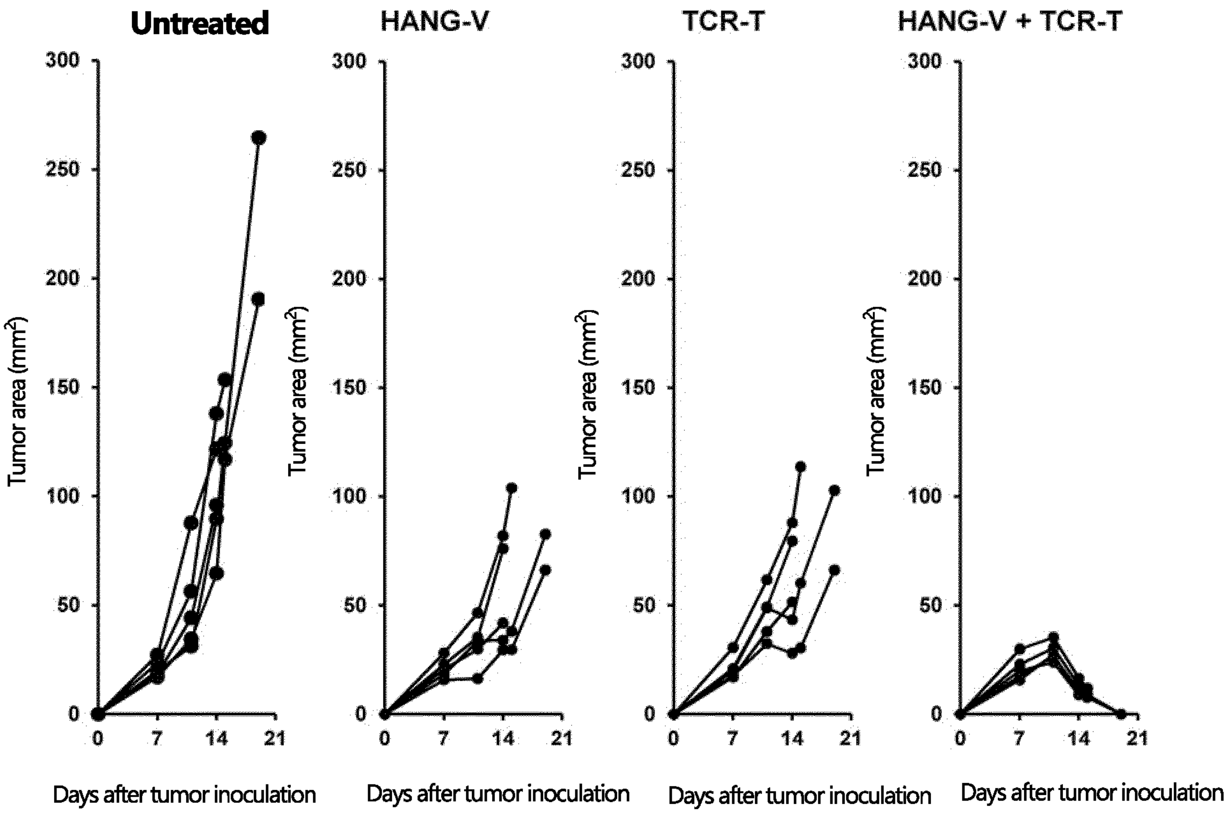
Untreated
HANG-V
TCR-T
HANG-V + TCR-T
Tumor area (mm2)
0
50
100
150
200
250
300
0
7
14
21
Days after tumor inoculation

Fig. 1-3

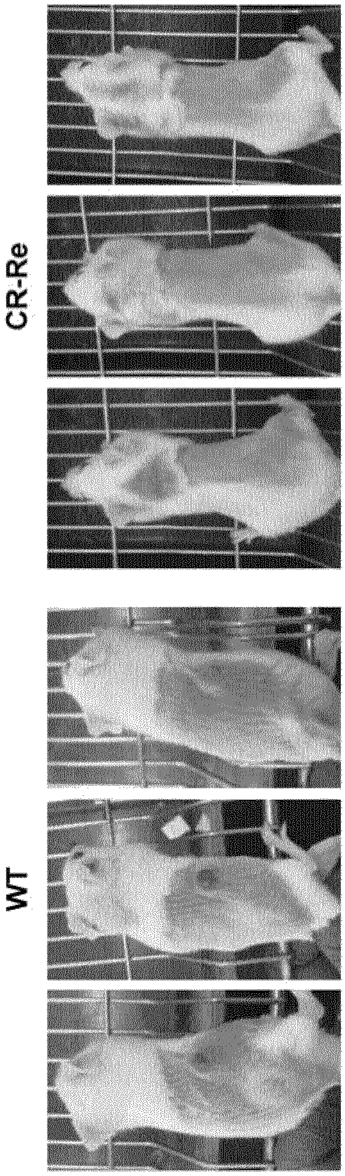

Fig. 2-1

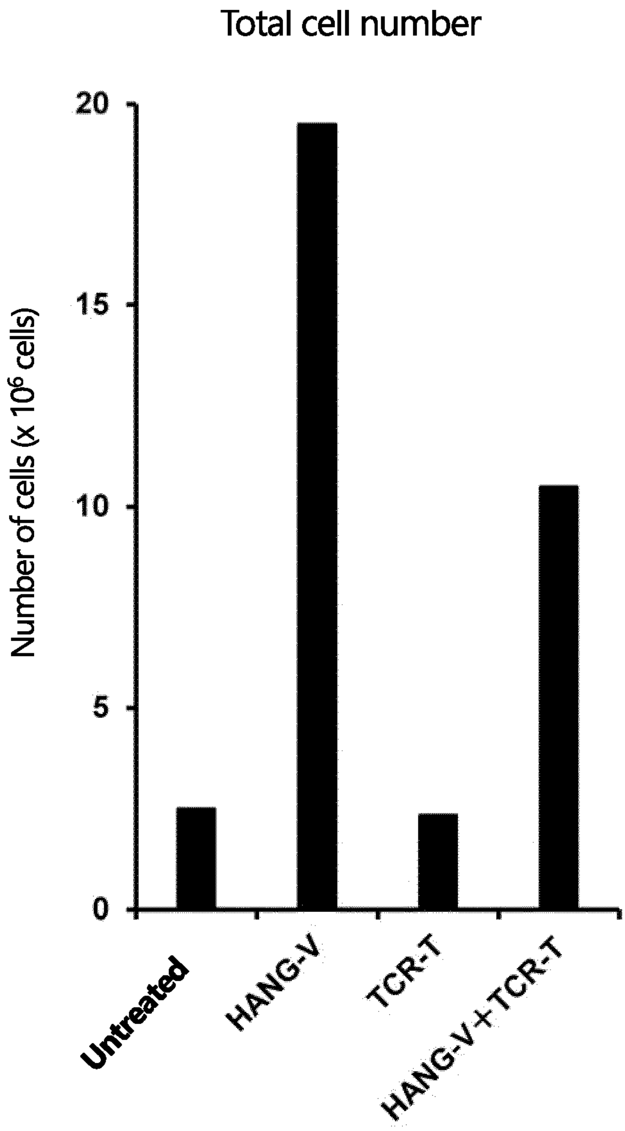
Total cell number
Number of cells (x 10^6 cells)
20
15
10
5
0
Untreated
HANG-V
TCR-T
HANG-V+TCR-T

Fig. 2-2

The draining lymph nodes

**Untreated** HANG-V TCR-T HANG-V+TCR-T

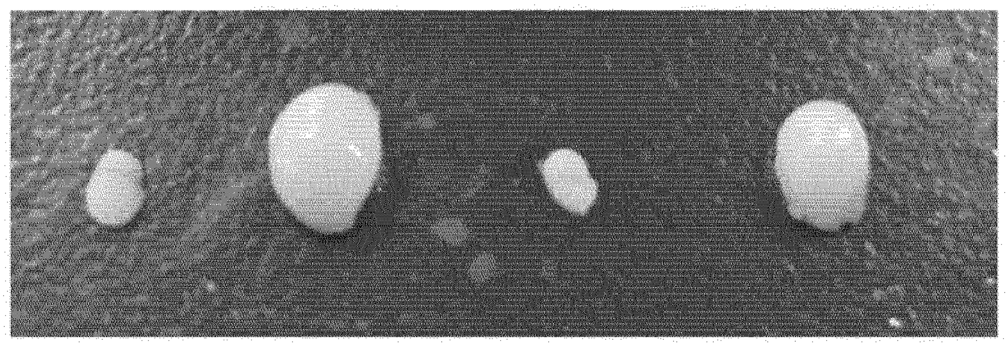

Tumors

**Untreated** HANG-V TCR-T

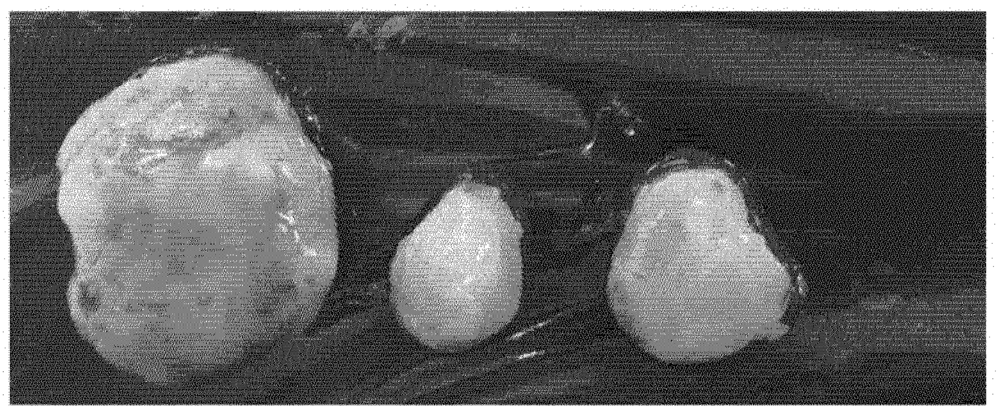

Fig. 2-3

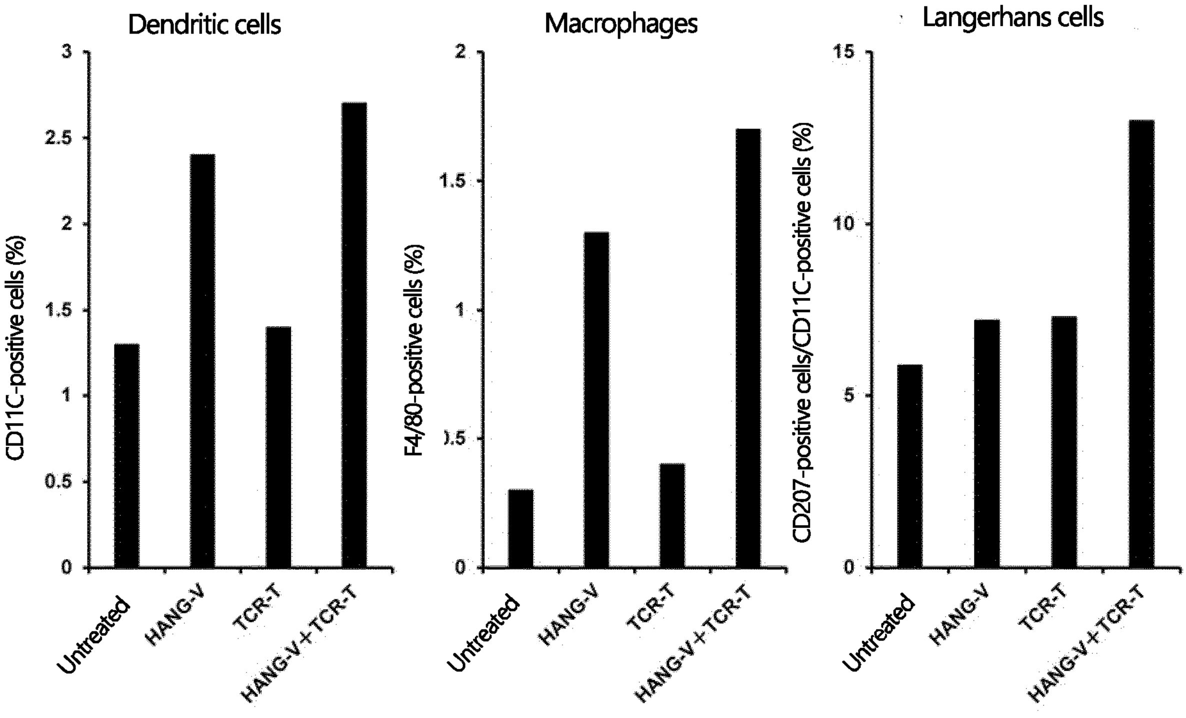
Dendritic cells
CD11C-positive cells (%)
3
2.5
2
1.5
1
0.5
0
Untreated
HANG-V
TCR-T
HANG-V+TCR-T
Macrophages
F4/80-positive cells (%)
2
1.5
1
).5
0
Untreated
HANG-V
TCR-T
HANG-V+TCR-T
Langerhans cells
CD207-positive cells/CD11C-positive cells (%)
15
10
5
0
Untreated
HANG-V
TCR-T
HANG-V+TCR-T

Fig. 2-4-1

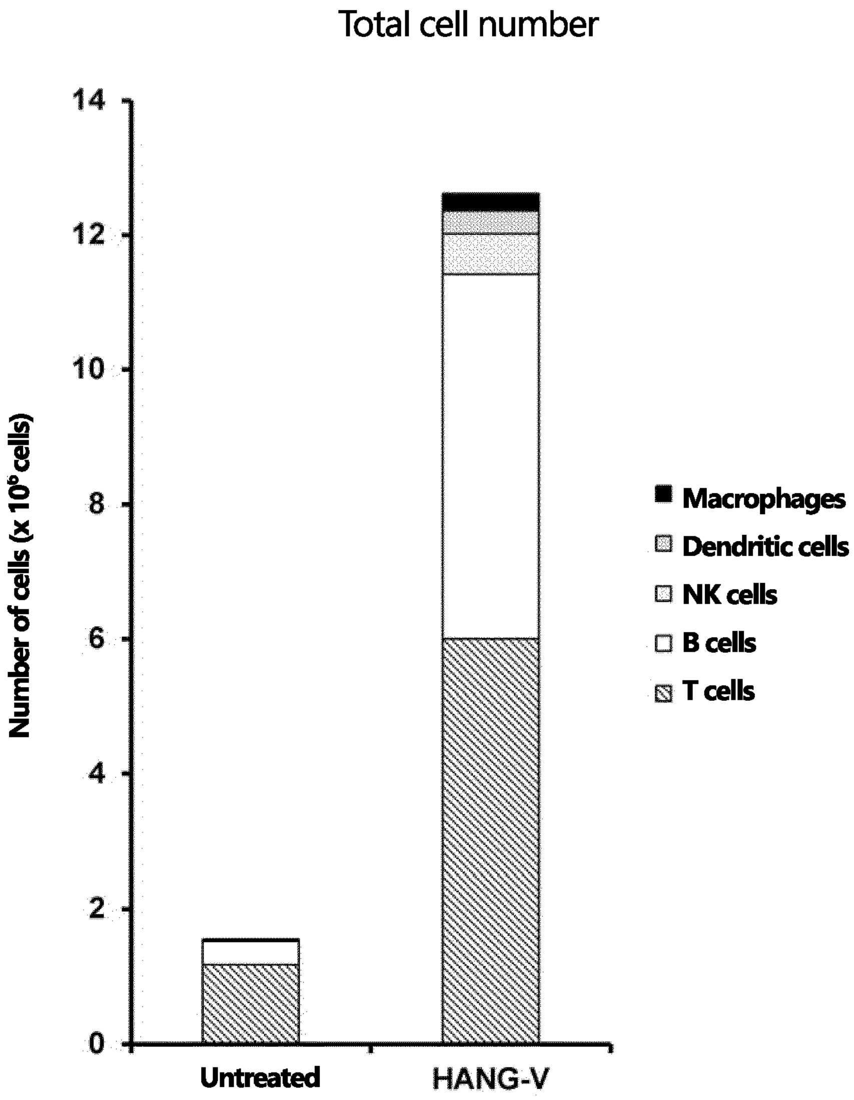
Total cell number
14
12
10
8
6
4
2
0
Number of cells (x $10^6$ cells)
Untreated
HANG-V
Macrophages
Dendritic cells
NK cells
B cells
T cells

Fig. 2-4-2

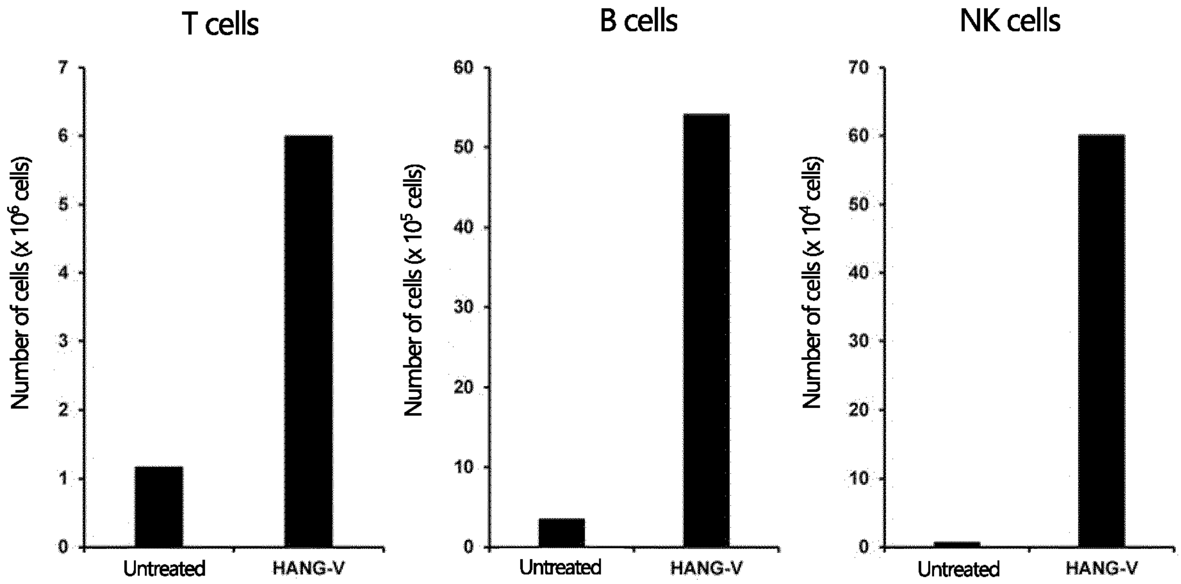
T cells
B cells
NK cells
Number of cells (x 10^6 cells)
Number of cells (x 10^5 cells)
Number of cells (x 10^4 cells)
Untreated
HANG-V
0
1
2
3
4
5
6
7
10
20
30
40
50
60
70

Fig. 2-4-3

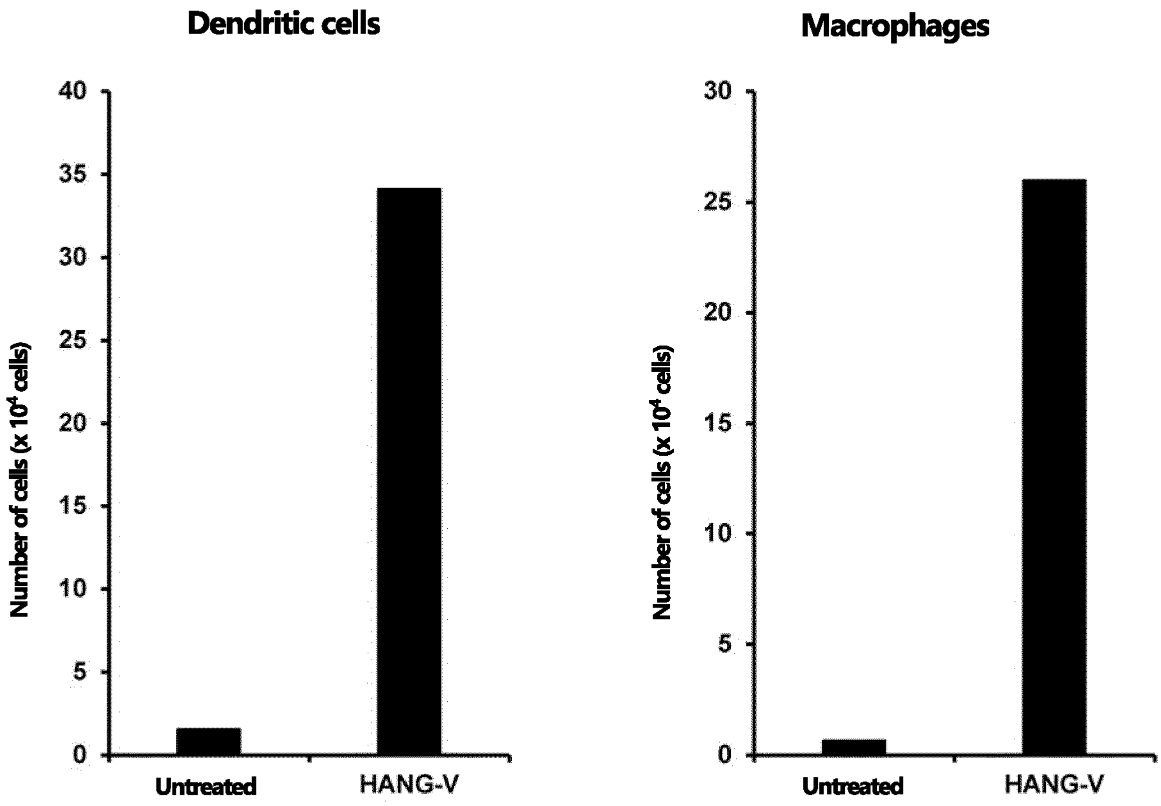
Dendritic cells
Number of cells (x 10^4 cells)
40
35
30
25
20
15
10
5
0
Untreated
HANG-V
Macrophages
Number of cells (x 10^4 cells)
30
25
20
15
10
5
0
Untreated
HANG-V

Fig. 2-5

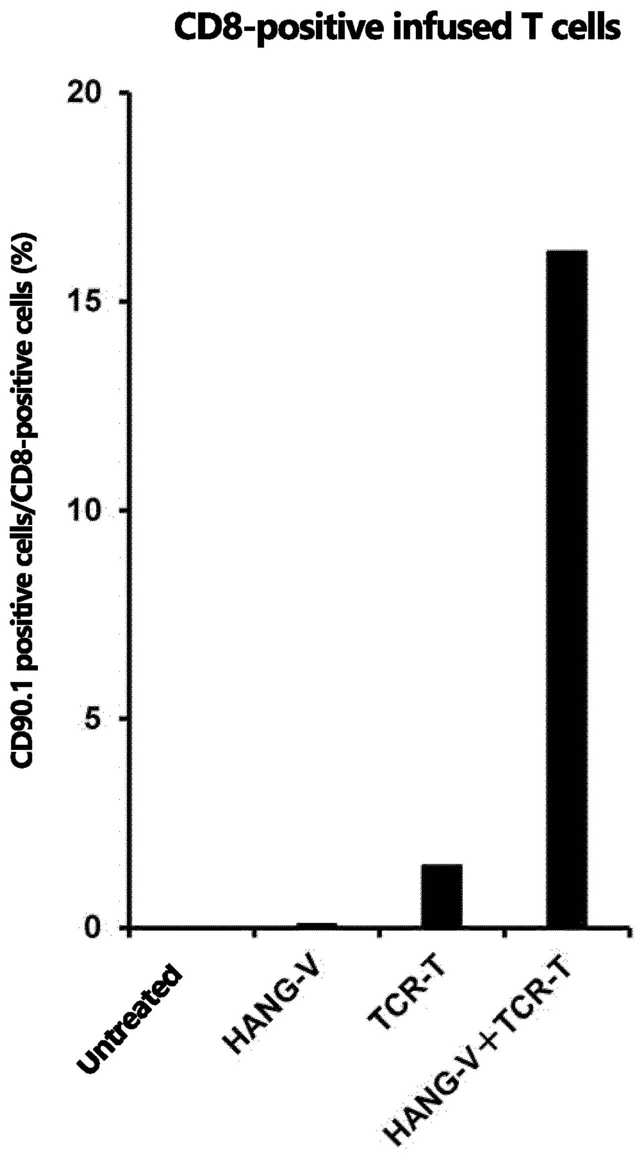
CD8-positive infused T cells
CD90.1 positive cells/CD8-positive cells (%)
20
15
10
5
0
Untreated
HANG-V
TCR-T
HANG-V+TCR-T

Fig. 2-6

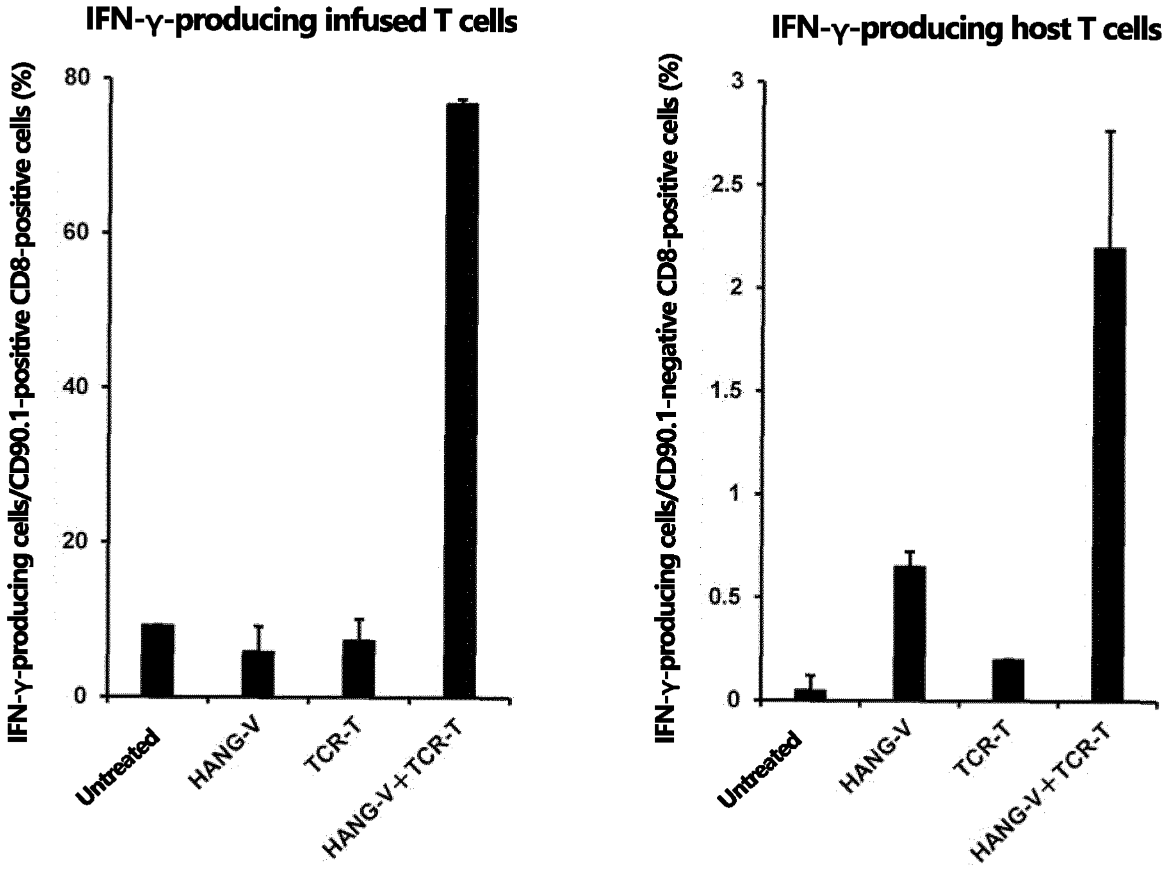
IFN-γ-producing infused T cells
IFN-γ-producing cells/CD90.1-positive CD8-positive cells (%)
80
60
40
20
0
Untreated
HANG-V
TCR-T
HANG-V+TCR-T
IFN-γ-producing host T cells
IFN-γ-producing cells/CD90.1-negative CD8-positive cells (%)
3
2.5
2
1.5
1
0.5
0
Untreated
HANG-V
TCR-T
HANG-V+TCR-T

Fig 3-1

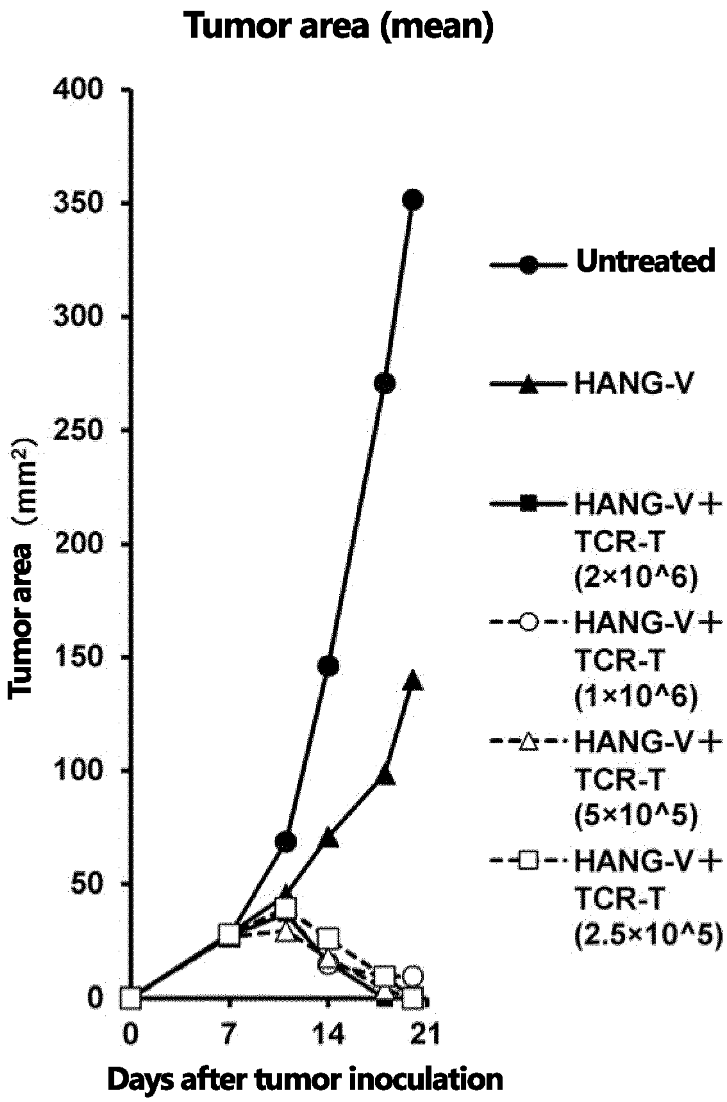
Tumor area (mean)
Tumor area (mm²)
400
350
300
250
200
150
100
50
0
0
7
14
21
Days after tumor inoculation
Untreated
HANG-V
HANG-V+ TCR-T (2×10^6)
HANG-V+ TCR-T (1×10^6)
HANG-V+ TCR-T (5×10^5)
HANG-V+ TCR-T (2.5×10^5)

Fig. 3-2

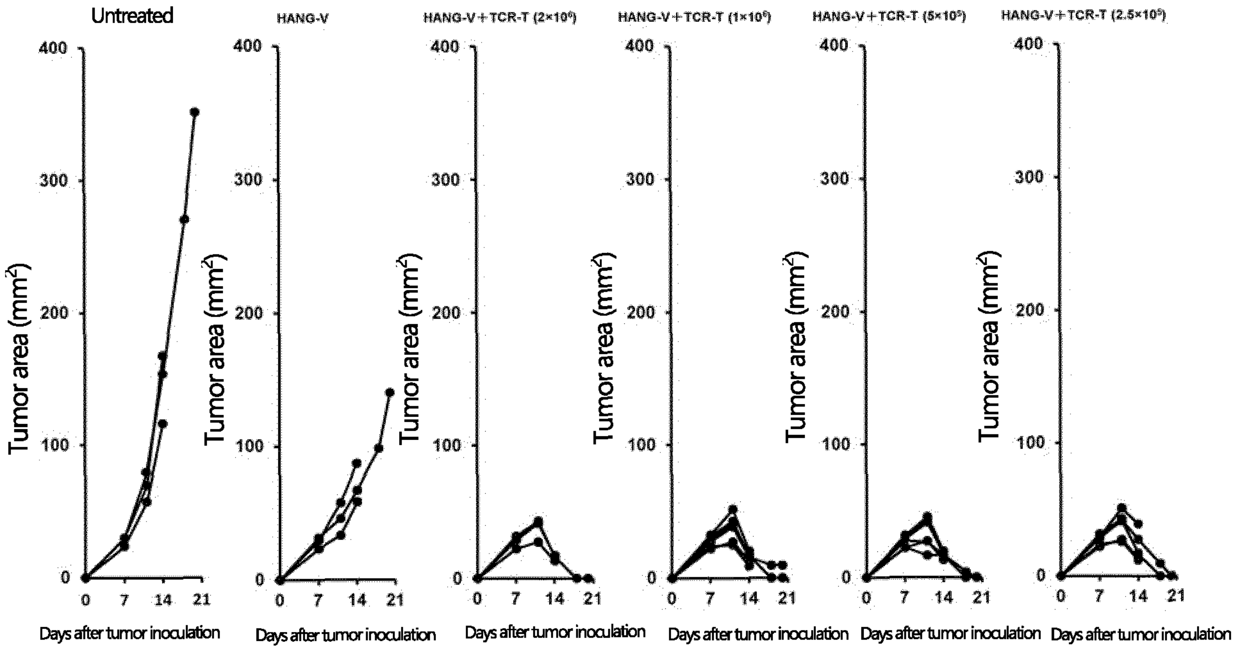
Untreated
HANG-V
HANG-V+TCR-T (2×10⁶)
HANG-V+TCR-T (1×10⁶)
HANG-V+TCR-T (5×10⁵)
HANG-V+TCR-T (2.5×10⁵)
Tumor area (mm²)
400
300
200
100
0
0 7 14 21
Days after tumor inoculation

Fig. 3-3

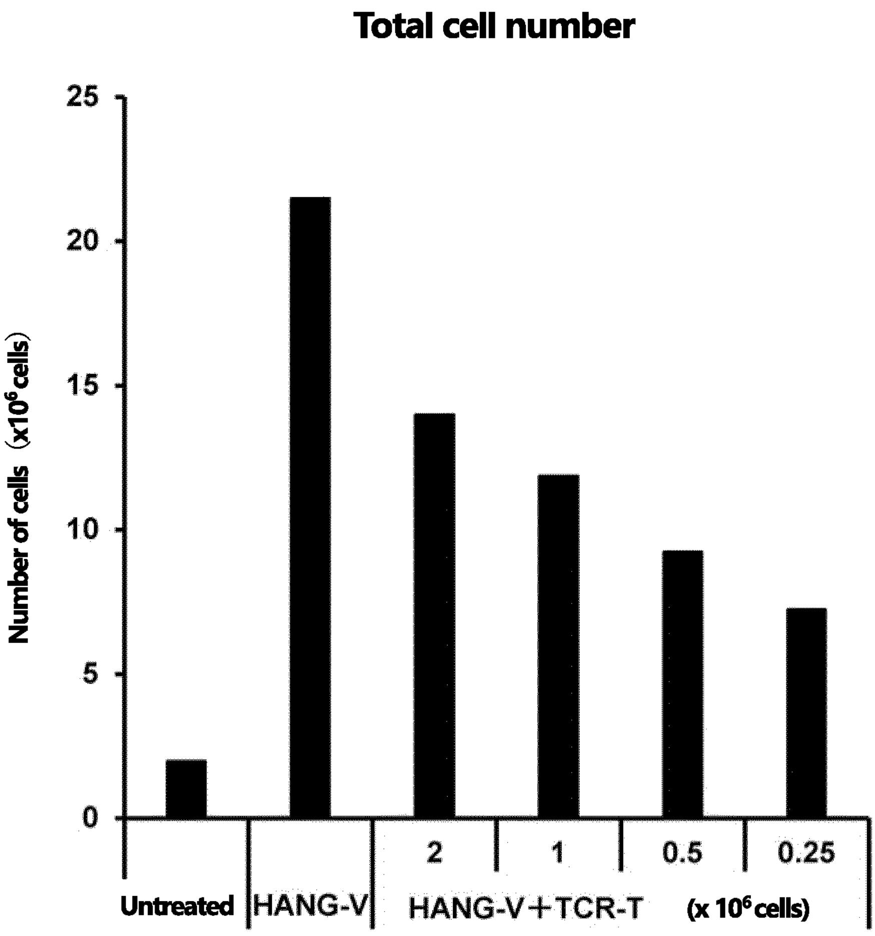
Total cell number
Number of cells (x10^6 cells)
25
20
15
10
5
0
2
1
0.5
0.25
Untreated
HANG-V
HANG-V+TCR-T
(x 10^6 cells)

Fig. 3-4

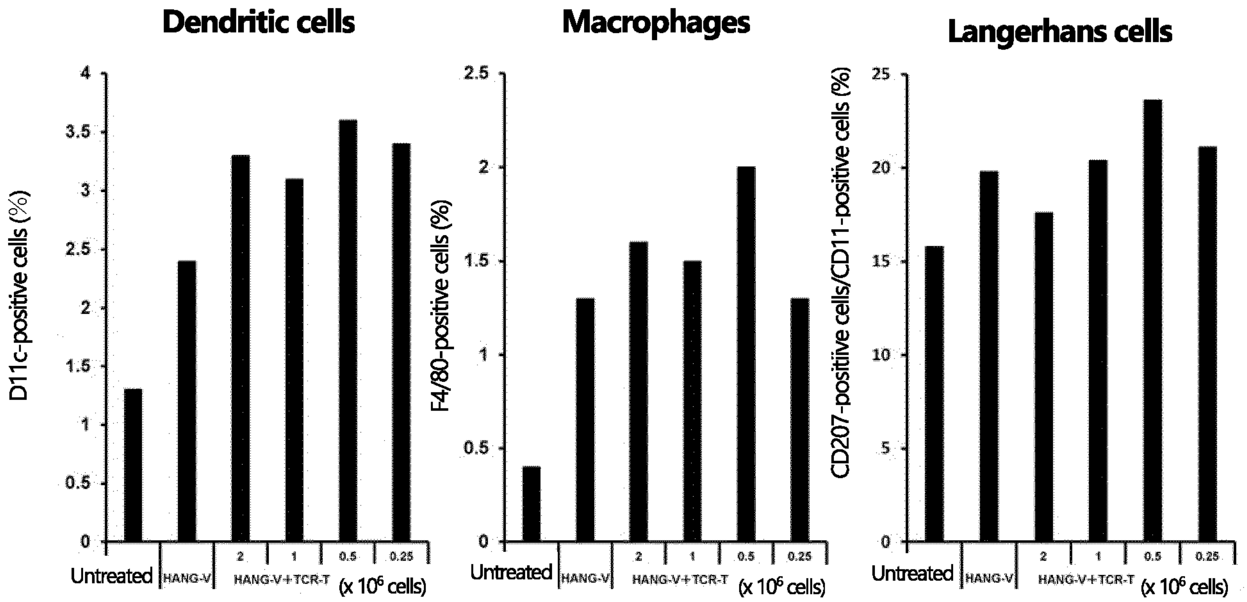
Dendritic cells
D11c-positive cells (%)
4
3.5
3
2.5
2
1.5
1
0.5
0
Untreated
HANG-V
HANG-V+TCR-T
2
1
0.5
0.25
(x 10⁶ cells)
Macrophages
F4/80-positive cells (%)
2.5
2
1.5
1
0.5
0
Untreated
HANG-V
HANG-V+TCR-T
2
1
0.5
0.25
(x 10⁶ cells)
Langerhans cells
CD207-positive cells/CD11-positive cells (%)
25
20
15
10
5
0
Untreated
HANG-V
HANG-V+TCR-T
2
1
0.5
0.25
(x 10⁶ cells)

Fig. 3-5

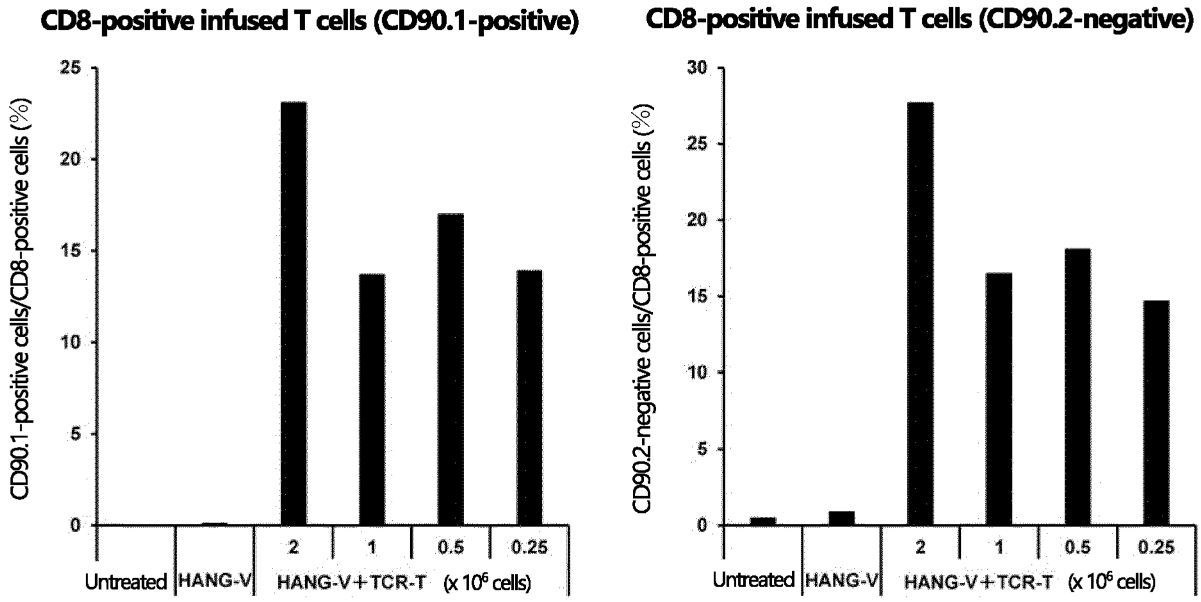
CD8-positive infused T cells (CD90.1-positive)
CD90.1-positive cells/CD8-positive cells (%)
25
20
15
10
5
0
2
1
0.5
0.25
Untreated
HANG-V
HANG-V+TCR-T (x 10^6 cells)
CD8-positive infused T cells (CD90.2-negative)
CD90.2-negative cells/CD8-positive cells (%)
30
25
20
15
10
5
0
2
1
0.5
0.25
Untreated
HANG-V
HANG-V+TCR-T (x 10^6 cells)

Fig. 3-6

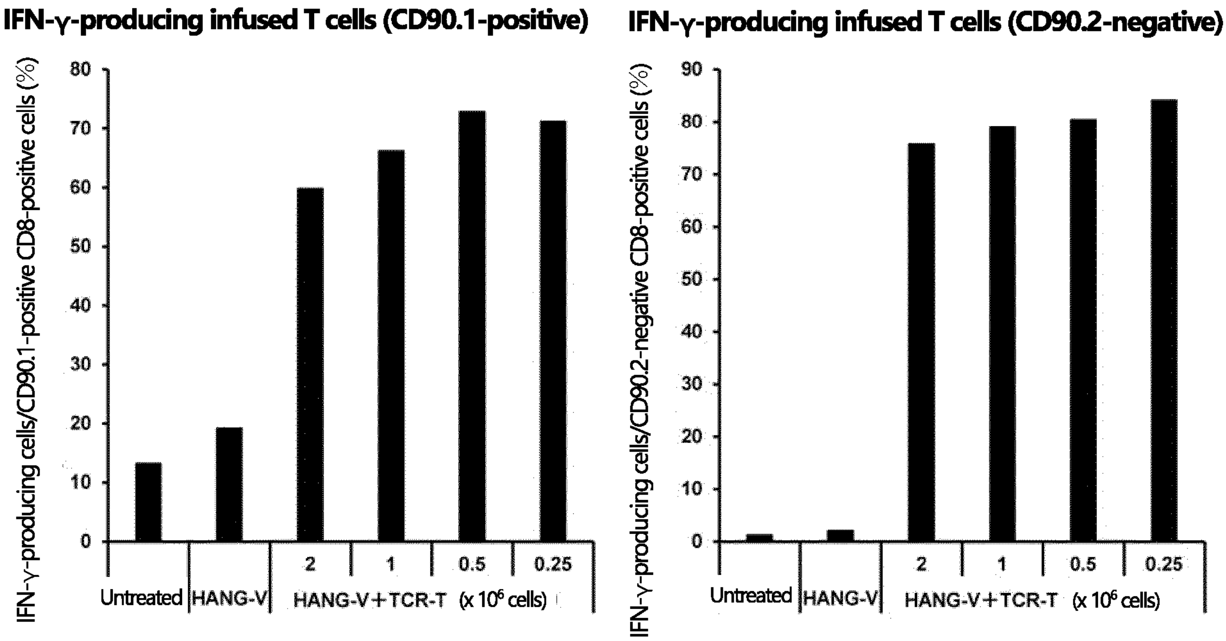
IFN-γ-producing infused T cells (CD90.1-positive)
IFN-γ-producing cells/CD90.1-positive CD8-positive cells (%)
80
70
60
50
40
30
20
10
0
Untreated
HANG-V
2
1
0.5
0.25
HANG-V+TCR-T (x 10^6 cells)
IFN-γ-producing infused T cells (CD90.2-negative)
IFN-γ-producing cells/CD90.2-negative CD8-positive cells (%)
90
80
70
60
50
40
30
20
10
0
Untreated
HANG-V
2
1
0.5
0.25
HANG-V+TCR-T (x 10^6 cells)

Fig. 3-7

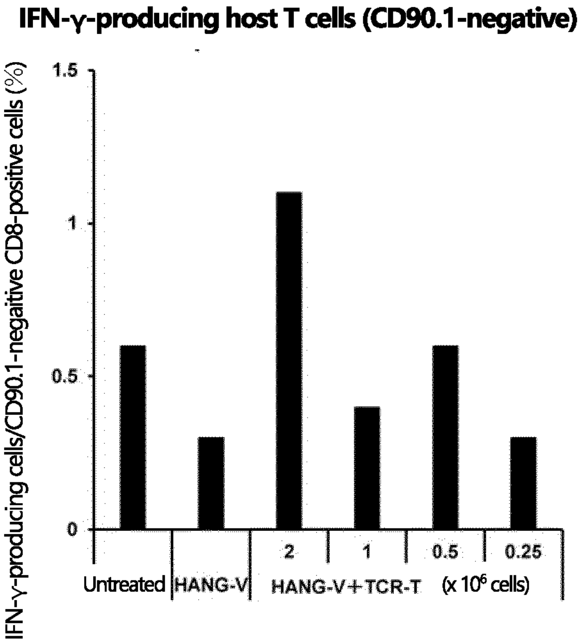
IFN-γ-producing host T cells (CD90.1-negative)
IFN-γ-producing cells/CD90.1-negative CD8-positive cells (%)
1.5
1
0.5
0
Untreated
HANG-V
HANG-V+TCR-T
2
1
0.5
0.25
(x 10^6 cells)

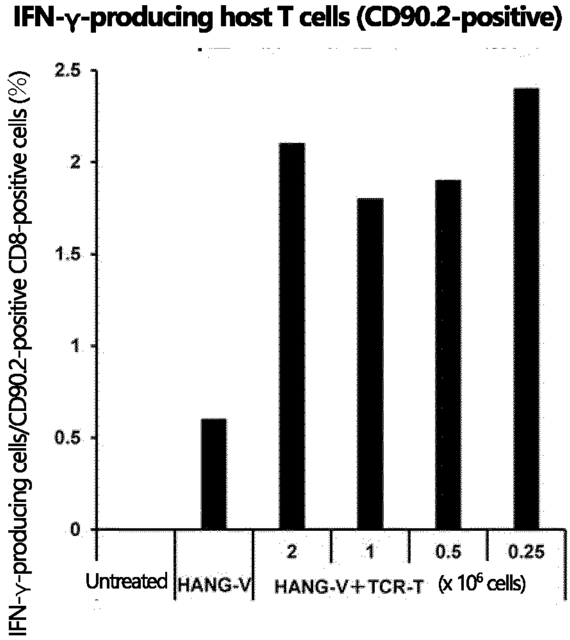
IFN-γ-producing host T cells (CD90.2-positive)
IFN-γ-producing cells/CD90.2-positive CD8-positive cells (%)
2.5
2
1.5
1
0.5
0
Untreated
HANG-V
HANG-V+TCR-T
2
1
0.5
0.25
(x 10^6 cells)

Fig. 4

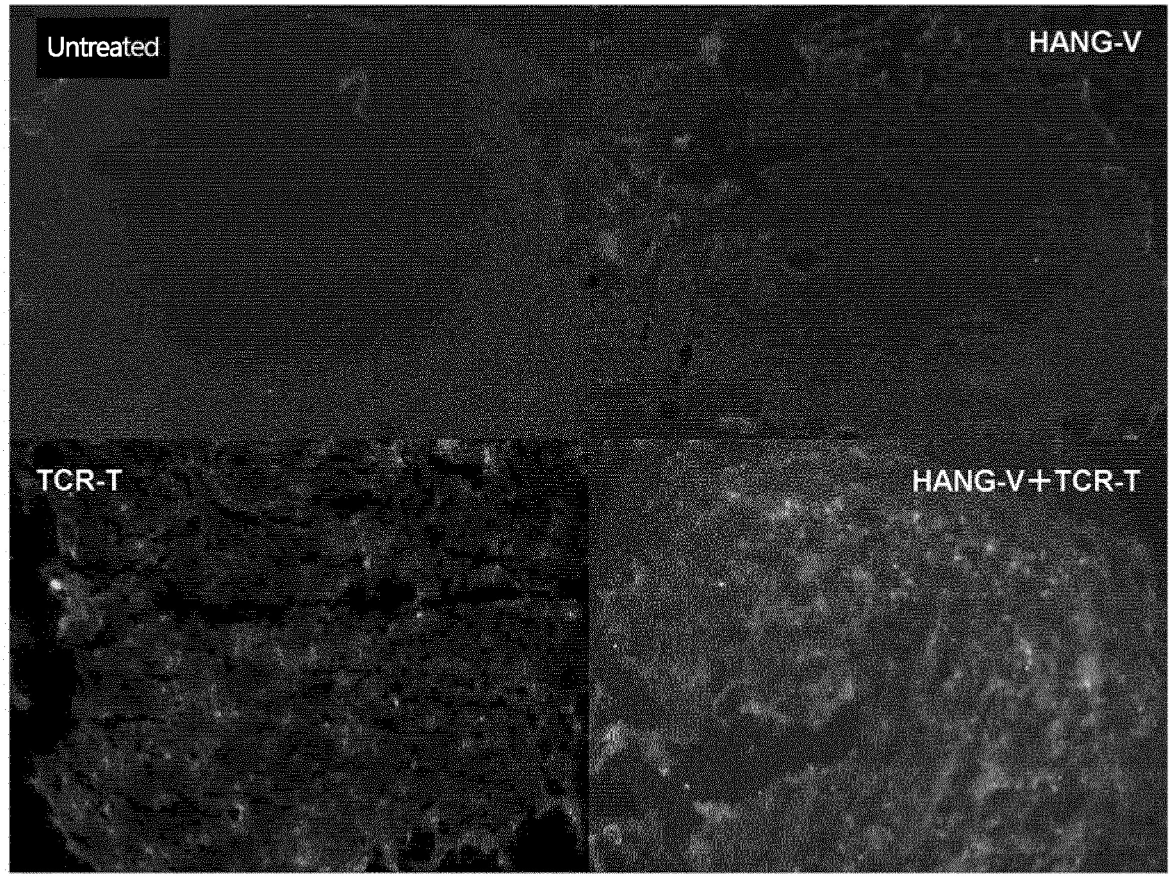

Fig. 5

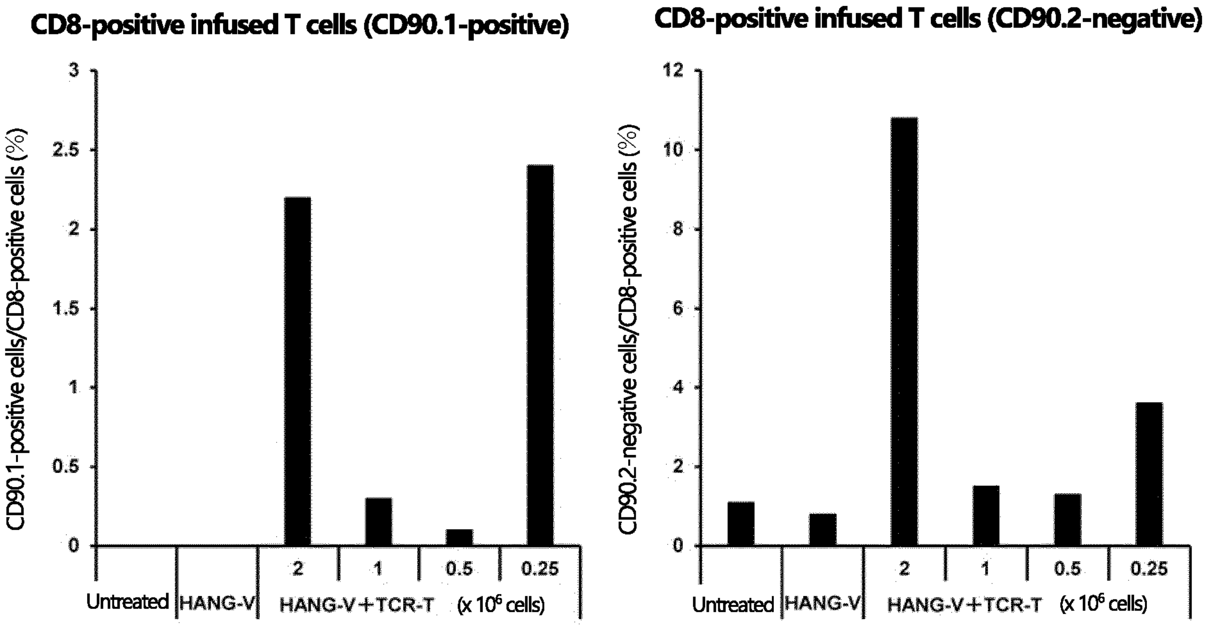
CD8-positive infused T cells (CD90.1-positive)
CD90.1-positive cells/CD8-positive cells (%)
3
2.5
2
1.5
1
0.5
0
2
1
0.5
0.25
Untreated
HANG-V
HANG-V+TCR-T
(x 10⁶ cells)
CD8-positive infused T cells (CD90.2-negative)
CD90.2-negative cells/CD8-positive cells (%)
12
10
8
6
4
2
0
2
1
0.5
0.25
Untreated
HANG-V
HANG-V+TCR-T
(x 10⁶ cells)

Fig. 6-1

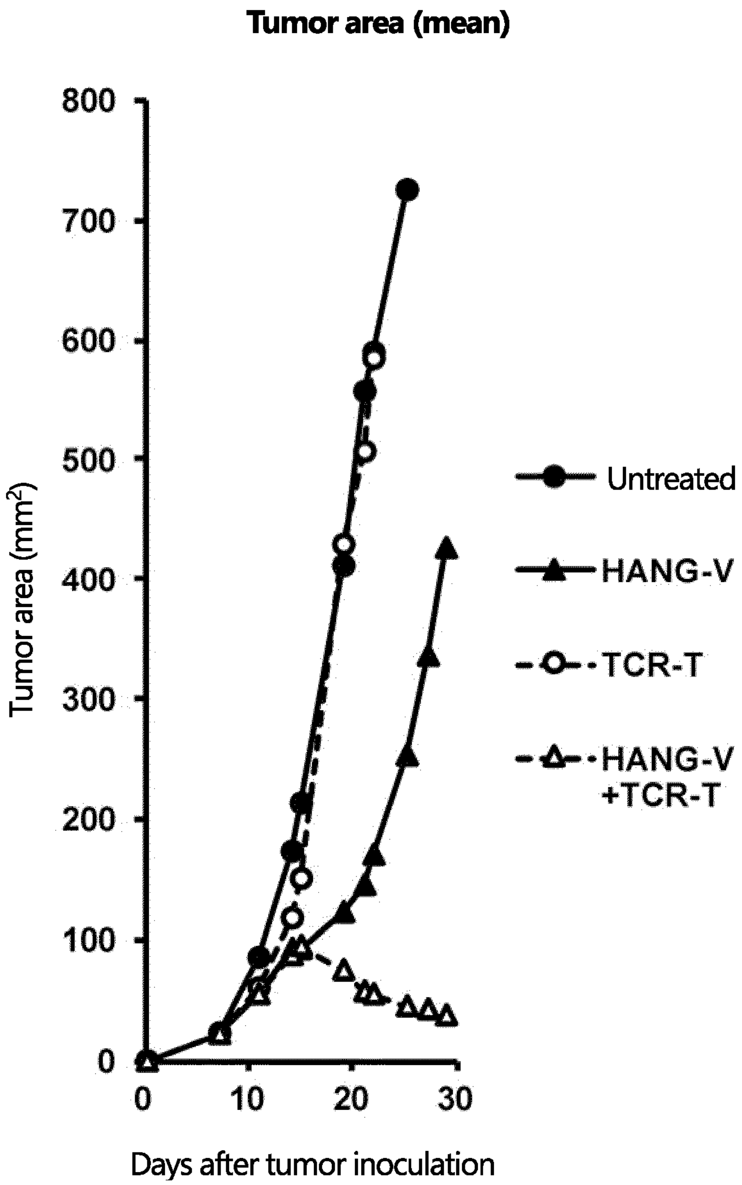
Tumor area (mean)
Tumor area (mm2)
800
700
600
500
400
300
200
100
0
0
10
20
30
Days after tumor inoculation
Untreated
HANG-V
TCR-T
HANG-V
+TCR-T

Fig. 6-2

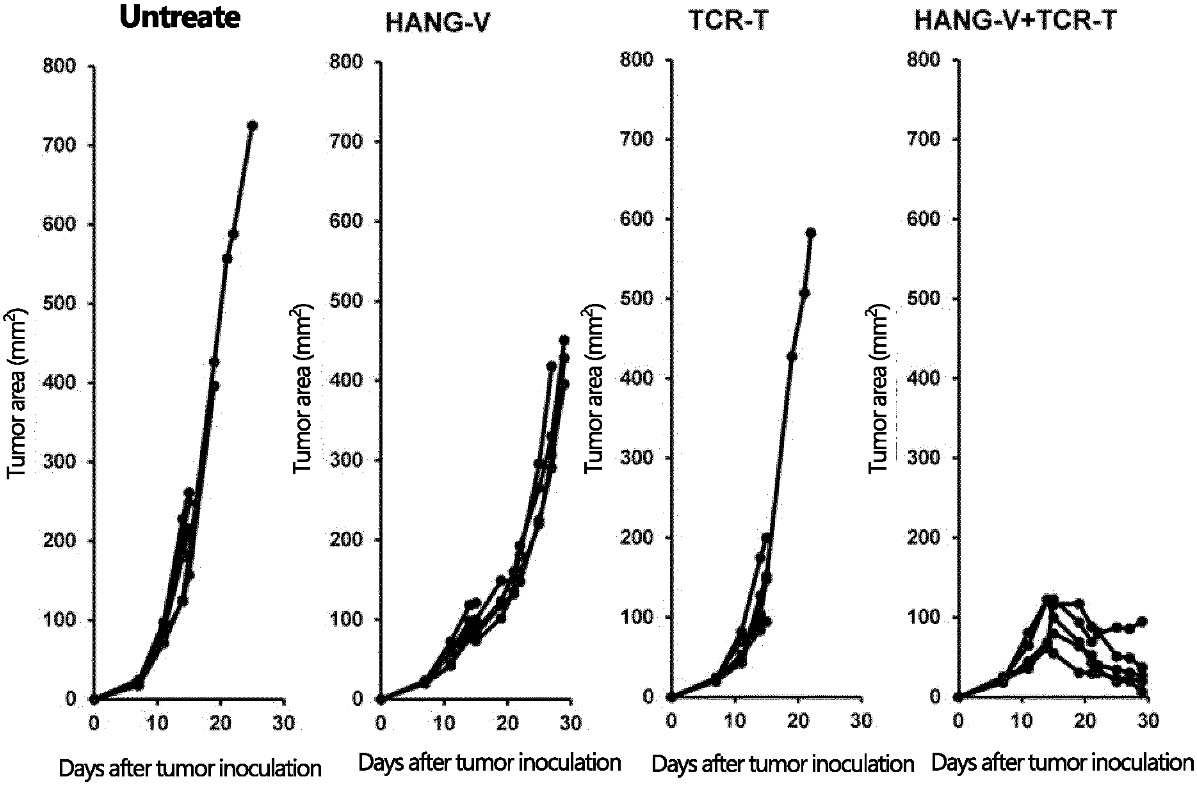
Untreate
HANG-V
TCR-T
HANG-V+TCR-T
Tumor area ($mm^2$)
Days after tumor inoculation
0
100
200
300
400
500
600
700
800
10
20
30

Fig. 6-3

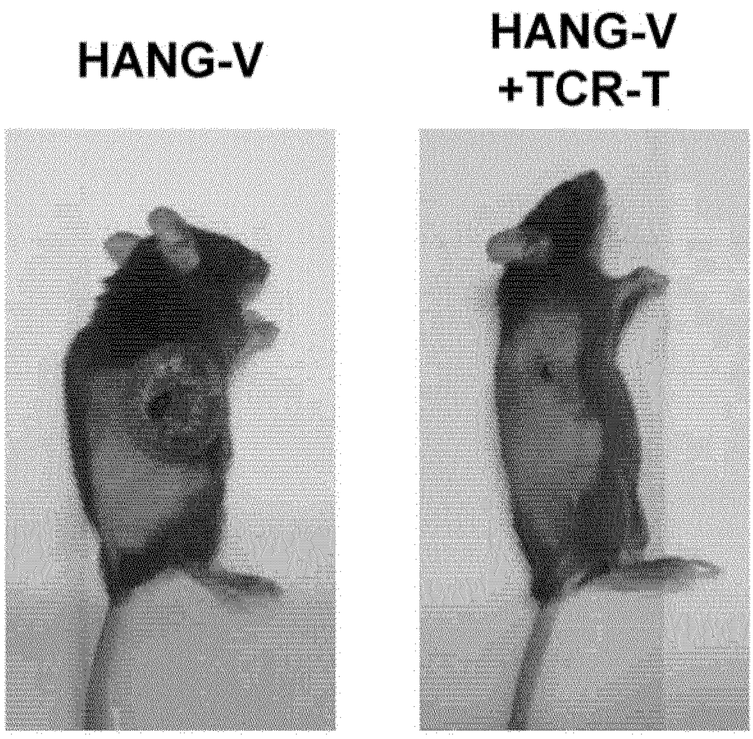
HANG-V
HANG-V
+TCR-T

Fig. 6-4

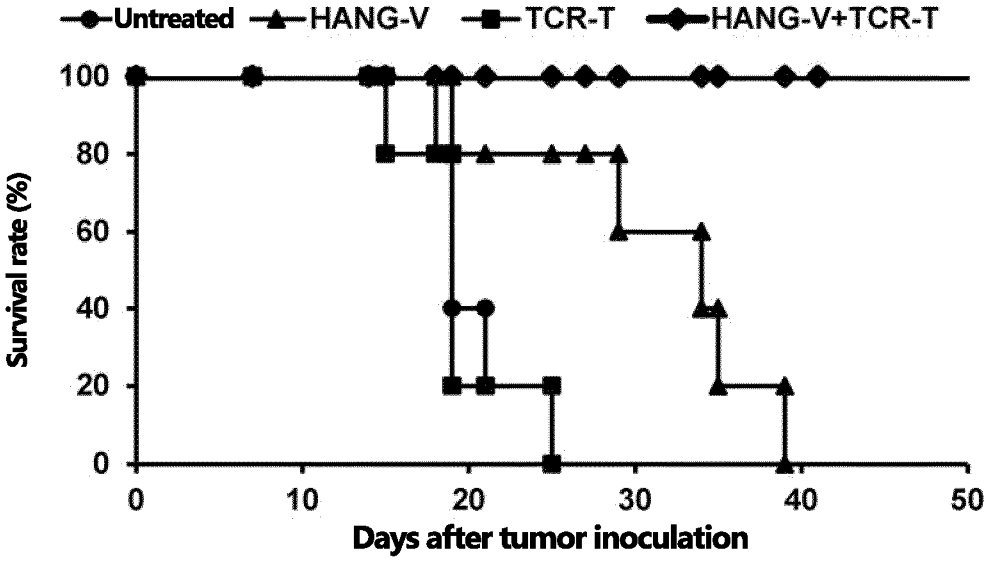
Untreated
HANG-V
TCR-T
HANG-V+TCR-T
100
80
60
40
20
0
Survival rate (%)
0
10
20
30
40
50
Days after tumor inoculation

Fig. 6-5

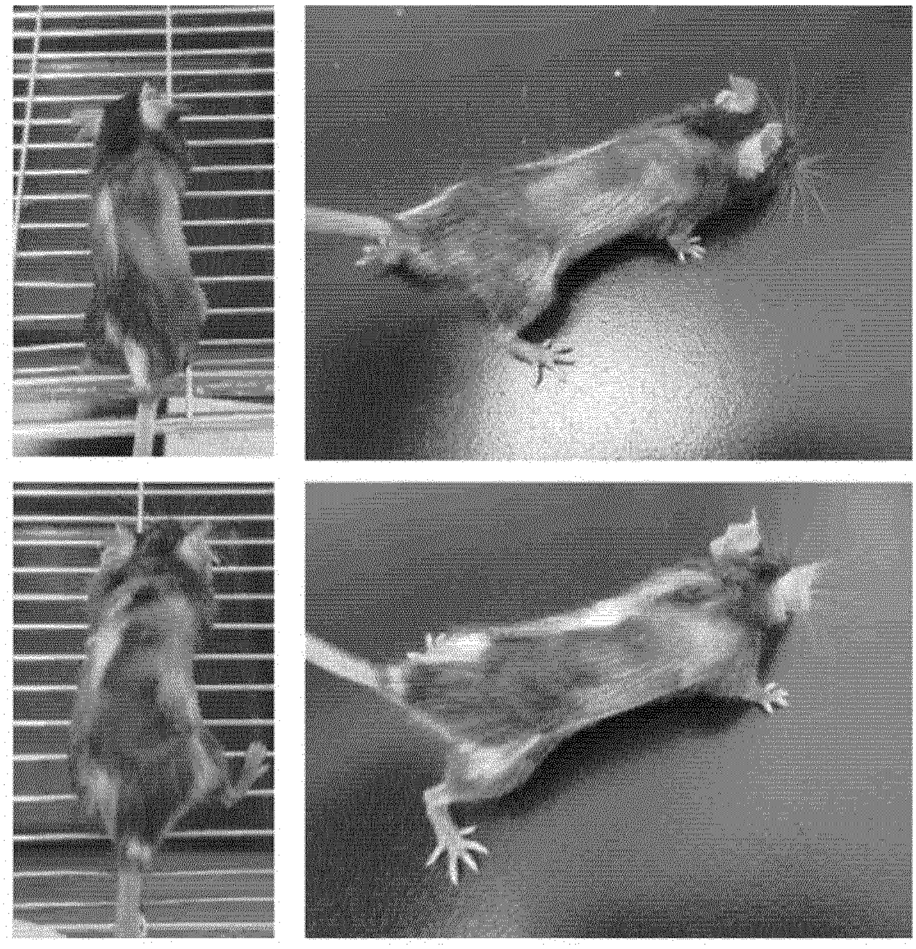

Fig. 7-1

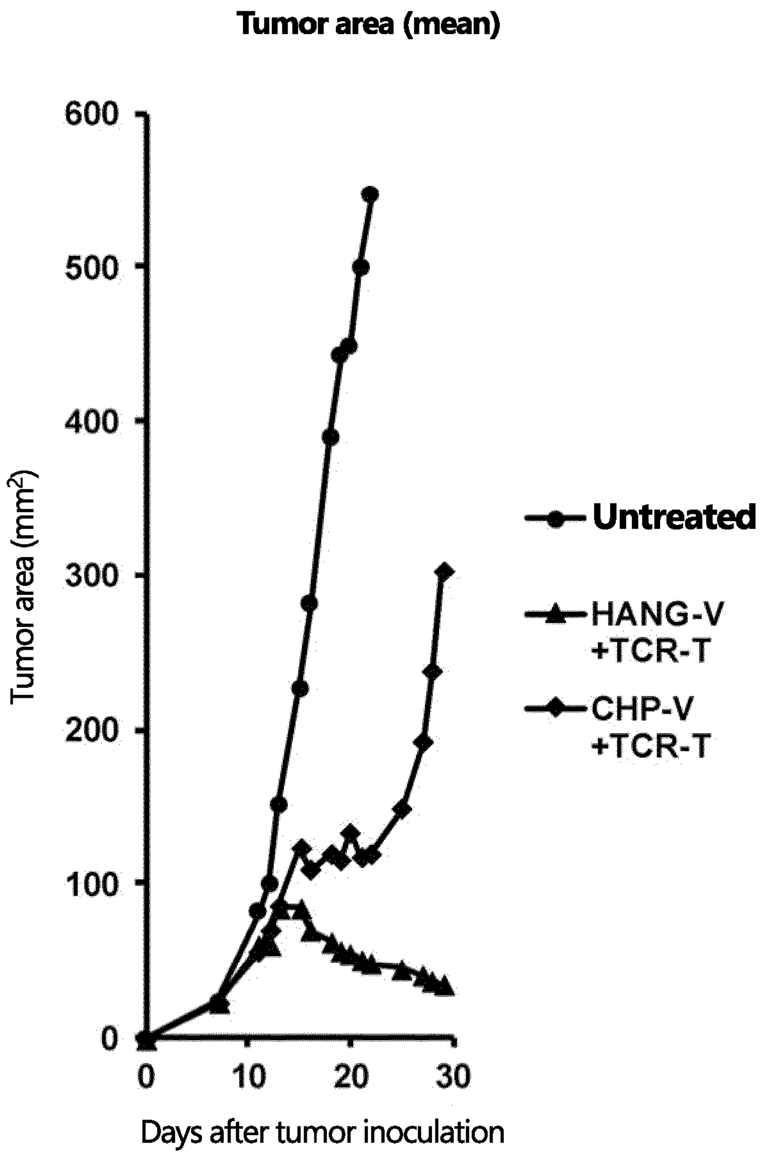
Tumor area (mean)
600
500
400
300
200
100
0
Tumor area (mm$^2$)
0
10
20
30
Days after tumor inoculation
Untreated
HANG-V
+TCR-T
CHP-V
+TCR-T

Fig. 7-2

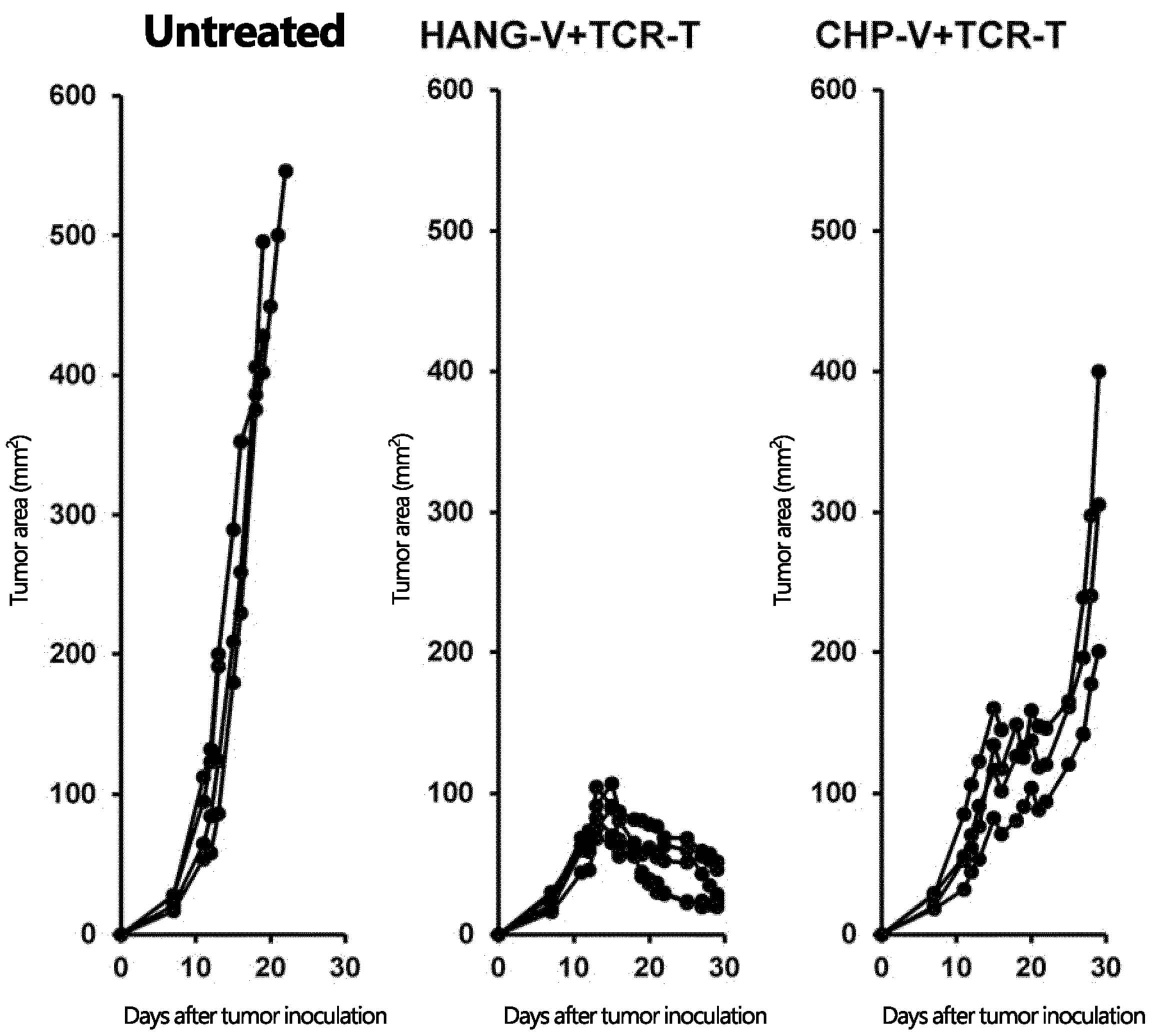
Untreated
HANG-V+TCR-T
CHP-V+TCR-T
Tumor area (mm²)
600
500
400
300
200
100
0
0
10
20
30
Days after tumor inoculation

Fig. 7-3

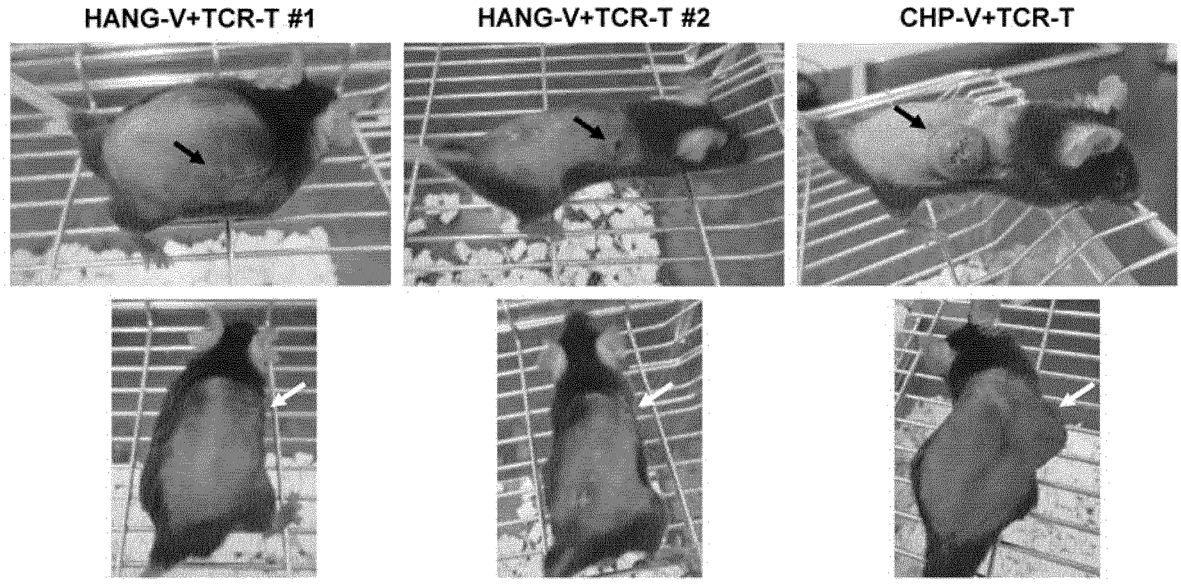
HANG-V+TCR-T #1
HANG-V+TCR-T #2
CHP-V+TCR-T

Fig. 7-4

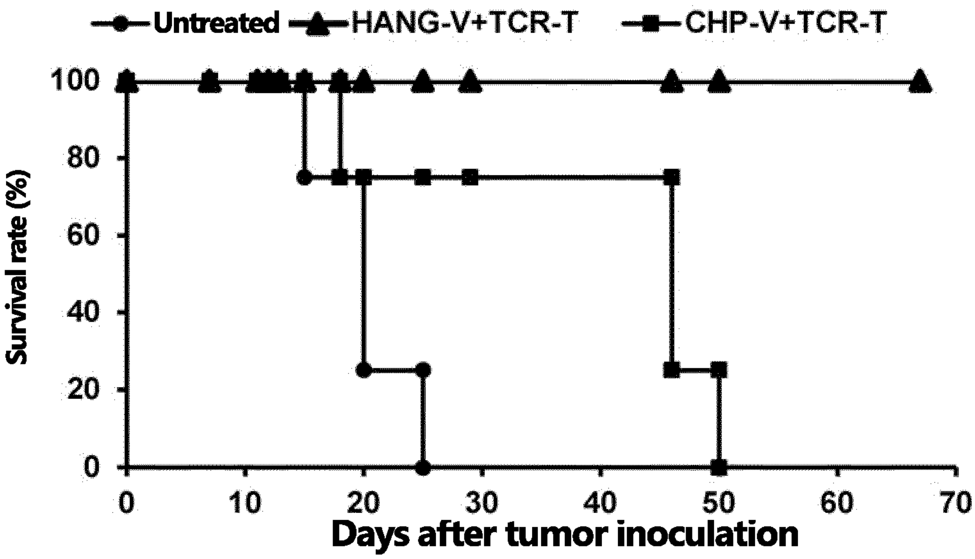
Untreated
HANG-V+TCR-T
CHP-V+TCR-T
Survival rate (%)
100
80
60
40
20
0
0
10
20
30
40
50
60
70
Days after tumor inoculation

Fig. 8-1

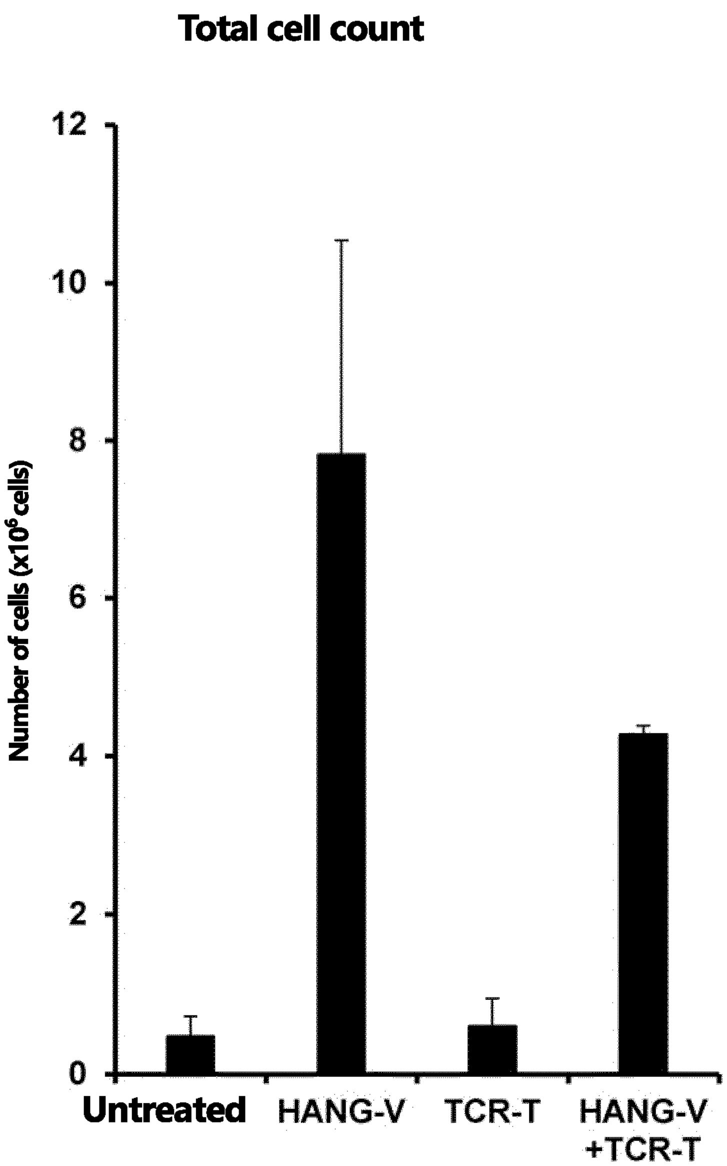
Total cell count
Number of cells (x10^6 cells)
12
10
8
6
4
2
0
Untreated
HANG-V
TCR-T
HANG-V
+TCR-T

Fig. 8-2

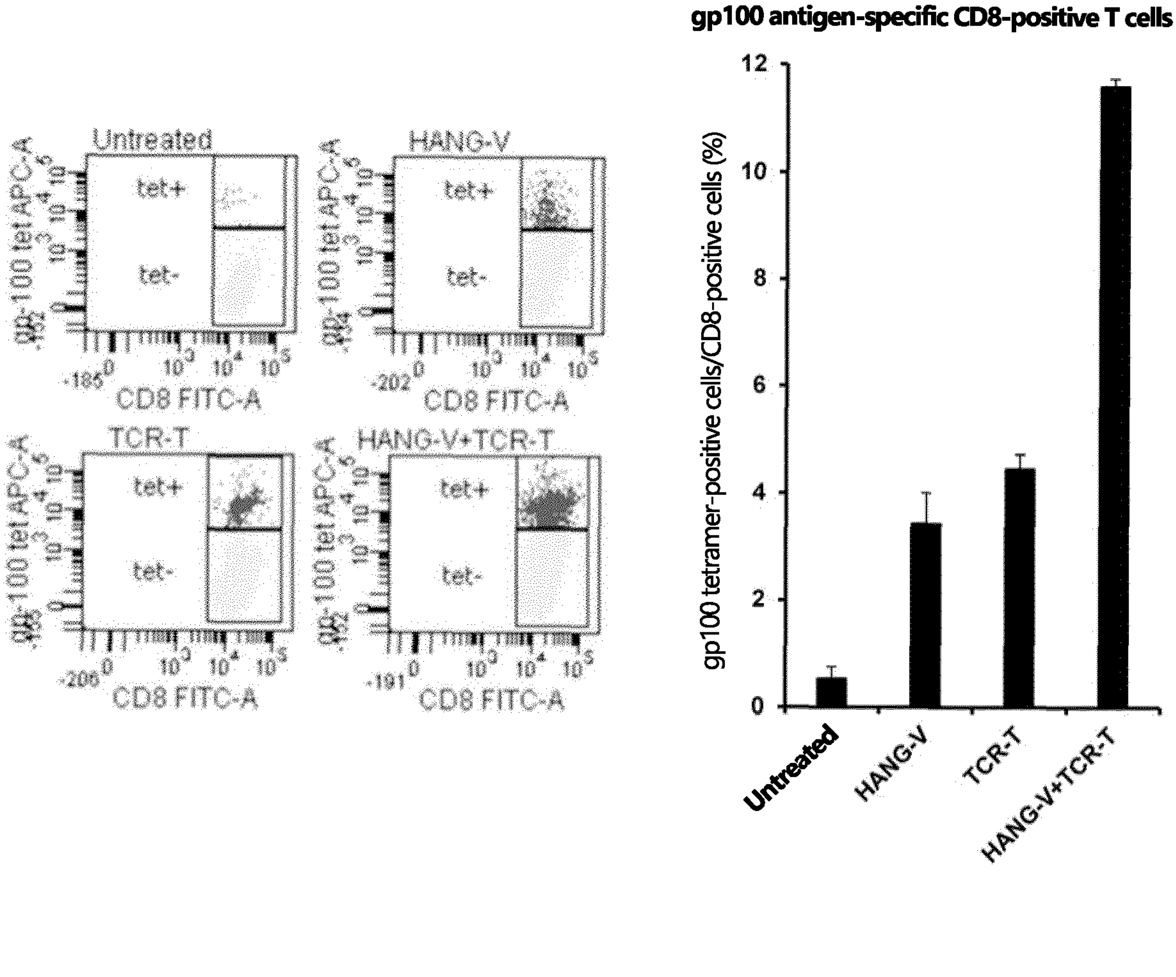
Untreated
HANG-V
TCR-T
HANG-V+TCR-T
tet+
tet-
gp-100 tet APC-A
CD8 FITC-A
gp100 antigen-specific CD8-positive T cells
gp100 tetramer-positive cells/CD8-positive cells (%)
0
2
4
6
8
10
12
Untreated
HANG-V
TCR-T
HANG-V+TCR-T

Fig. 8-3

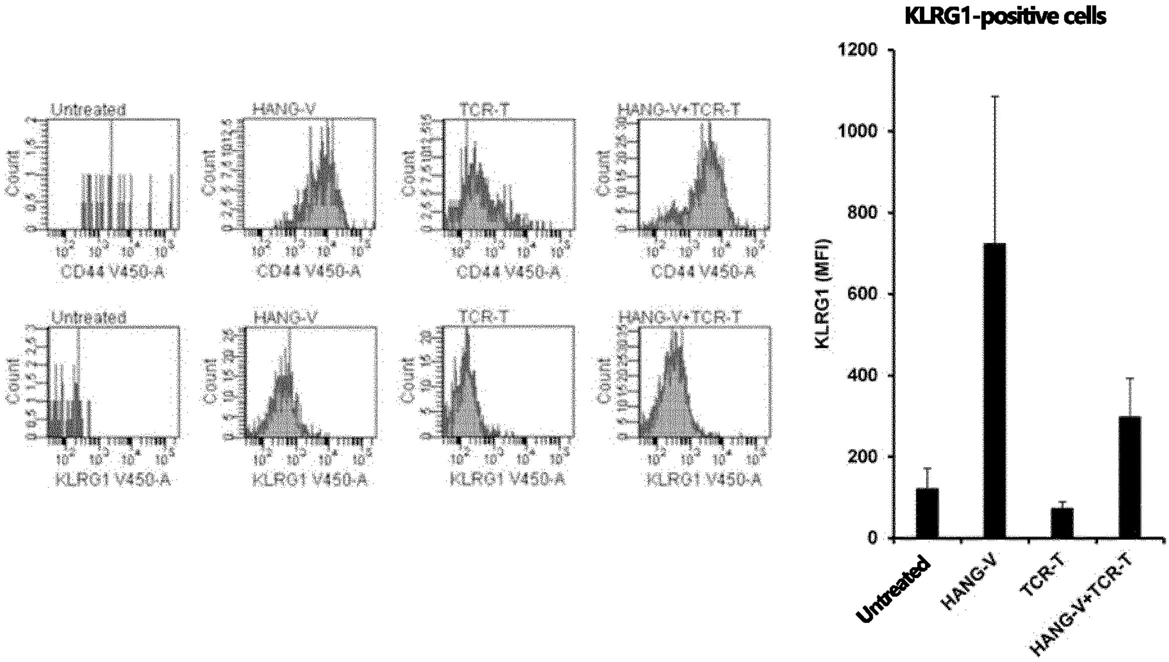
Untreated
HANG-V
TCR-T
HANG-V+TCR-T
Count
CD44 V450-A
KLRG1 V450-A
KLRG1-positive cells
KLRG1 (MFI)
1200
1000
800
600
400
200
0
Untreated
HANG-V
TCR-T
HANG-V+TCR-T

Fig. 8-4

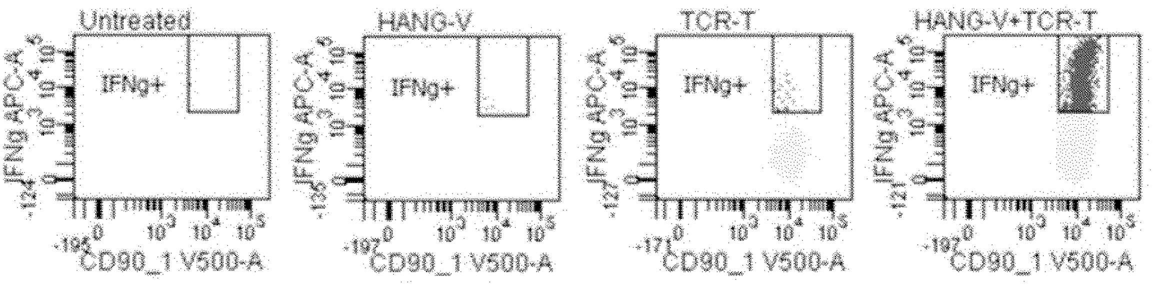
Untreated
HANG-V
TCR-T
HANG-V+TCR-T
IFNg+
IFNg APC-A
CD90_1 V500-A

Fig. 9-1

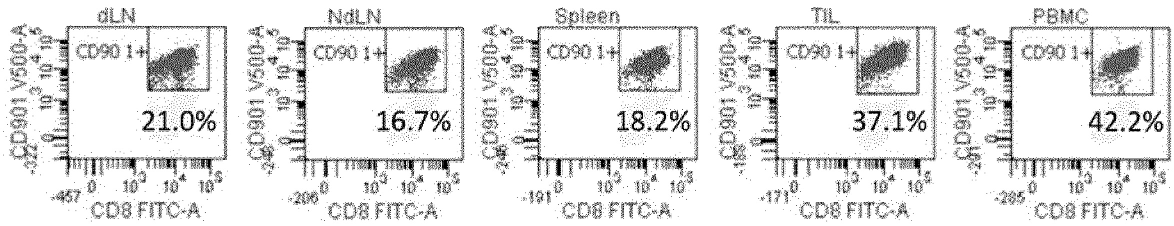
dLN
NdLN
Spleen
TIL
PBMC
CD90 1+
21.0%
16.7%
18.2%
37.1%
42.2%
CD901 V500-A
CD8 FITC-A

Fig. 9-2

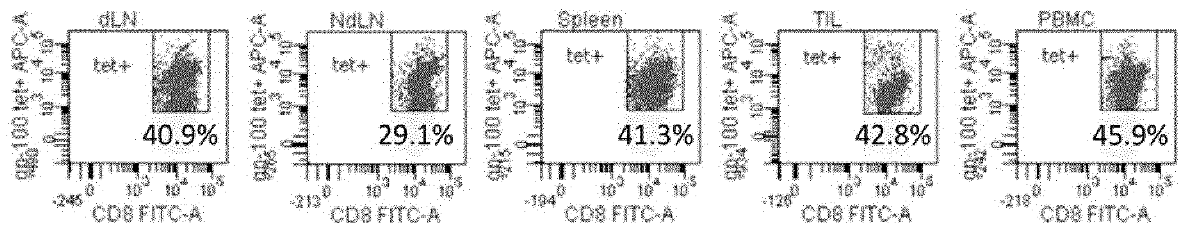
dLN
NdLN
Spleen
TIL
PBMC
tet+
40.9%
29.1%
41.3%
42.8%
45.9%
gp100 tet+ APC-A
CD8 FITC-A

Fig. 10-1

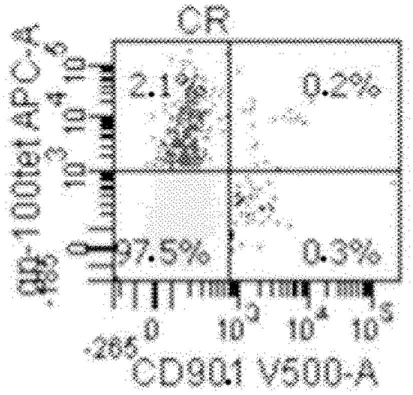


Fig. 10-2

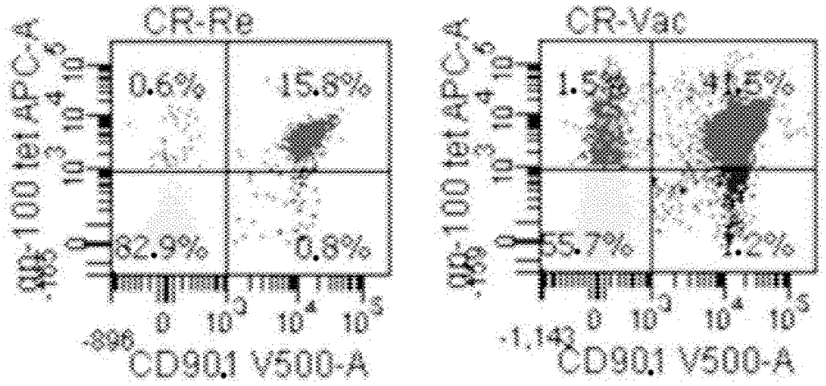


Fig. 10-3

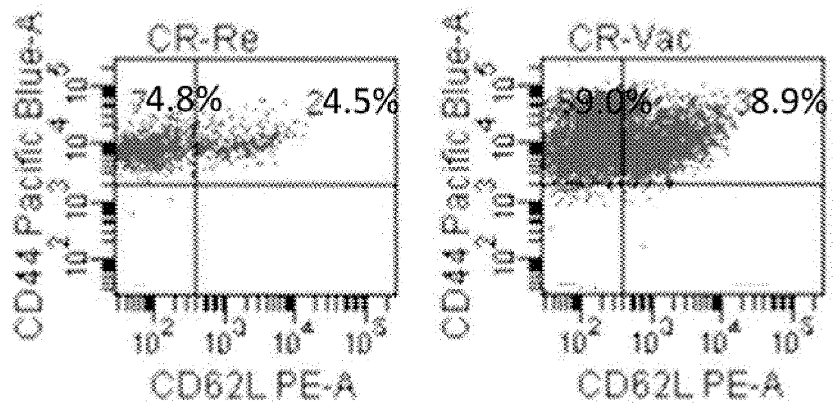

Fig. 10-4

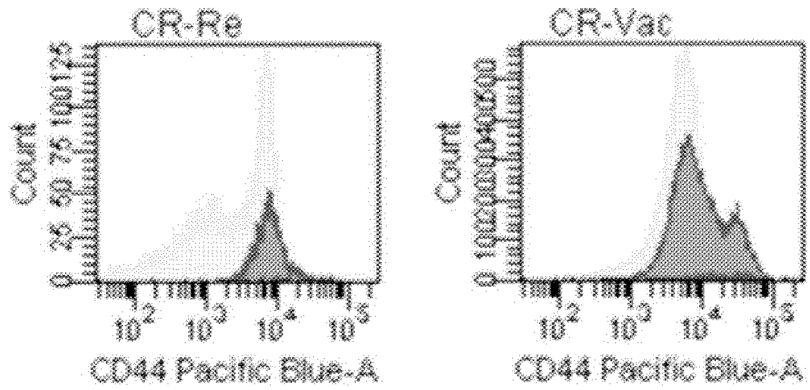


Fig. 10-5

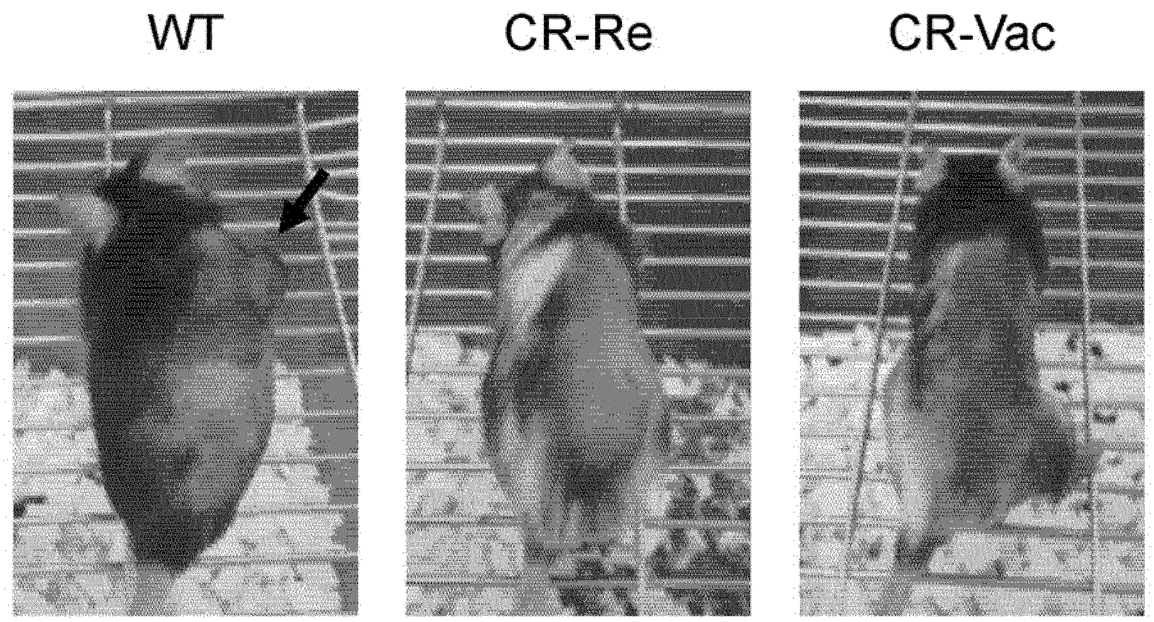

Fig. 11-1

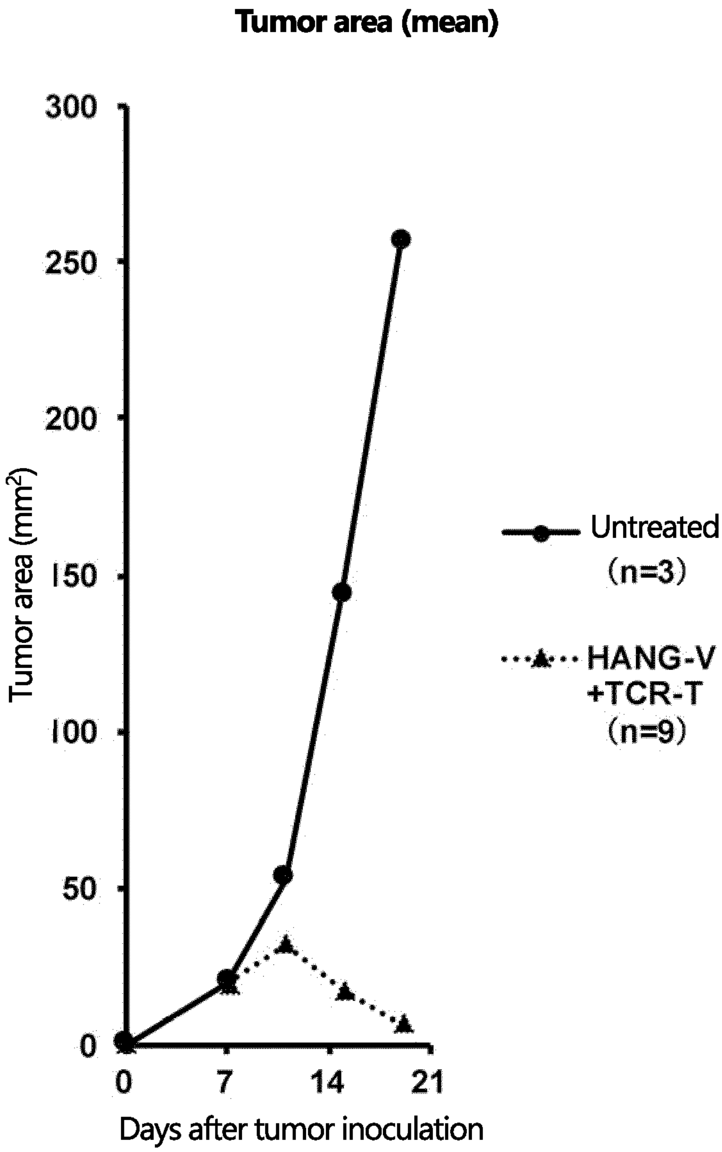
Tumor area (mean)
300
250
200
150
100
50
0
Tumor area (mm2)
0
7
14
21
Days after tumor inoculation
Untreated
(n=3)
HANG-V
+TCR-T
(n=9)

Fig. 11-2

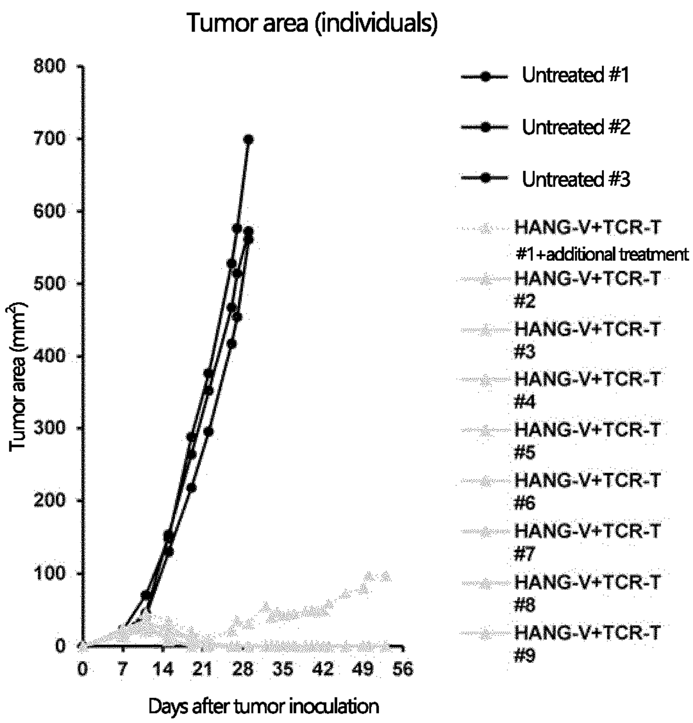
Tumor area (individuals)
800
700
600
500
400
300
200
100
0
Tumor area (mm²)
0 7 14 21 28 35 42 49 56
Days after tumor inoculation
Untreated #1
Untreated #2
Untreated #3
HANG-V+TCR-T #1+additional treatment
HANG-V+TCR-T #2
HANG-V+TCR-T #3
HANG-V+TCR-T #4
HANG-V+TCR-T #5
HANG-V+TCR-T #6
HANG-V+TCR-T #7
HANG-V+TCR-T #8
HANG-V+TCR-T #9

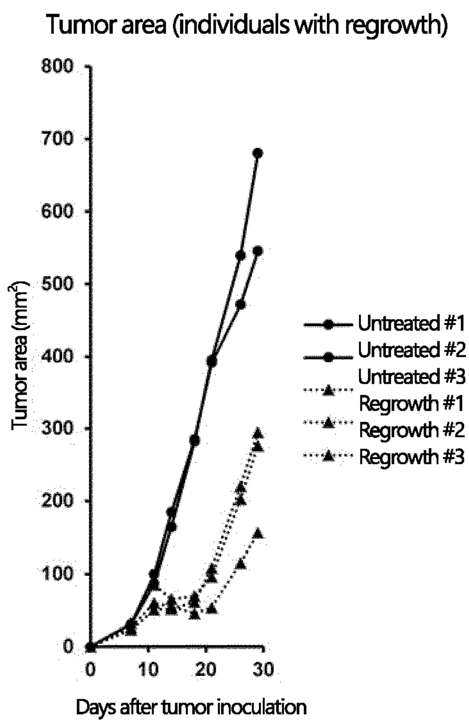
Tumor area (individuals with regrowth)
800
700
600
500
400
300
200
100
0
Tumor area (mm²)
0 10 20 30
Days after tumor inoculation
Untreated #1
Untreated #2
Untreated #3
Regrowth #1
Regrowth #2
Regrowth #3

Fig. 12-1

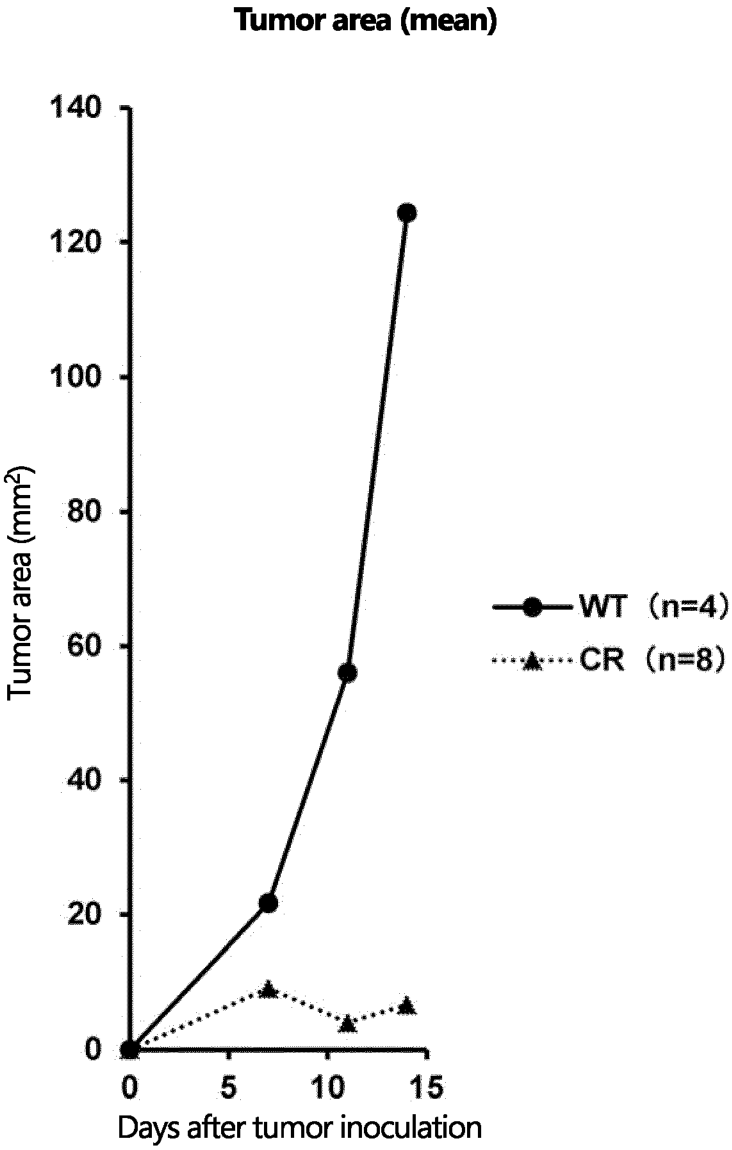
Tumor area (mean)
Tumor area (mm2)
140
120
100
80
60
40
20
0
0
5
10
15
Days after tumor inoculation
WT (n=4)
CR (n=8)

Fig. 12-2

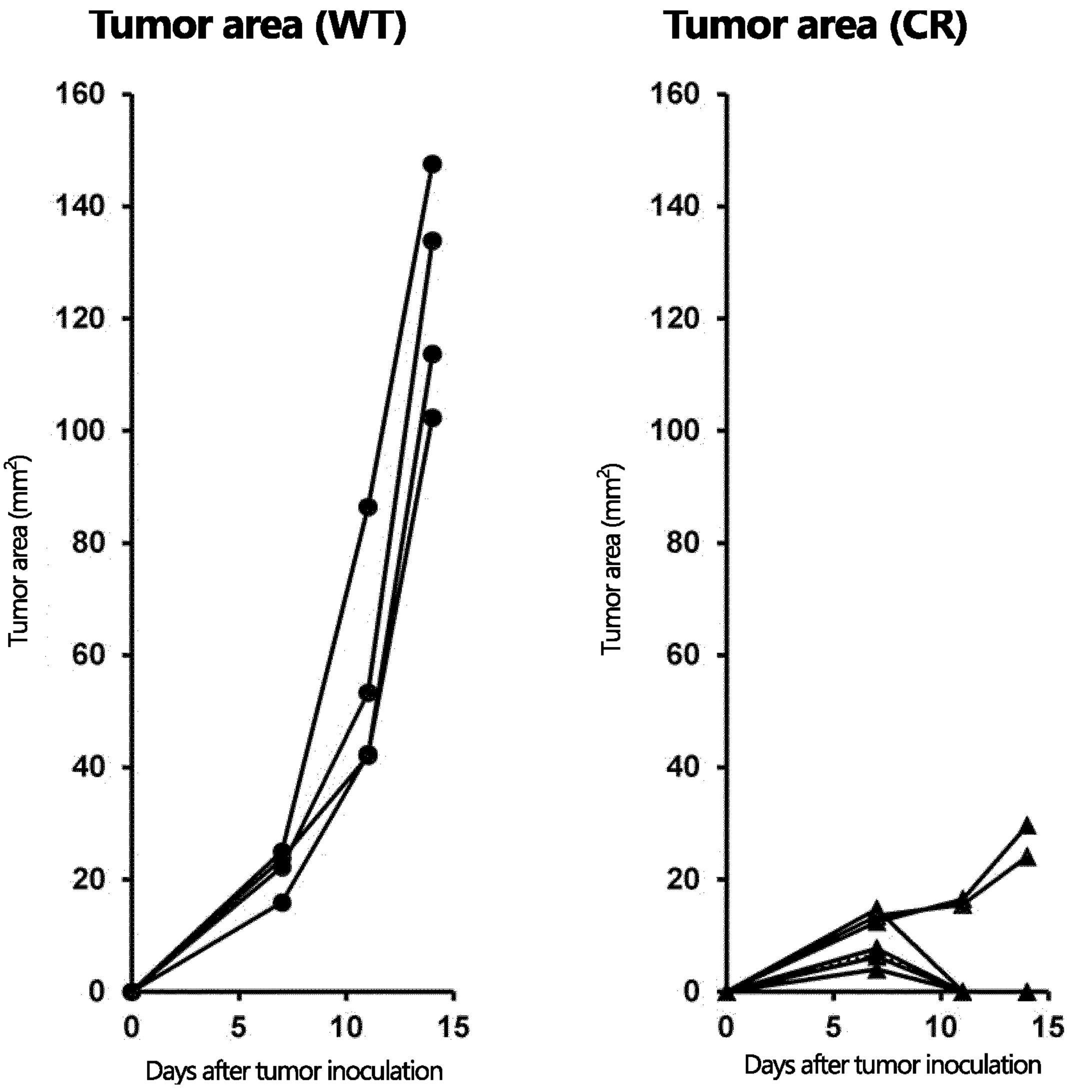
Tumor area (WT)
Tumor area (mm2)
160
140
120
100
80
60
40
20
0
0
5
10
15
Days after tumor inoculation
Tumor area (CR)
Tumor area (mm2)
160
140
120
100
80
60
40
20
0
0
5
10
15
Days after tumor inoculation

Fig. 13-1

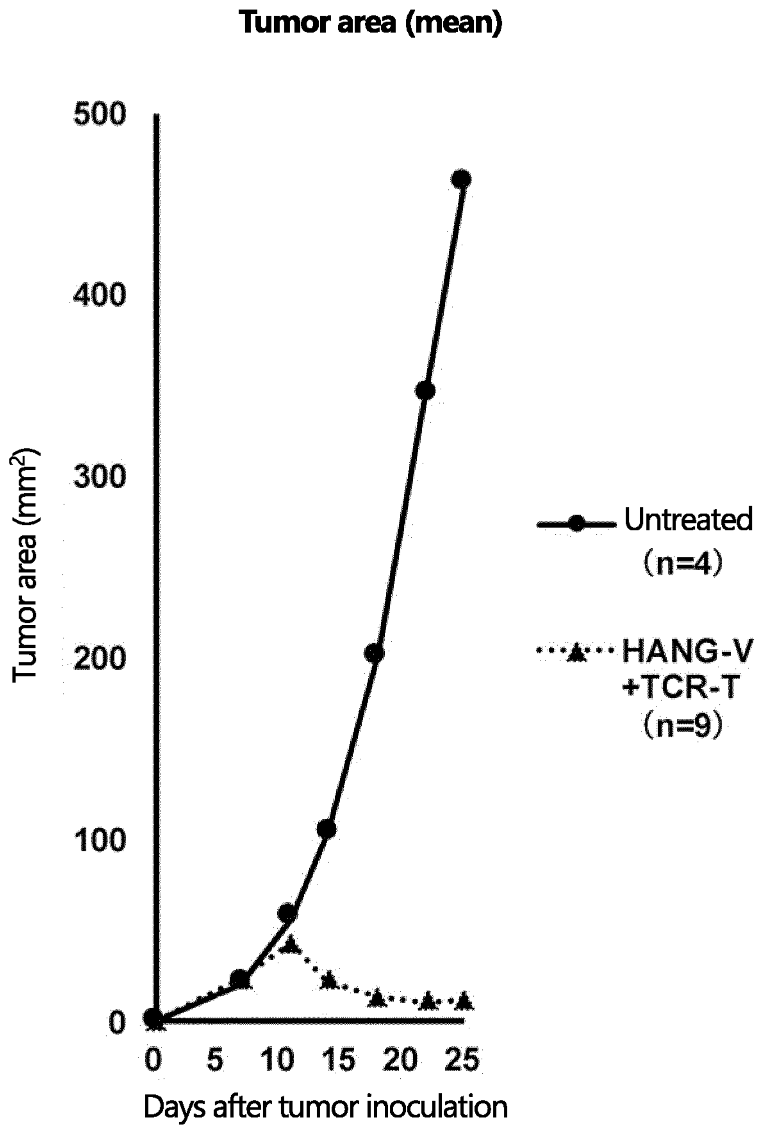
Tumor area (mean)
Tumor area ($mm^2$)
500
400
300
200
100
0
0 5 10 15 20 25
Days after tumor inoculation
Untreated (n=4)
HANG-V +TCR-T (n=9)

Fig. 13-2

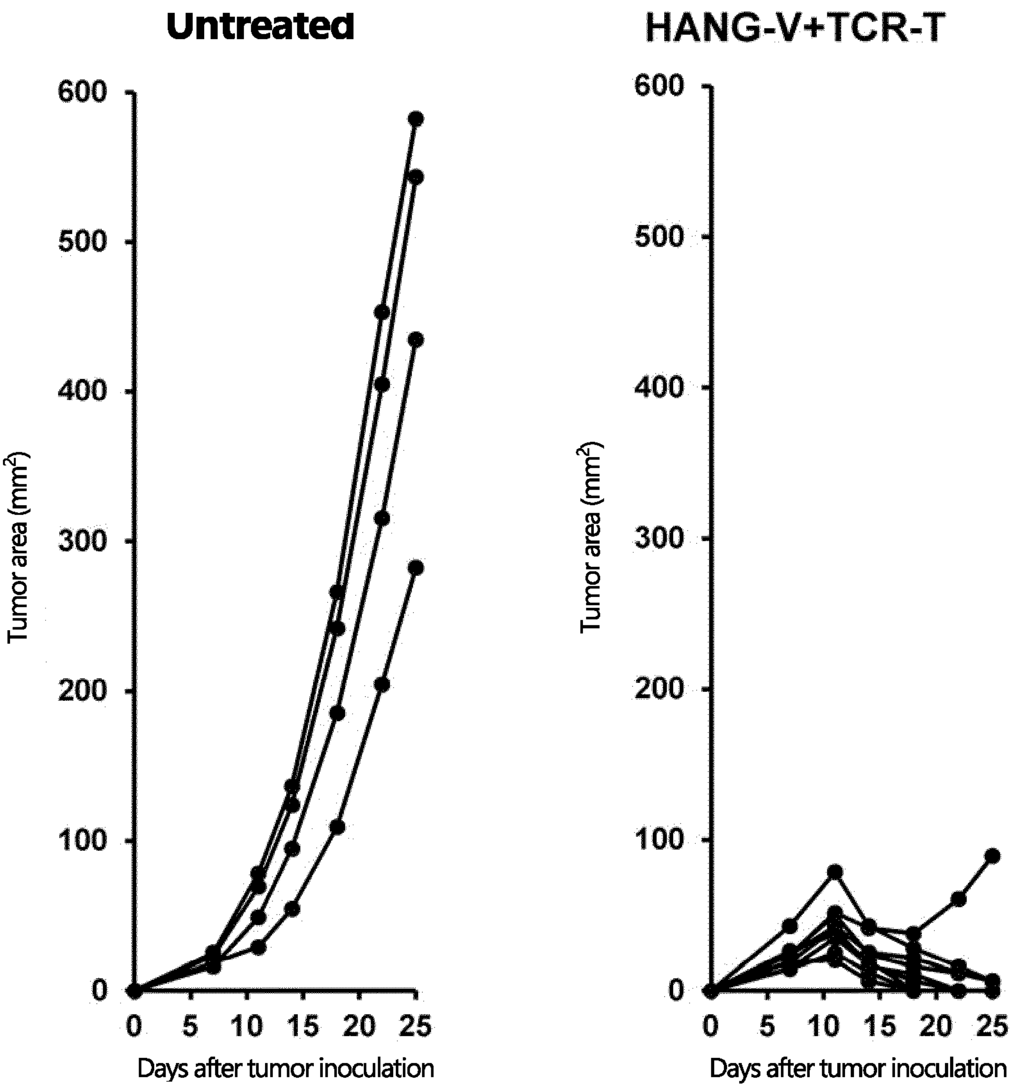
Untreated
HANG-V+TCR-T
600
500
400
300
200
100
0
0 5 10 15 20 25
Tumor area (mm2)
Days after tumor inoculation

Fig. 14

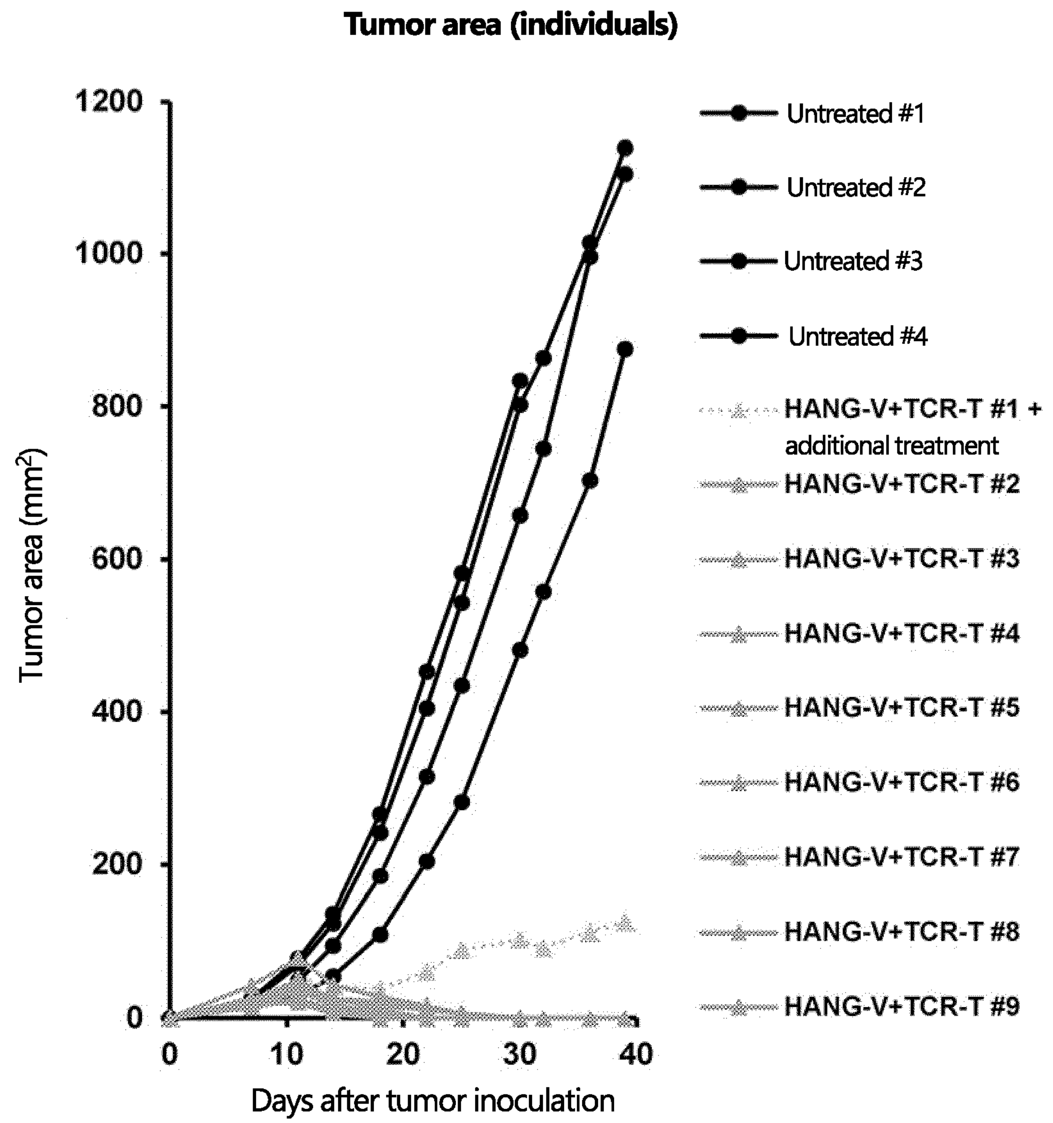
Tumor area (individuals)
Tumor area (mm²)
1200
1000
800
600
400
200
0
0
10
20
30
40
Days after tumor inoculation
Untreated #1
Untreated #2
Untreated #3
Untreated #4
HANG-V+TCR-T #1 + additional treatment
HANG-V+TCR-T #2
HANG-V+TCR-T #3
HANG-V+TCR-T #4
HANG-V+TCR-T #5
HANG-V+TCR-T #6
HANG-V+TCR-T #7
HANG-V+TCR-T #8
HANG-V+TCR-T #9

Fig. 15

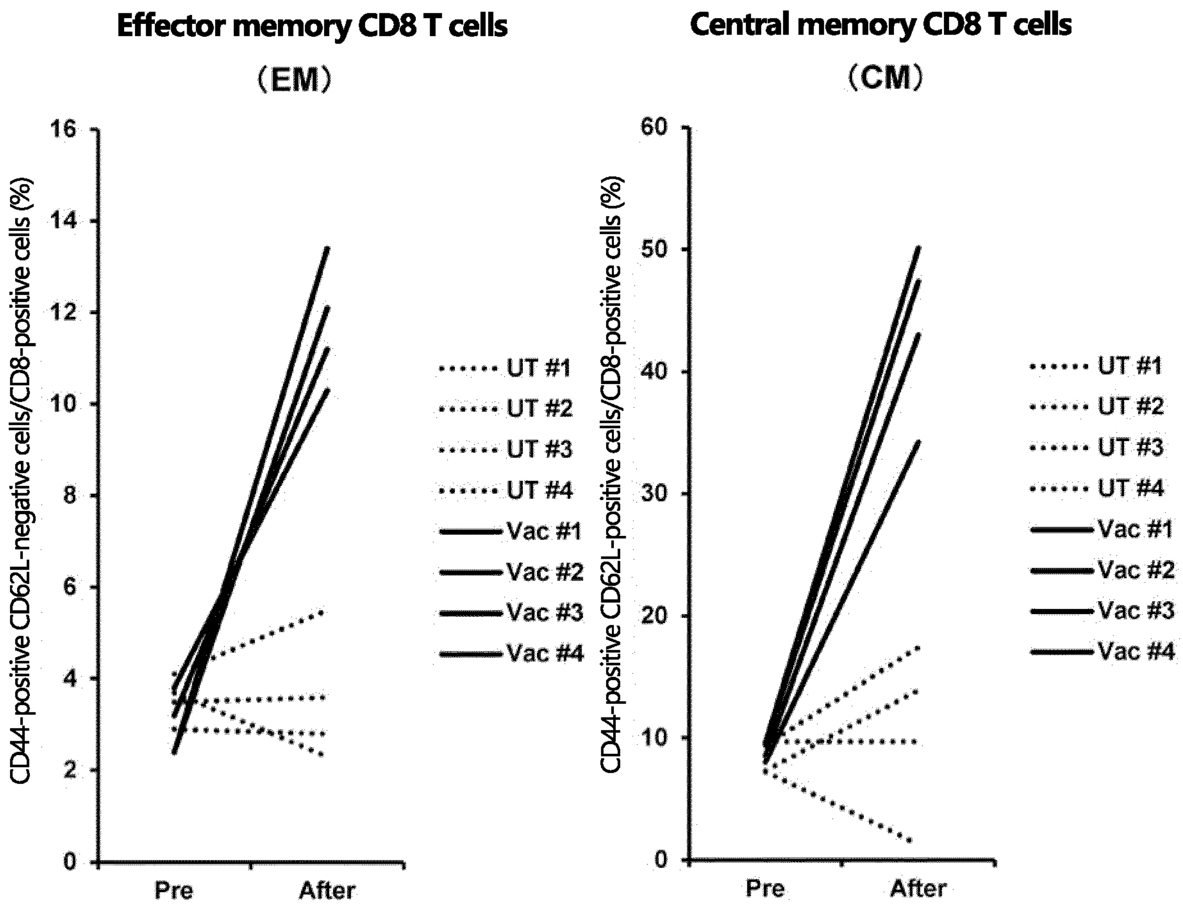
Effector memory CD8 T cells
(EM)
CD44-positive CD62L-negative cells/CD8-positive cells (%)
16
14
12
10
8
6
4
2
0
Pre
After
UT #1
UT #2
UT #3
UT #4
Vac #1
Vac #2
Vac #3
Vac #4
Central memory CD8 T cells
(CM)
CD44-positive CD62L-positive cells/CD8-positive cells (%)
60
50
40
30
20
10
0
Pre
After
UT #1
UT #2
UT #3
UT #4
Vac #1
Vac #2
Vac #3
Vac #4

Fig. 16

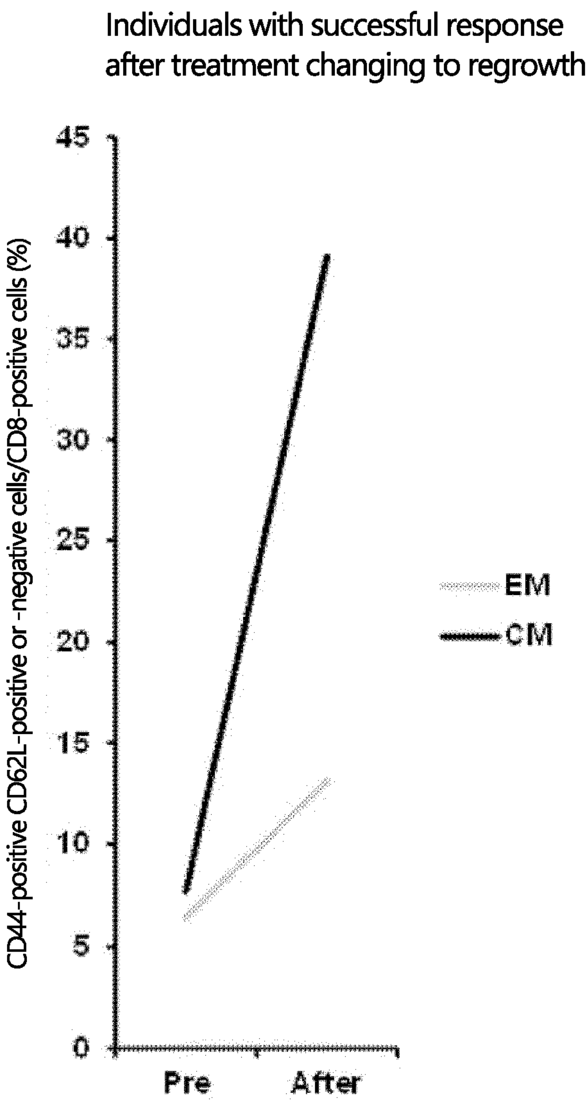
Individuals with successful response
after treatment changing to regrowth
CD44-positive CD62L-positive or -negative cells/CD8-positive cells (%)
45
40
35
30
25
20
15
10
5
0
Pre
After
EM
CM

Fig. 17-1a

Notl site Kozac sequence/VH single chain VL/Ascl site/mCD8 hinge, mCD28 TM, mCD3 zeta, mGITR ICD/Xhol site

GCGGCCGCAGATCCATGAGTGTGCCCACTCAGGTCCTGGGGTTGCTGCTGCTGTGGCTTACAGGTGCCAGATGTCAGGTCCAGCTGGTACAGTCTG
GGGCTGAGGTGAAGAAGCCTGGGTCCTCGGTGAAGGTCTCCTGCAAGGCTTCTGGAGGCACCTTCAGCAGCTATGCTATCAGCTGGGTGCGACAG
GCCCCTGGACAAGGGCTTGAGTGGATGGGAGGGATCATCCCTATCTTTGGTACAGCAAACTACGCACAGAAGTTCCAGGGCAGAGTCACGATTACC
GCGGACAAATCCACGAGCACAGCCTACATGGAGCTGAGCAGCCTGAGATCTGAGGACACGGCCGTGTATTACTGTGCGAGATCCCCCCGGCGGGC
ATATCATGATGCTTTTGATATCTGGGGCCAAGGGACAATGGTCACCGTCTCTTCAGGTGGAGGCGGTTCAGGCGGAGGTGGCAGCGGCGGTGGCG
GGAGTTCCTATGAGCTGACTCAGCCACCCTCGATGTCAGTGGCCCCAGGAAAGACGGCCAGCATTACCTGTGGCGGAGACCATATTGGAAGTAAAA
GTGTTCACTGGTACCAGCAGAAGCCAGGCCAGGCCCCTGTACTGGTCGTCTATGATGATAGCGACCGGCCCTCAGGGATCCCTGAGCGATTCTCTGG
CTCCAACTCTGGGAACACAGCCACTCTGACCATCAGCGGGACCCAGGCTATGGATGAGGCTGACTATTACTGTCTGGCGTGGGACAGCAGCACTGC
GATCTTCGGCGGAGGGACCAAGCTGACCGTCCTAGCGGCGCGCCAGATCAGCAACTCGGTGATGTACTTCAGTTCTGTCGTGCCAGTCCTTCAGAA
AGTGAACTCTACTACTACCAAGCCAGTGCTGCGAACTCCCTCACCTGTGCACCCTACCGGGACATCTCAGCCCCAGAGACCAGAAGATTGTCGGCCC
CGTGGCTCAGTGAAGGGGACCGGATTGGACTCTAGATTTTGGGCACTGGTCGTGGTTGCTGGAGTCCTGTTTTGTTATGGCTTGCTAGTGACAGTG
GCTCTTTGTGTTATCTGGACAAATCTGAGAGCAAAATTCAGCAGGAGTGCAGAGACTGCTGCCAACCTGCAGGACCCCAACCAGCTCTACAATGAG
CTCAATCTAGGGCGAAGAGAGGAATATGACGTCTTGGAGAAGAAGCGGGCTCGGGATCCAGAGATGGGAGGCAAACAGCAGAGGAGGAGGAAC
CCCCAGGAAGGCGTATACAATGCACTGCAGAAAGACAAGATGGCAGAAGCCTACAGTGAGATCGGCACAAAAGGCGAGAGGCGGAGAGGCAAG
GGGCACGATGGCCTTTACCAGGGTCTCAGCACTGCCACCAAGGACACCTATGATGCCCTGCATATGCAGACCCTGGCCCCTCGCAGGAGGCAACAC
ATGTGTCCTCGAGAGACCCAGCCATTCGCGGAGGTGCAGTTGTCAGCTGAGGATGCTTGCAGCTTCCAGTTCCCTGAGGAGGAACGCGGGGAGCA
GACAGAAGAAAAGTGTCATCTGGGGGGTCGGTGGCCATAATCGATTCTCGAG

Fig. 17-1b

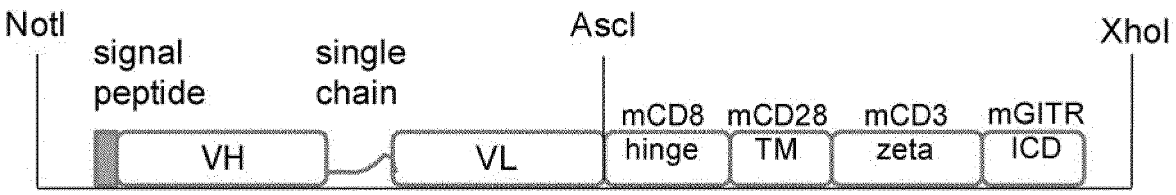

Fig. 17-2a

NotI site Kozac sequence/VH single chain VL/AscI site/mCD8 hinge, mCD28 TM, mCD28 ICD, mCD3 zeta/XhoI site

GCGGCCGCAGATCCATGAGTGTGCCCACTCAGGTCCTGGGGTTGCTGCTGCTGTGGCTTACAGGTGCCAGATGTCAGGTCCAGCTGGTACAGTCTG
GGGCTGAGGTGAAGAAGCCTGGGTCCTCGGTGAAGGTCTCCTGCAAGGCTTCTGGAGGCACCTTCAGCAGCTATGCTATCAGCTGGGTGCGACAG
GCCCCTGGACAAGGGCTTGAGTGGATGGGAGGGATCATCCCTATCTTTGGTACAGCAAACTACGCACAGAAGTTCCAGGGCAGAGTCACGATTACC
GCGGACAAATCCACGAGCACAGCCTACATGGAGCTGAGCAGCCTGAGATCTGAGGACACGGCCGTGTATTACTGTGCGAGATCCCCCCGGCGGGC
ATATCATGATGCTTTTGATATCTGGGGCCAAGGGACAATGGTCACCGTCTCTTCAGGTGGAGGCGGTTCAGGCGGAGGTGGCAGCGGCGGTGGCG
GGAGTTCCTATGAGCTGACTCAGCCACCCTCGATGTCAGTGGCCCCAGGAAAGACGGCCAGCATTACCTGTGGCGGAGACCATATTGGAAGTAAAA
GTGTTCACTGGTACCAGCAGAAGCCAGGCCAGGCCCCTGTACTGGTCGTCTATGATGATAGCGACCGGCCCTCAGGGATCCCTGAGCGATTCTCTGG
CTCCAACTCTGGGAACACAGCCACTCTGACCATCAGCGGGACCCAGGCTATGGATGAGGCTGACTATTACTGTCTGGCGTGGGACAGCAGCACTGC
GATCTTCGGCGGAGGGACCAAGCTGACCGTCCTAGCGGCGCGCCAGATCAGCAACTCGGTGATGTACTTCAGTTCTGTCGTGCCAGTCCTTCAGAA
AGTGAACTCTACTACTACCAAGCCAGTGCTGCGAACTCCCTCACCTGTGCACCCTACCGGGACATCTCAGCCCCAGAGACCAGAAGATTGTCGGCCC
CGTGGCTCAGTGAAGGGGACCGGATTGGACTCTAGATTTTGGGCACTGGTCGTGGTTGCTGGAGTCCTGTTTTGTTATGGCTTGCTAGTGACAGTG
GCTCTTTGTGTTATCTGGACAAATAGTAGAAGGAACAGACTCCTTCAAAGTGACTACATGAACATGACTCCCCGGAGGCCTGGGCTCACTCGAAAGC
CTTACCAGCCCTACGCCCCTGCCAGAGACTTTGCAGCGTACCGCCCCCTGAGAGCAAAATTCAGCAGGAGTGCAGAGACTGCTGCCAACCTGCAGG
ACCCCAACCAGCTCTACAATGAGCTCAATCTAGGGCGAAGAGAGGAATATGACGTCTTGGAGAAGAAGCGGGCTCGGGATCCAGAGATGGGAGGC
AAACAGCAGAGGAGGAGGAACCCCCAGGAAGGCGTATACAATGCACTGCAGAAAGACAAGATGGCAGAAGCCTACAGTGAGATCGGCACAAAA
GGCGAGAGGCGGAGAGGCAAGGGGCACGATGGCCTTTACCAGGGTCTCAGCACTGCCACCAAGGACACCTATGATGCCCTGCATATGCAGACCCT
GGCCCCTCGCTAATCGATTCTCGAG

Fig. 17-2b

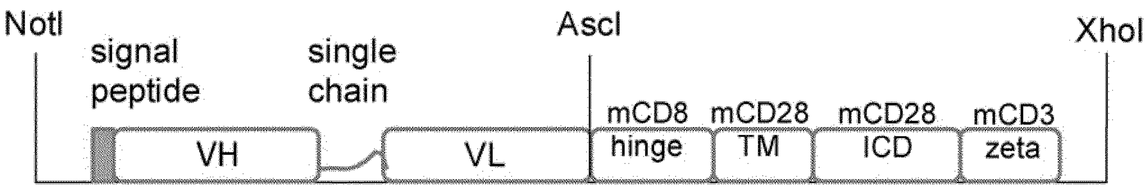

Fig. 17-3a

Notl site Kozac sequence/MAGE-A4/Clal site/P2A/truncated mouse NGFR/Xhol site

GCGGCCGCACCACCATGAGCTCCGAGCAGAAGAGCCAGCACTGCAAACCCGAGGAAGGCGTTGAAGCCCAGGAAGAAGCACTGGGTCTGGTTGGAGC
ACAAGCTCCCACTACCGAAGAACAGGAGGCTGCCGTTTCCAGTAGCTCTCCCCTTGTGCCAGGAACCCTGGAAGAGGTCCCTGCTGCCGAGTCTGCCGGT
CCTCCTCAGAGTCCTCAAGGCGCAAGCGCCTTGCCCACGACCATCAGCTTTACGTGCTGGAGACAACCCAATGAAGGATCAAGCTCTCAGGAGGAAGAA
GGCCCATCAACATCTCCGGATGCAGAATCACTCTTTCGCGAGGCACTCAGCAACAAAGTGGACGAATTGGCCCATTTCCTTCTGCGGAAGTACAGGGCTA
AAGAACTGGTGACAAAGGCGGAAATGCTGGAGAGGGTGATCAAGAACTACAAGCGGTGTTTCCCAGTGATCTTCGGCAAAGCATCCGAGAGTCTGAAG
ATGATCTTTGGCATTGACGTGAAGGAGGTCGATCCAGCCTCCAACACCTATACCCTGGTGACTTGTCTCGGGCTCTCCTATGATGGGCTTTTGGGTAACAAC
CAGATATTCCCCAAAACAGGGCTGCTGATCATTGTGCTGGGAACCATAGCCATGGAGGGCGATAGTGCCAGCGAAGAAGAGATTTGGGAGGAGCTGGGA
GTGATGGGTGTATATGACGGACGAGAGCACACTGTCTATGGCGAGCCTCGGAAACTCCTGACTCAGGACTGGGTCCAGGAGAATTACCTGGAGTATCGAC
AGGTACCAGGGTCAAATCCGGCTCGCTACGAGTTTCTTTGGGGCCCTCGTGCTTTGGCGGAGACATCCTACGTCAAGGTGCTGGAGCATGTGGTAAGGGT
GAATGCGAGAGTTCGCATTGCCTACCCATCTCTGAGAGAGGCCGCTCTCCTGGAGGAAGAGGAAGGGGTTATCGATGGAAGCGGAGCCACGAACTTCTC
TCTGTTAAAGCAAGCAGGAGATGTTGAAGAAAACCCCGGGCCTATGAGGAGGGCTGGAGCCGCCTGTTCCGCCATGGATCGACTGCGTTTGCTGCTTCTG
CTCCTGCTCCTTCTGGGTGTAAGCTTTGGAGGAGCCAAAGAGACATGCAGCACTGGCATGTATACCCACAGTGGAGAATGCTGCAAGGCATGCAACCTCG
GCGAAGGAGTTGCTCAGCCTTGTGGCGCAAACCAGACTGTGTGTGAACCCTGTCTGGACAGTGTGACATTCAGCGATGTGGTGTCTGCCACCGAGCCATG
CAAGCCATGTACCGAGTGTCTGGGTCTGCAATCCATGTCTGCACCTTGCGTGGAAGCTGATGACGCCGTTTGTCGCTGTAGTTACGGTTACTATCAGGACG
AGGAAACAGGCAGATGTGAGGCTTGCAGCGTTTGTGGTGTAGGGTCTGGACTGGTGTTCTCCTGCCAGGATAAGCAGAATACAGTGTGCGAAGAGTGTC
CCGAAGGCACCTATAGCGATGAGGCCAATCACGTCGATCCCTGCCTGCCGTGTACTGTCTGCGAGGATACAGAGCGCCAATTGCGCGAATGCACACCATGG
GCCGATGCGGAGTGTGAGGAGATCCCAGGAAGGTGGATTACGCGGTCTACCCCTCCCGAGGGCAGTGACGTAACCACACCCTCAACCCAGGAACCTGAG
GCACCACCGGAGCGGGACCTTATCGCGTCAACCGTCGCTGACACCGTCACGACAGTGATGGGTTCTAGCCAGCCAGTCGTGACTAGAGGCACTGCGGAC
AACTTGATTCCAGTGTACTGCTCTATTCTGGCTGCCGTCGTCGTTGGCCTCGTGGCGTATATCGCTTTCAAACGATGGAATTCCTGCAAACAGAACAAGCAA
GGGGCCAACTCAAGACCCGTGAATCAAACTCCTCCTCCCGAAGGGGAGAAACTGCACTCTGATAGCGGGATATCCTAACTCGAG

Fig. 17-3b

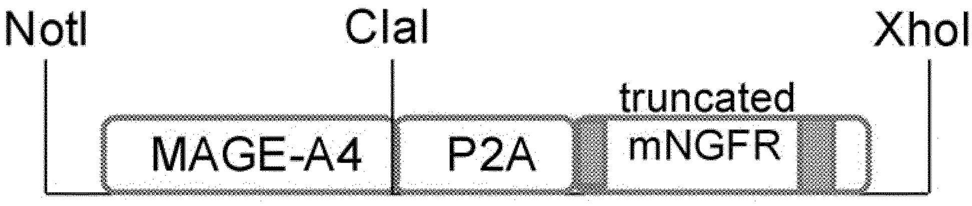

Fig. 17-4a

NotI site Kozac sequence/hbeta-2 microglobulin/single chain/HLA-A*02:01 alpha1, alpha2/H-2d alpha3, IM, ICD/XhoI site

GCGGCCGCACCACCATGGCCGTAATGGCTCCTAGGACTCTGGTGCTCCTTCTTAGTGGAGCTTTGGCTCTGACCCAGACCTGGGCCATTCAGCGGACTCC
CAAGATACAGGTGTACAGCAGGCATCCCGCTGAGAATGGGAAAAGCAACTTCCTGAACTGCTATGTGAGCGGATTTCACCCATCAGACATCGAAGTGGAT
CTGTTGAAGAATGGGGAGCGGATTGAGAAGGTCGAGCATTCCGACCTGTCATTCAGCAAAGACTGGAGCTTTTACCTCCTGTACTATACCGAGTTTACAC
CCACAGAGAAGGATGAATATGCATGCCGAGTGAATCACGTTACGCTGTCTCAGCCAAAGATCGTAAAGTGGGACCGGGATATGGGCGGTGGAGGTCAG
GCGGTGGCGGTTCAGGAGGAGGTGGGAGCGGAAGTCATTCCATGCGTTACTTCTTCACATCCGTGTCAAGACCTGGCAGAGGGGAGCCACGCTTCATC
GCAGTCGGCTACGTCGATGATACCCAGTTCGTCCGCTTTGACTCTGACGCAGCCAGTCAGCGTATGGAACCGCGTGCACCCTGGATTGAACAGGAAGGCC
CTGAGTATTGGGATGGGGAGACTAGGAAGGTGAAAGCCCATAGCCAGACACACCGAGTCGACCTTGGGACGCTGAGAGGATACTACAACCAGTCCGAA
GCTGGGAGTCACACCGTTCAGCGAATGTATGGCTGCGACGTAGGCTCCGATTGGAGATTCCTGAGGGGCTATCACCAGTACGCATACGATGGTAAGGACT
ATATCGCCCTCAAAGAGGATCTGCGGTCCTGGACAGCCGCTGACATGGCCGCGCAAACAACGAAGCACAAATGGGAGGCTGCGCATGTGGCCGAGCAA
CTGAGGGCCTACCTGGAGGGAACTTGTGTGGAGTGGTTGCGCAGATACCTGGAGAACGGCAAGGAAACTCTCCAACGCACCGACTCTCCGAAGGCACA
TGTGACTCACCACCCAAGGAGTAAGGGTGAAGTGACCTTGCGGTGTTGGGCACTGGGATTCTATCCCGCAGACATCACACTGACCTGGCAACTGAATGG
GGAAGAACTGACCCAGGACATGGAGCTCGTGGAGACAAGACCAGCCGGAGATGGGACATTTCAGAAATGGGCGTCTGTTGTTGTCCCTCTCGGCAAGG
AGCAGAACTACACCTGTCGCGTATACCACGAAGGGTTGCCCGAACCCCTGACCCTGCGATGGGAACCTCCACCTAGCACTGATTCCTATATGGTGATTGTG
GCCGTCCTTGGCGTTCTGGGAGCTATGGCCATCATAGGTGCTGTGGTTGCCTTTGTCATGAAAAGGCGCAGAAACACTGGTGGCAAAGGCGGCGACTAT
GCTCTCGCCCCTGGAAGCCAATCTAGCGAGATGTCTCTTCGGGATTGCAAAGCTTGACTCGAG

Fig. 17-4b

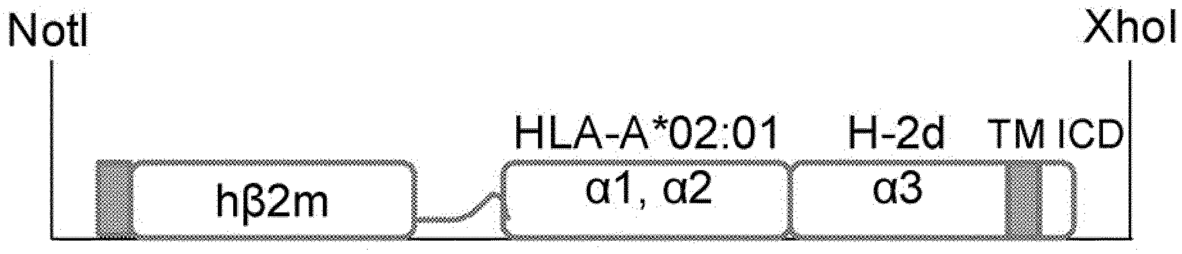

Fig. 17-5

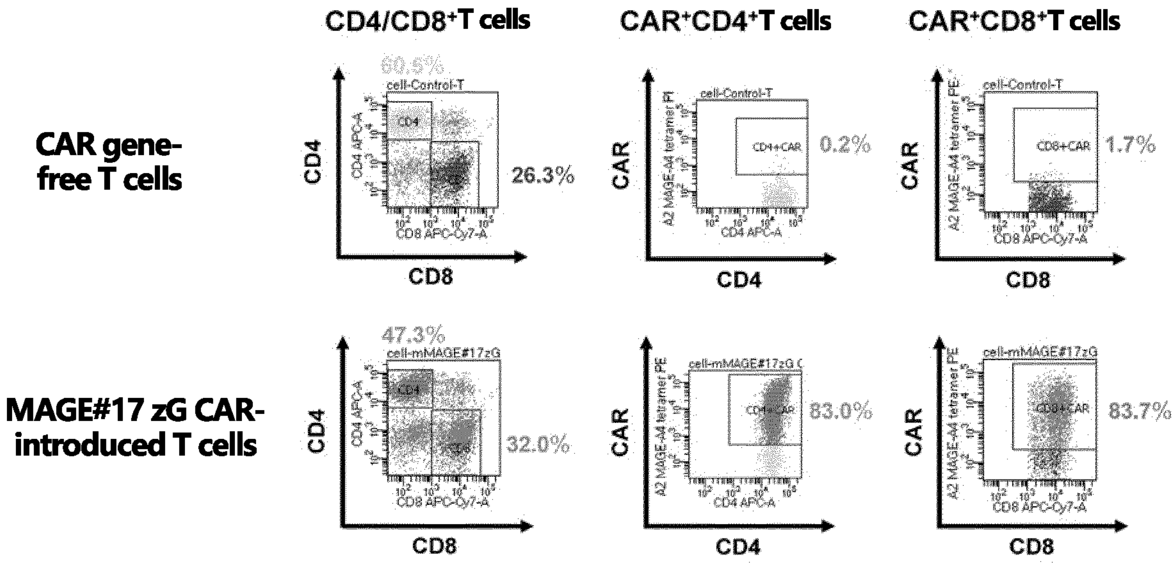


Fig. 17-6

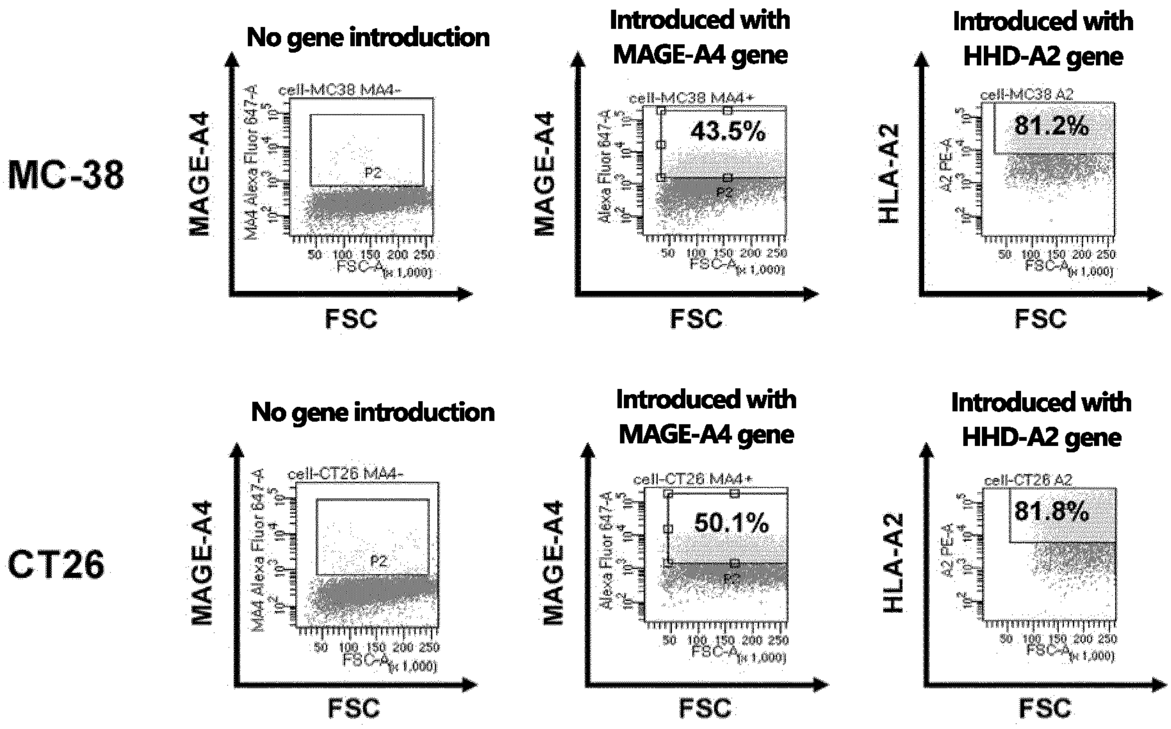

Fig. 17-7

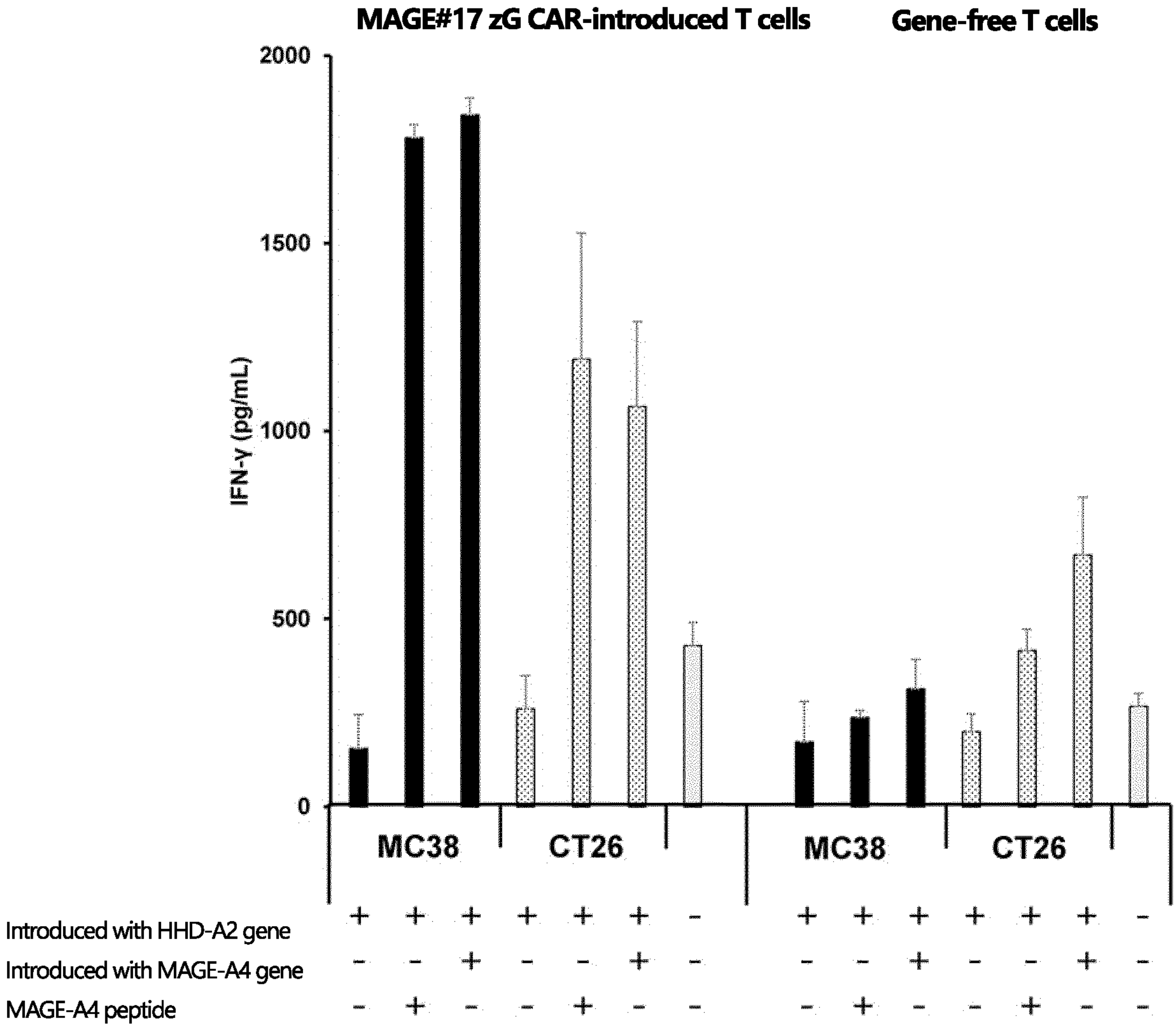
MAGE#17 zG CAR-introduced T cells
Gene-free T cells
2000
1500
1000
500
0
IFN-γ (pg/mL)
MC38
CT26
MC38
CT26
Introduced with HHD-A2 gene
Introduced with MAGE-A4 gene
MAGE-A4 peptide

## INTERNATIONAL SEARCH REPORT

International application No.
**PCT/JP2022/022211**

**A. CLASSIFICATION OF SUBJECT MATTER**

***A61K 39/00***(2006.01)i; ***A61K 35/17***(2015.01)i; ***A61K 39/39***(2006.01)i; ***A61K 47/61***(2017.01)i; ***A61P 35/00***(2006.01)i; ***A61P 43/00***(2006.01)i; *C07K 7/06*(2006.01)n
FI: A61K39/00 H ZNA; A61K35/17; A61K39/39; A61K47/61; A61P35/00; A61P43/00 121; C07K7/06

According to International Patent Classification (IPC) or to both national classification and IPC

**B. FIELDS SEARCHED**

Minimum documentation searched (classification system followed by classification symbols)

A61K39/00; A61K35/17; A61K39/39; A61K47/61; A61P35/00; A61P43/00; C07K7/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/MEDLINE/EMBASE/BIOSIS (STN), JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | WO 2017/138557 A1 (UNIV MIE) 17 August 2017 (2017-08-17)<br>claims 1, 4, 7 | 1-31 |
| Y | WO 2020/158771 A1 (UNIV MIE) 06 August 2020 (2020-08-06)<br>claims 1, 9, 11, 13, paragraphs [0113], [0123], [0127], [0182] | 1-31 |
| P, X | 百瀬文康　他, ヒアルロン酸ナノゲルワクチンとTCR-T細胞併用は所属リンパ節で強力に腫瘍抗原的CTLを誘導し、抗PD-1抵抗性腫瘍を消失させる, 第25回日本がん免疫学会総会　プログラム・抄録集, 07 June 2021, 25th, p. 143 P-6<br>method, reviews, (MOMOSE, Fumiyasu et al.), non-official translation (The combined use of hyaluronate nanogels vaccine and TCR-T cells potently induce tumor antigen CTL in regional lymph nodes and cause anti-PD-1-resistant tumors to disappear. Programs and abstracts of the 25th Annual Meeting of Japanese Association of Cancer Immunology.) | 1-31 |

☐ Further documents are listed in the continuation of Box C.　☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 June 2022** | **12 July 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/022211**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

**INTERNATIONAL SEARCH REPORT**
**Information on patent family members**

International application No.
**PCT/JP2022/022211**

| Patent document cited in search report | Publication date (day/month/year) | Patent family member(s) | Publication date (day/month/year) |
|---|---|---|---|
| WO 2017/138557 A1 | 17 August 2017 | US 2019/0111078 A1<br>claims 1, 4, 7<br>EP 3415159 A1<br>CA 3014027 A<br>KR 10-2018-0105232 A<br>CN 108697777 A | |
| WO 2020/158771 A1 | 06 August 2020 | EP 3919072 A1<br>claims 1, 9, 11, 13, paragraphs [0046], [0056], [0060], [0115]<br>CN 113329764 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020158771 A
- WO 2010053140 A
- WO 2014038641 A
- JP 2019523909 A


### Non-patent literature cited in the description


- **SEIICHI NAKAHAMA.** Essential Kobunshi Kagaku [Essential Polymer Science. Kodansha Ltd
- *Oncoimmunology,* 2017, vol. 6 (6), e1259049
- *Cancer Res.,* 2000, vol. 60, 5514-5521
- *Curr Opin Genet Dev.,* 2014, vol. 24, 46-51
- *Genomics,* 2020, vol. 21, 2
- *J Immunogenet,* 1989, vol. 16 (4-5), 291-303
- *Blood,* 2018, vol. 132 (11), 1134-1145
- *Science,* 24 January 2020, vol. 367 (6476), 446-453